(19) Europäisches Patentamt
European Patent Office
Office européen des brevets

(11) EP 1 344 823 A1

(12) **EUROPEAN PATENT APPLICATION**
published in accordance with Art. 158(3) EPC

(43) Date of publication:
**17.09.2003 Bulletin 2003/38**

(21) Application number: **01998185.1**

(22) Date of filing: **29.11.2001**

(51) Int Cl.[7]: **C12N 15/12**, C07K 14/705, C07K 16/28, C12P 21/02, C12Q 1/68, A61K 45/00, A61P 25/00, A61P 29/00, A61P 9/00, A61P 35/00, A61P 3/00, A61P 37/02, A61P 1/00

(86) International application number:
**PCT/JP01/10418**

(87) International publication number:
**WO 02/044368 (06.06.2002 Gazette 2002/23)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **30.11.2000 JP 2000364801**
**26.03.2001 JP 2001087482**
**15.05.2001 JP 2001145434**
**06.09.2001 JP 2001270838**

(71) Applicant: **Takeda Chemical Industries, Ltd.**
**Osaka-shi, Osaka 541-0045 (JP)**

(72) Inventors:
- **TERAO, Yasuko**
  **Higashinada-ku, Kobe-shi, Hyogo 658-0063 (JP)**
- **SHINTANI, Yasushi**
  **Toyonaka-shi, Osaka 560-0005 (JP)**
- **HARADA, Mioko**
  **Tsukuba-shi, Ibaraki 305-0046 (JP)**
- **SHIMOMURA, Yukio**
  **Tsukuba-shi, Ibaraki 305-0035 (JP)**
- **MORI, Masaaki**
  **Tsukuba-shi, Ibaraki 305-0821 (JP)**

(74) Representative: **Rickard, Timothy Mark Adrian**
**Takeda Euro IP Department,**
**11-12 Charles II Street**
**London SW1Y 4QU (GB)**

(54) **NOVEL G PROTEIN-COUPLED RECEPTOR PROTEINS AND DNAS THEREOF**

(57) The present invention intends to provide a novel protein useful for a screening of agonists/antagonists. Specifically, the present invention provides rat- and mouse-derived protein or its salt, DNA encoding the protein, a determination method of ligand to the protein, a screening method and a screening kit for a compound that alters a binding property between ligand and the protein, a compound or its salt obtainable by the screening, and the like.

The protein of the present invention or the DNA encoding the same can be used for, (1) a determination of ligand to the protein of the present invention, (2) a prophylactic and/or therapeutic agent for diseases associated with dysfunction of the protein of the present invention, (3) a screening of a compound (agonist/antagonist) that alters a binding property between the protein of the present invention and ligand, and the like.

EP 1 344 823 A1

## Description

FIELD OF THE INVENTION

The present invention relates to a novel G protein-coupled receptor proteins derived from rat whole brain or mouse whole brain, or salts thereof and polynucleotides encoding the same, etc.

BACKGROUND ART

Physiological active substances such as various hormones and neurotransmitters regulate the biological function via specific receptor proteins present on cell membranes. Many of these receptor proteins are coupled with guanine nucleotide-binding protein (hereinafter sometimes simply referred to as G protein) and mediate the intracellular signal transduction via activation of G protein. These receptor proteins possess the common structure containing seven transmembrane domains and are thus collectively referred to as G protein-coupled receptors or seven-transmembrane receptors (7TMR).

G protein-coupled receptor proteins present on the cell surface of each functional cell and organ in the body, and play important physiological roles as the target of the molecules that regulate the functions of the cells and organs, e.g., hormones, neurotransmitters, physiologically active substances and the like. Receptors transmit signals to cells via binding with physiologically active substances, and the signals induce various reactions such as activation and inhibition of the cells.

To clarify the relationship between substances that regulate complex biological functions in various cells and organs, and their specific receptor proteins, in particular, G protein-coupled receptor proteins, would elucidate the functional mechanisms in various cells and organs in the body to provide a very important means for development of drugs closely associated with the functions.

For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. However, it is supposed that many unknown hormones, neurotransmitters or many other physiologically active substances still exist in the body and, as to their receptor proteins, many of these proteins have not yet been reported. In addition, it is still unknown if there are subtypes of known receptor proteins.

It is very important for development of drugs to clarify the relationship between substances that regulate elaborated functions in vivo and their specific receptor proteins. Furthermore, for efficient screening of agonists and antagonists to receptor proteins in development of drugs, it is required to clarify functional mechanisms of receptor protein genes expressed in vivo and express the genes in an appropriate expression system.

In recent years, random analysis of cDNA sequences has been actively studied as a means for analyzing genes expressed in vivo. The sequences of cDNA fragments thus obtained have been registered on and published to databases as Expressed Sequence Tag (EST). However, since many ESTs contain sequence information only, it is difficult to predict their functions from the information.

Substances that inhibit binding between G protein-coupled proteins and physiologically active substances (i.e., ligands) and substances that bind and induce signals similar to those induced by physiologically active substances (i.e., ligands) have been used as pharmaceuticals, as antagonists and agonists specific to the receptors, that regulate the biological functions. Therefore, discovery and gene cloning (e.g., cDNA) of a novel G protein-coupled receptor that can be targeted for pharmaceutical development are very important means in search for a specific ligand, agonist, and antagonist of the novel G protein-coupled receptor.

However, not all G protein-coupled receptors have been discovered. There are unknown G protein-coupled receptors and many of these receptors in which the corresponding ligands are yet unidentified are called orphan receptors. Therefore, search and functional elucidation of a novel G protein-coupled receptor is awaited.

G protein-coupled receptors are useful in searching for a novel physiological active substance (i.e., ligand) using the signal transduction activity as the index and in search for agonists and antagonists of the receptor. Even if no physiological ligand is found, agonists and antagonist of the receptor may be prepared by analyzing the physiological action of the receptor through inactivation experiment of the receptor (knockout animal). Ligands, agonists, antagonists, etc. of the receptor are expected to be used as prophylactic/therapeutic and diagnostic agents for diseases associated with dysfunction of the G protein-coupled receptor.

Lowering or accentuation in functions of the G protein coupled receptor due to genetic aberration of the receptor in vivo causes some disorders in many cases. In this case, the G protein coupled receptor may be used not only for administration of antagonists or agonists of the receptor, but also for gene therapy by transfer of the receptor gene into the body (or some specific organs) or by introduction of the antisense nucleic acid of the receptor gene into the body (or the specific organ). In the gene therapy, information on the base sequence of the receptor gene is essentially required for investigating deletion or mutation in the gene. The receptor gene is also applicable as prophylactic/therapeutic and diagnostic agents for diseases associated with dysfunction of the receptor.

DISCLOSURE OF THE INVENTION

The present invention provides a novel and useful G protein-coupled receptor protein as described above. That is, the present invention provides a novel G protein-coupled receptor protein, its partial peptides and salts thereof, as well as polynucleotides (DNA and RNA, and derivatives thereof) containing the polynucleotides (DNA and RNA, and derivatives thereof) encoding the G protein-coupled receptor protein or its partial peptides, recombinant vectors containing the polynucleotides, transformants bearing the recombinant vectors, methods for manufacturing the G protein-coupled receptor protein or its salts, antibodies to the G protein-coupled receptor protein, its partial peptides and salts thereof, compounds that alter the expression level of said G protein-coupled receptor protein, methods for determination of ligands to the G protein-coupled receptor protein, methods for screening the compounds (antagonists and agonists) or salts thereof that alter the binding property of ligands and the G protein-coupled receptor protein, kits for use in the screening methods, compounds (antagonists and agonists) or salts thereof that alter the binding property of ligands obtainable by the screening methods or obtainable using the screening kits and the G protein-coupled receptor protein, and pharmaceutical compositions comprising the compounds (antagonists and agonists) that alter the binding property of ligands to the G protein-coupled receptor protein, or compounds or salts thereof that alter the expression level of the G protein-coupled receptor protein.

As a result of extensive investigations, the present inventors have succeeded in isolating cDNAs encoding novel G protein-coupled receptor proteins derived from rat whole brain, and in sequencing the full-length base sequences. When the base sequences were translated into the amino acid sequences, 1 to 7 transmembrane domains were found to be on the hydrophobic plot, establishing that the proteins encoded by these cDNAs are seven-transmembrane type G protein-coupled receptor proteins.

Based on these findings, the present inventors have continued further extensive studies and as a result, have come to accomplish'the present invention.

Thus, the present invention relates to the following features.

(1) A G protein-coupled receptor protein containing the same or substantially the same amino acid sequence as the amino acid sequence represented by SEQ ID NO: 1 or SEQ ID NO: 138, or a salt thereof.

(2) A G protein-coupled receptor protein according to (1), or a salt thereof, which comprises containing an amino acid sequence represented by SEQ ID NO: 1.

(3) A G protein-coupled receptor protein according to (1), or a salt thereof, which comprises containing an amino acid sequence represented by SEQ ID NO: 138.

(4) A partial peptide of the G protein-coupled receptor protein according to (1), or a salt thereof.

(5) A polynucleotide containing a polynucleotide encoding the G protein-coupled protein according to (1).

(6) A polynucleotide according to (5), which is DNA.

(7) A DNA according to (6), which is represented by SEQ ID NO: 2 or SEQ ID NO: 139.

(8) A recombinant vector containing the polynucleotide according to (5).

(9) A transformant transformed with the recombinant vector according to (8).

(10) A method of manufacturing the G protein-coupled receptor protein or its salt according to (1), which comprises culturing the transformant according to (9) and accumulating the G protein-coupled receptor protein according to (1).

(11) An antibody to the G protein-coupled receptor protein according to (1), the partial peptide according to (4), or a salt of said protein or partial peptide.

(12) An antibody according to (11), which is a neutralizing antibody capable of inactivating signal transduction of the G protein-coupled receptor protein according to (1).

(13) A diagnostic composition comprising an antibody according to (11).

(14) A ligand to the G protein-coupled receptor protein or its salt according to (1), which is obtainable using the G protein-coupled receptor protein according to (1) or the partial peptide according to (4), or a salt of said protein or partial peptide.

(15) A pharmaceutical composition comprising the ligand to the G protein-coupled receptor according to (14).

(16) A method of determining a ligand to the G protein-coupled receptor protein or its salt according to (1), which comprises using the G protein-coupled receptor protein according to (1) or the partial peptide according to (4), or a salt of said protein or partial peptide.

(17) A method of screening a compound that alters the binding property between a ligand and the G protein-coupled receptor protein or its salt according to (1), which comprises using the G protein-coupled receptor protein according to (1) or the partial peptide according to (4), or a salt of said protein or partial peptide.

(18) A kit for screening a compound or its salt that alters the binding property between a ligand and the G protein-coupled receptor protein or its salt according to (1), comprising the G protein-coupled receptor protein according to (1) or the partial peptide according to (4), or a salt of said protein or partial peptide.
(19) A compound or its salt that alters the binding property between a ligand and the G protein-coupled receptor protein or its salt according to (1), which is obtainable using the screening method according to (17) or the screening kit according to (18).
(20) A pharmaceutical composition comprising a compound or its salt that alters the binding property between a ligand and the G protein-coupled receptor protein or its salt according to (1), which is obtainable using the screening method according to (17) or the screening kit according to (18).
(21) A polynucleotide that hybridizes to the polynucleotide according to (5) under a highly stringent condition.
(22) A polynucleotide comprising a base sequence complementary to the polynucleotide according to (5) or a part of the base sequence.
(23) A method of quantifying mRNA of the G protein-coupled receptor protein according to (1), which comprises using the polynucleotide according to (5) or a part of the polynucleotide.
(24) A method of quantifying the G protein-coupled receptor protein according to (1), which comprises using the antibody according to (11).
(25) A diagnostic method for a disease associated with functions of the G protein-coupled receptor protein according to (1), which comprises using the quantification method according to (23) or (24).
(26) A method of screening a compound or its salt that alters the expression level of the G protein-coupled receptor protein according to (1), which comprises using the quantification method according to (23).
(27) A method of screening a compound or its salt that alters the amount of the G protein-coupled receptor protein according to (1) in cell membrane, which comprises using the quantification method according to (24).
(28) A compound or its salt that alters the expression level of the G protein-coupled receptor protein according to (1), which is obtainable using the screening method according to (26).
(29) A compound or its salt that alters the amount of the G protein-coupled receptor protein according to (1) in cell membrane, which is obtainable using the screening method according to (27).
(30) The screening method according to (17), which comprises comparing (i) the case where the G protein-coupled receptor protein according to (1) or its salt, or the partial peptide according to (4) or its salt is contacted with ligand, with (ii) the case where the G protein-coupled receptor protein according to (1) or its salt, or the partial peptide according to (4) or its salt is contacted with ligand and test compound.
(31) The screening method according to (30), wherein the ligand is the polypeptide containing an identical or substantially identical amino acid sequence to that represented by SEQ ID NO: 8.
(32) The screening method according to (30), wherein the ligand is the polypeptide containing an amino acid sequence represented by SEQ ID NO: 8.
(33) The screening method according to (30), wherein the ligand is the polypeptide having an amino acid sequence represented by SEQ ID NO: 8.
(34) The screening method according to (30), wherein the ligand is the polypeptide having an amino acid sequence represented by SEQ ID NO: 9.
(35) The diagnostic according to (13), which is a diagnostic for obesity.
(36) The medicament according to (15) or (20), which is an anti-obesity drug.
(37) The medicament according to (15) or (20), which is an appetite enhancer.
(38) The medicament according to (15) or (20), which is an inhibitor of prolactin production..
(39) A method for prevention and/or treatment of obesity, which comprises administrating an effective amount of a compound or a salt thereof that alters a binding property between the ligand according to (19) and the G protein-coupled receptor protein according to claim 1 or a salt thereof, to mammal.
(40) A method for enhancing appetite, which comprises administrating an effective amount of a compound or a salt thereof that alters a binding property between the ligand according to (19) and the G protein-coupled receptor protein according to claim 1 or a salt thereof, to mammal.
(41) A method for inhibiting a prolactin production, which comprises administrating an effective amount of a compound or a salt thereof that alters a binding property between the ligand according to (19) and the G protein-coupled receptor protein according to claim 1 or a salt thereof, to mammal.
(42) Use of a compound or a salt thereof that alters a binding property between the ligand according to (19) and the G protein-coupled receptor protein according to claim 1 or a salt thereof for manufacturing an anti-obesity drug.
(43) Use of a compound or a salt thereof that alters a binding property between the ligand according to (19) and the G protein-coupled receptor protein according to claim 1 or a salt thereof for manufacturing an appetite enhancer.
(44) Use of a compound or a salt thereof that alters a binding property between the ligand according to (19) and the G protein-coupled receptor protein according to claim 1 or a salt thereof for manufacturing an inhibitor of prolactin production.

(45) A non-human transgenic animal, which has exogenous DNA encoding the G protein-coupled receptor protein according to (1) or mutated DNA thereof.
(46) The animal according to (45), wherein the non-human animal is a rodent.
(47) The animal according to (46), wherein the rodent is mouse or rat.
(48) A recombinant vector, which contains exogenous DNA encoding the G protein-coupled receptor protein according to (1) or mutated DNA thereof, and is capable of expressing in non-human animal.
(49) A non-human mammalian embryonic stem cell, wherein the DNA encoding the G protein-coupled receptor protein according to (1) is inactivated.
(50) The embryonic stem cell according to (49), wherein the non-human mammal is a rodent.
(51) The embryonic stem cell according to (50), wherein the rodent is mouse.
(52) A non-human mammal barely expressing DNA, wherein the DNA encoding the G protein-coupled receptor protein according to (1).
(53) The non-human mammal according to (52), wherein the non-human mammal is a rodent.
(54) The non-human mammal according to (53), wherein the rodent is mouse.

The present invention further relates to the following features.

(55) A G protein-coupled receptor protein or its salt according to (1), wherein said protein contains <1> (i) the amino acid sequence shown by SEQ ID NO: 1, of which at least 1 or 2 (preferably approximately 1 to 30, more preferably approximately 1 to 10, most preferably several (1 to 5)) amino acids are deleted, (ii) the amino acid sequence shown by SEQ ID NO: 1, to which at least 1 or 2 (preferably approximately 1 to 30, more preferably approximately 1 to 10, most preferably several (1 to 5)) amino acids are added; (iii) the amino acid sequence shown by SEQ ID NO: 1, in which at least 1 or 2 (preferably approximately 1 to 30, more preferably approximately 1 to 10, most preferably several (1 to 5)) amino acids are substituted; or (iv) the amino acid sequence containing a combination of these amino acid sequences, or <2> (i) the amino acid sequence shown by SEQ ID NO: 138, of which at least 1 or 2 (preferably approximately 1 to 30, more preferably approximately 1 to 10, most preferably several (1 to 5)) amino acids are deleted, (ii) the amino acid sequence shown by SEQ ID NO: 138, to which at least 1 or 2 (preferably approximately 1 to 30, more preferably approximately 1 to 10, most preferably several (1 to 5)) amino acids are added; (iii) the amino acid sequence shown by SEQ ID NO: 138, in which at least 1 or 2 (preferably approximately 1 to 30, more preferably approximately 1 to 10, most preferably several (1 to 5)) amino acids are substituted; or (iv) the amino acid sequence containing a combination of these amino acid sequences.
(56) A method of determining a ligand according to (16), which comprises contacting the G protein-coupled receptor protein or its salt according to (1) or the partial peptide or its salt according to (4) with a test compound.
(57) A method of determining a ligand according to (56), in which said ligand is, for example, angiotensin, bombesin, canavinoid, cholecystokinin, glutamine, serotonin, melatonin, neuropeptide Y, an opioid, a purine, vasopressin, oxytocin, PACAP (e.g., PACAP27, PACAP38), secretin, glucagon, calcitnonin, adrenomedulin, somatostatin, GHRH, CRF, ACTH, GRP, PTH, vasoactive intestinal and related polypeptide (VIP), somatostatin, dopamine, motilin, amylin, bradykinin, calcitonin gene-related peptide (CGRP), a leukotriene, pancreastatin, a prostaglandin, thromboxane, adenosine, adrenaline, a chemokine superfamily (e.g., IL-8, GRO$\alpha$, GRO$\beta$, GRO$\gamma$, NAP-2, ENA-78, GCP-2, PF4, IP10, Mig, CXC chemokine subfamily such as PBSF/SDF-1, etc.; CC chemokine subfamily such as MCAF/MCP-1, MCP-2, MCP-3, MCP-4, eotaxin, RANTES, MIP1-$\alpha$, MIP-1$\beta$, HCC-1, MIP-3$\alpha$/LARC, MIP-3$\beta$/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc., etc.), endothelin, enterogastrin, histamine, neurotensin, TRH, pancreatic polypeptide, galanin, lysophosphatidic acid (LPA), sphingosine 1-phosphate or a polypeptide containing an identical or substantially identical amino acid sequence to that represented by SEQ ID NO: 8.
(58) A method of screening a compound or its salt that alters the binding property between a ligand and the G protein-coupled receptor protein or its salt according to (1), which comprises measuring the amounts of a labeled ligand bound to the G protein-coupled receptor protein or its salt according to (1) or to the partial peptide or its salt according to (4), (i) when the labeled ligand is brought in contact with the G protein-coupled receptor protein or its salt according to (1) or with the partial peptide or its salt according to (4), and (ii) when the labeled ligand and a test compound are brought in contact with the G protein-coupled receptor protein or its salt according to (1) or with the partial peptide or its salt according to (4); and comparing the amounts measured in (i) and (ii).
(59) A method of screening a compound or its salt that alters the binding property between a ligand and the G protein-coupled receptor protein or its salt according to (1), which comprises measuring the amounts of a labeled ligand bound to a cell containing the G protein-coupled receptor protein according to (1), (i) when the labeled ligand is brought in contact with the cell containing the G protein-coupled receptor protein according to (1), and (ii) when the labeled ligand and a test compound are brought in contact with the cell containing the G protein-coupled receptor protein according to (1); and comparing the amounts measured in (i) and (ii).

(60) A method of screening a compound or its salt that alters the binding property between a ligand and the G protein-coupled receptor protein or its salt according to (1), which comprises measuring the amounts of a labeled ligand bound to a cell membrane fraction containing the G protein-coupled receptor protein according to (1), (i) when the labeled ligand is brought in contact with the cell membrane fraction, and (ii) when the labeled ligand and a test compound are brought in contact with the cell membrane fraction; and comparing the amounts measured in (i) and (ii).

(61) A method of screening a compound or its salt that alters the binding property between a ligand and the G protein-coupled receptor protein or its salt according to (1), which comprises measuring the amounts of a labeled ligand bound to a G protein-coupled receptor protein expressed in a cell membrane, (i) when the labeled ligand is brought in contact with the G protein-coupled receptor protein expressed in a cell membrane of the transformant according to (9) by culturing the transformant and (ii) when the labeled ligand and a test compound are brought in contact with the G protein-coupled receptor protein expressed in a cell membrane of the transformant according to (9) by culturing the transformant; and comparing the amounts measured in (i) and (ii).

(62) A method of screening a compound or its salt that alters the binding property between a ligand and the G protein-coupled receptor protein or its salt according to (1), which comprises measuring the G protein-coupled receptor protein-mediated cell stimulating activities, (i) when a compound that activates the G protein-coupled receptor protein or its salt according to (1) is brought in contact with a cell containing the G protein-coupled receptor protein according to (1), and (ii) when a compound that activates the G protein-coupled receptor protein or its salt according to (1) and a test compound are brought in contact with a cell containing the G protein-coupled receptor protein according to (1); and comparing the activities measured in (i) and (ii).

(63) A method of screening a compound or its salt that alters the binding property between a ligand and the G protein-coupled receptor protein or its salt according to (1), which comprises measuring the G protein-coupled receptor protein-mediated cell stimulating activities, when a compound that activates the G protein-coupled receptor protein or its salt according to (1) is brought in contact with a G protein-coupled receptor protein expressed in a cell membrane of the transformant according to (9) by culturing the transformant, and when the compound that activates the G protein-coupled receptor protein or its salt according to (1) and a test compound are brought in contact with the G protein-coupled receptor protein expressed in a cell membrane of the transformant according to (9) by culturing the transformant; and comparing the protein-mediated activities measured in (i) and (ii).

(64) A method of screening according to (62) or (63), in which said compound that activates the protein according to (1) is angiotensin, bombesin, canavinoid, cholecystokinin, glutamine, serotonin, melatonin, neuropeptide Y, an opioid, a purine, vasopressin, oxytocin, PACAP (e.g., PACAP27, PACAP38), secretin, glucagon, calcitnonin, adrenomedulin, somatostatin, GHRH, CRF, ACTH, GRP, PTH, vasoactive intestinal and related polypeptide (VIP), somatostatin, dopamine, motilin, amylin, bradykinin, calcitonin gene-related peptide (CGRP), a leukotriene, pancreastatin, a prostaglandin, thromboxane, adenosine, adrenaline, a chemokine superfamily (e.g., IL-8, GROα, GROβ, GROγ, NAP-2, ENA-78, GCP-2, PF4, IP10, Mig, CXC chemokine subfamily such as PBSF/SDF-1, etc.; CC chemokine subfamily such as MCAF/MCP-1, MCP-2, MCP-3, MCP-4, eotaxin, RANTES, MIP1-α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc., etc.), endothelin, enterogastrin, histamine, neurotensin, TRH, pancreatic polypeptide, galanin, lysophosphatidic acid (LPA), sphingosine 1-phosphate or a polypeptide containing an identical or substantially identical amino acid sequence to that represented by SEQ ID NO: 8.

(65) A compound or its salt that alters the binding property between a ligand and the G protein-coupled receptor protein or its salt according to (1), which is obtainable by the screening methods according to (30) through (34) or (58) through (64).

(66) A pharmaceutical composition comprising a compound or its salt that alters the binding property between a ligand and the G protein-coupled receptor protein or its salt according to (1), which is obtainable by the screening methods according to (30) through (34) or (58) through (64).

(67) A kit for screening according to (18), comprising a cell containing the G protein-coupled receptor protein according to (1).

(68) A screening kit according to (18), comprising a cell membrane fraction containing the G protein-coupled receptor protein according to (1).

(69) A screening kit according to (18), comprising a G protein-coupled receptor protein expressed on the cell membrane of the transformant according to (9) by culturing the transformant.

(70) A compound or its salt that alters the binding property of a ligand and the G protein-coupled receptor protein or its salt according to (1), which is obtainable using the screening kits according to (67) through (69).

(71) A pharmaceutical composition comprising a compound or its salt that alters the binding property of a ligand compound or its salt that alters the binding property between a ligand and the G protein-coupled receptor protein or its salt according to (1), which is obtainable using the screening kits according to (67) through (69).

(72) A method of quantifying the G protein-coupled receptor protein according to (1), the partial peptide according to (4), or a salt thereof, which comprises contacting the antibody according to (11) with the G protein-coupled receptor protein according to (1), the partial peptide according to (4), or a salt thereof.

(73) A method of quantifying the G protein-coupled receptor protein according to (1), the partial peptide according to (4) or salts thereof in a test fluid, which comprises competitively reacting the antibody according to (11) with a test fluid and a labeled form of the G protein-coupled receptor protein according to (1), the partial peptide according to (4) or salts thereof; and measuring the ratios bound to the antibody of the labeled form of the G protein-coupled receptor protein according to (1), the partial peptide or its salts according to (4).

(74) A method of quantifying the G protein-coupled receptor protein according to (1), the partial peptide according to (4), or salts thereof in a test fluid, which comprises reacting a test fluid simultaneously or sequentially with the antibody according to (11) immobilized on a carrier and the labeled antibody according to (11), and then measuring the activity of the label on the immobilizing carrier.

BRIEF DESCRIPTION OF THE DRAWINGS

FIG. 1 shows a base sequence of DNA encoding TGR26 and an amino acid sequence of TGR26 (continued to FIG. 2).

FIG. 2 shows a base sequence of DNA encoding TGR26 and an amino acid sequence of TGR26 (continued from FIG. 1).

FIG. 3 shows a plot for hydrophobicity of TGR26.

FIG 4 shows an inhibiting activity for cAMP production of hGPR8L (1-23) and hGPR8L (1-30) on CHO/TGR26 cells. In the figure, open circle and open triangle show the administration of hGPR8L (1-23) and hGPR8L (1-30), respectively.

FIG 5 shows an enhancing activity for GTPγS binding of hGPR8L (1-23) and hGPR8L (1-30) on CHO/TGR26 cells. In the figure, open circle and open triangle show the administration of hGPR8L (1-23) and hGPR8L (1-30), respectively.

FIG. 6 shows an influence of GPR8 ligand peptide on an amount of food ingested. Each value shows the mean value plus/minus SEM.

FIG. 7 shows an inhibiting activity for binding of a various concentration of hGPR8L (1-23) and hGPR8L (1-30) on the binding of [$^{125}$I]-labeled human GPR8 ligand, which consists of 23 residues, to a cell membrane fraction prepared from TGR26-expressing CHO cells. In the figure, open circle and open triangle show the administration of hGPR8L (1-23) and hGPR8L (1-30), respectively.

FIG 8 shows a DNA sequence of human GPR7 ligand precursor H.

FIG. 9 shows an amino acid sequence of human GPR7 ligand precursor H.

FIG. 10 shows a DNA sequence of mouse GPR7 ligand precursor. H.

FIG. 11 shows an amino acid sequence of mouse GPR7 ligand precursor H.

FIG. 12 shows a DNA sequence of rat GPR7 ligand precursor H.

FIG. 13 shows an amino acid sequence of rat GPR7 ligand precursor H.

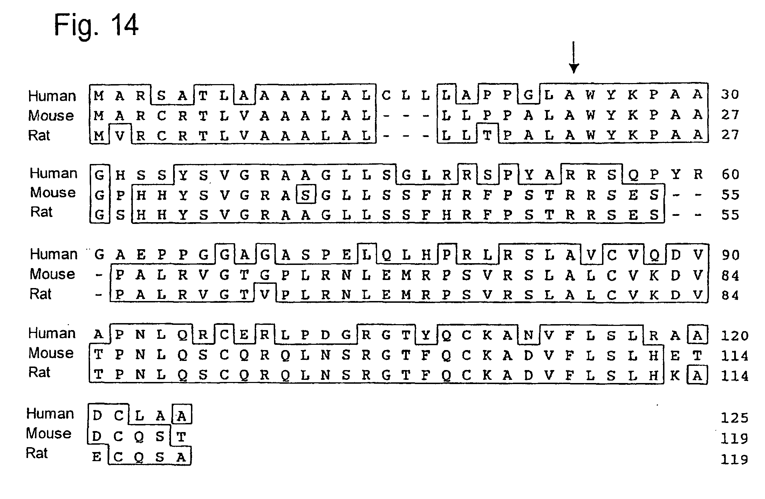

FIG. 14 shows comparison among human, rat and mouse GPR7 ligand precursor H. The same amino acids are enclosed by box. Also, the arrow shows a predicted cleavage site of secretion signal.

FIG. 15 shows detection of inhibition of luciferase activity by ligand stimulus caused by adding culture supernatant of CHO cells, in which the ligand expression vector pAK-S64 and the expression vector without insertion (pAKKO-111H) are expressed, to medium of CHO cells, in which the expression vector inserted with GPR7 cDNA is transiently expressed, with forskolin (FSK).

FIG. 16 shows detection of inhibition of luciferase activity when culture supernatant of CHO cells, in which S64 is transiently expressed, is added to medium of CHO cells, in which TGR26 is transiently expressed, with FSK.

BEST MODE FOR CARRYING OUT THE INVENTION

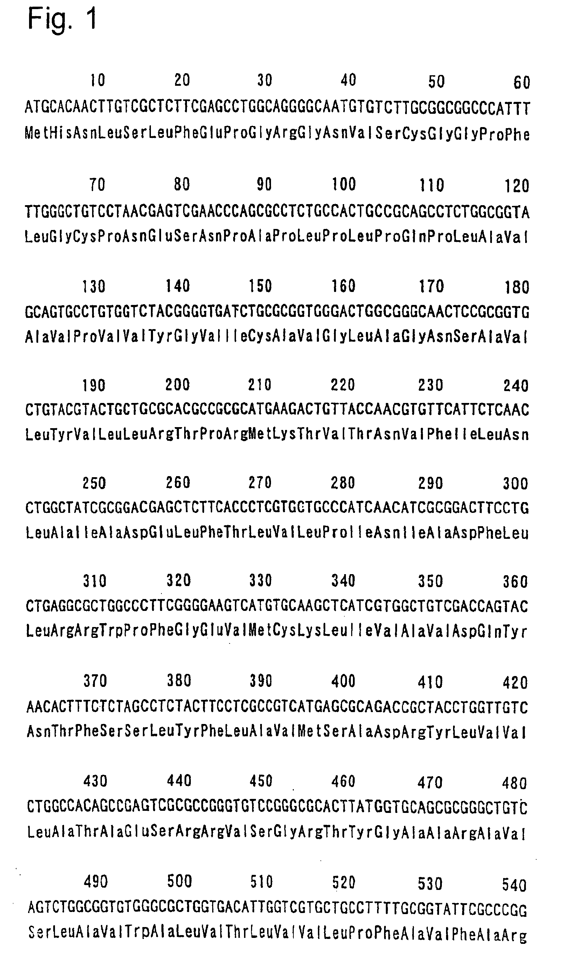

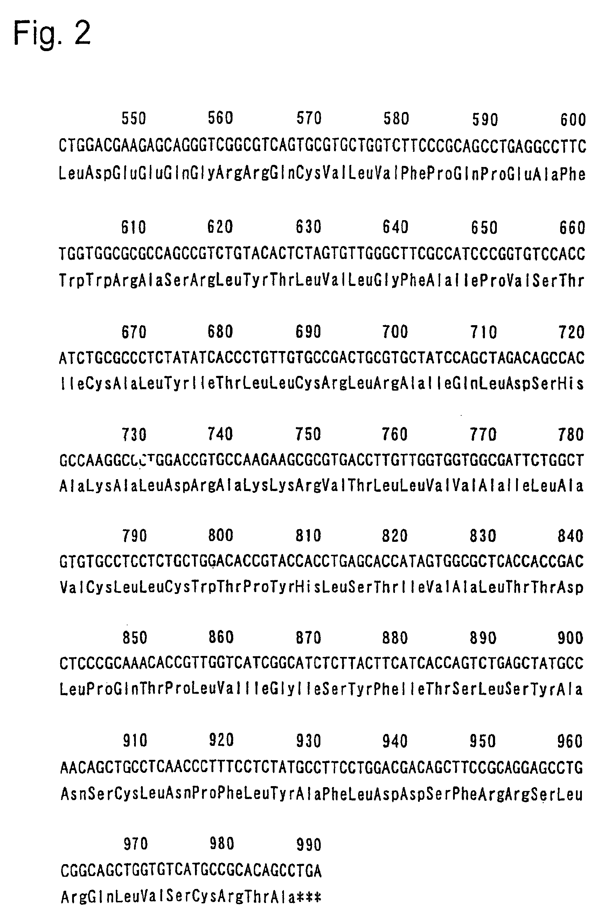

The G protein-coupled receptor protein of the present invention (hereinafter sometimes merely referred to as the receptor protein) is a receptor protein, which contains the same or substantially the same amino acid sequence as the amino acid sequence shown by SEQ ID NO: 1 (FIGs. 1 and 2) or a receptor protein, which contains the same or substantially the same amino acid sequence as the amino acid sequence shown by SEQ ID NO: 138.

The receptor protein of the present invention may be any protein derived from any cells (e.g., retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g.,

macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testis, ovary, placenta, uterus, bone, joint, skeletal muscle, etc. from human and other mammals (e.g., guinea pigs, rats, mice, rabbits, swine, sheep, bovine, monkeys, etc.). The receptor protein may also be a synthetic protein.

The amino acid sequence which has substantially the same amino acid sequence as that represented by SEQ ID NO: 1 includes an amino acid sequence having at least about 85% homology, preferably at least about 90% homology, more preferably at least about 95% homology, to the amino acid sequence represented by SEQ ID NO: 1.

Examples of the protein which contains substantially the same amino acid sequence as that shown by SEQ ID NO: 1 include a protein having substantially the same amino acid sequence as that shown by SEQ ID NO: 1 and having the activity substantially equivalent to the amino acid sequence represented by SEQ ID NO: 1, etc.

The amino acid sequence which has substantially the same amino acid sequence as that represented by SEQ ID NO: 138 includes an amino acid sequence having at least about 86% homology, preferably at least about 90% homology, more preferably at least about 95% homology, to the amino acid sequence represented by SEQ ID NO: 138.

Examples of the protein which contains substantially the same amino acid sequence as that shown by SEQ ID NO: 138 include a protein having substantially the same amino acid sequence as that shown by SEQ ID NO: 138 and having the activity substantially equivalent to the amino acid sequence represented by SEQ ID NO: 138, etc.

Examples of the substantially equivalent activity include a ligand binding activity, a signal transduction activity, etc. The term "substantially equivalent" is used to mean that the nature of the activity is the same. Therefore, although it is preferred that activities such as the ligand binding and signal transduction activities, etc. be equivalent (e.g., about 0.01- to about 100-fold, preferably about 0.5- to about 20-fold, more preferably about 0.5- to about 2-fold), quantitative factors such as a level of the activity, a molecular weight of the protein, etc. may differ.

The activities such as ligand binding and signal transduction activities or the like can be determined according to a publicly known method with some modifications, for example, by the ligand determination methods or the screening methods that will be later described.

Proteins containing the following amino acid sequences are used as the receptor protein of the present invention: (1) (i) amino acid sequences represented by SEQ ID NO: 1, wherein at least 1 or 2 amino acids (preferably approximately 1 to 30 amino acids, more preferably approximately 1 to 10 amino acids, most preferably several (1 to 5) amino acids) are deleted; (ii) amino acid sequences represented by SEQ ID NO: 1, to which at least 1 or 2 amino acids (preferably approximately 1 to 30 amino acids, more preferably approximately 1 to 10 amino acids, and most preferably several (1 to 5) amino acids) are added; (iii) amino acid sequences represented by SEQ ID NO: 1, in which at least 1 or 2 amino acids (preferably approximately 1 to 30 amino acids, more preferably approximately 1 to 10 amino acids, and most preferably several (1 to 5) amino acids) are substituted by other amino acids; or (iv) combination of the amino acid sequences described in the above, and (2) (i) amino acid sequences represented by SEQ ID NO: 138, wherein at least 1 or 2 amino acids (preferably approximately 1 to 30 amino acids, more preferably approximately 1 to 10 amino acids, most preferably several (1 to 5) amino acids) are deleted; (ii) amino acid sequences represented by SEQ ID NO: 138, to which at least 1 or 2 amino acids (preferably approximately 1 to 30 amino acids, more preferably approximately 1 to 10 amino acids, and most preferably several (1 to 5) amino acids) are added; (iii) amino acid sequences represented by SEQ ID NO: 138, in which at least 1 or 2 amino acids (preferably approximately 1 to 30 amino acids, more preferably approximately 1 to 10 amino acids, and most preferably several (1 to 5) amino acids) are substituted by other amino acids; or (iv) combination of the amino acid sequences described in the above.

Throughout the present specification, the receptor proteins are represented in accordance with the conventional way of describing peptides, that is, the N-terminus (amino terminus) at the left hand and the C-terminus (carboxyl terminus) at the right hand. In the receptor proteins of the present invention including the receptor proteins containing the amino acid sequence shown by SEQ ID NO: 1, the C-terminus is usually in the form of a carboxyl group (-COOH) or a carboxylate ($-COO^-$) but may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the C-terminus described above.

Furthermore, examples of the receptor protein of the present invention include variants of the above receptor proteins, wherein the amino group at the N-terminal methionine residue of the protein supra is protected with a protecting group (for example, a $C_{1-6}$ acyl group such as a $C_{2-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins bound to sugar chains.

Specific examples of the receptor protein of the present invention which can be used include a receptor protein containing an amino acid sequence represented by SEQ ID NO: 1, a receptor protein containing an amino acid sequence represented by SEQ ID NO: 138, etc.

As partial peptides of the receptor protein of the present invention (hereinafter sometimes referred to as the partial peptides), any partial peptide can be used so long as it can be a partial peptide of the receptor protein. Among the receptor protein molecules of the present invention, for example, those having a site exposed to the outside of a cell membrane and having a substantially equivalent receptor binding activity can be used.

Specifically, the partial peptide of the receptor protein having the amino acid sequence represented by SEQ ID NO: 1 or SEQ ID NO: 138 is a peptide containing the parts analyzed to be extracellular domains (hydrophilic domains) in the hydrophobic plotting analysis. A peptide containing a hydrophobic domain in part can be used as well. In addition, the peptide may contain each domain separately or plural domains together.

In the receptor protein of the present invention, preferred partial peptides are those having at least 20, preferably at least 50, and more preferably at least 100 amino acids, in the amino acid sequence which constitutes the receptor protein of the present invention.

Herein, the term "activity substantially equivalent" refers to the same significance as defined above. The "activity substantially equivalent' can be assayed in the same manner as given above.

The partial peptide of the present invention may contain an amino acid sequence, wherein (i) at least 1 or 2 amino acids (preferably approximately 1 to 10 amino acids, more preferably several (1 to 5) amino acids) are deleted; (ii) to which at least 1 or 2 amino acids (preferably approximately 1 to 20 amino acids, more preferably approximately 1 to 10 amino acids, and most preferably several (1 to 5) amino acids) are added; or, (iii) in which at least 1 or 2 amino acids (preferably approximately 1 to 10 amino acids, more preferably several and most preferably approximately 1 to 5 amino acids) are substituted by other amino acids.

More specifically, the partial peptide of the present invention includes a peptide, which contains (i) a partial amino acid sequence consisting of the 1st (Met) through the 85th (Asp) amino acid residue from N-terminus, or its portion, or (ii) a partial amino acid sequence consisting of the 222nd (Cys) through the 329th (Ala) amino acid residue from N-terminus, or its portion, in the amino acid sequence represented by SEQ ID NO: 1.

In the partial peptide of the present invention, the C-terminus is normally a carboxyl group (-COOH) or carboxylate ($-COO^-$) but the C-terminus may be in the form of an amide ($-CONH_2$) or an ester (-COOR) (R means the same significance as described above). When the partial peptide of the present invention has carboxyl group (or carboxylate) apart from the C-terminus, a peptide which carboxyl group is amidated or esterified is also included in the partial peptide of the present invention. As the ester, for example, an ester of the C-terminus described above is used.

As in the receptor protein of the present invention described above, the partial peptide of the present invention further includes those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycoproteins, to which sugar chains are bound, and the like.

For salts of the receptor protein or the partial peptide of the present invention, preferred are salts with physiologically acceptable acids, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide having an identical or substantially identical amino acid sequence to that represented by SEQ ID NO: 8 (hereinafter, referred to as the polypeptide used in the present invention) may be any protein derived from any cells (e.g., retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), or any tissues where such cells are present, e.g., brain or any region of the brain (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc., or hemocyte type cells or its cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.) from human and warm-blooded animals (e.g., guinea pigs, rats, mice, chicken, rabbits, swine, sheep, bovine, monkeys, etc.). The receptor protein may also be a synthetic protein.

The amino acid sequence which has substantially the same amino acid sequence as that represented by SEQ ID NO: 8 includes an amino acid sequence having at least about 90% homology, preferably at least about 95% homology, more preferably at least about 98% homology, to the amino acid sequence represented by SEQ ID NO: 8.

In particular, the amino acid sequence which has substantially the same amino acid sequence as that represented by SEQ ID NO: 8 includes, except for the above-mentioned amino acid sequences, (i) amino acid sequences represented by SEQ ID NO: 8, wherein at least 1 or 5 amino acids (preferably 1 to 3 amino acids, further preferably approximately 1 to 2 amino acids, more preferably 1 amino acid) are deleted; (ii) amino acid sequences represented by SEQ ID NO: 8, to which at least 1 or 5 amino acids (preferably 1 to 3 amino acids, further preferably approximately 1 to 2 amino acids, more preferably 1 amino acid) are added; (iii) amino acid sequences represented by SEQ ID NO: 8, to which at least 1 or 5 amino acids (preferably 1 to 3 amino acids, further preferably approximately 1 to 2 amino acids, more preferably 1 amino acid) are inserted; (iv) amino acid sequences represented by SEQ ID NO: 8, in which at least 1 or 2 amino acids (preferably approximately 1 to 30 amino acids, more preferably approximately 1 to 10 amino acids, and most preferably several (1 to 5) amino acids) are substituted by other amino acids; or (v) combination of the amino acid sequences described in the above (i) through (iv).

Examples of the protein which contains substantially the same amino acid sequence as that shown by SEQ ID NO: 8 include a protein having substantially the same amino acid sequence as that shown by SEQ ID NO: 8 and having the activity substantially equivalent to the amino acid sequence represented by SEQ ID NO: 8, etc.

Examples of the substantially equivalent activity include an activity which the polypeptide used in the present invention possesses (e.g., an activity for prevention and/or treatment of diseases described below, a binding activity to receptor, cell stimulating activities on the receptor-expressing cells (e.g., the activities that accelerate arachidonic acid release, acetylcholine release, intracellular $Ca^{2+}$ release, enhancement and/or inhibition of intracellular cAMP production, intracellular cGMP production, inositol phosphate production, change in cell membrane potential, phosphorylation of intracellular proteins, activation of c-fos, pH reduction, GTPγS binding activity, etc.).

The phrase "substantially equivalent" indicates that these activities are equivalent concerning the properties (e.g., physiochemically, or pharmaceutically).

Specific examples of the protein which contains substantially the same amino acid sequence as that shown by SEQ ID NO: 8 include amino acid sequences represented by SEQ ID NO: 14, SEQ ID NO: 9, SEQ ID NO: 128, SEQ ID NO: 129, SEQ ID NO: 130, SEQ ID NO: 131, SEQ ID NO: 24, SEQ ID NO: 25, SEQ ID NO: 56, SEQ ID NO: 57, SEQ ID NO: 73, SEQ ID NO: 74, SEQ ID NO: 91, SEQ ID NO: 92, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, or SEQ ID NO: 113.

Specific examples of the polypeptide used in the present invention include a polypeptide having the ability of specific binding to the receptor protein of the present invention such as a polypeptide having an amino acid sequence represented by SEQ ID NO: 8, a polypeptide having an amino acid sequence represented by SEQ ID NO: 14, a polypeptide having an amino acid sequence represented by SEQ ID NO: 9, a polypeptide having an amino acid sequence represented by SEQ ID NO: 128, a polypeptide having an amino acid sequence represented by SEQ ID NO: 129, a polypeptide having an amino acid sequence represented by SEQ ID NO: 130, a polypeptide having an amino acid sequence represented by SEQ ID NO: 131, a polypeptide having an amino acid sequence represented by SEQ ID NO: 24, a polypeptide having an amino acid sequence represented by SEQ ID NO: 25, a polypeptide having an amino acid sequence represented by SEQ ID NO: 56, a polypeptide having an amino acid sequence represented by SEQ ID NO: 57, a polypeptide having an amino acid sequence represented by SEQ ID NO: 73, a polypeptide having an amino acid sequence represented by SEQ ID NO: 74, a polypeptide having an amino acid sequence represented by SEQ ID NO: 91, a polypeptide having an amino acid sequence represented by SEQ ID NO: 92, a polypeptide having an amino acid sequence represented by SEQ ID NO: 95, a polypeptide having an amino acid sequence represented by SEQ ID NO: 96, a polypeptide having an amino acid sequence represented by SEQ ID NO: 97, a polypeptide having an amino acid sequence represented by SEQ ID NO: 98, a polypeptide having an amino acid sequence represented by SEQ ID NO: 99, a polypeptide having an amino acid sequence represented by SEQ ID NO: 100, a polypeptide having an amino acid sequence represented by SEQ ID NO: 101, a polypeptide having an amino acid sequence represented by SEQ ID NO: 102, a polypeptide having an amino acid sequence represented by SEQ ID NO: 103, a polypeptide having an amino acid sequence represented by SEQ ID NO: 104, a polypeptide having an amino acid sequence represented by SEQ ID NO: 105, a polypeptide having an amino acid sequence represented by SEQ ID NO: 106, a polypeptide having an amino acid sequence represented by SEQ ID NO: 107, a polypeptide having an amino acid sequence represented by SEQ ID NO: 108, a polypeptide having an amino acid sequence represented by SEQ ID NO: 109, a polypeptide having an amino acid sequence represented by SEQ ID NO: 110, a polypeptide having an amino acid sequence represented by SEQ ID NO: 111, a polypeptide having an amino acid sequence represented by SEQ ID NO: 112, or a polypeptide having an amino acid sequence represented by SEQ ID NO: 113. The polypeptide may further have an ability of specific binding to human GPR8 and/or GPR7, preferably have an ability of specific binding to human GPR8 and GPR7.

Further, the polypeptide used in the present invention encompasses a precursor polypeptide of the polypeptide having the binding activity to the protein of the present invention or the cell stimulating activity on the protein-expressing cells (e.g., the activities that accelerate arachidonic acid release, acetylcholine release, intracellular $Ca^{2+}$ release, enhancement and/or inhibition of intracellular cAMP production, intracellular cGMP production, inositol phosphate production, change in cell membrane potential, phosphorylation of intracellular proteins, activation of c-fos, pH reduction, GTPγS binding activity, etc.) in the addition of the binding activity and the cell stimulating activity.

More specifically, the amino acid sequence which has substantially the same amino acid sequence as that represented by SEQ ID NO: 23 includes an amino acid sequence having at least about 80% homology, preferably at least about 90% homology, more preferably at least about 95% homology, to the amino acid sequence represented by SEQ ID NO: 23.

In particular, the amino acid sequence which has substantially the same amino acid sequence as that represented by SEQ ID NO: 23 includes, except for the above-mentioned amino acid sequences, (i) amino acid sequences represented by SEQ ID NO: 23, wherein at least 1 or 5 amino acids (preferably 1 to 3 amino acids, further preferably approximately 1 to 2 amino acids, more preferably 1 amino acid) are deleted; (ii) amino acid sequences represented by SEQ ID NO: 23, to which at least 1 or 5 amino acids (preferably 1 to 3 amino acids, further preferably approximately 1 to 2 amino acids, more preferably 1 amino acid) are added; (iii) amino acid sequences represented by SEQ ID NO: 23, to which at least 1 or 5 amino acids (preferably 1 to 3 amino acids, further preferably approximately 1 to 2 amino acids, more preferably 1 amino acid) are inserted; (iv) amino acid sequences represented by SEQ ID NO: 23, in which at least 1 or 2 amino acids (preferably approximately 1 to 30 amino acids, more preferably approximately 1 to 10 amino acids, and most preferably several (1 to 5) amino acids) are substituted by other amino acids; or (v) combination of the amino acid sequences described in the above (i) through (iv).

Specific examples of the protein which contains substantially the same amino acid sequence as that shown by SEQ ID NO: 23 include amino acid sequences represented by SEQ ID NO: 42, SEQ ID NO: 55, SEQ ID NO: 72, or SEQ ID NO: 90.

Specific examples of the precursor polypeptide described above include a polypeptide having an amino acid sequence represented by SEQ ID NO: 23, a polypeptide having an amino acid sequence represented by SEQ ID NO: 42, a polypeptide having an amino acid sequence represented by SEQ ID NO: 55, a polypeptide having an amino acid sequence represented by SEQ ID NO: 72, and a polypeptide having an amino acid sequence represented by SEQ ID NO: 90.

When the polypeptide used in the present invention has carboxyl group (or carboxylate) apart from the C-terminus, a polypeptide which carboxyl group is amidated or esterified is also included in the polypeptide used in the present invention. As the ester, for example, an ester of the C-terminus described above is used.

For salts of the polypeptide used in the present invention, preferred are salts with physiologically acceptable acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide used in the present invention has, for example, an appetitive action (enhancement of feeding) and/or influence of enhancing a prolactin production.

The receptor protein of the present invention or salts thereof may be manufactured by a publicly known method used to purify a receptor protein from human and other mammalian cells or tissues described above, or by culturing a transformant that contains the DNA encoding the receptor protein of the present invention, as will be later described. Furthermore, the receptor protein or its salts may also be manufactured by the methods for synthesizing proteins or by modifications thereof, which will also be described hereinafter.

Where the receptor protein or its salts are manufactured from human or other mammalian tissues or cells, human or other mammalian tissues or cells are homogenized, then extracted with an acid or the like, and the extract obtained is isolated and purified by a combination of chromatography techniques such as reverse phase chromatography, ion exchange chromatography, and the like.

To synthesize the receptor protein of the present invention, its partial peptide, or salts or amides thereof according to the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the receptor protein is cut out from the resin and at the same time, the protecting groups are removed. Then, intramolecular disulfide bond-forming reaction is performed in a highly diluted solution to obtain the objective protein or peptide, or amides thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be acomplished by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts in the protein of the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part. Where the product contains protecting groups, these protecting groups are removed to give the desired peptide. Publicly known methods for condensation and elimination of the protecting groups are described in (i) - (v) below.

(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: *Peptide Gosei-no-Kiso to Jikken* (Basics and experiments of peptide synthesis), published by Maruzen Co. (1975)
(iv) Haruaki Yajima & Shunpei Sakakibara: *Seikagaku Jikken* Koza (Biochemical Experiment) 1, *Tanpakushitsu no Kagaku* (Chemistry of Proteins) IV, 205 (1977)
(v) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The polypeptide used in the present invention can also be manufactured according to the above method.

The polynucleotide encoding the receptor protein of the present invention may be any polynucleotide so long as it contains the base sequence (DNA or RNA, preferably DNA) encoding the receptor protein of the present invention described above. Such a polynucleotide may also be any one of DNA encoding the receptor protein of the present invention, RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the receptor protein of the present invention, mRNA of the receptor protein of the present invention can be quantified by, for example, the publicly known method published in separate volume of *Jikken Igaku* 15 (7) "New PCR and its application" (1997), or by its modifications.

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be (1) DNA having the base sequence shown by SEQ ID NO: 2, or DNA having the base sequence hybridizable to the base sequence represented by SEQ ID NO: 2 under highly stringent conditions and encoding a receptor protein having the activities substantially equivalent to those of the receptor protein of the present invention (e.g., a ligand binding activity, a signal transduction activity, etc.), or (2) DNA having the base sequence shown by SEQ ID NO: 139, or DNA having the base sequence hybridizable to the base sequence represented by SEQ ID NO: 139 under highly stringent conditions and encoding a receptor protein having the activities substantially equivalent to those of the receptor protein of the present invention (e.g., a ligand binding activity, a signal transduction activity, etc.).

Specific examples of the DNA hybridizable to the base sequence represented by SEQ ID NO: 2 or SEQ ID NO: 139 under highly stringent conditions include DNA containing a base sequence having at least about 85% homology, preferably at least about 90% homology, and more preferably at least about 95% homology, to the base sequence represented by SEQ ID NO: 2 or SEQ ID NO: 139.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, for the DNA encoding the receptor protein having the amino acid sequence represented by SEQ ID NO: 1, there may be employed DNA having the base sequence represented by SEQ ID NO: 2. For the DNA encoding the receptor protein having the amino acid sequence represented by SEQ ID NO: 138, there may be employed DNA having the base sequence represented by SEQ ID NO: 139.

The polynucleotide comprising a part of the base sequence of the DNA encoding the receptor protein of the present invention or a part of the base sequence complementary to the DNA is used to mean to embrace not only the DNA encoding the partial peptide of the present invention described below but also RNA.

According to the present invention, antisense polynucleotides (nucleic acids) that can inhibit the replication or expression of G protein-coupled receptor protein genes can be designed and synthesized based on the base sequence information of the cloned or determined DNA encoding the G protein-coupled receptor protein. Such a polynucleotide (nucleic acid) is capable of hybridizing to RNA of G protein-coupled receptor protein gene to inhibit the synthesis or function of said RNA or capable of modulating or controlling the expression of a G protein-coupled receptor protein gene via interaction with G protein-coupled receptor protein-associated RNA. Polynucleotides complementary to the selected sequences of RNA associated with G protein-coupled receptor protein and polynucleotides specifically hybridizable to the G protein-coupled receptor protein-associated RNA are useful in modulating or controlling the expression of a G protein-coupled receptor protein gene in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the G protein-coupled receptor protein genes, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the G protein-coupled receptor protein genes.

The relationship between the targeted nucleic acids and the polynucleotides complementary to at least a part of the target, that is, the relationship between the target and the polynucleotides hybridizable to the target, can be denoted to be "antisense". Examples of the antisense polynucleotides include polydeoxynucleotides containing 2-deoxy-D-ribose, polynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by RT-PCR using mRNA fraction prepared from the cells and tissues described above.

Specifically, the DNA encoding the partial peptide of the present invention may be any one of, for example, (1) DNA containing a partial base sequence of the DNA containing the base sequence represented by SEQ ID NO: 2 or SEQ ID NO: 139, or (2) any DNA containing a partial base sequence of the DNA having a DNA hybridizable to the DNA containing a base sequence represented by SEQ ID NO: 2 or SEQ ID NO: 139 under highly stringent conditions and encoding a protein which has the activities (e.g., a ligand-biding activity, a signal transduction activity, etc.) substantially equivalent to those of the protein peptide of the present invention.

Specific examples of the DNA that is hybridizable to the DNA containing the base sequence represented by SEQ ID NO: 2 or SEQ ID NO: 139 under highly stringent conditions include DNA containing a base sequence having at least about 85% homology, preferably at least about 90% homology, and more preferably at least about 95% homology, to the base sequence represented by SEQ ID NO: 2.

More specifically, DNA encoding the partial peptide of the present invention includes (i) base sequence encoding a partial amino acid sequence consisting of the 1st (Met) through the 85th (Asp) amino acid residue from N-terminus in the amino acid sequence represented by SEQ ID NO: 1 (e.g., the base sequence consisting of the 1st through the 255th base from the 5'-terminus in the base sequence represented by SEQ ID NO: 2), or its portion, or (ii) base sequence encoding a partial amino acid sequence consisting of the 222nd (Cys) through the 329th (Ala) amino acid residue from N-terminus (e.g., the base sequence consisting of the 664th through the 987th base from the 5'-terminus in the base sequence represented by SEQ ID NO: 2), or DNA encoding a peptide containing its portion.

The DNA encoding the polypeptide used in the present invention may be, for example, (i) DNA containing the base sequence represented by SEQ ID NO: 126, SEQ ID NO: 127, SEQ ID NO: 26, SEQ ID NO: 27, SEQ ID NO: 28, SEQ ID NO: 29, SEQ ID NO: 30, SEQ ID NO: 31, SEQ ID NO: 58, SEQ ID NO: 59, SEQ ID NO: 75, SEQ ID NO: 76, SEQ ID NO: 93, SEQ ID NO: 94, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, or SEQ ID NO: 125; (ii) DNA containing a base sequence which hybridizes under high stringent conditions to the base sequence represented by SEQ ID NO: 126, SEQ ID NO: 127, SEQ ID NO: 26, SEQ ID NO: 27, SEQ ID NO: 28, SEQ ID NO: 29, SEQ ID NO: 30, SEQ ID NO: 31, SEQ ID NO: 58, SEQ ID NO: 59, SEQ ID NO: 75, SEQ ID NO: 76, SEQ ID NO: 93, SEQ ID NO: 94, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, or SEQ ID NO: 125, and encoding a polypeptide which have an activity substantially equivalent to that of the polypeptide used in the present invention; (iii) DNA containing the base sequence represented by SEQ ID NO: 22, SEQ ID NO: 41, SEQ ID NO: 54, SEQ ID NO: 71 or SEQ ID NO: 89; or (iv) DNA containing a base sequence which hybridizes under high stringent conditions to the base sequence represented by SEQ ID NO: 22, SEQ ID NO: 41, SEQ ID NO: 54, SEQ ID NO: 71 or SEQ ID NO: 89.

For the DNA containing a base sequence which hybridizes under high stringent conditions to the base sequence represented by SEQ ID NO: 126, SEQ ID NO: 127, SEQ ID NO: 26, SEQ ID NO: 27, SEQ ID NO: 28, SEQ ID NO: 29, SEQ ID NO: 30, SEQ ID NO: 31, SEQ ID NO: 58, SEQ ID NO: 59, SEQ ID NO: 75, SEQ ID NO: 76, SEQ ID NO: 93, SEQ ID NO: 94, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, or SEQ ID NO: 125, or SEQ ID NO: 22, SEQ ID NO: 41, SEQ ID NO: 54, SEQ ID NO: 71 or SEQ ID NO: 89, for example, DNA containing a base sequence which have at least about 70% homology, preferably at least about 80% homology, more preferably at least about 90% homology, and further preferably at least about 95% homology, to the base sequence represented by SEQ ID NO: 126, SEQ ID NO: 127, SEQ ID NO: 26, SEQ ID NO: 27, SEQ ID NO: 28, SEQ ID NO: 29, SEQ ID NO: 30, SEQ ID NO: 31, SEQ ID NO: 58, SEQ ID NO: 59, SEQ ID NO: 75, SEQ ID NO: 76, SEQ ID NO: 93, SEQ ID NO: 94, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, or SEQ ID NO: 125, or SEQ ID NO: 22, SEQ ID NO: 41, SEQ ID NO: 54, SEQ ID NO: 71 or SEQ ID NO: 89.

Hybridization can be carried out according to the publicly known methods or its compliant procedures, for example, the methods described in Molecular Cloning 2nd (J. Sambrook et al., Cold Spring Harbor Lab. Press, 1989). In addition, where libraries commercially available are used, it can be performed in accordance with the method described in the attached instructions. More preferably, it can be performed under high stringent conditions.

The high stringent conditions mean, for example, the conditions including about 19 to 40 mM, preferably about 19 to 20 mM as to sodium, and about 50°C to 70°C, preferably about 60°C to 65°C as to temperature. In particular, the most preferred are about 19 mM of sodium concentration and about 65°C of temperature.

More specifically, DNA containing the following sequence can be used:

(i) For DNA encoding a polypeptide which contains the amino acid sequence represented by SEQ ID NO: 8, DNA containing the base sequence represented by SEQ ID NO: 126;
(ii) For DNA encoding a polypeptide which contains the amino acid sequence represented by SEQ ID NO: 9, DNA containing the base sequence represented by SEQ ID NO: 127;
(iii) For DNA encoding a polypeptide which contains the amino acid sequence represented by SEQ ID NO: 128, DNA containing the base sequence represented by SEQ ID NO: 26;
(iv) For DNA encoding a polypeptide which contains the amino acid sequence represented by SEQ ID NO: 129, DNA containing the base sequence represented by SEQ ID NO: 27;
(v) For DNA encoding a polypeptide which contains the amino acid sequence represented by SEQ ID NO: 130, DNA containing the base sequence represented by SEQ ID NO: 28;
(vi) For DNA encoding a polypeptide which contains the amino acid sequence represented by SEQ ID NO: 131, DNA containing the base sequence represented by SEQ ID NO: 29;
(vii) For DNA encoding a polypeptide which contains the amino acid sequence represented by SEQ ID NO: 24, DNA containing the base sequence represented by SEQ ID NO: 30;
(viii) For DNA encoding a polypeptide which contains the amino acid sequence represented by SEQ ID NO: 25, DNA containing the base sequence represented by SEQ ID NO: 31;
(ix) For DNA encoding a polypeptide which contains the amino acid sequence represented by SEQ ID NO: 56, DNA containing the base sequence represented by SEQ ID NO: 58;
(x) For DNA encoding a polypeptide which contains the amino acid sequence represented by SEQ ID NO: 57, DNA containing the base sequence represented by SEQ ID NO: 59;
(xi) For DNA encoding a polypeptide which contains the amino acid sequence represented by SEQ ID NO: 73, DNA containing the base sequence represented by SEQ ID NO: 75;
(xii) For DNA encoding a polypeptide which contains the amino acid sequence represented by SEQ ID NO: 74, DNA containing the base sequence represented by SEQ ID NO: 76;
(xiii) For DNA encoding a polypeptide which contains the amino acid sequence represented by SEQ ID NO: 91, DNA containing the base sequence represented by SEQ ID NO: 93;
(xiv) For DNA encoding a polypeptide which contains the amino acid sequence represented by SEQ ID NO: 92, DNA containing the base sequence represented by SEQ ID NO: 94;
(xv) For DNA encoding a polypeptide which contains the amino acid sequence represented by SEQ ID NO: 95, DNA containing the base sequence represented by SEQ ID NO: 126;
(xvi) For DNA encoding a polypeptide which contains the amino acid sequence represented by SEQ ID NO: 96, DNA containing the base sequence represented by SEQ ID NO: 114;
(xvii) For DNA encoding a polypeptide which contains the amino acid sequence represented by SEQ ID NO: 97, DNA containing the base sequence represented by SEQ ID NO: 115;
(xviii) For DNA encoding a polypeptide which contains the amino acid sequence represented by SEQ ID NO: 98, DNA containing the base sequence represented by SEQ ID NO: 116;
(xix) For DNA encoding a polypeptide which contains the amino acid sequence represented by SEQ ID NO: 99, DNA containing the base sequence represented by SEQ ID NO: 117;
(xx) For DNA encoding a polypeptide which contains the amino acid sequence represented by SEQ ID NO: 100, DNA containing the base sequence represented by SEQ ID NO: 118;
(xxi) For DNA encoding a polypeptide which contains the amino acid sequence represented by SEQ ID NO: 101, DNA containing the base sequence represented by SEQ ID NO: 119;
(xxii) For DNA encoding a polypeptide which contains the amino acid sequence represented by SEQ ID NO: 102, DNA containing the base sequence represented by SEQ ID NO: 120;
(xxiii) For DNA encoding a polypeptide which contains the amino acid sequence represented by SEQ ID NO: 103, DNA containing the base sequence represented by SEQ ID NO: 58;
(xxiv) For DNA encoding a polypeptide which contains the amino acid sequence represented by SEQ ID NO: 104, DNA containing the base sequence represented by SEQ ID NO: 75;
(xxv) For DNA encoding a polypeptide which contains the amino acid sequence represented by SEQ ID NO: 105, DNA containing the base sequence represented by SEQ ID NO: 126;
(xxvi) For DNA encoding a polypeptide which contains the amino acid sequence represented by SEQ ID NO: 106, DNA containing the base sequence represented by SEQ ID NO: 126;
(xxvii) For DNA encoding a polypeptide which contains the amino acid sequence represented by SEQ ID NO: 107, DNA containing the base sequence represented by SEQ ID NO: 121;
(xxviii) For DNA encoding a polypeptide which contains the amino acid sequence represented by SEQ ID NO: 108, DNA containing the base sequence represented by SEQ ID NO: 122;
(xxix) For DNA encoding a polypeptide which contains the amino acid sequence represented by SEQ ID NO: 109, DNA containing the base sequence represented by SEQ ID NO: 123;
(xxx) For DNA encoding a polypeptide which contains the amino acid sequence represented by SEQ ID NO: 110, DNA containing the base sequence represented by SEQ ID NO: 124;
(xxxi) For DNA encoding a polypeptide which contains the amino acid sequence represented by SEQ ID NO: 14, DNA containing the base sequence represented by SEQ ID NO: 125;
(xxxii) For DNA encoding a polypeptide which contains the amino acid sequence represented by SEQ ID NO: 111, DNA containing the base sequence represented by SEQ ID NO: 121;
(xxxiii) For DNA encoding a polypeptide which contains the amino acid sequence represented by SEQ ID NO: 112, DNA containing the base sequence represented by SEQ ID NO: 126;
(xxxiv) For DNA encoding a polypeptide which contains the amino acid sequence represented by SEQ ID NO: 113, DNA containing the base sequence represented by SEQ ID NO: 121.

The above-mentioned DNA encoding receptor protein or polypeptide may be labeled by a publicly known method, and specifically includes isotope-labeled DNA, fluorescence-labeled DNA (e.g., fluorescence labeling with fluorescein), biotinylated DNA or enzyme-labeled DNA.

For cloning of the DNA that completely encodes the receptor protein of the present invention or its partial peptide (hereinafter sometimes collectively referred to as the receptor protein of the present invention), the DNA may be either amplified by PCR using synthetic DNA primers containing a part of the base sequence of DNA encoding the peptide of the present invention, or the DNA inserted into an appropriate vector can be selected by hybridization with a labeled DNA fragment or synthetic DNA that encodes a part or entire region of the receptor protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the ODA-LA PCR method, the Gupped duplex method or the Kunkel method or its modification by using a publicly known kit available as Mutan™-G or Mutan™-K (both manufactured by Takara Shuzo Co., Ltd.).

The cloned DNA encoding the receptor protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the receptor protein of the present invention can be manufactured, for example, by (a) excising the desired DNA fragment from the DNA containing the DNA encoding the receptor protein of the present invention (e.g., cDNA), and then (b) ligating the DNA fragment with an appropriate expression vector downstream a promoter in the vector.

Examples of the vector include plasmids derived form E. coli (e.g., pCR4, pCR2.1, pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, $\lambda P_L$ promoter, Ipp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker in CHO ($dhfr^-$) cells, selection can also be made on thymidine free media.

If necessary and desired, a signal sequence that matches with a host is added to the N-terminus of the receptor protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the receptor protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), DH5α (Inoue, H., Nojima, H., Gene, 96, 23-28 (1990)), DH10B (Proc. Natl. Acad. Sci. USA, 87, 4645-4649 (1990)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO($dhfr^-$) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the G protein-coupled receptor protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the G protein-coupled receptor protein of the present invention can be produced into the cell, in the cell membrane or out of the cell of the transformant.

The receptor protein of the present invention can be separated and purified from the culture described above by the following procedures.

When the receptor protein of the present invention is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the receptor protein of the present invention can be obtained. The buffer used for the procedures may contain a protein modifier such as urea or guanidine hydrochloride, or a surfactant such as Triton X-100™, etc. When the receptor protein is secreted in the culture, after completion of the cultivation the supernatant can be separated from the transformants or cells to collect the supernatant by a publicly known method.

The receptor protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the receptor protein thus obtained is in a free form, it can be converted into the salt by publicly known methods or modifications thereof. On the other hand, when the receptor protein is obtained in the form of a salt, it can be converted into the free form or in the form of a different salt by publicly known methods or modifications thereof.

The receptor protein produced by the recombinant can be treated, prior to or after the purification, with an appropriate protein modifying enzyme so that the receptor protein can be appropriately modified to partially remove a polypeptide. Examples of the protein-modifying enzyme include trypsin, chymotrypsin, arginyl endopeptidase, protein kinase, glycosidase or the like.

The activity of the thus produced receptor protein of the present invention or salts thereof can be determined by a test binding to a labeled ligand, by an enzyme immunoassay using a specific antibody, or the like.

Antibodies to the receptor protein of the present invention, its partial peptides, or salts thereof may be any of polyclonal antibodies and monoclonal antibodies, as long as they are capable of recognizing the receptor protein of the present invention, its partial peptides, or salts thereof.

The antibodies to the receptor protein of the present invention, its partial peptides, or salts thereof (hereinafter sometimes merely referred to as the receptor protein of the present invention) may be manufactured by publicly known methods for manufacturing antibodies or antisera, using as antigens the receptor protein of the present invention.

[Preparation of monoclonal antibody]

(a) Preparation of monoclonal antibody-producing cells

The receptor protein of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the receptor protein etc. as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein labeled with a radioactive substance or an enzyme and detecting the monoclonal antibody bound to the solid phase.

The monoclonal antibody can be selected by publicly known methods or by modifications of these methods.

In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (antigen such as the protein of the present invention) and a carrier protein is prepared, and a mammal is immunized with the complex in a manner similar to the method described above for the manufacture of monoclonal antibodies. The product containing the antibody to the receptor protein of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

The receptor protein of the present invention, its salts, its partial peptides, or salts thereof, and the DNA encoding the receptor protein or the partial peptide can be used for: (1) determination of ligands (agonists) to the G protein-coupled receptor protein of the present invention, (2) prophylactic and/or therapeutic agents for diseases associated with dysfunction of the G protein-coupled receptor protein of the present invention, (3) agents for gene diagnosis, (4) methods of screening compounds that alter the expression level of the receptor protein of the present invention or its partial peptides, (5) prophylactic and/or therapeutic agents for various diseases comprising a compound that alters the expression level of the receptor protein of the present invention or its partial peptides, (6) methods of quantification of ligands to the G protein-coupled receptor protein of the present invention, (7) methods of screening compounds (agonists, antagonists, etc.) that alter the binding property between the G protein-coupled receptor protein of the present invention and ligands, (8) prophylactic and/or therapeutic agents for various diseases comprising a compound (an agonist or an antagonist) that alters the binding property between the G protein-coupled receptor protein of the present invention and ligands, (9) quantification of the receptor protein of the present invention, its partial peptides or salts thereof, (10) methods of screening compounds that alter the amount of the receptor protein of the present invention or its partial peptides in cell membranes, (11) prophylactic and/or therapeutic agents for various diseases comprising a compound that alters the amount of the receptor protein of the present invention or its partial peptides in cell membranes, (12) neutralization by antibodies to the receptor protein of the present invention, its partial peptides, or salts thereof, (13) preparation of non-human animals that possess the DNA of the present invention and (14) a non-human mammalian embryonic stem cell, wherein the DNA of the present invention is inactivated, and a non-human mammal, wherein the DNA of the present invention is barely expressed..

In particular, by the use of the receptor binding assay system using the expression system of the recombinant G protein-coupled receptor protein of the present invention, compounds (e.g., agonists, antagonists, etc.) that alter the binding property of human or other mammal-specific ligands for the G protein-coupled receptor protein can be screened, and the agonists or antagonists can be used as prophylactic and therapeutic agents for various diseases.

Hereinafter, the receptor protein of the present invention, its partial peptides, or salts thereof (hereinafter sometimes referred to as the receptor protein of the present invention), the DNA encoding the receptor protein of the present invention or its partial peptides (hereinafter sometimes referred to as the DNA of the present invention) and the antibodies to the receptor protein of the present invention (hereinafter sometimes referred to as the antibodies of the present invention) are specifically described for the use or applications.

(1) Determination of a ligand (agonist) to the G protein-coupled receptor protein of the present invention

The receptor protein of the present invention or its salts, or the partial peptide or its salts of the present invention are useful as reagents for searching and determining ligands (agonists) to the receptor protein of the present invention or its salts.

That is, the present invention provides a method for determining a ligand to the receptor protein of the present invention, which comprises bringing the receptor protein of the present invention or its salts, or the partial peptide of the present invention or its salts, in contact with a test compound.

For the test compound, other than publicly known substance (e.g., angiotensin, bombesin, canavinoid, cholecystokinin, glutamine, serotonin, melatonin, neuropeptide Y, opioid, purines, vasopressin, oxytocin, PACAP (e.g., PACAP27, PACAP38), secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, a chemokine superfamily (e.g., IL-8, GROα, GROβ, GROγ, NAP-2, ENA-78, GCP-2, PF4, IP10, Mig, CXC chemokine subfamily such as PBSF/SDF-1, etc.; CC chemokine subfamily such as MCAF/MCP-1, MCP-2, MCP-3, MCP-4, eotaxin, RANTES, MIP1-α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc., etc.), endothelin, enterogastrin, histamine, neurotensin, TRH, pancreatic polypeptide, galanin, lysophosphatidic acid (LPA) or sphingosine 1-phosphate, etc.), the above-mentioned polypeptide containing the amino acid sequence represented by SEQ ID NO: 8, the polypeptide containing the same or substantially the same amino acid sequence as that represented by SEQ ID NO: 140, SEQ ID NO: 143, SEQ ID NO: 146, SEQ ID NO: 152 or SEQ ID NO: 155, and the like can be used. Further, tissue extracts from human or mammals (e.g., mouse, rat, swine, bovine, sheep, monkey), supernatant of cell culture and the like can be used. Among them, the polypeptide used in the present invention (preferably, the polypeptide containing the amino acid sequence shown by SEQ ID NO: 8, etc.) can preferably be used. For example, the tissue extract or cell culture supernatant is added to the receptor protein of the present invention and fractionated while assaying the cell stimulating activities, etc. to finally give a single ligand.

Specific example of the same or substantially the same amino acid sequence as the amino acid sequence represented by SEQ ID NO: 140 includes the amino acid sequence represented by SEQ ID NO: 140, SEQ ID NO: 141 or SEQ ID NO: 142. Specific example of the same or substantially the same amino acid sequence as the amino acid sequence represented by SEQ ID NO: 143 includes the amino acid sequence represented by SEQ ID NO: 143, SEQ ID NO: 144 or SEQ ID NO: 145. Specific example of the same or substantially the same amino acid sequence as the amino acid sequence represented by SEQ ID NO: 146 includes the amino acid sequence represented by SEQ ID NO: 146, SEQ ID NO: 147, SEQ ID NO: 148, SEQ ID NO: 149, SEQ ID NO: 150 or SEQ ID NO: 151. Specific example of the same or substantially the same amino acid sequence as the amino acid sequence represented by SEQ ID NO: 152 includes the amino acid sequence represented by SEQ ID NO: 152, SEQ ID NO: 153 or SEQ ID NO: 154. Specific example of the same or substantially the same amino acid sequence as the amino acid sequence represented by SEQ ID NO: 155 includes the amino acid sequence represented by SEQ ID NO: 155, SEQ ID NO: 156 or SEQ ID NO: 157.

Specifically, the method for determining ligands of the present invention comprises determining compounds (e.g., peptides, proteins, non-peptide compounds, synthetic compounds, fermentation products, etc.) or salts thereof that bind to the receptor protein of the present invention to provide cell stimulating activities (e.g., the activities that accelerate or suppress arachidonic acid release, acetylcholine release, intracellular $Ca^{2+}$ release, enhancement and inhibition of intracellular cAMP production, intracellular cGMP production, inositol phosphate production, change in cell membrane potential, phosphorylation of intracellular proteins, activation of c-fos, pH reduction, etc.), using the receptor of the present invention, its partial peptides or salts thereof, or by the receptor binding assay using the constructed recombinant receptor protein expression system.

The method for determining ligands of the present invention is characterized, for example, by measurement of the amount of the test compound bound to the receptor protein or the partial peptide, or by assaying the cell-stimulating activities, etc., when the test compound is brought in contact with the receptor protein of the present invention or its partial peptides.

More specifically, the present invention provides the following features:

(i) A method for determining a ligand to the receptor protein of the present invention or its salt, which comprises bringing a labeled test compound in contact with the receptor protein of the present invention or its salt or the partial peptide of the present invention or its salt and measuring the amount of the labeled test compound bound to the receptor protein or its salt or to the partial peptide or its salt;

(ii) A method for determining ligands to the receptor protein of the present invention or its salt, which comprises bringing a labeled test compound in contact with cells or cell membrane fraction containing the receptor protein of the present invention, and measuring the amount of the labeled test compound bound to the cells or the membrane fraction;

(iii) A method for determining ligands to the receptor protein of the present invention, which comprises culturing a transformant containing the DNA encoding the receptor protein of the present invention, bringing a labeled test compound in contact with the receptor protein expressed on the cell membrane by said culturing, and measuring the amount of the labeled test compound bound to the receptor protein or its salt;

(iv) A method for determining ligands to the receptor protein of the present invention or its salt, which comprises bringing a test compound in contact with cells containing the receptor protein of the present invention and measuring the receptor protein-mediated cell stimulating activities (e.g., the activities that promote or suppress arachidonic acid release, acetylcholine release, intracellular $Ca^{2+}$ release, enhancement and inhibition of intracellular cAMP production, intracellular cGMP production, inositol phosphate production, change in cell membrane potential, phosphorylation of intracellular proteins, activation of c-fos, pH reduction, etc.); and,

(v) A method for determining ligands to the receptor protein of the present invention or its salt, which comprises culturing a transformant containing DNA encoding the receptor protein of the present invention, bringing a labeled test compound in contact with the receptor protein expressed on the cell membrane by said culturing, and measuring the receptor protein-mediated cell stimulating activities (e.g., the activities that promote or suppress arachidonic acid release, acetylcholine release, intracellular $Ca^{2+}$ release, enhancement and inhibition of intracellular cAMP production, intracellular cGMP production, inositol phosphate production, change in cell membrane potential, phosphorylation of intracellular proteins, activation of c-fos, pH reduction, etc.).

It is particularly preferred to perform the tests (i) to (iii) described above, thereby to confirm that the test compound can bind to the receptor protein of the present invention, followed by the tests (iv) and (v) described above.

Any protein exemplified to be usable as the receptor protein for determining ligands, so long as it contains the receptor protein of the present invention or the partial peptide of the present invention. However, the receptor protein that is abundantly expressed using animal cells is appropriate.

The receptor protein of the present invention can be manufactured by the method for expression described above, preferably by expressing DNA encoding the receptor protein in mammalian or insect cells. As DNA fragments encoding the desired portion of the protein, complementary DNA is generally used but not necessarily limited thereto. For example, gene fragments or synthetic DNA may also be used. For introducing a DNA fragment encoding the receptor protein of the present invention into host animal cells and efficiently expressing the same, it is preferred to insert the DNA fragment downstream a polyhedrin promoter of nuclear polyhedrosis virus (NPV), which is a baculovirus having insect hosts, an SV40-derived promoter, a retrovirus promoter, a metallothionein promoter, a human heat shock promoter, a cytomegalovirus promoter, an SR $\alpha$ promoter or the like. The amount and quality of the receptor expressed can be determined by a publicly known method. For example, this determination can be made by the method described in the literature (Nambi, P., et al., J. Biol. Chem., 267, 19555-19559 (1992)).

Accordingly, the subject containing the receptor protein of the present invention, its partial peptides or salts thereof in the method for determining the ligand according to the present invention may be the receptor protein, its partial peptides or salts thereof purified by publicly known methods, cells containing the receptor protein, or membrane fractions of such cells.

Where cells containing the receptor protein of the present invention are used in the method of the present invention for determination of ligands, the cells may be fixed using glutaraldehyde, formalin, etc. The fixation can be made by a publicly known method.

The cells containing the receptor protein of the present invention are host cells that have expressed the receptor protein of the present invention, which host cells include Escherichia coli, Bacillus subtilis, yeast, insect cells, animal cells, and the like.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cells containing the receptor protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (i) through (iii) supra for determination of a ligand to the receptor protein of the present invention or its salt, an appropriate receptor fraction and a labeled test compound are required.

The receptor protein fraction is preferably a fraction of naturally occurring receptor protein or a recombinant receptor fraction having an activity equivalent to that of the natural protein. Herein, the term "equivalent activity" is intended to mean a ligand binding activity, a signal transduction activity or the like that is equivalent to that possessed by naturally occurring receptor proteins.

Preferred examples of labeled test compounds include angiotensin, bombesin, canavinoid, cholecystokinin, glutamine, serotonin, melatonin, neuropeptide Y, opioid, purines, vasopressin, oxytocin, PACAP (e.g., PACAP27, PACAP38), secretin, glucagon, calcitonin, adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leukotrienes, pancreastatin, prostaglandins, thromboxane, adenosine, adrenaline, a chemokine superfamily (e.g., IL-8, GROα, GROβ, GROγ, NAP-2, ENA-78, GCP-2, PF4, IP10, Mig, CXC chemokine subfamily such as PBSF/SDF-1, etc.; CC chemokine subfamily such as MCAF/MCP-1, MCP-2, MCP-3, MCP-4, eotaxin, RANTES, MIP1-α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc., etc.), endothelin, enterogastrin, histamin, neurotensin, TRH, pancreatic polypeptide, galanin, lysophosphatidic acid (LPA), sphingosine 1-phosphate, polypeptides used in the present invention, etc., which are labeled with [$^3$H], [$^{125}$I], [$^{14}$C], [$^{35}$S], etc. Among them, the polypeptide containing the amino acid sequence shown by SEQ ID NO: 8 described above is preferred. Moreover, the polypeptide containing the same or substantially the same amino acid sequence as that represented by SEQ ID NO: 140, SEQ ID NO: 143, SEQ ID NO: 146, SEQ ID NO: 152 or SEQ ID NO: 155 is used.

More specifically, the ligand to the receptor protein of the present invention or its salt is determined by the following procedures. First, a standard receptor preparation is prepared by suspending cells containing the receptor protein of the present invention or the membrane fraction thereof in a buffer appropriate for use in the determination method. Any buffer can be used so long as it does not inhibit the ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween-80™ (manufactured by Kao-Atlas Inc.), digitonin or deoxycholate, and various proteins such as bovine serum albumin or gelatin, may optionally be added to the buffer. Further for the purpose of suppressing the degradation of the receptors or ligands by proteases, a protease inhibitor such as PMSF, leupeptin, E-64 (manufactured by Peptide Institute, Inc.) and pepstatin may also be added. A given amount (5,000 to 500,000 cpm) of the test compound labeled with [$^3$H], [$^{125}$I], [$^{14}$C], [$^{35}$S] or the like is added to 0.01 ml to 10 ml of the receptor solution. To determine the amount of non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. A test compound exceeding 0 cpm in count obtained by subtracting nonspecific binding (NSB) from the total binding (B) (B minus NSB) may be selected as a ligand (agonist) to the receptor protein of the present invention or its salt.

The method (iv) or (v) supra for determination of a ligand to the receptor protein of the present invention or its salt can be performed as follows. The receptor protein-mediated cell-stimulating activities (e.g., the activities that promote or suppress arachidonic acid release, acetylcholine release, intracellular $Ca^{2+}$ release, enhancement and inhibition of intracellular cAMP production, intracellular cGMP production, inositol phosphate production, change in cell membrane potential, phosphorylation of intracellular proteins, activation of c-fos, pH reduction, etc.) may be determined by a publicly known method, or using an assay kit commercially available. Specifically, cells containing the receptor protein are first cultured on a multi-well plate, etc. Prior to the ligand determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the CAMP production suppression activity, the baseline production in the cells is increased by forskolin or the like and the suppressing effect on the increased baseline production may then be detected.

The kit of the present invention for determination of the ligand that binds to the receptor protein of the present invention or its salt comprises the receptor protein of the present invention or its salt, the partial peptide of the present invention or its salt, cells containing the receptor protein of the present invention, or the membrane fraction of the cells containing the receptor protein of the present invention.

Examples of the ligand determination kit of the present invention are given below.

1. Reagents for determining ligands

(i) Buffers for assay and washing

Hanks' Balanced Salt Solution (manufactured by Gibco Co.) supplemented with 0.05% bovine serum albumin (Sigma Co.).

The solution is sterilized by filtration through a 0.45 μm filter and stored at 4°C. Alternatively, the solution may be prepared at use.

(ii) Standard G protein-coupled receptor protein

CHO cells on which the receptor protein of the present invention has been expressed are passaged in a 12-well plate in a density of $5 \times 10^5$ cells/well followed by culturing at 37°C under 5% $CO_2$ and 95% air for 2 days.

(iii) Labeled test compounds

Compounds labeled with [$^3$H], [$^{125}$I], [$^{14}$C], [$^{35}$S], etc., which are commercially available labels, or compounds labeled by appropriate methods.

An aqueous solution of the compound is stored at 4°C or -20°C. The solution is diluted to 1 μM with an assay buffer at use. A sparingly water-soluble test compound is dissolved in dimethylformamide, DMSO, methanol, etc.

(iv) Non-labeled compounds

A non-labeled form of the same compound as the labeled compound is prepared in a concentration 100 to 1,000-fold higher than that of the labeled compound.

2. Method for assay

(i) CHO cells expressing the receptor protein of the present invention are cultured in a 12-well culture plate. After washing twice with 1 ml of an assay buffer, 490 μl of the assay buffer is added to each well.
(ii) After 5 μl of the labeled test compound is added, the resulting mixture is incubated at room temperature for an hour. To determine the non-specific binding, 5 μl of the non-labeled compound is added to the system.
(iii) The reaction mixture is removed and the wells are washed 3 times with 1 ml of washing buffer. The labeled test compound bound to the cells is dissolved in 0.2N NaOH-1% SDS and then mixed with 4 ml of liquid scintillatorA (manufactured by Wako Pure Chemical Industries, Ltd.).
(iv) The radioactivity is measured using a liquid scintillation counter (manufactured by Beckman Co.).

The ligands that bind to the receptor protein of the present invention or its salt include substances specifically present in brain, large intestine, small intestine, pancreas, ovary, stomach, heart, liver, testis, placenta, lung, spinal cord, spleen, thymus, kidney, duodenum, adrenal, prostate, pituitary, uterus, etc. Examples of such ligands are angiotensin, bombesin, canavinoid, cholecystokinin, glutamine, serotonin, melatonin, neuropeptide Y, opioids, purines, vasopressin, oxytocin, PACAP (e.g., PACAP27, PACAP38), secretin, glucagon, calcitonin, adrenomedulin, somatostatin, GHRH, CRF, ACTH, GRP, PTH, VIP (vasoactive intestinal peptide), somatostatin, dopamine, motilin, amylin, bradykinin, CGRP (calcitonin gene-related peptide), leukotriens, pancreastatin, prostaglandins, thromboxane, adenosine, adrenaline, a chemokine superfamily (e.g., IL-8, GROα, GROβ, GROγ, NAP-2, ENA-78, GCP-2, PF4, IP10, Mig, CXC chemokine subfamily such as PBSF/SDF-1, etc.; CC chemokine subfamily such as MCAF/MCP-1, MCP-2, MCP-3, MCP-4, eotaxin, RANTES, MIP1-α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc., etc.), endothelin, enterogastrin, histamine, neurotensin, TRH, pancreatic polypeptide, galanin, lysophosphatidic acid (LPA) or sphingosine 1-phosphate, etc.

Specific example of ligand, which is capable of binding to the receptor protein or its salt of the present invention, includes polypeptides used in the present invention (preferably, the polypeptide containing the same or substantially the same amino acid sequence as that represented by SEQ ID NO: 8 and the like).

The polypeptide containing the same or substantially the same amino acid sequence as that represented by SEQ ID NO: 8 includes the polypeptide containing the amino acid sequence represented by SEQ ID NO: 8, the polypeptide containing the amino acid sequence represented by SEQ ID NO: 9 and the like.

The polypeptide containing the same or substantially the same amino acid sequence as that represented by SEQ ID NO: 8 possesses, for example, an appetitive function (enhancement of feeding) and/or an enhancing activity of prolactin production.

The polypeptide containing the amino acid sequence represented by SEQ ID NO: 8 can be produced from cells or tissues of the human or mammals described above by the publicly known methods for purifying proteins. Alternatively, it can be manufactured by the methods described in reference examples 12 and 13 below, or by the methods according to those.

Specific example of ligand, which is capable of binding to the receptor protein or its salt of the present invention, includes polypeptides containing the same or substantially the same amino acid sequence as that shown by SEQ ID NO: 140, SEQ ID NO: 143, SEQ ID NO: 146, SEQ ID NO: 142 or SEQ ID NO: 145 described above. The polypeptides can be manufactured from the above-mentioned cells or tissues of the human or mammals by the publicly known methods for purifying proteins.

(2) Prophylactic and/or therapeutic agents for diseases associated with dysfunction of the G protein-coupled receptor protein of the present invention

When a compound is clarified to be a ligand of the receptor protein of the present invention by the methods described in (1), (i) the receptor protein of the present invention, or (ii) the DNA encoding the receptor protein can be used, depending on the activities possessed by the ligand, as a prophylactic and/or therapeutic agent for diseases associated with dysfunction of the receptor protein of the present invention.

For example, when the physiological activity of the ligand cannot be expected in a patient (deficiency of the receptor protein) due to a decrease in the receptor protein of the present invention, the activity of the ligand can be exhibited by: (i) administering the receptor protein of the present invention to the patient thereby to supplement the amount of the receptor protein; or (ii) by increasing the amount of the receptor protein in the patient through: i) administration of the DNA encoding the receptor protein of the present invention to express the same in the patient; or ii) insertion and expression of the DNA encoding the receptor protein of the present invention in the objective cells to transplant the cells to the patient, whereby the activity of the ligand can be sufficiently exhibited. That is, the DNA encoding the receptor protein of the present invention is useful as a safe and low toxic prophylactic and/or therapeutic agent for diseases associated with dysfunction of the receptor protein of the present invention.

The receptor protein having the amino acid sequence shown by SEQ ID NO: 1, one of the receptor proteins of the present invention, is a novel 7 transmembrane receptor protein that is recognized to have 84.8% and 58.1% homology on an amino acid sequence level to human GPR7 [Genomics, Vol. 28, pp. 84-91, 1995] and human GPR8 [Genomics, Vol. 28, pp. 84-91, 1995], respectively, which are a G protein-coupled receptor protein. Also, the receptor protein having the amino acid sequence shown by SEQ ID NO: 138, is a novel 7 transmembrane receptor protein that is recognized to have 85.1% homology on an amino acid sequence level to human GPR7 [Genomics, Vol. 28, pp. 84-91, 1995], which are a G protein-coupled receptor protein.

The receptor protein or the DNA encoding the receptor protein of the present invention is useful for the prevention and/or treatment of central nerve system dysfunction (e.g., Alzheimer's disease, dementia, eating disorder), endocrine disorders [e.g., hypertension, hypogonadism, thyroid insufficiency, dyspituitarism, hyposecretion of pituitary hormone (e.g., hyposecretion of prolactin (e.g., ovarian dysfunction, ateliosis of seminal vesicle, menopausal disorder, hypothroidism)), etc.], metabolic disorders (e.g., diabetes, metabolic disorder of lipid, hyperlipemia), cancers (e.g., non-small-cell lung cancer, ovarian cancer, prostate cancer, stomach cancer, bladder carcinoma, breast cancer, cancer of uterine cervix, colon cancer, rectum cancer), heart diseases (e.g., angina, heart infarction), and the like. Further, it is also useful for the prevention and/or treatment of anorexia, improvement in appetite (enhancement of feeding), the prevention and/or treatment of adiposis (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity), hyperphagia and the like.

When the receptor protein of the present invention is used as the prophylactic/therapeutic agents supra, the receptor protein can be prepared into a pharmaceutical composition in a conventional manner.

On the other hand, where the DNA encoding the receptor protein of the present invention (hereinafter sometimes referred to as the DNA of the present invention) is used as the prophylactic/therapeutic agents described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, (i) the receptor protein of the present invention or (ii) the DNA encoding the receptor protein can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing (i) the receptor protein of the present invention or (ii) the DNA encoding the receptor protein with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human and other mammals (e.g., rats, mice, rabbits, sheep, swine, bovine, cats, dogs, monkeys, etc.).

The dose of the receptor protein of the present invention varies depending on subject to be administered, organs to be administered, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(3) Gene diagnostic agent

By using the DNA of the present invention as a probe, an abnormality (gene abnormality) of the DNA or mRNA encoding the receptor protein of the present invention or its partial peptide in human and other mammals (e.g., rats, mice, rabbits, sheep, swine, bovine, cats, dogs, monkeys, etc.) can be detected. Therefore, the DNA of the present invention is useful as a gene diagnostic agent for the damage against the DNA or mRNA, its mutation, or its decreased expression, or increased expression or overexpression of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

(4) Methods of screening compounds that alter the expression level of the receptor protein of the present invention or its partial peptide

By using the DNA of the present invention as a probe, the DNA can be used for screening of compounds that alter the amount of the receptor protein of the present invention or its partial peptide.

That is, the present invention provides methods of screening compounds that alter the amount of the receptor protein or its partial peptide, which comprises measuring the amount of mRNA in the receptor protein of the present invention or its partial peptide contained in, for example, (i) (a) blood, (b) specific organs, (c) tissues or cells isolated from the organs of non-human mammals, or in (ii) transformants, etc.

The amount of mRNA in the receptor protein of the present invention or its partial peptide can be specifically measured as follows.

(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., anti-dementia agents, hypotensive agents, anticancer agents, antiobestic agents, etc.) or physical stress (e.g., soaking stress, electric shock, light and darkness, low temperature, etc.), and the blood, specific organs (e.g., brain, lung, large intestine, etc.), or tissues or cells isolated from the organs are obtained after a specified period of time.

The mRNA of the receptor protein of the present invention or its partial peptide contained in the thus obtained cells is extracted from the cells, for example, in a conventional manner and quantified using, e.g., TaqManPCR, or may also be analyzed by northern blot technique by publicly known methods.

(ii) Transformants that express the receptor protein of the present invention or its partial peptide are prepared according to the methods described above, and the mRNA of the receptor protein of the present invention or its partial peptide can be quantified and analyzed, as described above.

Compounds that alter the expression level of the receptor protein of the present invention or its partial peptide can be screened by the following procedures.

(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA in the receptor protein of the present invention or its partial peptide contained in cells are quantified and analyzed.

(ii) Transformants are cultured in a conventional manner and a test compound is mixed in the culture medium. After a specified time (after 1 day to 7 days, preferably after 1 day to 3 days, more preferably after 2 to 3 days), the amount of mRNA in the receptor protein of the present invention or its partial peptide contained in the transformants can be quantified and analyzed.

The compounds or their salts, which are obtainable by the screening methods of the present invention, are compounds that alter the expression level of the receptor protein of the present invention or its partial peptide. Specifically, (a) compounds that potentiate the cell stimulating activities mediated by the G protein-coupled receptor (e.g., activities that promote or suppress arachidonic acid release, acetylcholine release, intracellular $Ca^{2+}$ release, enhancement and inhibition of intracellular cAMP production, intracellular cGMP production, inositol phosphate production, alters in cell membrane potential, phosphorylation of intracellular proteins, activation of c-fos, pH reduction, etc.) by increasing the expression level of the receptor protein of the present invention or its partial peptide ; and (b) compounds that decrease the cell-stimulating activities by reducing the expression level of the receptor protein of the present invention or its partial peptide.

The compounds include peptides, proteins, non-peptide compounds, synthetic compounds, and fermentation products. They may be novel or known compounds.
The compounds that increase the cell-stimulating activities are useful as safe and low toxic pharmaceuticals for potentiation of the physiological activity of the receptor protein of the present.
The compounds that decrease the cell-stimulating activities are useful as safe and low toxic pharmaceuticals for reducing the physiological activity of the receptor protein or its other forms of the present invention.
When the compounds or their salt forms, which are obtainable by the screening methods of the present invention, are used as pharmaceutical components, the compounds can be formulated by the conventional methods. For example, as described for the pharmaceuticals containing the receptor protein of the present invention, the compounds can be prepared into tablets, capsules, elixir, microcapsules, aseptic solution, or suspension.
The preparations obtained as described above are safe and low toxic, and can be administered to human and other mammals (e.g., rats, mice, rabbits, sheep, swine, bovine, cats, dogs, monkeys, etc.).
The dose of the compounds or their salt forms varies depending on subject to be administered, target organs, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(5) Prophylactic and/or therapeutic agents for various diseases comprising the compounds that alter the expression level of the receptor protein of the present invention or its partial peptide

As described above, the receptor protein of the present invention is considered to play some important role such as a role in various tissues (e.g., brain, large intestine, small intestine, pancreas, ovary, stomach, heart, liver, testis, placenta, lung, spinal cord, spleen, thymus, kidney, duodenum, adrenal, prostate, pituitary, uterus, etc.). Thus, the compounds that alter the expression level of the receptor protein of the present invention or its partial peptide can be used as prophylactic and/or therapeutic agents for diseases associated with dysfunction of the receptor protein of the present invention.
Where these compounds are used as prophylactic and/or therapeutic agents for diseases associated with dysfunction of the receptor protein of the present invention, the preparations can be obtained by the conventional methods.
For example, the compounds can be administered orally as a sugar coated tablet, capsule, elixir, and microcapsule, or non-orally as injection such as aseptic solution or suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.
For the additive that may be mixed in tablets and capsules, for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation procedure such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.
The prophylactic/therapeutic agents described above may be combined with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants, and the like. The preparation for injection is usually filled in appropriate ampoules.
The preparations obtained as described above are safe and low toxic, and can be administered to, for example, human and other mammals (e.g., rats, mice, rabbits, sheep, swine, bovine, cats, dogs, monkeys, etc.).
The dose of the compounds or their salt forms varies depending on subject to be administered, target organs, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(6) Methods of quantifying ligands for the G protein-coupled protein of the present invention

Since the receptor protein etc. of the present invention has binding affinity to ligands, the ligand concentration can be quantified in vivo with good sensitivity.
The quantification methods of the present invention can be used in combination with, for example, a competitive method. The ligand concentration in a test sample can be measured by contacting the test sample to the receptor protein etc. of the present invention. Specifically, the methods can be used by following, for example, the methods described in (i) and (ii) or its modified methods.

(i) Hiroshi Irie, ed. "Radioimmunoassay," Kodansha, published in 1974
(ii) Hiroshi Irie, ed. "Sequel to the Radioimmunoassay," Kodansha, published in 1979

(7) Methods of screening compounds (agonists, antagonists, or the like) that alter the binding property between the G protein-coupled receptor protein of the present invention and ligands

Using the receptor protein etc. of the present invention, or using the receptor binding assay system of the expression system constructed using the recombinant receptor protein etc., compounds (e.g., peptides, proteins, non-peptide compounds, synthetic compounds, fermentation products, etc.) or salt forms thereof that alter the binding property between ligands and the receptor protein of the present invention can be efficiently screened.
Such compounds include (a) compounds that have the G protein-coupled receptor-mediated cell-stimulating activities (e.g., activities that promote or suppress arachidonic acid release, acetylcholine release, intracellular $Ca^{2+}$ release, enhancement and inhibition of intracellular cAMP production, intracellular cGMP production, inositol phosphate production, changes in cell membrane potential, phosphorylation of intracellular proteins, activation of c-fos, pH reduction, etc.) (so-called agonists to the receptor protein of the present invention); (b) compounds that do not have the cell-stimulating activity (so-called antagonists to the receptor protein of the present invention); (c) compounds that potentiate the binding affinity between ligands and the G protein-coupled receptor protein of the present invention; and (d) compounds that reduce the binding affinity between ligands and the G protein-coupled receptor protein of the present invention (it is preferred to screen the compounds described in (a) using the ligand determination methods described above).
That is, the present invention provides methods of screening compounds or their salt forms that alter the binding property between ligands and the receptor protein, its partial peptide or salts thereof, which comprises comparing (i) the case wherein the receptor protein of the present invention, its partial peptide or salts thereof are brought in contact with a ligand, with (ii) the case wherein the receptor protein of the present invention, its partial peptide or salts thereof are brought in contact with a ligand and a test compound.
Specific example of the ligand includes the polypeptide used in the present invention (preferably, the polypeptide containing the same or substantially the same amino acid sequence as that shown by SEQ ID NO: 8 described above and the like). In addition, the polypeptide containing the same or substantially the same amino acid sequence as that represented by SEQ ID NO: 140, SEQ ID NO: 143, SEQ ID NO: 146, SEQ ID NO: 152 or SEQ ID NO: 155 may be included.
For the polypeptide containing the same or substantially the same amino acid sequence as that represented by SEQ ID NO: 8, the polypeptide having the amino acid shown by SEQ ID NO: 8, the polypeptide having the amino acid shown by SEQ ID NO: 9 and the like can be included.
The screening methods of the present invention are characterized by assaying, for example, the amount of ligand bound to the receptor protein etc., the cell-stimulating activity, etc., and comparing the property between (i) and (ii).
More specifically, the present invention provides the following screening methods:

(i) A method of screening a compound or its salt that alters the binding property between a ligand and the receptor protein etc. of the present invention, which comprises: measuring the amount of a labeled ligand bound to the receptor protein etc., when the labeled ligand is brought in contact with the receptor protein etc. of the present invention and when the labeled ligand and a test compound are brought in contact with the receptor protein etc. of the present invention, and, comparing the binding property between them;

(ii) A method of screening a compound or its salt that alters the binding property between a ligand and the receptor protein etc. of the present invention, which comprises: measuring the amount of a labeled ligand bound to cells or the membrane fraction of the cells, when the labeled ligand is brought in contact with the cells or cell membrane fraction containing the receptor protein etc. of the present invention and when the labeled ligand and a test compound are brought in contact with the cells or cell membrane fraction containing the receptor protein etc. of the present invention, and, comparing the binding property between them;

(iii) A method of screening a compound or its salt that alters the binding property between a ligand and the receptor protein etc. of the present invention, which comprises: measuring the amount of a labeled ligand to the receptor protein etc., when the labeled ligand is brought in contact with the receptor protein etc. expressed on the cell membrane induced by culturing a transformant containing the DNA of the present invention and when the labeled ligand and a test compound are brought in contact with the receptor protein etc. of the present invention expressed on the cell membrane induced by culturing a transformant containing the DNA of the present invention, and, comparing the binding property between them;

(iv) A method of screening a compound or its salt that alters the binding property between a ligand and the receptor protein etc. of the present invention, which comprises: measuring the receptor-mediated cell-stimulating activity (e.g., the activity that promotes or suppresses arachidonic acid release, acetylcholine release, intracellular $Ca^{2+}$ release, enhancement and inhibition of intracellular CAMP production, intracellular cGMP production, inositol phosphate production, changes in cell membrane potential, phosphorylation of intracellular proteins, activation of c-fos, pH reduction, etc.), when a compound (e.g., a ligand to the receptor protein etc. of the present invention) that activates the receptor protein etc. of the present invention is brought in contact with cells containing the receptor protein etc. of the present invention and when the compound that activates the receptor protein etc. of the present invention and a test compound are brought in contact with cells containing the receptor protein etc. of the present invention, and, comparing the binding property between them; and,

(v) A method of screening a compound or its salt that alters the binding property between a ligand and the receptor protein etc. of the present invention, which comprises: measuring the receptor-mediated cell-stimulating activity (e.g., the activity that promotes or suppresses arachidonic acid release, acetylcholine release, intracellular $Ca^{2+}$ release, enhancement and inhibition of intracellular cAMP production, intracellular cGMP production, inositol phosphate production, changes in cell membrane potential, phosphorylation of intracellular proteins, activation of c-fos, pH reduction, etc.), when a compound (e.g., a ligand for the receptor protein etc. of the present invention) that activates the receptor protein etc. of the present invention is brought in contact with the receptor protein etc. of the present invention expressed on the cell membrane induced by culturing a transformant containing the DNA of the present invention and when the compound that activates the receptor protein etc. of the present invention and a test compound are brought in contact with the receptor protein etc. of the present invention expressed on the cell membrane induced by culturing a transformant containing the DNA of the present invention, and, comparing the binding property between them.

Before the receptor protein etc. of the present invention was obtained, it was required for screening G protein-coupled receptor agonists or antagonists to obtain candidate compounds first, using cells or tissues containing the G protein-coupled receptor protein or the cell membrane fraction from rats or other animals (primary screening), and then examine the candidate compounds whether the compounds actually inhibit the binding between human G protein-coupled receptor protein and ligands (secondary screening). When cells, tissues, or the cell membrane fractions were directly used, it was practically difficult to screen agonists or antagonists to the objective receptor protein, since other receptor proteins were present together.

However, using, for example, the human-derived receptor protein of the present invention, the primary screening becomes unnecessary, and compounds that inhibit the binding between ligands and the G protein-coupled receptor protein can be efficiently screened. Furthermore, it is easy to assess whether the obtained compound is an agonist or antagonist.

Hereinafter, the screening methods of the present invention are described more specifically.

First, for the receptor protein etc. of the present invention used for the screening methods of the present invention, any substance may be used so long as it contains the receptor protein etc. of the present invention described above. The cell membrane fraction from mammalian organs containing the receptor protein etc. of the present invention is preferred. However, it is very difficult to obtain human organs. It is thus preferable to use rat-derived receptor proteins or the like, produced by large-scale expression using recombinants.

To manufacture the receptor protein etc. of the present invention, the methods described above are used, and it is preferred to express the DNA of the present invention in mammalian and insect cells. For the DNA fragment encoding the objective protein region, the complementary DNA (cDNA), but not necessarily limited thereto, is employed. For example, the gene fragments and synthetic DNA may also be used. To introduce a DNA fragment encoding the receptor protein of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SR $\alpha$ promoter. The amount and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening methods of the present invention, the material that contains the receptor protein etc. of the present invention may be the receptor protein etc. purified by publicly known methods, cells containing the receptor protein etc., or the cell membrane fraction containing the receptor protein or the like.

In the screening methods of the present invention, when cells containing the receptor protein etc. of the present invention are used, the cells may be fixed with glutaraldehyde, formalin, etc. The cells can be fixed by publicly known methods.

The cells containing the receptor protein etc. of the present invention are host cells that express the receptor protein or the like. For the host cells, Escherichia coli, Bacillus subtilis, yeast, insect cells, animal cells and the like are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein etc. expressed and membrane components such as cell-derived phospholipids and membrane proteins.

The amount of the receptor protein in the cells containing the receptor protein etc. and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To screen the compounds that alter the binding property between ligands and the receptor protein etc. of the present invention described in (i) to (iii), for example, an appropriate receptor protein fraction and a labeled ligand are necessary.

The receptor protein fraction is preferably a fraction of naturally occurring receptor protein or a recombinant receptor fraction having an activity equivalent to that of the natural protein. Herein, the equivalent activity is intended to mean a ligand binding activity, a signal transduction activity or the like that is equivalent to that possessed by naturally occurring receptor proteins.

For the labeled ligand, a labeled ligand and a labeled ligand analogue are used. For example, ligands labeled with [$^3$H], [$^{125}$I], [$^{14}$C], [$^{35}$S], etc. are used.

Specifically, to screen the compounds that alter the binding property between ligands and the receptor protein etc. of the present invention, first, the receptor protein standard is prepared by suspending cells or cell membrane fraction containing the receptor protein etc. of the present invention in a buffer appropriate for the screening. For the buffer, any buffer that does not interfere with the binding of ligands to the receptor protein is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween-80™ (Kao-Atlas Co.), digitonin, deoxycholate, etc. may be added to the buffer. To inhibit degradation of the receptor and ligands by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of labeled ligand is added, and $10^{-4}$ M - $10^{-10}$ M of a test compound is simultaneously added to be co-present. To examine non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or $\gamma$-counter. Regarding the count obtained by subtracting the amount of non-specific binding (NSB) from the count obtained in the absence of any competitive substance ($B_0$) as 100%, when the amount of specific binding (B-NSB) is, for example, 50% or less, the test compound can be selected as a candidate substance having a potential of competitive inhibition.

To perform the methods (iv) and (v) supra of screening the compounds that alter the binding property between ligands and the receptor protein etc. of the present invention, the receptor protein-mediated cell-stimulating activity (e.

g., activity that promotes or inhibits arachidonic acid release, acetylcholine release, intracellular Ca release, enhancement and inhibition of intracellular cAMP production, intracellular cGMP production, inositol phosphate production, changes in cell membrane potential, phosphorylation of intracellular proteins, activation of c-fos, pH reduction, etc.) can be measured using publicly known methods or commercially available kits.

Specifically, the cells containing the receptor protein etc. of the present invention are first cultured on a multiwell plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

Screening by assaying the cell-stimulating activity requires cells that have expressed an appropriate receptor protein. For the cells that have expressed the receptor protein etc. of the present invention, the cell line possessing the native receptor protein etc. of the present invention, the cell line expressing the recombinant receptor protein described above and the like are desirable.

For the test compound, for example, peptides, proteins, non-peptide compounds, synthetic compounds, fermentation products, cell extracts, plant extracts, and animal tissue extracts are used. These compounds may be novel or known compounds.

The kits for screening the compounds or their salts that alter the binding property between ligands and the receptor protein etc. of the present invention comprise the receptor protein etc. of the present invention, cells containing the receptor protein etc. of the present invention, or the membrane fraction of cells containing the receptor protein etc. of the present invention.

Examples of the screening kits of the present invention are as follow.

1. Reagents for screening

(i) Buffer for measurement and washing

Hanks' balanced salt solution (manufactured by Gibco Co.) supplemented with 0.05% bovine serum albumin (manufactured by Sigma Co.).

The solution is sterilized by filtration through a 0.45 μm filter, and stored at 4°C or may be prepared at use.

(ii) Standard G protein-coupled receptor

CHO cells expressing the receptor protein of the present invention are passaged in a 12-well plate at a density of $5 \times 10^5$ cells/well followed by culturing at 37°C under 5% $CO_2$ and 95% air for 2 days.

(iii) Labeled ligands

Aqueous solutions of ligands labeled with commercially available [$^3$H], [$^{125}$I], [$^{14}$C], [$^{35}$S], etc. are stored at 4°C or -20°C, and diluted to 1 μM with the measurement buffer.

(iv) Standard ligand solution

The ligand is dissolved in and adjusted to 1 mM with PBS containing 0.1% bovine serum albumin (manufactured by Sigma Co.) and stored at -20°C.

2. Measurement method

(i) CHO cells expressing the receptor protein of the present invention are cultured in a 12-well culture plate and washed twice with 1 ml of the measurement buffer, and 490 μl of the measurement buffer is added to each well.
(ii) After adding 5 μl of $10^{-3}$ - $10^{-10}$ M test compound solution, 5 μl of a labeled ligand is added to the mixture, and the cells are incubated at room temperature for an hour. To determine the amount of the non-specific binding, 5 μl of the non-labeled ligand is added in place of the test compound.

(iii) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)

(iv) The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB : Percent maximum binding
B : Value obtained in the presence of a test compound
NSB : Non-specific binding
$B_0$ : Maximum binding

The compounds or their salts, which are obtainable using the screening methods or the screening kits of the present invention, are the compounds that alter the binding property between ligands and the receptor protein etc. of the present invention. Specifically, these compounds are: (a) compounds that have the G protein-coupled receptor-mediated cell-stimulating activity (e.g., activity that promotes or inhibits arachidonic acid release, acetylcholine release, intracellular $Ca^{2+}$ release, enhancement and inhibition of intracellular cAMP production, intracellular cGMP production, inositol phosphate production, changes in cell membrane potential, phosphorylation of intracellular proteins, activation of c-fos, pH reduction, etc.) (so-called agonists to the receptor protein of the present invention); (b) compounds having no cell stimulating-activity (so-called antagonists to the receptor protein of the present invention); (c) compounds that increase the binding affinity between ligands and the G protein-coupled receptor protein of the present invention; and (d) compounds that reduce the binding affinity between ligands and the G protein-coupled receptor protein of the present invention.

The compounds may be peptides, proteins, non-peptide compounds, synthetic compounds, fermentation products, and may be novel or known compounds.

Since the compound having a function that alters the binding property between ligand and the receptor protein of the present invention, which is obtainable by using the screening method or the screening kit of the present invention, can modulate alters the binding property between ligand and the receptor protein of the present invention, it is useful as a safe and low toxic medicament. The medicament includes, for example, a prophylactic and/or therapeutic medicine for anorexia; an appetite (feeding) enhancer; a prophylactic and/or therapeutic medicine for hyposecretion of pituitary hormone [e.g., hyposecretion of prolactin (e.g., ovarian dysfunction, ateliosis of seminal vesicle, menopausal disorder, hypothroidism)]; a prophylactic and/or therapeutic medicine for adiposis (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity), hyperphagia and the like; an safe and low toxic prophylactic and/or therapeutic agent for pituitary adenomatoid tumor, diencephalons tumor, emmeniopathy, autoimmune disease, prolactinoma, infertile, impotence, amenia, galactorrhea, acromegaly, Chiari-Frommel syndrome, Argonz-Del Castillo syndrome, Forbes-Albright syndrome, lymphoma, Sheehan's syndrome, dysspermatogenesis, etc. (inhibitor of prolactin production). Preferably, an safe and low toxic prophylactic and/or therapeutic medicine for adiposis, hyperphagia and the like and an appetite enhancer are included.

Among them, since an agonist to the receptor protein of the present invention has a similar function to the physiological activity that the ligand for the receptor protein of the present invention has, it is useful as a safe and low toxic pharmaceutical depending on the ligand activity. For the pharmaceutical, for example, a prophylactic and/or therapeutic medicine for anorexia, an appetite (feeding) enhancer, and a prophylactic and/or therapeutic medicine for hyposecretion of pituitary hormone [e.g., hyposecretion of prolactin (e.g., ovarian dysfunction, ateliosis of seminal vesicle, menopausal disorder, hypothroidism)] can be included.

On the other hand, since an antagonist to the receptor protein of the present invention can suppress the physiological activity that the ligand for the receptor protein of the present invention has, it is useful as a safe and low toxic medicine that can suppress the ligand activity. For the medicine, for example, a safe and low toxic prophylactic and/or therapeutic medicine for adiposis (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity), hyperphagia and the like; an safe and low toxic prophylactic and/or therapeutic agent for pituitary adenomatoid tumor, diencephalons tumor, emmeniopathy, autoimmune disease, prolactinoma, infertile, impotence, amenia, galactorrhea, acromegaly, Chiari-Frommel syndrome, Argonz-Del Castillo syndrome, Forbes-Albright syndrome, lymphoma, Sheehan's syndrome, dysspermatogenesis, etc. (inhibitor of prolactin production), preferably, a safe and low toxic prophylactic and/or therapeutic medicine for adiposis, hyperphagia and the like can be included.

The compounds that increase the binding affinity between ligands and the G protein-coupled receptor protein of the present invention are useful as safe and low toxic pharmaceuticals to potentiate the physiological activities that the ligands for the receptor protein etc. of the present invention possess.

The compounds that reduce the binding affinity between ligands and the G protein-coupled receptor protein of the present invention are useful as safe and low toxic pharmaceuticals that decrease the physiological activities of ligands for the receptor protein etc. of the present invention.

When compounds or their salt forms, which are obtainable by the screening methods or using the screening kits of the present invention, are employed as ingredients of the pharmaceuticals described above, the compounds can be formulated in the pharmaceuticals in a conventional manner. For example, the compounds can be prepared into tablets, capsules, elixir, microcapsules, aseptic solution, suspension, etc., as described for pharmaceuticals containing the receptor protein of the present invention.

The preparations thus obtained are safe and low toxic, and can be administered to, for example, human and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(8) Prophylactic and/or therapeutic agents for various diseases comprising the compounds (agonists or antagonists) that alter the binding property between the G protein-coupled receptor protein of the present invention and ligands

As described above, the receptor protein of the present invention may play some important role in the body such as a role in the central function, circulatory function, alimentary function and heart function. Therefore, the compounds (agonists or antagonists) that alter the binding property between the G protein-coupled receptor protein of the present invention and ligands to the receptor protein of the present invention can be used as prophylactic and/or therapeutic agents for diseases associated with dysfunction of the receptor protein of the present invention.

When the compounds and the ligand are used as the prophylactic and/or therapeutic agents for diseases associated with dysfunction of the receptor protein of the present invention, the pharmaceutical preparations can be obtained in a conventional manner.

For example, the compounds and the ligand can be administered orally as sugar coated tablet, capsule, elixir, and microcapsule, or non-orally as injection such as aseptic solution or suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.

For the additive that may be mixed in tablets, capsules, etc., for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined, for example, with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), and antioxidants. The preparation for injection is usually filled in appropriate ampoules.

In addition, the prophylactic/therapeutic agent described above can be used in combination with an appropriate pharmaceutical, as, for example, DDS formulation preparation, to which organs or tissues that highly express the receptor protein of the present invention are specifically targeted.

The preparations obtained as described above are safe and low toxic, and can be administered to, for example, human and other mammals (e.g., rats, mice, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(9) Quantification of the receptor protein of the present invention, its partial peptide, or its salt form

The antibodies of the present invention are capable of specifically recognizing the receptor protein etc. of the present invention. Therefore, the antibodies can be used to quantify the receptor protein etc. of the present invention in a test fluid, especially for quantification by the sandwich immunoassay. That is, the present invention provides, for example, the following quantification methods:

(i) A method of quantifying the receptor protein etc. of the present invention in a test fluid, which comprises competitively reacting the antibody of the present invention with the test fluid and a labeled form of the receptor protein etc. of the present invention, and measuring the ratio of the labeled receptor protein etc. bound to the antibody; and,
(ii) A method of quantifying the receptor protein etc. of the present invention in a test fluid, which comprises reacting the test fluid with the antibody of the present invention immobilized on a carrier and a labeled form of the antibody of the present invention simultaneously or sequentially, and measuring the activity of the label on the immobilized carrier.

In (ii) described above, it is preferred that one antibody recognizes the N-terminal region of the receptor protein etc. of the present invention, and another antibody reacts with the C-terminal region of the receptor protein etc. of the present invention.

Using monoclonal antibodies to the receptor protein etc. of the present invention (hereinafter sometimes referred to as the monoclonal antibodies of the present invention), the receptor protein etc. of the present invention can be assayed and also detected by tissue staining or the like. For this purpose, an antibody molecule itself may be used, or $F(ab')_2$, Fab' or Fab fractions of the antibody molecule may also be used. Assay methods using antibodies to the receptor protein etc. of the present invention are not particularly limited. Any assay method can be used, so long as the amount of antibody, antigen, or antibody-antigen complex corresponding to the amount of antigen (e.g., the amount of the receptor protein) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [$^{125}I$], [$^{131}I$], [$^{3}H$] and [$^{14}C$] are used. As the enzyme described above, stable enzymes with high specific activity are preferred; for example, β-galactosidase, β-glucosidase, alkaline phosphatase, peroxidase, malate dehydrogenase and the like are used. Example of the fluorescent substance used is fluorescamine and fluorescein isothiocyanate are used. For the luminescent substance, for example, luminol, luminol derivatives, luciferin, and lucigenin can be used, Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like. are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the receptor protein of the present invention in the test fluid can be quantified. The order of the primary and secondary reactions may be reversed, and the reactions may be performed simultaneously or with an interval. The methods of labeling and immobilization can be performed by the methods described above.

In the immunoassay by the sandwich method, the antibody used for immobilized or labeled antibodies is not necessarily one species, but a mixture of two or more species of antibody may be used to increase the measurement sensitivity.

In the methods of assaying the receptor protein etc. of the present invention by the sandwich method, antibodies that bind to different sites of the receptor protein etc. are preferably used as the monoclonal antibodies of the present invention for the primary and secondary reactions. That is, in the antibodies used for the primary and secondary reactions are, for example, when the antibody used in the secondary reaction recognizes the C-terminal region of the receptor protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the receptor protein of the present invention or its salts are constructed by adding the usual technical consideration in the art to the conventional conditions and procedures. For the details of these general technical means, reference can be made to the following reviews and texts. [For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the receptor protein of the present invention or its salts can be quantified with high sensitivity, using the antibodies of the present invention.

By quantifying the receptor protein of the present invention or its salts in vivo using the antibodies of the present invention, diagnosis can be made on various diseases associated with dysfunction of the receptor protein of the present invention.

The antibodies of the present invention can also be used for specifically detecting the receptor protein etc. of the present invention present in test samples such as body fluids or tissues. The antibodies may also be used for preparation of antibody columns for purification of the receptor protein etc. of the present invention, for detection of the receptor protein etc. of the present invention in each fraction upon purification, and for analysis of the behavior of the receptor protein of the present invention in the test cells.

(10) Methods of screening compounds that alter the amount of the receptor protein of the present invention or its partial peptide in cell membranes

Since the antibodies of the present invention specifically recognize the receptor protein, its partial peptide, or its salt of the present invention, the antibodies can be used for screening of the compounds that alter the amount of the receptor protein of the present invention or its partial peptide in cell membranes.

That is, the present invention provides, for example, the following methods:

(i) A method of screening compounds that alter the amount of the receptor protein of the present invention or its partial peptides in cell membranes, which comprises disrupting (a) blood, (b) specific organs, (c) tissues or cells isolated from the organs of non-human mammals, isolating the cell membrane fraction and then quantifying the receptor protein of the present invention or its partial peptide contained in the cell membrane fraction;

(ii) A method of screening compounds that alter the amount of the receptor protein of the present invention or its partial peptides in cell membranes, which comprises disrupting transformants, etc. expressing the receptor protein of the present invention or its partial peptides, isolating the cell membrane fraction, and then quantifying the receptor protein of the present invention or its partial peptides contained in the cell membrane fraction;

(iii) A method of screening compounds that alter the amount of the receptor protein of the present invention or its partial peptides in cell membranes, which comprises sectioning (a) blood, (b) specific organs, (c) tissues or cells isolated from the organs of non-human mammals, immunostaining, and then quantifying the staining intensity of the receptor protein in the cell surface layer to confirm the protein on the cell membrane; and,

(iv) a method of screening compounds that alter the amount of the receptor protein of the present invention or its partial peptides in cell membranes, which comprises sectioning transformants, etc. expressing the receptor protein of the present invention or its partial peptides , immunostaining, and then quantifying the staining intensity of the receptor protein in the cell surface layer to confirm the protein on the cell membrane.

Specifically, the receptor protein and its partial peptides of the present invention contained in cell membrane fractions are quantified as follows.

(i) Normal or non-human mammals of disease models (e.g., mice, rats, rabbits, sheep, swine, bovine, cats, dogs, monkeys, more specifically, rats with dementia, obese mice, rabbits with arteriosclerosis, tumor-bearing mice, etc.) are administered with a drug (e.g., anti-dementia agents, hypotensive agents, anticancer agents, antiobestic agents) or physical stress (e.g., soaking stress, electric shock, light and darkness, low temperature, etc.), and the blood, specific organs (e.g., brain, lung, large intestine, etc.), or tissue or cells isolated from the organs are obtained after a specified period of time. The obtained organs, tissues or cells are suspended in, for example, an appropriate buffer (e.g., Tris hydrochloride buffer, phosphate buffer, Hepes buffer), and the organs, tissues, or cells are disrupted, and the cell membrane fraction is obtained using surfactants (e.g., Triton-X 100™, Tween 20™) and further using techniques such as centrifugal separation, filtration, and column fractionation.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein etc. expressed and membrane components such as cell-derived phospholipids and membrane proteins.

The receptor protein of the present invention or its partial peptides contained in the cell membrane fraction can be quantified by, for example, the sandwich immunoassay and western blot analysis using the antibodies of the present invention.

The sandwich immunoassay can be performed as described above, and the western blot can be performed by publicly known methods.

(ii) Transformants expressing the receptor protein of the present invention or its partial peptides are prepared following the method described above, and the receptor protein of the present invention or its partial peptides contained in the cell membrane fraction can be quantified.

The compounds that alter the amount of the receptor protein of the present invention or its partial peptides in cell membranes can be screened as follows.

(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of the receptor protein of the present invention or its partial peptides contained in cell membranes are quantified.

(ii) Transformants are cultured in a conventional manner and a test compound is mixed in the culture medium. After a specified time (after 1 day to 7 days, preferably after 1 day to 3 days, more preferably after 2 to 3 days), the amount of the receptor protein of the present invention or its partial peptides contained in the cell membranes can be quantified.

Specifically, the receptor protein of the present invention or its partial peptides contained in cell membrane fractions is confirmed as follows.

(iii) Normal or non-human mammals of disease models (e.g., mice, rats, rabbits, sheep, swine, bovine, cats, dogs, monkeys, more specifically, rats with dementia, obese mice, rabbits with arteriosclerosis, tumor-bearing mice, etc.) are administered with a drug (e.g., anti-dementia agents, hypotensive agents, anticancer agents, antiobestic agents) or physical stress (e.g., soaking stress, electric shock, light and darkness, low temperature, etc.), and the blood, specific organs (e.g., brain, large intestine, small intestine, pancreas, ovary, stomach, heart, liver, testis, placenta, lung, spinal cord, spleen, thymus, kidney, duodenum, adrenal, prostate, pituitary, uterus, etc.), or tissue or cells isolated from the organs are obtained after a specified period of time. Tissue sections are prepared from the thus obtained organs, tissues or cells in a conventional manner followed by immunostaining with the antibody of the present invention. The staining intensity of the receptor protein in the cell surface layer is quantified to confirm the protein on the cell membrane, and the amount of the receptor protein of the present invention or its partial peptides in the cell membrane can be quantitatively or qualitatively confirmed.

(iv) The confirmation can also be made by the similar method, using transformants expressing the receptor protein of the present invention or its partial peptides.

The compounds or its salts, which is obtainable by the screening methods of the present invention, are the compounds that alter the amount of the receptor protein or its peptide fragments of the present invention. Specifically, these compounds are; (a) compounds that potentiate the G protein-coupled receptor-mediated cell-stimulating activity (e.g., activity that promotes or inhibits arachidonic acid release, acetylcholine release, intracellular $Ca^{2+}$ release, enhancement and inhibition of intracellular cAMP production, intracellular cGMP production, inositol phosphate production, changes in cell membrane potential, phosphorylation of intracellular proteins, activation of c-fos, pH reduction, etc.) (so-called agonists to the receptor protein of the present invention), by increasing thea mount of the receptor protein of the present invention or its partial peptides ; and (b) compounds that lower the cell stimulating-activity by decreasing the amount of the receptor protein of the present invention.

The compounds may be peptides, proteins, non-peptide compounds, synthetic compounds, fermentation products, and may be novel or known compounds.

The compounds that increase the cell-stimulating activity are useful as safe and low toxic pharmaceuticals for potentiation of the physiological activity of the receptor protein etc. of the present invention.

The compounds that decrease the cell-stimulating activity are useful as safe and low toxic pharmaceuticals for reduction of the physiological activity of the receptor protein etc. of the present invention.

When compounds or their salt forms, which are obtainable by the screening methods of the present invention, are used as for pharmaceutical compositions, preparations can be prepared following the conventional methods. For example, as described above for preparation of the pharmaceuticals containing the receptor protein of the present invention, the compounds can be prepared into tablets, capsules, elixir, microcapsules, aseptic solution, suspension, etc.

Since the preparations thus obtained are safe and low toxic, the preparations can be administered to human and other mammals (e.g., rats, mice, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(11) Prophylactic and/or therapeutic agents for various diseases comprising compounds that alter the amount of the receptor protein of the present invention or its partial peptides in cell membrane

As described above, the receptor protein of the present invention is considered to play some important role in vivo, such as a role in various tissues (e.g., brain, large intestine, small intestine, pancreas, ovary, stomach, heart, liver, testis, placenta, lung, spinal cord, spleen, thymus, kidney, duodenum, adrenal, prostate, pituitary, uterus, etc.). Therefore, the compounds that alter the amount of the receptor protein of the present invention or its partial peptide in cell membrane can be used as prophylactic and/or therapeutic agents for diseases associated with dysfunction of the receptor protein of the present invention.

When the compounds are used as prophylactic and/or therapeutic agents for diseases associated with dysfunction of the receptor protein of the present invention, the preparations can be obtained in a conventional manner.

For example, the compounds can be administered orally as a sugar coated tablet, capsule, elixir, and microcapsule, or parenterally as injection such as aseptic solution and suspension in water or other pharmaceutically acceptable liquid. For example, preparations of the compounds can be manufactured by mixing with physiologically acceptable known carrier, flavor, filler, vehicle, antiseptic, stabilizer, and binder in a unit-dosage form required for generally approved drug preparation. The amount of the active ingredient is set to an appropriate volume within the specified range.

For the additive that may be mixed in tablets and capsules, for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry are used. When the dosage form is a capsule, liquid carrier such as fat and oil may be contained. Aseptic compositions for injection can be formulated following the usual preparation such as dissolving or suspending the active substance in vehicle, e.g., water for injection, and natural plant oils e.g., sesame oil and coconut oil. For the aqueous solution for injection, for example, physiological saline and isotonic solutions (e.g., D-sorbitol, D-mannitol, sodium hydrochloride) containing glucose and other adjuvant are used. Appropriate dissolution-assisting agents, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agents described above may be combined with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), and antioxidants. The preparation for injection is usually filled in appropriate ampoules.

Since the preparations thus obtained are safe and low toxic, the preparation can be administered to, for example, human and other mammals (e.g., rats, mice, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the patient with adiposis, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with adiposis, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(12) Neutralization with antibodies to the receptor protein, its partial peptides, or their salts of the present invention

The neutralizing activity of antibodies to the receptor protein of the present invention, its partial peptides, or its salts refers to an activity of inactivating the signal transduction function involving the receptor protein. Therefore, when the antibody has the neutralizing activity, the antibody can inactivate the signal transduction in which the receptor protein participates, for example, inactivate the receptor protein-mediated cell-stimulating activity (e.g., activity that promotes or inhibits arachidonic acid release, acetylcholine release, intracellular $Ca^{2+}$ release, enhancement and inhibition of intracellular cAMP production, intracellular cGMP production, inositol phosphate production, changes in cell membrane potential, phosphorylation of intracellular proteins, activation of c-fos, pH reduction, etc.). Therefore, the antibody can be used for the prevention and/or treatment of diseases caused by overexpression of the receptor protein.

(13) Animals carrying the DNA encoding the G protein-coupled receptor protein of the present invention

Using the DNA of the present invention, transgenic animals expressing the receptor protein etc. of the present invention can be prepared. Examples of the animals include mammals (e.g., rats, mice, rabbits, sheep, swine, bovine, cats, dogs, monkeys, etc.) (hereinafter merely referred to as animals) can be used, with mice and rabbits being particularly appropriate.

To transfer the DNA of the present invention to target animals, it is generally advantageous to use the DNA in a gene construct ligated downstream of a promoter that can express the DNA in animal cells. For example, when the DNA of the present invention derived from rabbit is transferred, e.g., the gene construct, in which the DNA is ligated downstream of a promoter that can expresses the DNA of the present invention derived from animals containing the DNA of the present invention highly homologous to the rabbit-derived DNA, is microinjected to rabbit fertilized ova; thus, the DNA-transferred animal, which is capable of producing a high level of the receptor protein etc. of the present invention, can be produced. Examples of the promoters that are usable include virus-derived promoters and ubiquitous expression promoters such as metallothionein promoter, but promoters of gene that are specifically expressed in brain, lung, stomach, heart, kidney and the like, are preferably used.

The introduction of the DNA of the present invention at the fertilized egg cell stage secures the presence of the DNA in all germ and somatic cells in the produced animal. The presence of the receptor protein etc. of the present invention in the germ cells in the DNA-introduced animal means that all germ and somatic cells contain the gene (the DNA) of the receptor protein etc. of the present invention in all progenies of the animal. The progenies of the animal that took over the gene contain the receptor protein etc. of the present invention in all germ and somatic cells.

The DNA-introduced animals of the present invention can be maintained and bled in the conventional environment as animals carrying the DNA after confirming the stable retention of the gene in the animals through mating. Furthermore, mating male and female animals containing the objective DNA results in acquiring homozygote animals having the transferred gene on both homologous chromosomes. By mating the male and female homozygotes, bleeding can be performed so that all progenies contain the DNA.

Since the receptor protein etc. of the present invention is highly expressed in the animals in which the DNA of the present invention has been introduced, the animals are useful for screening of agonists or antagonists to the receptor protein etc. of the present invention.

The animals in which the DNA of the present invention has been introduced can also be used as cell sources for tissue culture. The receptor protein of the present invention can be analyzed by, for example, directly analyzing the DNA or RNA in tissues from the mouse in which the DNA of the present invention has been introduced, or by analyzing tissues containing the receptor protein etc. expressed from the gene. Cells from tissues containing the receptor protein etc. of the present invention are cultured by the standard tissue culture technique. Using these cells, for example, the function of tissue cells such as cells derived from the brain or peripheral tissues, which are generally difficult to culture, can be studied. Using these cells, for example, it is possible to select pharmaceuticals that increase various tissue functions. When a highly expressing cell line is available, the receptor protein etc. of the present invention can be isolated and purified from the cell line.

(14) Knockout animals

The present invention provides a non-human mammalian embryonic stem cell, wherein the DNA of the present invention is inactivated, and a non-human mammal, which DNA is barely expressed.

That is, the present invention provides:

1) A non-human embryonic stem cell in which the DNA of the present invention is inactivated;
2) The embryonic stem cell according to 1), wherein the DNA is inactivated by introducing a reporter gene (e.g., β-galactosidase gene derived from Escherichia coli);
3) The embryonic stem cell according to 1), which is resistant to neomycin;
4) The embryonic stem cell according to 1), wherein the non-human mammal is a rodent;
5) An embryonic stem cell according to 4), wherein the rodent is mouse;
6) A non-human mammal deficient in expressing the DNA of the present invention, wherein the DNA of the present invention is inactivated;
7) The non-human mammal according to 6), wherein the DNA is inactivated by inserting a reporter gene (e.g., β-galactosidase derived from Escherichia coli) therein and the reporter gene is capable of being expressed under control of a promoter for the DNA of the present invention;
8) The non-human mammal according to 6), which is a rodent;
9) The non-human mammal according to 8), wherein the rodent is mouse; and,
10) A method for screening a compound or its salt that promotes or inhibits the promoter activity for the DNA of the present invention, which comprises administering a test compound to the mammal of 7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the polypeptide of the present invention (hereinafter sometimes referred to as the knockout DNA of the present invention) by substantially inactivating the activities of the polypeptide of the present invention encoded by the DNA (hereinafter merely referred to as ES cell).

As the non-human mammal, the same examples as described above apply.

Methods for artificially mutating the DNA of the present invention include, for example, deletion of a part or all of the DNA sequence and insertion of or substitution with other DNA, by genetic engineering. By these mutations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the subject animal by, e.g., homologous recombination, a DNA sequence which terminates gene transcription (e.g., polyA additional signal, etc.) in the intron between exons to, thus inhibit the synthesis of complete messenger RNA and eventually destroy the gene (hereinafter simply referred to as targeting vector). The thus-obtained ES cells to the Southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may be originally established in accordance with a modification of the publicly known method by Evans and Kaufman *supra.* For example, in the case of mouse ES cells, currently it is common practice to use ES cells of the 129 strain. However, since their immunological background is obscure, the C57BL/6 mouse or the $BDF_1$ mouse ($F_1$ hybrid between C57BU6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BU6 mouse, since its background is of the C57BU6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is desirable to identify sexes as soon as possible also in order to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to spontaneously differentiate them to various cell types, for example, pariental muscle, visceral muscles, cardiac muscle or the like (M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985). The cells deficient in expression of the DNA of the present invention, which are obtainable from the differentiated ES cells of the present invention, are useful for studying the functions of the polypeptide of the present invention cytologically or molecular biologically.

The non-human mammal deficient in expression of the DNA of the present invention can be identified from a normal animal by measuring the amount of mRNA in the subject animal by a publicly known method, and indirectly comparing the degrees of expression.

As the non-human mammal, the same examples *supra* can be applied.

With respect to the non-human mammal deficient in expression of the DNA of the present invention, the DNA of the present invention can be made knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse oocyte.

The knockout cells with the DNA of the present invention disrupted can be identified by Southern hybridization analysis with a DNA sequence on or near the DNA of the present invention as a probe, or by PCR analysis using a DNA sequence on the targeting vector and another DNA sequence derived from mouse, which is not included in the targeting vector, as primers. When non-human mammalian embryonic stem cells are used, a cell line wherein the DNA of the present invention is inactivated by homologous recombination is cloned; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The prepared animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the receptor protein of the present invention. The individuals deficient in homozygous expression of the receptor protein of the present invention can be obtained from offspring of the intercross between the heterozygotes.

When an oocyte is used, a DNA solution may be injected, e.g., to the nucleus of the oocyte by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals that the DNA of the present invention is rendered knockout permit passage rearing under ordinary rearing condition, after the individuals obtained by their crossing have proven to be knockout.

Furthermore, the genital system may be obtained and maintained by conventional methods. That is, by crossing male and female animals each having the inactivated DNA, homozygoous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell in which the DNA of the present invention is inactivated is very useful for preparing a non-human mammal deficient in expression of the DNA of the present invention.

Since the non-human mammal in which the DNA of the present invention is inactivated lacks various biological activities derived from the receptor protein of the present invention, such an animal can be a disease model suspected of inactivated biological activities of the receptor protein of the present invention and thus, offers an effective study to investigate causes for and therapy for these diseases.

(14a) Methods for screening of compounds having therapeutic and/or prophylactic effects for diseases caused by deficiency, damages, etc. of the DNA of the present invention

The non-human mammal deficient in expression of the DNA of the present invention can be employed for the screening of compounds having therapeutic and/or prophylactic effects for diseases caused by deficiency, damages, etc. of the DNA of the present invention.

That is, the present invention provides a method for screening of a compound having therapeutic and/or prophylactic effects for diseases caused by deficiency, damages, etc. of the DNA of the present invention, which comprises administering a test compound to the non-human mammal deficient in expression of the DNA of the present invention and observing and measuring a change occurred in the animal.

As the non-human mammal deficient in expression of the DNA of the present invention, which can be employed for the screening method, the same examples as given hereinabove apply.

Examples of the test compounds include peptides, proteins, non-peptide compounds, synthetic compounds, fermentation products, cell extracts, vegetable extracts, animal tissue extracts, blood plasma and the like and these compounds may be novel compounds or publicly known compounds.

Specifically, the non-human mammal deficient in the expression of the DNA of the present invention is treated with a test compound, and by comparison with an intact animal for control, a change in each organ, tissue, disease conditions, etc. of the animal is used as an index to assess the therapeutic and/or prophylactic effects of the test compound.

For treating an animal to be tested with a test compound, for example, oral administration, intravenous injection, etc. are applied and the treatment is appropriately selected depending upon conditions of the test animal, properties of the test compound, etc. Furthermore, an amount of a test compound administered can be selected depending on administration route, nature of the test compound, and the like.

For example, in the case of screening a compound having a therapeutic and/or prophylactic effect for central nerve system dysfunction (e.g., Alzheimer's disease, dementia, eating disorder), endocrine disorders [e.g., hypertension, hypogonadism, thyroid insufficiency, dyspituitarism, hyposecretion of pituitary hormone (e.g., hyposecretion of prolactin (e.g., ovarian dysfunction, ateliosis of seminal vesicle, menopausal disorder, hypothroidism)), etc.], metabolic disorders (e.g., diabetes, metabolic disorder of lipid, hyperlipemia), cancers (e.g., non-small-cell lung cancer, ovarian cancer, prostate cancer, stomach cancer, bladder carcinoma, breast cancer, cancer of uterine cervix, colon cancer, rectum cancer), heart diseases (e.g., angina, heart infarction), anorexia, adiposis, hyperphagia and the like, the non-human mammal deficient in expression of the DNA of the present invention is subjected to a sugar loading treatment, and a test compound is administered to the animal before or after the sugar loading treatment. Then, blood sugar level, body weight change, etc. of the animal is measured with passage of time.

In the screening method, when a test compound is administered to an animal to be tested and found to reduce the blood sugar level of the animal to at least about 10%, preferably at least about 30% and more preferably at least about 50%, the test compound can be selected as a compound having the therapeutic and/or prophylactic effect for the above-mentioned diseases.

The compound obtained using the above screening method is a compound selected from the test compounds described above and exhibits a therapeutic and/or prophylactic effect for the diseases caused by deficiencies, damages, etc. of the receptor protein of the present invention. Therefore, the compound can be employed as a safe and low toxic pharmaceutical for the treatment and prevention of these diseases. Furthermore, compounds derived from such a compound obtained by the above screening can be likewise employed.

The compound obtained by the screening method may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical containing the compound obtained by the above screening method or salts thereof may be manufactured in a manner similar to the method for preparing the pharmaceutical comprising the receptor protein of the present invention described hereinabove.

Since the pharmaceutical thus obtained is safe and low toxic, it can be administered to human or other mammals (e.g., rat, mouse, guinea pig, rabbit, sheep, swine, bovine, horse, cat, dog, monkey, etc.).

Although the amount of the compound or its salt to be administered varies depending upon particular disease, subject to be administered, route of administration, etc., but when the compound is orally administered, the compound is generally administered to an adult patient with anorexia (as 60 kg body weight) in a dose of about 0.1 mg/day to about 100 mg/day, preferably about 1.0 mg/day to about 50 mg/day, more preferably about 1.0 mg to about 20 mg. For parenteral administration, a single dose of the compound varies depending upon subject to be administered, target disease, etc., but when the compound is administered to an adult patient with anorexia (as 60 kg body weight) in the form of an injectable preparation, it is generally advantageous to administer the compound intravenously in a dose of about 0.01 mg/day to about 30 mg/day, preferably about 0.1 mg/day to about 20 mg/day, more preferably about 0.1 mg/day to about 10 mg/day. As for other animals, the compound can be administered in the above amount with converting it into that for the body weight of 60 kg.

(14b) Method for screening a compound that promotes or inhibits the activities of a promoter to the DNA of the present invention

The present invention provides a method for screening a compound or its salt that promotes or inhibits the activities of a promoter to the DNA of the present invention, which comprises administering a test compound to a non-human mammal deficient in expression of the DNA of the present invention and detecting expression of the reporter gene.

In the screening method described above, as the non-human mammal deficient in expression of the DNA of the present invention, an animal in which the DNA of the present invention is inactivated by introducing a reporter gene and the reporter gene can be expressed under control of a promoter to the DNA of the present invention is used from the aforementioned non-human mammal deficient in expression of the DNA of the present invention.

The same examples of the test compound apply to specific compounds used for the screening.

As the reporter gene, the same specific examples described above apply, and β-galactosidase (lacZ), soluble alkaline phosphatase gene, luciferase gene and the like are preferably employed.

Since a reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the receptor protein of the present invention is substituted with, e.g., β-galactosidase gene (lacZ) derived from Escherichia coli, β-galactosidase is expressed in a tissue where the receptor protein of the present invention should originally be expressed, instead of the receptor protein of the present invention. Thus, the state of expression of the receptor protein of the present invention can be readily observed in vivo of an animal by staining with a reagent, e.g., 5-bromo-4-chloro-3-indolyl-β-galactopyranoside (X-gal), which is substrate for β-galactosidase. Specifically, a mouse deficient in the receptor protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After that, by washing the tissue preparation with 1 mM EDTA/PBS solution, the β-galactosidase reaction is terminated, and the color formed is observed. Alternatively, mRNA encoding lacZ may be detected in a conventional manner.

The compound or salts thereof obtained using the above screening method, are compounds that are selected from the test compounds described above and that promote or inhibit the promoter activity to the DNA of the present invention.

The compound obtained by the screening method above may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Since the compounds or salts thereof that enhance the promoter activity to the DNA of the present invention can promote the expression of the receptor protein of the present invention, or can promote the functions of the receptor protein, they are useful as safe and low toxic pharmaceuticals for the treatment and/or prevention of central nerve system dysfunction (e.g., Alzheimer's disease, dementia, eating disorder), endocrine disorders [e.g., hypertension, hypogonadism, thyroid insufficiency, dyspituitarism, hyposecretion of pituitary hormone (e.g., hyposecretion of prolactin (e.g., ovarian dysfunction, ateliosis of seminal vesicle, menopausal disorder, hypothroidism)), etc.], metabolic disorders (e.g., diabetes, metabolic disorder of lipid, hyperlipemia), cancers (e.g., non-small-cell lung cancer, ovarian cancer, prostate cancer, stomach cancer, bladder carcinoma, breast cancer, cancer of uterine cervix, colon cancer, rectum cancer), heart diseases (e.g., angina, heart infarction), diseases such as anorexia, adiposis, hyperphagia and the like (preferably, anorexia, adiposis, etc.).

Further, the compounds or salts thereof that inhibit the promoter activity to the DNA of the present invention can inhibit the expression of the receptor protein of the present invention, or can inhibit the functions of the protein, they are useful as safe and low toxic pharmaceuticals such as a prophylactic and/or therapeutic agent for adiposis (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity), hyperphagia, pituitary adenomatoid tumor, diencephalons tumor, emmeniopathy, autoimmune disease, prolactinoma, infertile, impotence, amenia, galactorrhea, acromegaly, Chiari-Frommel syndrome, Argonz-Del Castillo syndrome, Forbes-Albright syndrome, lymphoma, Sheehan's syndrome, dysspermatogenesis, etc. (inhibitor of prolactin production), preferably a prophylactic and/or therapeutic agent for adiposis, hyperphagia and the like.

In addition, compound derived from the compounds obtained by the screening above may be likewise employed.

A pharmaceutical containing the compounds or salts thereof obtained by the screening method may be manufactured in a manner similar to the method for preparing the pharmaceutical containing the receptor protein of the present invention described above.

Since the pharmaceutical preparation thus obtained is safe and low toxic, it can be administered to human or other mammals (e.g., rat, mouse, guinea pig, rabbit, sheep, swine, bovine, horse, cat, dog, monkey, etc.).

The dose of the compound or salts thereof varies depending on target disease, subject to be administered, route for administration, etc.; for example, when the compound that enhances the promoter activity to the DNA of the present invention is orally administered, the dose is normally about 0.1 to about 100 mg, preferably about 1.0 to about 50 mg, more preferably about 1.0 to about 20 mg per day for adult patient with anorexia (as 60 kg body weight). In parenteral administration, a single dose of the compound varies depending on subject to be administered, target disease, etc. but when the compound that enhances the promoter activity to the DNA of the present invention is administered in the form of injectable preparation, it is advantageous to administer the compound intravenously at a single dose of about 0.01 to about 30 mg/day, preferably about 0.1 to about 20 mg/day, more preferably about 0.1 to about 10 mg/day for adult patient with anorexia (as 60 kg body weight). For other animal species, the corresponding dose as converted per 60 kg weight can be administered.

On the other hand, when the compound that inhibits the promoter activity to the DNA of the present invention is orally administered, the dose is normally about 0.1 to about 100 mg, preferably about 1.0 to about 50 mg, more preferably about 1.0 to about 20 mg per day for adult patient with adiposis (as 60 kg body weight). In parenteral administration, a single dose of the compound varies depending on subject to be administered, target disease, etc. but when the compound that inhibits the promoter activity to the DNA of the present invention is administered in the form of injectable preparation, it is advantageous to administer the compound intravenously at a single dose of about 0.01 to about 30 mg/day, preferably about 0.1 to about 20 mg/day, more preferably about 0.1 to about 10 mg/day for adult patient with adiposis (as 60 kg body weight). For other animal species, the corresponding dose as converted per 60 kg weight can be administered.

As stated above, the non-human mammal deficient in expression of the DNA of the present invention is extremely useful for screening the compound or its salt that promotes or inhibits the activity of a promoter to the DNA of the present invention and can greatly contribute to the elucidation of causes for various diseases suspected of deficiency in expression of the DNA of the present invention and for the development of medicine for prevention and/or treatment of these diseases.

Furthermore, a so-called transgenic animal (gene introduced animal) can be prepared by using DNA containing a promoter region of the receptor protein of the present invention, ligating genes encoding various proteins downstream and injecting the same into oocyte of an animal. It is then possible to synthesize the protein therein specifically and study its activity in vivo. When an appropriate reporter gene is ligated to the promoter site above and a cell line that express the gene is established, the resulting system can be utilized as the survey system for a low molecular compound having the action of specifically promoting or inhibiting the in vivo productivity of the receptor protein per se of the present invention.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| I | : inosine |
| R | : adenine (A) or guanine (G) |
| Y | : thymine (T) or cytosine (C) |
| M | : adenine (A) or cytosine (C) |
| K | : guanine (G) or thymine (T) |
| S | : guanine (G) or cytosine (C) |
| W | : adenine (A) or thymine (T) |
| B | : guanine (G), guanine (G) or thymine (T) |
| D | : adenine (A), guanine (G) or thymine (T) |
| V | : adenine (A), guanine (G) or cytosine (C) |
| N | : adenine (A), guanine (G), cytosine (C) or thymine (T), or unknown or other bases |
| RNA | : ribonucleic acid |
| MRNA | : messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |
| SDS | : sodium dodecyl sulfate |
| BHA | : benzhydrilamine |
| pMBH | : p-methylbenzhydrilamine |
| DCM | : dichloromethane |
| TFA | : trifluoroacetic acid |
| DIEA | : diisopropylethylamine |
| Gly or G | : glycine |
| Ala or A | : alanine |

| | |
|---|---|
| Val or V | : valine |
| Leu or L | : leucine |
| lie or I | : isoleucine |
| Ser or S | : serine |
| Thr or T | : threonine |
| Cys or C | : cysteine |
| Met or M | : methionine |
| Glu or E | : glutamic acid |
| Asp or D | : aspartic acid |
| Lys or K | : lysine |
| Arg or R | : arginine |
| His or H | : histidine |
| Phe or F | : phenylalanine |
| Tyr or Y | : tyrosine |
| Trp or W | : tryptophan |
| Pro or P | : proline |
| Asn or N | : asparagine |
| Gln or Q | : glutamine |
| pGlu | : pyroglutamic acid |
| Tyr(I) | : 3-iode tyrosine |
| * | : corresponding stop codon |
| Me | : methyl |
| Et | : ethyl |
| Bu | : butyl |
| Ph | : phenyl |
| TC | : thiazolidine-4(R)-carboxamide |

The substituents, protective groups and reagents, which are frequently used throughout the specification, are shown by the following abbreviations.

| | |
|---|---|
| Tos | : p-toluenesulfonyl |
| CHO | : formyl |
| Bzl | : benzyl |
| $Cl_2Bl$ | : 2,6-dichlorobenzyl |
| Bom | : benzyloxymethyl |
| Z | : benzyloxycarbonyl |
| Cl-Z | : 2-chlorobenzyloxycarbonyl |
| Br-Z | : 2-bromobenzyloxycarbonyl |
| Boc | : t-butoxycarbonyl |
| DNP | : dinitrophenol |
| Trt | : trityl |
| Bum | : t-butoxymethyl |
| Fmoc | : N-9-fluorenylmethoxycarbonyl |
| HOBt | : 1-hydroxybenztriazole |
| HOOBt | : 3,4-dihydro-3-hydroxy-4-oxo-1,2,3-benzotriazine |
| HONB | : 1-hydroxy-5-norbornene-2,3-dicarboximide |
| DCC | : N,N'-dicyclohexylcarbodiimide |
| DMP | : N,N'-dimethylformamide |
| Pbf | : 2,2,4,6,7-pentamethyldihydrobenzofuran-5-sulfonyl |
| Clf | : 2-chlorotrityl |
| $Bu^t$ | : t-buthyl |
| Met(O) | : methionine sulfoxide |

The sequence identification numbers in the sequence listing of the specification indicates the following sequence, respectively.

[SEQ ID NO: 1]

This shows the amino acid sequence of TGR26, the rat-derived novel G protein-coupled receptor protein of the present invention.

[SEQ ID NO: 2]

This shows the base sequence of cDNA encoding TGR26, the rat-derived novel G protein-coupled receptor protein of the present invention.

[SEQ ID NO: 3]

This shows the base sequence of primer 1 used in the PCR reaction of Example 1 described below.

[SEQ ID NO: 4]

This shows the base sequence of primer 2 used in the PCR reaction of Example 1 described below.

[SEQ ID NO: 5]

This shows the base sequence of primer used for determining an expression level of TGR26 receptor gene on the TGR26-expressing CHO cells in Example 3 described below.

[SEQ ID NO: 6]

This shows the base sequence of primer used for determining an expression level of TGR26 receptor gene on the TGR26-expressing CHO cells in Example 3 described below.

[SEQ ID NO: 7]

This shows the base sequence of probe used for determining an expression level of TGR26 receptor gene on the TGR26-expressing CHO cells in Example 3 described below.

[SEQ ID NO: 8]

This shows the amino acid sequence of human homologue of ligand peptide to GPR8.

[SEQ ID NO: 9]

This shows the amino acid sequence of human homologue of ligand peptide to GPR8.

[SEQ ID NO: 10]

This shows the synthetic DNA used in the screening of cDNA encoding human GPR8 protein.

[SEQ ID NO: 11]

This shows the synthetic DNA used in the screening of cDNA encoding human GPR8 protein.

[SEQ ID NO: 12]

This shows whole base sequence of cDNA encoding human GPR8 protein, wherein the base sequence recognized by restriction enzyme ClaI is added to the 5'-end and the base sequence recognized by restriction enzyme SpeI is added to the 3'-end.

[SEQ ID NO: 13]

This shows the sequence of riboprobe used for determining an expression level of mRNA coding for GPR8 receptor protein in each clone of the GPR8-expressing CHO cell line.

[SEQ ID NO: 14]

This shows the amino acid sequence obtained from the result of amino acid sequence analysis of the amino terminus of ligand peptide to GPR8 purified from swine hypothalamus.

[SEQ ID NO: 15]

This shows the EST sequence (Accession Number: AW007531), wherein a portion of the precursor protein of human homologue of the GPR8 ligand peptide is supposed to be encoded by its complementary strand.

[SEQ ID NO: 16]

This shows the EST sequence (Accession Number: AI500303), wherein a portion of the precursor protein of human homologue of the GPR8 ligand peptide is supposed to be encoded by its complementary strand.

[SEQ ID NO: 17]

This shows the EST sequence (Accession Number: AI990964), wherein a portion of the precursor protein of human homologue of the GPR8 ligand peptide is supposed to be encoded by its complementary strand.

[SEQ ID NO: 18]

This shows the EST sequence (Accession Number: AA744804), wherein a portion of the precursor protein of human homologue of the GPR8 ligand peptide is supposed to be encoded by its complementary strand.

[SEQ ID NO: 19]

This shows the EST sequence (Accession Number: H31598), wherein a portion of the precursor protein of rat homologue of the GPR8 ligand peptide is supposed to be encoded.

[SEQ ID NO: 20]

This shows the synthetic DNA used in the screening of cDNA encoding a portion of the precursor protein of human homologue of ligand peptide to GPR8.

[SEQ ID NO: 21]

This shows the synthetic DNA used in the screening of cDNA encoding a portion of the precursor protein of human homologue of ligand peptide to GPR8.

[SEQ ID NO: 22]

This shows the DNA sequence encoding a portion of the precursor protein of human homologue of ligand peptide to GPR8, which was amplified from cDNA derived from human brain.

[SEQ ID NO: 23]

This shows the amino acid sequence of a portion of the precursor protein of human homologue of ligand peptide to GPR8.

[SEQ ID NO: 24]

This shows the amino acid sequence of human GPR ligand (1-25) synthesized in Reference Example 20 described below.

[SEQ ID NO: 25]

This shows the amino acid sequence of human GPR ligand (1-24) synthesized in Reference Example 21 described below.

[SEQ ID NO: 26]

This shows the base sequence encoding the amino acid sequence represented by SEQ ID NO: 128.

[SEQ ID NO: 27]

This shows the base sequence encoding the amino acid sequence represented by SEQ ID NO: 129.

[SEQ ID NO: 28]

This shows the base sequence encoding the amino acid sequence represented by SEQ ID NO: 130.

[SEQ ID NO: 29]

This shows the base sequence encoding the amino acid sequence represented by SEQ ID NO: 131.

[SEQ ID NO: 30]

This shows the base sequence encoding the amino acid sequence represented by SEQ ID NO: 24.

[SEQ ID NO: 31]

This shows the base sequence encoding the amino acid sequence represented by SEQ ID NO: 25.

[SEQ ID NO: 32]

This shows whole amino acid sequence of human GPR8 protein.

[SEQ ID NO: 33]

This shows the synthetic DNA used for obtaining 5' upstream sequence of cDNA encoding the precursor protein of human homologue of ligand peptide to GPR8.

[SEQ ID NO: 34]

This shows the synthetic DNA used for obtaining 5' upstream sequence of cDNA encoding the precursor protein of human homologue of ligand peptide to GPR8.

[SEQ ID NO: 35]

This shows the 5' upstream DNA sequence of cDNA encoding the precursor protein of human homologue of ligand peptide to GPR8.

[SEQ ID NO: 36]

This shows the synthetic DNA used for obtaining 3' downstream sequence of cDNA encoding the precursor protein of human homologue of ligand peptide to GPR8.

[SEQ ID NO: 37]

This shows the synthetic DNA used for obtaining 3' downstream sequence of cDNA encoding the precursor protein of human homologue of ligand peptide to GPR8.

[SEQ ID NO: 38]

This shows the 3' downstream DNA sequence of cDNA encoding the precursor protein of human homologue of ligand peptide to GPR8.

[SEQ ID NO: 39]

This shows the synthetic DNA used for obtaining cDNA encoding the precursor protein of human homologue of ligand peptide to GPR8.

[SEQ ID NO: 40]

This shows the synthetic DNA used for obtaining cDNA encoding the precursor protein of human homologue of ligand peptide to GPR8.

[SEQ ID NO: 41]

This shows the cDNA sequence, which encodes the precursor protein of human homologue of ligand peptide to GPR8.

[SEQ ID NO: 42]

This shows the amino acid sequence of the precursor protein of human homologue of ligand peptide to GPR8.

[SEQ ID NO: 43]

This shows the synthetic DNA used for obtaining 5' upstream sequence of cDNA encoding the precursor protein of porcine homologue of ligand peptide to GPR8.

[SEQ ID NO: 44]

This shows the synthetic DNA used for obtaining 5' upstream sequence of cDNA encoding the precursor protein of porcine homologue of ligand peptide to GPR8.

[SEQ ID NO: 45]

This shows the 5' upstream DNA sequence of cDNA encoding the precursor protein of porcine homologue of ligand peptide to GPR8.

[SEQ ID NO: 46]

This shows the synthetic DNA used for obtaining 5' upstream sequence of cDNA encoding the precursor protein of porcine homologue of ligand peptide to GPR8.

[SEQ ID NO: 47]

This shows the synthetic DNA used for obtaining 5' upstream sequence of cDNA encoding the precursor protein of porcine homologue of ligand peptide to GPR8.

[SEQ ID NO: 48]

This shows the 5' upstream DNA sequence of cDNA encoding the precursor protein of porcine homologue of ligand peptide to GPR8.

[SEQ ID NO: 49]

This shows the synthetic DNA used for obtaining 3' downstream sequence of cDNA encoding the precursor protein of porcine homologue of ligand peptide to GPR8.

[SEQ ID NO: 50]

This shows the synthetic DNA used for obtaining 3' downstream sequence of cDNA encoding the precursor protein of porcine homologue of ligand peptide to GPR8.

[SEQ ID NO: 51]

This shows the 3' downstream DNA sequence of cDNA encoding the precursor protein of porcine homologue of ligand peptide to GPR8.

[SEQ ID NO: 52]

This shows the synthetic DNA used for obtaining cDNA encoding the precursor protein of porcine homologue of ligand peptide to GPR8.

[SEQ ID NO: 53]

This shows the synthetic DNA used for obtaining cDNA encoding the precursor protein of porcine homologue of ligand peptide to GPR8.

[SEQ ID NO: 54]

This shows the cDNA sequence, which encodes the precursor protein of porcine homologue of ligand peptide to GPR8.

[SEQ ID NO: 55]

This shows the amino acid sequence of the precursor protein of porcine homologue of ligand peptide to GPR8.

[SEQ ID NO: 56]

This shows the amino acid sequence of porcine homologue of ligand peptide to GPR8 deduced from SEQ ID NO: 55.

[SEQ ID NO: 57]

This shows the amino acid sequence of porcine homologue of ligand peptide to GPR8 deduced from SEQ ID NO: 55.

[SEQ ID NO: 58]

This shows the base sequence encoding the amino acid sequence represented by SEQ ID NO: 56.

[SEQ ID NO: 59]

This shows the base sequence encoding the amino acid sequence represented by SEQ ID NO: 57.

[SEQ ID NO: 60]

This shows the synthetic DNA used for obtaining the cDNA encoding a portion of the precursor protein of rat homologue of ligand peptide to GPR8.

[SEQ ID NO: 61]

This shows the synthetic DNA used for obtaining the cDNA encoding a portion of the precursor protein of rat homologue of ligand peptide to GPR8.

[SEQ ID NO: 62]

This shows the cDNA sequence, which encodes a portion of the precursor protein of rat homologue of ligand peptide to GPR8.

[SEQ ID NO: 63]

This shows the synthetic DNA used for obtaining 5' upstream sequence of cDNA encoding the precursor protein of rat homologue of ligand peptide to GPR8.

[SEQ ID NO: 64]

This shows the synthetic DNA used for obtaining 5' upstream sequence of cDNA encoding the precursor protein of rat homologue of ligand peptide to GPR8.

[SEQ ID NO: 65]

This shows the 5' upstream DNA sequence of cDNA encoding the precursor protein of rat homologue of ligand peptide to GPR8.

[SEQ ID NO: 66]

This shows the synthetic DNA used for obtaining 3' downstream sequence of cDNA encoding the precursor protein of rat homologue of ligand peptide to GPR8.

[SEQ ID NO: 67]

This shows the synthetic DNA used for obtaining 3' downstream sequence of cDNA encoding the precursor protein of rat homologue of ligand peptide to GPR8.

[SEQ ID NO: 68]

This shows the 3' downstream DNA sequence of cDNA encoding the precursor protein of rat homologue of ligand peptide to GPR8.

[SEQ ID NO: 69]

This shows the synthetic DNA used for obtaining the cDNA encoding the precursor protein of rat homologue of ligand peptide to GPR8.

[SEQ ID NO: 70]

This shows the synthetic DNA used for obtaining the cDNA encoding the precursor protein of rat homologue of ligand peptide to GPR8.

[SEQ ID NO: 71]

This shows the cDNA sequence, which encodes the precursor protein of rat homologue of ligand peptide to GPR8.

[SEQ ID NO: 72]

This shows the amino acid sequence of the precursor protein of rat homologue of ligand peptide to GPR8.

[SEQ ID NO: 73]

This shows the amino acid sequence of rat homologue of ligand peptide to GPR8 deduced from SEQ ID NO: 72.

[SEQ ID NO: 74]

This shows the amino acid sequence of rat homologue of ligand peptide to GPR8 deduced from SEQ ID NO: 72.

[SEQ ID NO: 75]

This shows the base sequence encoding the amino acid sequence represented by SEQ ID NO: 73.

[SEQ ID NO: 76]

This shows the base sequence encoding the amino acid sequence represented by SEQ ID NO: 74.

[SEQ ID NO: 77]

This shows the sequence of mouse genome fragment supposed to be coded for a portion of the precursor protein of mouse homologue of ligand peptide to GPR8.

[SEQ ID NO: 78]

This shows the synthetic DNA used in the screening of the cDNA encoding a portion of the precursor protein of mouse homologue of ligand peptide to GPR8.

[SEQ ID NO: 79]

This shows the synthetic DNA used in the screening of the cDNA encoding a portion of the precursor protein of mouse homologue of ligand peptide to GPR8.

[SEQ ID NO: 80]

This shows the DNA sequence encoding a portion of the precursor protein of human homologue of ligand peptide to GPR8, which was amplified from cDNA derived from mouse testis.

[SEQ ID NO: 81]

This shows the synthetic DNA used for obtaining 5' upstream sequence of cDNA encoding the precursor protein of mouse homologue of ligand peptide to GPR8.

[SEQ ID NO: 82]

This shows the synthetic DNA used for obtaining 5' upstream sequence of cDNA encoding the precursor protein of mouse homologue of ligand peptide to GPR8.

[SEQ ID NO: 83]

This shows the 5' upstream DNA sequence of cDNA encoding the precursor protein of mouse homologue of ligand peptide to GPR8.

[SEQ ID NO: 84]

This shows the synthetic DNA used for obtaining 3' downstream sequence of cDNA encoding the precursor protein of mouse homologue of ligand peptide to GPR8.

[SEQ ID NO: 85]

This shows the synthetic DNA used for obtaining 3' downstream sequence of cDNA encoding the precursor protein of mouse homologue of ligand peptide to GPR8.

[SEQ ID NO: 86]

This shows the 3' downstream DNA sequence of cDNA encoding the precursor protein of mouse homologue of ligand peptide to GPR8.

[SEQ ID NO: 87]

This shows the synthetic DNA used for obtaining the cDNA encoding the precursor protein of mouse homologue of ligand peptide to GPR8.

[SEQ ID NO: 88]

This shows the synthetic DNA used for obtaining the cDNA encoding the precursor protein of mouse homologue of ligand peptide to GPR8.

[SEQ ID NO: 89]

This shows the cDNA sequence encoding the precursor protein of mouse homologue of ligand peptide to GPR8.

[SEQ ID NO: 90]

This shows the amino acid sequence of the precursor protein of mouse homologue of ligand peptide to GPR8.

[SEQ ID NO: 91]

This shows the amino acid sequence of mouse homologue of ligand peptide to GPR8 deduced from SEQ ID NO: 90.

[SEQ ID NO: 92]

This shows the amino acid sequence of mouse homologue of ligand peptide to GPR8 deduced from SEQ ID NO: 90.

[SEQ ID NO: 93]

This shows the base sequence encoding the amino acid sequence represented by SEQ ID NO: 91.

[SEQ ID NO: 94]

This shows the base sequence encoding the amino acid sequence represented by SEQ ID NO: 92.

[SEQ ID NO: 95]

This shows the amino acid sequence of human GPR8 ligand (1-23) oxidant, which was synthesized in Reference Example 40 described below.

[SEQ ID NO: 96]

This shows the amino acid sequence of human GPR8 ligand (1-22), which was synthesized in Reference Example 41 described below.

[SEQ ID NO: 97]

This shows the amino acid sequence of human GPR8 ligand (1-21), which was synthesized in Reference Example 42 described below.

[SEQ ID NO: 98]

This shows the amino acid sequence of human GPR8 ligand (1-20), which was synthesized in Reference Example 43 described below.

[SEQ ID NO: 99]

This shows the amino acid sequence of human GPR8 ligand (1-19), which was synthesized in Reference Example 44 described below.

[SEQ ID NO: 100]

This shows the amino acid sequence of human GPR8 ligand (1-18), which was synthesized in Reference Example 45 described below.

[SEQ ID NO: 101]

This shows the amino acid sequence of human GPR8 ligand (1-17), which was synthesized in Reference Example 46 described below.

[SEQ ID NO: 102]

This shows the amino acid sequence of human GPR8 ligand (1-16), which was synthesized in Reference Example 47 described below.

[SEQ ID NO: 103]

This shows the amino acid sequence of porcine GPR8 ligand (1-23) oxidant, which was synthesized in Reference Example 50 described below.

[SEQ ID NO: 104]

This shows the amino acid sequence of rat or mouse GPR8 ligand (1-23) oxidant, which was synthesized in Reference Example 51 described below.

[SEQ ID NO: 105]

This shows the amino acid sequence of human GPR8 ligand (1-23) modified by Fmoc, which was synthesized in Reference Example 12 described below.

[SEQ ID NO: 106]

This shows the amino acid sequence of [$N^{\alpha}$-Acetyl-$Trp^1$]-human GPR8 ligand (1-23) oxidant, which was synthesized in Reference Example 50 described below.

[SEQ ID NO: 107]

This shows the amino acid sequence of human GPR8 ligand (2-23), which was synthesized in Reference Example 53 described below.

[SEQ ID NO: 108]

This shows the amino acid sequence of human GPR8 ligand (4-23), which was synthesized in Reference Example 54 described below.

[SEQ ID NO: 109]

This shows the amino acid sequence of human GPR8 ligand (9-23), which was synthesized in Reference Example 55 described below.

[SEQ ID NO: 110]

This shows the amino acid sequence of human GPR8 ligand (15-23), which was synthesized in Reference Example 56 described below.

[SEQ ID NO: 111]

This shows the amino acid sequence of [N-Acetyl-$Tyr^2$]-human GPR8 ligand (2-23), which was synthesized in Reference Example 57 described below.

[SEQ ID NO: 112]

This shows the amino acid sequence of [D-$Trp^1$]-human GPR8 ligand (1-23), which was synthesized in Reference Example 58 described below.

[SEQ ID NO: 113]

This shows the amino acid sequence of [N-3-Indolepropanoyl-$Tyr^2$]-human GPR8 ligand (2-23), which was synthesized in Reference Example 59 described below.

[SEQ ID NO: 114]

This shows the base sequence encoding the amino acid sequence represented by SEQ ID NO: 96.

[SEQ ID NO: 115]

This shows the base sequence encoding the amino acid sequence represented by SEQ ID NO: 97.

[SEQ ID NO: 116]

This shows the base sequence encoding the amino acid sequence represented by SEQ ID NO: 98.

[SEQ ID NO: 117]

This shows the base sequence encoding the amino acid sequence represented by SEQ ID NO: 99.

[SEQ ID NO: 118]

This shows the base sequence encoding the amino acid sequence represented by SEQ ID NO: 100.

[SEQ ID NO: 119]

This shows the base sequence encoding the amino acid sequence represented by SEQ ID NO: 101.

[SEQ ID NO: 120]

This shows the base sequence encoding the amino acid sequence represented by SEQ ID NO: 102.

[SEQ ID NO: 121]

This shows the base sequence encoding the amino acid sequence represented by SEQ ID NO: 107.

[SEQ ID NO: 122]

This shows the base sequence encoding the amino acid sequence represented by SEQ ID NO: 108.

[SEQ ID NO: 123]

This shows the base sequence encoding the amino acid sequence represented by SEQ ID NO: 109.

[SEQ ID NO: 124]

This shows the base sequence encoding the amino acid sequence represented by SEQ ID NO: 110.

[SEQ ID NO: 125]

This shows the base sequence encoding the amino acid sequence represented by SEQ ID NO: 14.

[SEQ ID NO: 126]

This shows the base sequence encoding the amino acid sequence represented by SEQ ID NO: 8.

[SEQ ID NO: 127]

This shows the base sequence encoding the amino acid sequence represented by SEQ ID NO: 9.

[SEQ ID NO: 128]

This shows the amino acid sequence of human GPR ligand (1-29), which was synthesized in Reference Example 16 described below.

[SEQ ID NO: 129]

This shows the amino acid sequence of human GPR ligand (1-28), which was synthesized in Reference Example 17 described below.

[SEQ ID NO: 130]

This shows the amino acid sequence of human GPR ligand (1-27), which was synthesized in Reference Example 18 described below.

[SEQ ID NO: 131]

This shows the amino acid sequence of human GPR ligand (1-26), which was synthesized in Reference Example 19 described below.

[SEQ ID NO: 132]

This shows the base sequence of the primer used in the PCR reaction of Example 10 below.

[SEQ ID NO: 133]

This shows the base sequence of the primer used in the PCR reaction of Example 10 below.

[SEQ ID NO: 134]

This shows the base sequence of 5' upstream end of the DNA encoding mouse TGR26, which was obtained in Example 10 below.

[SEQ ID NO: 135]

This shows the base sequence of the primer used in the PCR reaction of Example 11 below.

[SEQ ID NO: 136]

This shows the base sequence of the primer used in the PCR reaction of Example 11 below.

[SEQ ID NO: 137]

This shows the base sequence of the cDNA encoding mouse TGR26, which was obtained in Example 10 below.

[SEQ ID NO: 138]

This shows the amino acid sequence of TGR26, the mouse-derived novel G protein-coupled receptor protein of the present invention.

[SEQ ID NO: 139]

This shows the base sequence of the cDNA encoding TGR26, the mouse-derived novel G protein-coupled receptor protein of the present invention.

[SEQ ID NO: 140]

This shows the amino acid sequence of human GPR7 ligand A.

[SEQ ID NO: 141]

This shows the amino acid sequence of mouse GPR7 ligand A.

[SEQ ID NO: 142]

This shows the amino acid sequence of rat GPR7 ligand A.

[SEQ ID NO: 143]

This shows the amino acid sequence of human GPR7 ligand B.

[SEQ ID NO: 144]

This shows the amino acid sequence of mouse GPR7 ligand B.

[SEQ ID NO: 145]

This shows the amino acid sequence of rat GPR7 ligand B.

[SEQ ID NO: 146]

This shows the amino acid sequence of human GPR7 ligand C.

[SEQ ID NO: 147]

This shows the amino acid sequence of human GPR7 ligand D.

[SEQ ID NO: 148]

This shows the amino acid sequence of mouse GPR7 ligand C.

[SEQ ID NO: 149]

This shows the amino acid sequence of mouse GPR7 ligand D.

[SEQ ID NO: 150]

This shows the amino acid sequence of rat GPR7 ligand C.

[SEQ ID NO: 151]

This shows the amino acid sequence of rat GPR7 ligand D.

[SEQ ID NO: 152]

This shows the amino acid sequence of human GPR7 ligand E.

[SEQ ID NO: 153]

This shows the amino acid sequence of mouse GPR7 ligand E.

[SEQ ID NO: 154]

This shows the amino acid sequence of rat GPR7 ligand E.

[SEQ ID NO: 155]

This shows the amino acid sequence of human GPR7 ligand F.

[SEQ ID NO: 156]

This shows the amino acid sequence of mouse GPR7 ligand F.

[SEQ ID NO: 157]

This shows the amino acid sequence of rat GPR7 ligand F.

[SEQ ID NO: 158]

This shows the base sequence of the DNA encoding human GPR7 ligand A.

[SEQ ID NO: 159]

This shows the base sequence of the DNA encoding mouse GPR7 ligand A.

[SEQ ID NO: 160]

This shows the base sequence of the DNA encoding rat GPR7 ligand A.

[SEQ ID NO: 161]

This shows the base sequence of the DNA encoding human GPR7 ligand B.

[SEQ ID NO: 162]

This shows the base sequence of the DNA encoding mouse GPR7 ligand B.

[SEQ ID NO: 163]

This shows the base sequence of the DNA encoding rat GPR7 ligand B.

[SEQ ID NO: 164]

This shows the base sequence of the DNA encoding human GPR7 ligand C.

[SEQ ID NO: 165]

This shows the base sequence of the DNA encoding human GPR7 ligand D.

[SEQ ID NO: 166]

This shows the base sequence of the DNA encoding mouse GPR7 ligand C.

[SEQ ID NO: 167]

This shows the base sequence of the DNA encoding mouse GPR7 ligand D.

[SEQ ID NO: 168]

This shows the base sequence of the DNA encoding rat GPR7 ligand C.

[SEQ ID NO: 169]

This shows the base sequence of the DNA encoding rat GPR7 ligand D.

[SEQ ID NO: 170]

This shows the base sequence of the DNA encoding human GPR7 ligand E.

[SEQ ID NO: 171]

This shows the base sequence of the DNA encoding mouse GPR7 ligand E.

[SEQ ID NO: 172]

This shows the base sequence of the DNA encoding rat GPR7 ligand E.

[SEQ ID NO: 173]

This shows the base sequence of the DNA encoding human GPR7 ligand F.

[SEQ ID NO: 174]

This shows the base sequence of the DNA encoding mouse GPR7 ligand F.

[SEQ ID NO: 175]

This shows the base sequence of the DNA encoding rat GPR7 ligand F.

[SEQ ID NO: 176]

This shows the amino acid sequence of the human GPR7 ligand precursor containing a secretory signal.

[SEQ ID NO: 177]

This shows the amino acid sequence of the mouse GPR7 ligand precursor containing a secretory signal.

[SEQ ID NO: 178]

This shows the amino acid sequence of the rat GPR7 ligand precursor containing a secretory signal.

[SEQ ID NO: 179]

This shows the base sequence of the DNA encoding the human GPR7 ligand precursor containing a secretory signal, which was obtained in Reference Example 61.

[SEQ ID NO: 180]

This shows the base sequence of the DNA encoding the mouse GPR7 ligand precursor containing a secretory signal, which was obtained in Reference Example 62.

[SEQ ID NO: 181]

This shows the base sequence of the DNA encoding the rat GPR7 ligand precursor containing a secretory signal, which was obtained in Reference Example 63.

[SEQ ID NO: 182]

This shows the amino acid sequence of human GPR7.

[SEQ ID NO: 183]

This shows the base sequence of the DNA encoding human GPR7.

[SEQ ID NO: 184]

This shows the synthetic DNA used in Reference Example 61.

[SEQ ID NO: 185]

This shows the synthetic DNA used in Reference Example 61.

[SEQ ID NO: 186]

This shows the synthetic DNA used in Reference Example 62.

[SEQ ID NO: 187]

This shows the synthetic DNA used in Reference Example 62.

[SEQ ID NO: 188]

This shows the synthetic DNA used in Reference Example 63.

[SEQ ID NO: 189]

This shows the synthetic DNA used in Reference Example 63.

[SEQ ID NO: 190]

This shows the base sequence of the primer used in Reference Example 60.

[SEQ ID NO: 191]

This shows the base sequence of the primer used in Reference Example 60.

The transformant Escherichia coli DH10B/pAK-rGPR7 obtained in Example 1 described below was on deposit with Institute for Fermentation (IFO), located at 2-17-85 Juso-honmachi, Yodogawa-ku, Osaka-shi, Osaka, Japan, as the Accession Number IFO 16496 on October 31, 2000, and with International Patent Organisms Depository, National Institute of Advanced Industrial Science and Technology (formerly, National Institute of Bioscience and Human-Technology (NIBH), Ministry of International Trade and Industry), located at Central 6, 1-1-1 Higashi, Tsukuba, Ibaraki, 305-8566, Japan, as the Accession Number FERM BP-7365 on November 13, 2000.

The transformant Escherichia coli TOP10/pCR2.1-TOPO Mouse GPR7 obtained in Example 11 described below was on deposit with Institute for Fermentation (IFO), located at 2-17-85 Juso-honmachi, Yodogawa-ku, Osaka-shi, Osaka, Japan, as the Accession Number IFO 16704 on September 20, 2001, and with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7775 on October 15, 2001.

The transformant Escherichia coli DH5α/pAKKO-GPR8 obtained in Reference Example 3 described below was on deposit with Institute for Fermentation (IFO), located at 2-17-85 Juso-honmachi, Yodogawa-ku, Osaka-shi, Osaka, Japan, as the Accession Number IFO 16564 on February 27, 2001, and with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7540 on April 11, 2001.

The transformant Escherichia coli TOP10/pCR2.1-TOPO Human GPR8 Ligand Precursor obtained in Reference Example 25 described below was on deposit with Institute for Fermentation (IFO), located at 2-17-85 Juso-honmachi, Yodogawa-ku, Osaka-shi, Osaka, Japan, as the Accession Number IFO 16568 on February 27, 2001, and with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7544 on April 11, 2001.

The transformant Escherichia coli TOP10/pCR2.1-TOPO Porcine GPR8 Ligand Precursor obtained in Reference Example 29 described below was on deposit with Institute for Fermentation (IFO), located at 2-17-85 Juso-honmachi, Yodogawa-ku, Osaka-shi, Osaka, Japan, as the Accession Number IFO 16565 on February 27, 2001, and with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7541 on April 11, 2001.

The transformant Escherichia coli TOP10/pCR2.1-TOPO Rat GPR8 Ligand Precursor obtained in Reference Example 33 described below was on deposit with Institute for Fermentation (IFO), located at 2-17-85 Juso-honmachi, Yodogawa-ku, Osaka-shi, Osaka, Japan, as the Accession Number IFO 16567 on February 27, 2001, and with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7543 on April 11, 2001.

The transformant Escherichia coli TOP10/pCR2.1-TOPO Mouse GPR8 Ligand Precursor obtained in Reference Example 38 described below was on deposit with Institute for Fermentation (IFO), located at 2-17-85 Juso-honmachi, Yodogawa-ku, Osaka-shi, Osaka, Japan, as the Accession Number IFO 16566 on February 27, 2001, and with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7542 on April 11, 2001.

The transformant Escherichia coli JM109/pTAhGPR7-1 obtained in Reference Example 61 described below was as Escherichia coli JM109/pTAhGPR7L-1 on deposit with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7640 on June 27, 2001, and with Institute for Fermentation (IFO), located at 2-17-85 Juso-honmachi, Yodogawa-ku, Osaka-shi, Osaka, Japan, as the Accession Number IFO 16644 on June 19, 2001.

The transformant Escherichia coli JM109/pTAmGPR7-1 obtained in Reference Example 62 described below was as Escherichia coli JM109/pTAmGPR7L-1 on deposit with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7641 on June 27, 2001, and with Institute for Fermentation (IFO), located at 2-17-85 Juso-honmachi, Yodogawa-ku, Osaka-shi, Osaka, Japan, as the Accession Number IFO 16656 on June 19, 2001.

The transformant Escherichia coli JM109/pTArGPR7-1 obtained in Reference Example 63 described below was as Escherichia coli JM109/pTArGPR7L-1 on deposit with International Patent Organisms Depository, National Institute of Advanced Industrial Science and Technology, located at Central 6, 1-1-1 Higashi, Tsukuba, Ibaraki, 305-8566, Japan, as the Accession Number FERM BP-7642 on June 27, 2001, and with Institute for Fermentation (IFO), located at 2-17-85 Juso-honmachi, Yodogawa-ku, Osaka-shi, Osaka, Japan, as the Accession Number IFO 16657 on June 19, 2001.

EXAMPLES

The present invention is described in detail below with reference to EXAMPLES and REFERENCE EXAMPLES, but is not deemed to limit the scope of the present invention thereto. The gene manipulation procedures using Escherichia coli were performed according to the methods described in the Molecular Cloning.

EXAMPLE 1

Cloning of the cDNA encoding the rat whole brain-derived G protein-coupled receptor protein and determination of the base sequence

Using rat whole brain-derived cDNA (CLONTECH) as a template and two primers, namely, primer 1 (SEQ ID NO: 3) and primer 2 (SEQ ID NO: 4), which were designed based on the base sequence of the DNA encoding human GPR8, PCR reaction was carried out. The reaction solution in the above reaction comprised of 1/10 volume of the cDNA as a template, 1/50 volume of Advantage-2 cDNA Polymerase Mix (CLONTECH), 0.2 μM of primer 3, 0.2 μM of primer 2, 200 μM of dNTPs, and a buffer attached to the enzyme to make the total volume 25 μl. The PCR reaction was carried out (i) by reaction of 94°C for 2 minutes, then (ii) a cycle set to include 94°C for 20 seconds followed by 72°C for 2 minutes, which was repeated 3 times, (iii) 94°C for 20 seconds followed by 66°C for 20 seconds and 68°C for 2 minutes, which was repeated 3 times, (iv) 94°C for 20 seconds followed by 60°C for 20 seconds and 68°C for 2 minutes, which was repeated 36 times, and finally, extension reaction at 68°C for 7 minutes. The PCR product was subcloned to plasmid vector pCR2.1-TOPO (Invitrogen) following the instructions attached to the TA Cloning Kit (Invitrogen). The plasmid was then introduced into Escherichia coli DH5α, and the clones containing the cDNA were selected on LB agar plates containing ampicillin. As a result of analysis for sequence of each clone, a base sequence of the cDNA encoding the novel G protein-coupled receptor protein was obtained (SEQ ID NO: 2). The novel G protein-coupled receptor protein containing the amino acid sequence (SEQ ID NO: 1) encoded by the base sequence of this DNA was designated TGR26 (FIG. 1 and FIG. 2).

The amino acid sesquence represented by SEQ ID NO: 1 has a 84.8% homology to GPR7, the well known human G protein-coupled receptor protein [Genomics, Vol. 28, 84-91, 1995].

One clone of the aforementioned transformants harboring a plasmid, in which the DNA encoding TGR26 was inserted, was selected, cultured in LB medium containing ampicillin with shaking. As a result, the plasmid was obtained. The plasmid was treated with restriction enzymes ClaI and SpeI, and the insert encoding TGR26 was excised. Using pAKKO-1.11 H treated with restrictionenzymes ClaI and SpeI in a similar manner and Ligaation Expression Kit (CLONTECH), the insert was ligated, and the plasmid obtained was introduced into Escherichia coli DH10B by electroporation. A structure of the plasmid used for construction of cells for expression, in which the obtained clone contained, was confirmed by treatment of restriction enzymes and by analysis of the sequence. Then, the clone was designated Escherichia coli DH10B/pAK-rGPR7.

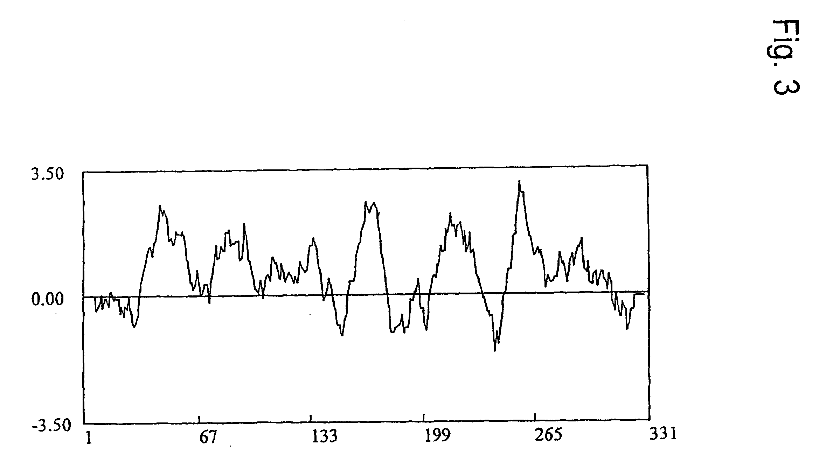

The hydrophobicity plot of TGR26 is indicated in FIG. 3.

EXAMPLE 2

Preparation of TGR26 expressing CHO cells

After completion of culture of Escherichia coli DH5α (TOYOBO) transformed with the expression plasmid pAK-rGPR7 described in Example 1, pAK-rGPR7 plasmid DNA was prepared using Plasmid Midi Kit (QIAGEN). This plasmid was introduced into CHO dhfr$^-$ cells using CellPhect Transfection Kit (Amersham Pharmacia Biotech) in accordance with the attached protocol. Co-precipitate suspension of 5 μg of DNA with calcium phosphate was prepared and added to each of two 6cm-diameter dishes, on which 3x$10^5$ CHO dhfr$^-$ cells were seeded before 48 hours. The cells were cultivated in MEMα medium containing 10% fetal bovine serum for one day and were subcultured. Subsequently, the cells were cultured in the selection medium, MEMα medium containing 10% dialyzed fetal bovine serum and no nucleic acid. The 44 clones of transformant colonies that are TGR26 expressing CHO cells grown in the selection medium were selected.

EXAMPLE 3

Quantification of an expression level of TGR26 in TGR26 expressing CHO cell line using TaqMan PCR method

The 44 clones of TGR26 expressing cell line obtained in Example 2 were cultured in 25 cm$^2$ flask, respectively. After preparation of total RNA using Rneasy Mini Kits (Qiagen), RNA was treated with DNase using RNase-free DNase Set (Qiagen). Four micrograms of total RNA obtained was dissolved in 12 μl of solution containing 500pmol of random primers (Takara Shuzo), was treated at 70°C for 10 minutes and was chilled on ice. Further, to the mixture, 1 x First Strand Buffer, 10 mM DTT, 500 μM dA/dC/dG/dTTP and 200 units of SUPERSCRIPT II (GIBCO) were added. Subsequently, for 20 μl of the mixture, reverse transcription was carried out by treatment at 30°C for 10 minutes, followed by 42°C for 60 minutes, 51°C for 30 minutes and 70°C for 15 minute. For 25 μl of the reaction mixture containing the obtained reverse transcripts corresponding to 5 ng of total RNA or 10 to 1 x $10^7$ copies of standard cDNA prepared by the method described below, 1 x Universal PCR Master Mix (PE Biosystems), 100 nM each of primers represented by SEQ ID NO: 5 and SEQ ID NO: 6, and 100 nM TaqMan probe represented by SEQ ID NO: 7 (Fam-tcctctgctg gacaccgtac cacctga-Tamra; in the sequence, Fam and Tamra represent 6-carboxy-fluorescein and 6-carboxy-tetramethyl-rhodamine, respectively), using ABI PRISM 7700 Sequence Detector (PE Biosystems), PCR was carried out. PCR was performed by heating of 50°C for 2 minutes and 95°C for 10 minutes, then a cycle set to include 95°C for 15 seconds followed by 60°C for 60 seconds, which was repeated 40 times.

The standard cDNA was prepared by PCR amplification, wherein the PCR was performed using 200 μl of the reaction mixture containing 100 pg of TGR26 expressing plasmid DNA (pAK-rGPR7), 500 nM each of primers represented by SEQ ID NO: 5 and SEQ ID NO: 6, 1 x PCR Gold Buffer, 2.5 mM $MgCl_2$, 200 μM dA/dC/dG/dTTP and 200 units of AmpliTaq Gold (PE Biosystems) and GeneAmp PCR System 9700 (PE Biosystems), by treatment at 95°C for 10 minutes, then a cycle set to include 95°C for 10 seconds followed by 63°C for 15 seconds and 72°C for 10 seconds, which was repeated 40 times. Concentration of the synthetic cDNA purified using QIAquick PCR Purification Kit (Qiagen) was calculated by measurement of absorbance at 260 nm. Further, accurate copy number of standard cDNA was calculated, and subsequently, standard cDNA solution was prepared at the concentration of 1 x $10^8$ copies/μl by dilution with 10 mM Tris-HCl (pH8.0) containing 1 mM EDTA. Probe and primers for TaqMan PCR were designed by using Primer Express (Version 1.0) (PE Biosystems).

The expression level was calculated by using ABI PRISM 7700 SDS Software. Cycle numbers at the moment when fluorescent intensity of reporter comes to preset values indicated as a vertical axis, and logarithm of an initial concentration of the standard cDNA as a horizontal axis. Subsequently, a standard curve was prepared. By calculating an initial concentration of each reverse transcript from a standard curve, an expression level of TGR26 gene per total RNA of each clone was estimated. As a result, it was found that clone numbers 18 and 28 of the TGR26 expressing cell line exhibit high expression level. Hereafter, these two clones of expression cells were utilized in experiments.

## EXAMPLE 4

### Assay for a level of intracellular cAMP production using TGR26 expressing CHO cells

TGR26 expressing CHO cells prepared in Example 3 were seeded on 24-well plate at 5x$10^4$ cells/well andcultivated for 48 hours. The cells were washed with MEMα buffer (pH7.4) containing 0.2 mM 3-isobutyl-methylxanthine, 0.05% BSA (bovine serum albumine) and 20 mM HEPES [hereafter, MEMα buffer (pH7.4) containing 0.2 mM 3-isobutylmethylxanthine, 0.05% BSA (bovine serum albumine) and 20 mM HEPES may be referred to as reaction buffer]. Then, 0.5 ml of the reaction buffer was added to the cells and the suspension was incubated for 30 minutes in the incubator. After removal of the reaction buffer and newly adding 0.25 ml of the reaction buffer to the cells, an appropriate concentration of sample in DMSO solution and 0.25 ml of the reaction buffer containing 2 μM forskolin were added to the cells and the mixture was reacted at 37°C for 30 minutes. For termination of the reaction, 100 μl of 20% perchloric acid was added. Subsequently, the mixture was stood on ice for an hour to extract intracellular CAMP. The cAMP level in the extract was measured using the cAMP EIA Kit (Amersham Pharmacia Biotech).

## EXAMPLE 5

### Inhibiting activity for intracellular cAMP production of human homologue of GPR8 ligand peptide consisting of 23 or 30 residues, which was measured using TGR26 expressing CHO cells

The human homologue of GPR8 ligand peptide consisting of 23 residues represented by SEQ ID NO: 8, which was obtained in Reference Example 12 (hereafter, sometimes referred to as hGPR8L (1-23)), or the human homologue of GPR8 ligand peptide consisting of 30 residues represented by SEQ ID NO: 8, which was obtained in Reference Example 13 (hereafter, sometimes referred to as hGPR8L (1-30)) was administered at various concentrations to membrane fraction of the TGR26 expressing CHO cells by the method described in Example 4, and an inhibiting activity of intracellular cAMP production was measured.

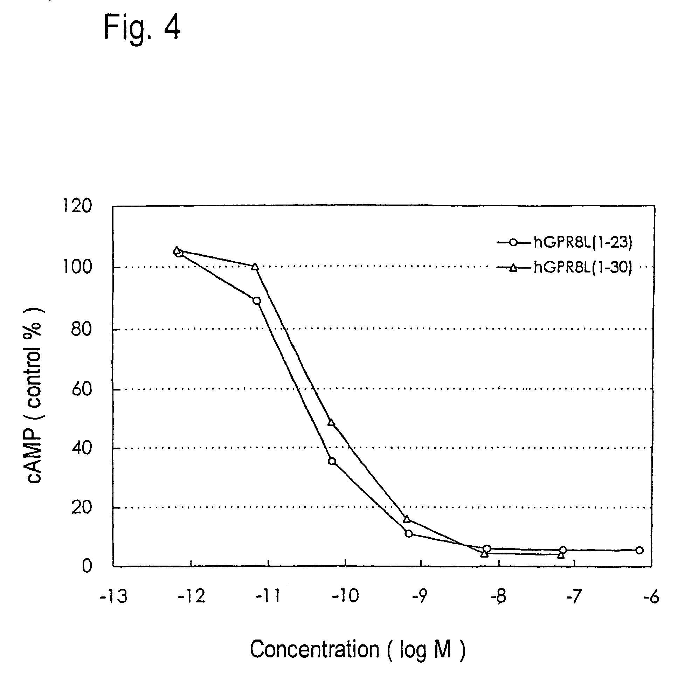

The result was shown in FIG. 4.

From this result, hGPR8L (1-23) and hGPR8L (1-30) apparently inhibited intracellular cAMP production of TGR26 expressing CHO cells depending on the concentration.

In the figure, when an amount subtracted intracellular cAMP level with the reaction buffer from intracellular cAMP level with the reaction buffer containing forskolin is indicated as 100%, the inhibiting activity of cAMP synthesis is indicated as an amount subtracted intracellular cAMP level with hGPR8L (1-23) or hGPR8L (1-30) from intracellular cAMP level with the reaction buffer by representation of percentage (%).

From this result, it became clear that hGPR8L (1-23) or hGPR8L (1-30) is a ligand to TGR26.

When porcine-, rat- and mouse- homologues (SEQ ID NO: 56, SEQ ID NO: 73 and SEQ ID NO: 91) of hGPR8L (1-23) and porcine-, rat- and mouse- homologues (SEQ ID NO: 57, SEQ ID NO: 74 and SEQ ID NO: 92) of hGPR8L (1-30) were also used, as described above, it can be confirmed that intracellular cAMP production of the TGR26 expressing CHO cells was inhibited depending on the concentration.

EXAMPLE 6

Assay for a GTPyS binding activity using a membrane fraction of TGR26 expressing CHO cells

Promoting activity for [$^{35}$S]-guanosine 5'-(γ-thio) triphosphate (GTPγS) binding to a membrane fraction of TGR26 expressing cells was assayed in accordance with the following method.

1) Preparing method of membrane fraction

To 1 x $10^8$ of TGR26 expressing CHO cells, 10 ml of homogenating buffer (10 mM $NaHCO_3$, 5 mM EDTA, 0.5 mM PMSF (phenylmethylsulfonyl fluoride), 1 μg/ml Pepstatin, 4 μg/ml E-64, 20 μg/ml Leupeptin) was added, and the cells were disrupted using Polytron (12,000 rpm, 1 minute). Supernatant was obtained from the disrupted cell suspension by centrifugation (1,000 g, 15 minutes). In addition, the supernatant was ultracentrifuged (Beckman Type 30 rotor, 30,000 rpm, 1 hour), and precipitate obtained was kept as a membrane fraction of TGR26 expressing CHO cells.

2) Measurement of GTPyS binding activity

The membrane fraction of TGR26 expressing CHO cells was diluted with membrane dilution buffer (50 mM Tris HCl buffer (pH7.4), 5 mM $MgCl_2$, 150 mM NaCl, 1 μM GDP, 0.1% BSA) to prepare a cell membrane fraction solution for assay at 30 μg/ml of protein concentration. To 200 μl of membrane fraction solution for assay, 2 μl of 50 nM [$^{35}$S]-guanosine 5'-(γ-thio) triphosphate (NEN) and 2 μl of sample/DMSO solution having an appropriate concentration were added. Then the mixture was incubated at 25°C for one hour. The mixture was filtrated, and further the filter was washed twice with 1.5 ml of washing buffer (50 mM Tris HCl buffer (pH7.4), 5 mM $MgCl_2$, 1 mM EDTA, 0.1% BSA). Finally, a radioactivity on the filter was determined by liquid scintillation counter.

EXAMPLE 7

Promoting activity for GTPγS binding of human homologue of GPR8 ligand peptide consisting of 23 or 30 residues, which was measured using a membrane fraction of TGR26 expressing CHO cells

Various concentration of hGPR8L (1-23) or hGPR8L (1-30) was mixed with a membrane fraction of the TGR26 expressing cell in accordance with the method described in Example 6, and a promoting activity of GTPγS binding was assayed.

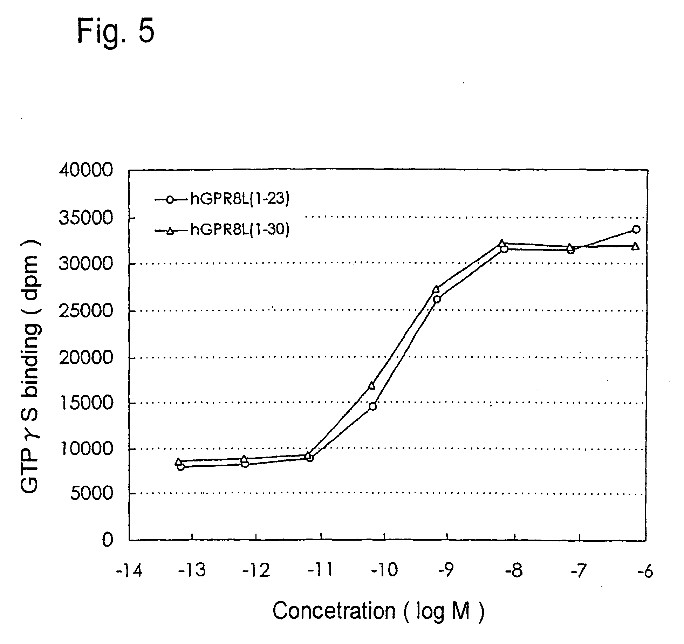

The result was shown in FIG. 5.

From this result, hGPR8L (1-23) and hGPR8L (1-30) apparently promoted GTPγS binding of TGR26 expressing CHO cells depending on the concentration.

When porcine-, rat- and mouse- homologues (SEQ ID NO: 56, SEQ ID NO: 73 and SEQ ID NO: 91) of hGPR8L (1-23) and porcine-, rat- and mouse- homologues (SEQ ID NO: 57, SEQ ID NO: 74 and SEQ ID NO: 92) of hGPR8L (1-30) were also used, as described above, it can be confirmed that GTPγS binding of the TGR26 expressing CHO cells was promoted depending on the concentration.

EXAMPLE 8

Experiment for receptor binding using [$^{125}$I-Tyr$^{10}$]-hGPR8L (1-23)

Using [$^{125}$I-Tyr$^{10}$]-hGPR8L (1-23) prepared by the method described in Reference Example 15 and a cell membrane fraction prepared from TGR26 expressing cells as described in Example 6, a receptor binding assay was carried out as follows.

Cell membrane fraction prepared from TGR26 expressing cells was diluted to various concentration with assay buffer (25 mM Tris-HCl, 5 mM EDTA, 0.05% CHAPS (3-[(3-Cholamidopropyl) Dimethyl-Ammonio]-1-Propanesulfonate), 0.1% BSA, 0.5 mM PMSF, 1 μg/ml Pepstatin, 20 μg/ml Leupeptin, 4 μg/ml E-64, pH7.4), and 200 μl each of the diluent was dispensed into polypropilene test tube (Falcon 2053). In order to determine an amount of maximum binding, 2 μl of DMSO and 2 μl of 7 nM [$^{125}$I-Tyr$^{10}$]-hGPR8L (1-23) were added to the membrane fraction solution. Further, in order to determine a non-specific binding, 2 μl of 100 μM hGPR8L (1-23)/DMSO solution and 2 μl of 7 nM [$^{125}$I-Tyr$^{10}$]-hGPR8L (1-23) were added to the membrane fraction solution. The reaction was done at 25°C for 75 minutes, and the reaction mixture was filtered by suction filtration using Whatman glassfilter (GF-F) treated with polyethyleneimine. In addition, the filter was washed twice with 1.5 ml of washing buffer (25 mM Tris-HCl, 5 mM EDTA, 0.05% CHAPS, 0.1% BSA, pH7.4). After filtration, a radioactivity remaining on the filter was counted using γ-counter, and an amount of specific binding was estimated by subtracting an amount of non-specific binding from an amount of maximum binding.

When the concentration of membrane fraction was changed, a specific binding of [$^{125}$I-Tyr$^{10}$]-hGPR8L (1-23) was perceived depending on the concentration of membrane fraction. Where the concentration of membrane fraction was set to 3 μg/ml, an inhibition for the specific binding of [$^{125}$I-Tyr$^{10}$]-hGPR8L (1-23) to the membrane fraction of TGR26 expressing cells by hGPR8L (1-23) and hGPR8L (1-30) was investigated. When concentration of 50% inhibition ($IC_{50}$ values) was calculated from the inhibition rate, it was found that the $IC_{50}$ values for hGPR8L (1-23) and hGPR8L (1-30) were 0.12 nM and 0.028 nM, respectively.

From this result, it was shown that both hGPR8L (1-23) and hGPR8L (1-30) have a high affinity for the membrane fraction of TGR26 expressing cells. That is, this means hGPR8L (1-23) and hGPR8L (1-30) is a high affinity ligand for TGR26 receptor.

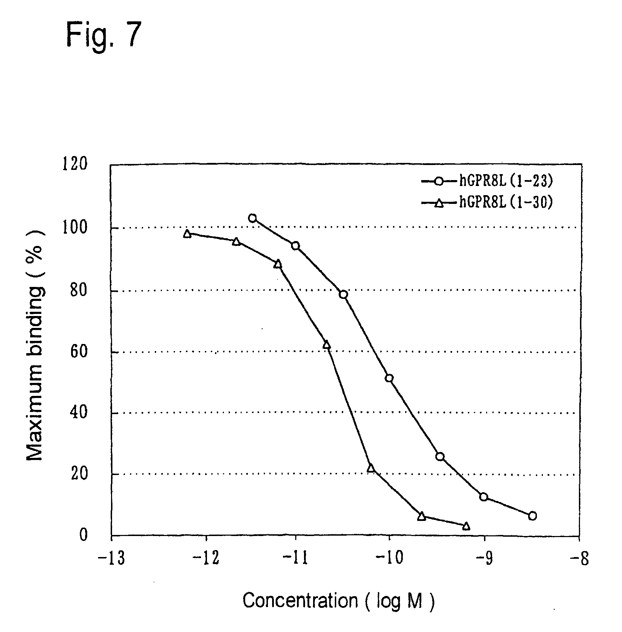

An inhibition of binding of hGPR8L (1-23) and hGPR8L (1-30) on the various concentration was shown in FIG. 7.

Using rat and mouse homologues of hGPR8L (1-23) (SEQ ID NO: 73 and SEQ ID NO: 91) and porcine, rat and mouse homologues of hGPR8L (1-30) (SEQ ID NO: 57, SEQ ID NO: 74 and SEQ ID NO: 92), as well as the above, an inhibition for the specific binding of [$^{125}$I-Tyr$^{10}$]-hGPRBL (1-23) to the membrane fraction of TGR26 expressing cells can also be confirmed.

EXAMPLE 9

Receptor binding activity of human- and porcine-homologue derivatives of GPR8 ligand peptide, which was measured using a membrane fraction of TGR26 expressing CHO cells and [$^{125}$I-Tyr$^{10}$]-hGPR8L (1-23)

A receptor binding activity of human- and porcine-homologue derivatives of GPR8 ligand peptide obtained in the reference examples was measured using a membrane fraction of TGR26 expressing CHO cells and [$^{125}$I-Tyr$^{10}$]-hGPR8L (1-23) by the method described in Example 8. SEQ ID NO of the derivatives measured and receptor binding activity thereof are shown in Table 1. The receptor binding activity is represented by concentration of 50% inhibition for binding ($IC_{50}$ value).

Table 1

| Derivatives | SEQ ID NO | receptor binding activity ($IC_{50}$ value) |
|---|---|---|
| (Met(O)]-hGPR8L (1-23) | 95 | 0.29 |
| Fmoc-hGPR8L (1-23) | 105 | 0.23 |
| Ac-hGPR8L (1-23) | 106 | 0.27 |
| [D-Trp$^1$]-hGPR8L (1-23) | 112 | 1.3 |
| hGPRBL (2-23) | 107 | 240 |
| Ac-hGPR8L (2-23) | 111 | 570 |
| IndPr-hGPR8L (2-23) | 113 | 0.12 |
| hGPR8L (4-23) | 108 | 2000 |
| hGPRBL (9-23) | 109 | 2500 |
| hGPR8L (1-20) | 98 | 0.17 |
| hGPR8L (1-19) | 99 | 9.9 |
| hGPR8L (1-18) | 100 | 760 |
| pGPR8L (1-23) | 24 | 0.12 |

Table 1 (continued)

| Derivatives | SEQ ID NO | receptor binding activity ($IC_{50}$ value) |
|---|---|---|
| [Met(O)]- pGPR8L (1-23) | 103 | 0.28 |

EXAMPLE 10

Cloning of 5' upstream end of the cDNA encoding mouse TGR26

By 5' RACE PCR cloning, a base sequence of 5' upstream region of cDNA encoding mouse TGR26 was elucidated.

The 5' RACE PCR cloning was accomplished by PCR reaction using mouse brain cDNA described in Reference Example 35 as a template, Universal Primer Mix attached to SMART™ RACE cDNAAmplification Kit, and synthetic primer represented by SEQ ID NO: 132 followed by PCR reaction using the above PCR reaction mixture as a template, Nested Universal Primer attached to the kit, and synthetic primer represented by SEQ ID NO: 133. The primers represented by SEQ ID NO: 132 and SEQ ID NO: 133 were designed based on the sequence of mouse GPR7 cDNA fragment registered on Genbank (Accession: U23807). The composition of the reaction solution and the conditions for PCR are as follows. The reaction solution comprised of 1 μl of mouse brain cDNA, 2 μl of Universal Primer Mix, 0.2 μM of synthetic DNA primer represented by SEQ ID NO: 132, 0.8 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 120 seconds, which was repeated 30 times, and finally, incubation at 72°C for 10 minutes. Subsequently, the reaction solution comprised of 0.5 μl of the above PCR reaction solution diluted to 50-fold with Tricine-EDTA Buffer attached to the kit, 0.5 μM of Nested Universal Primer, 0.5 μM of synthetic DNA primer represented by SEQ ID NO: 133, 0.8 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 30 seconds and 72°C for 60 seconds, which was repeated 30 times, and finally, incubation at 72°C for 10 minutes. After isolating the amplified DNA by 1.5% agarose gel electrophoresis, DNA having about 450 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 134, was obtained.

EXAMPLE 11

Cloning of the cDNA encoding mouse TGR26

Using the mouse brain-derived cDNA as a template and the following synthetic DNA primers, namely the synthetic primer shown by SEQ ID NO: 135 and the synthetic primer shown by SEQ ID NO: 136, amplification of mouse TGR26 DNA by PCR method was performed.

The reaction solution in the above reaction comprised of 1 μl of mouse brain cDNA, 0.2 μM each of synthetic DNA primers, 0.8 mM dNTPs, 0.4 μl of Advantage cDNA Polymerase Mix (CLONTECH) and a buffer attached to the enzyme to make the total volume 20 μl. The PCR reaction was carried out using a thermal cycler (Applied Biosystems) by heating of 96°C for 2 minutes, then a cycle set to include 96°C for 30 seconds followed by 64°C for 30 seconds and 72°C for 1 minute, which was repeated 30 times, and finally, extension reaction at 72°C for 10 minutes. The amplified DNA fragment in the PCR reaction mixture, of which is about 1100 bases, was cloned to pCR2.1-TOPO in accordance with the protocol of TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 137, was obtained.

Tlanslation from the base sequence represented by SEQ ID NO: 137 to an amino acid sequence was set as an amino acid sequence of mouse TGR26 and was represented by SEQ ID NO: 138.

When SEQ ID NO: 138 was compared with the amino acid sequence of the rat-derived TGR26 (SEQ ID NO: 1), it was found that 96.0% of amino acids are identical.

Escherichia coli transformed with a plasmid containing the DNA having the base sequence shown by SEQ ID NO: 137 was designated Escherichia coli TOP10/pCR2.1-TOPO mouse GPR7.

REFERENCE EXAMPLE 1

Amplification of human GPR8 cDNA by PCR method using human brain-derived cDNA

Using the human brain-derived poly(A)$^+$ RNA (CLONTECH) as a template and random primers, reverse transcription reaction was carried out. For the reverse transcription, reagents for RNA PCR ver 2.1 Kit (Takra Shuzo) were used. Subsequently, using the reverse transcript as a template and the synthetic DNA primers represented by SEQ ID NO: 10 and SEQ ID NO: 11, amplification by PCR method was performed. The synthetic primers were constructed to allow a region of the gene to be translated to the receptor protein to amplify. Therewith, at the 5' end of the gene, the base sequence recognized by restriction enzyme ClaI was added, and at the 3' end, the base sequence recognized by restriction enzyme SpeI was added. The reaction solution in the above reaction comprised of 5 μl of cDNA template, 0.4 μM each of synthetic DNA primers, 0.8 mM dNTPs, 0.5 μl of pfu Polymerase (STRATAGENE) and a buffer attached to the enzyme to make the total volume 50 μl. The PCR reaction was carried out using a thermal cycler (PE Biosystems) by heating of 94°C for 60 seconds, then a cycle set to include 94°C for 60 seconds followed by 65°C for 60 seconds and 72°C for 150 seconds, which was repeated 35 times. The amplified product was confirmed by 0.8% agarose gel electrophoresis follwed by ethidium bromide staining.

REFERENCE EXAMPLE 2

Subcloning of PCR product into plasmid vector and confirmation of amplified cDNA sequence by decoding a base sequence of the inserted cDNA region

Using the PCR reaction solution in Reference Example 1, DNA was isolated by 0.8% low melting agarose gel electrophoresis. The DNA band was excised from the gel with razor, and was recovered by crashing the pieces of agarose, phenol extraction, phenol-chroloform extraction and ethanol precipitation. In the manner prescribed in PCR-Script™ Amp SK(+) Cloning Kit (Stratagene), the recovered DNA was subcloned to plasmid vector pCR-Script Amp SK(+). After transformation of Escherichia coli DH5α competent cell (TOYOBO) by introducing the above-mentioned vector, clones harboring cDNA insert fragment was selected on LB agar medium containing ampicillin, IPTG and X-gal. All the white-colored clones were isolated with sterilized toothpick, and then the transformant E. coli DH5α/GPR8 was obtained. Respective clones were cultured in LB medium containing ampicillin for overnight. Subsequently, the plasmid DNA was prepared using QIAwell 8 Plasmid Kit (Qiagen). A portion of the prepared DNA was cleaved with the restriction enzymes ClaI and SpeI, and a size of the receptor cDNA fragment inserted was confirmed. The reaction for determination of the base sequence was carried out using DyeDeoxy Terminator Cycle Sequence Kit (PE Biosystems PE Biosystems). As a result, after decoding with the fluorescent automated sequencer, the DNA sequence represented by SEQ ID NO: 12, was obtained.

REFERENCE EXAMPLE 3

Preparation of GPR8 expressing CHO cells

Using Plasmid Midi Kit (Qiagen), plasmid DNA was prepared from clones of E. coli transformed by the plasmid encoding the human brain-derived GPR8 full-length amino acid sequence, which sequence was confirmed in Reference Example 2, with the ClaI recognition sequence added at the 5' side and with the SpeI recognition sequence added at the 3' side. The plasmid DNA was digested with restriction enzymes ClaI and SpeI to excise the insert part out. After electrophoresis, the insert DNA was excised from the agarose gel with a razor and then homogenized. The homogenate was extracted with phenol and then with phenol/chloroform, followed by precipitation in ethanol. Thus, the insert DNA was recovered. The insert DNA was added to ClaI- and SpeI-cleaved expression vector plasmid pAKK0-111H for animal cell (the same as the vector plasmid pAKK01.11G described in Hinuma, S. et al., Biochim. Biophys. Acta, Vol. 1219, pp. 251-259 (1994)) followed by ligation using T4 ligase (Takara Shuzo). Thus, plasmid pAKKO-GPR8 for protein expression was constructed.

After E. coli DH5α (TOYOBO) transformed by pAKKO-GPR8 was cultured, plasmid DNA of pAKKO-GPR8 was prepared using Plasmid Midi Kit (Qiagen). Using CellPhect Transfection Kit (Amersham Pharmacia Biotech Co.), the plasmid DNA was introduced into CHO dhfr⁻ cells in accordance with the protocol attached to the kit. The DNA, 4.5 μg, was co-precipitated with calcium phosphate in suspension. The resulting suspension was added to a 6 cm Petri dish in which $5 \times 10^5$ or $1 \times 10^6$ CHO dhfr⁻ cells had been seeded before 24 hours. The cells were cultured in MEMα containing 10% fetal calf serum for one day. After passage, the cells were cultured in nucleic acid-free selection medium MEMα containing 10% dialyzed fetal calf serum and 47 clones of the transformant GPR8 expressing CHO cells, growing in the selection medium, were selected.

REFERENCE EXAMPLE 4

Selection of CHO/GPR8 cell line, in which an expression level of mRNA for full length of human GPR8 protein is high

The expression level of mRNA of the full-length GPR8 protein of 47 clones from the CHO/GPR8 cell line established in Reference Example 3 was measured as follows using Cytostar T Plate (Amersham Pharmacia Biotech Co.), in accordance with the protocol attached thereto. Each clone of the CHO/GPR8 cell line was inoculated on Cytostar T Plate in 2.5 x $10^4$ cells/well. After culturing for 24 hours, the cells were fixed with 10% formalin. After 0.25% Triton X-100 was added to each well to increase cell permeability, $^{35}$S-labeled riboprobe represented by SEQ ID NO: 13 was added to the cells for hybridization. By adding 20 μg/ml RNaseA to each well, free riboprobe was digested. After the plate was thoroughly washed, radioactivity of the riboprobe hybridized was measured with Topcounter. The cell line with a high radioactivity provides a high expression amount of mRNA. Three clones (#17, #41 and #46) that showed high expression level of mRNA were used for the following experiment, especially clone #17 as a main clone.

REFERENCE EXAMPLE 5

Assay for a level of intracellular cAMP production using GPR8 expressing CHO cells

The CHO/GPR8 cells prepared in Reference Example 4 and mock CHO cells were inoculated on a 24-well plate in $5 \times 10^4$ cells/well followed by cultivation for 48 hours. The cells were then washed with Hanks' buffer (pH 7.4) containing 0.2 mM 3-isobutyl-methylxanthine, 0.05% BSA and 20 mM HEPES (hereinafter Hanks' buffer (pH 7.4) containing 0.2 mM 3-isobutyl-methylxanthine, 0.05% BSA and 20 mM HEPES is referred to as a reaction buffer). Thereafter, 0.5 ml of the reaction buffer was added to the system, which was kept in the incubator for 30 minutes. The reaction buffer was removed and 0.25 ml of a fresh reaction buffer was added to the cells. Then, 0.25 ml of the reaction buffer containing sample and 2 μM forskolin was added to the cells followed by reacting at 37°C for 24 minutes. By adding 100 μl of 20% perchloric acid, the reaction was terminated. The reaction mixture was then allowed to stand on ice for an hour to extract intracellular CAMP. The amount of cAMP in the extract was measured using cAMP EIA kit (Amersham Pharmacia Biotech).

REFERENCE EXAMPLE 6

Assay for a GTPγS binding activity using a membrane fraction of GPR8 expressing CHO cells

The binding promoting activity of [$^{35}$S]-guanosine 5'-(γ-thio) triphosphate to membrane fraction of the GPR8 expressing CHO cell was assayed by the following method. First, preparation of the membrane fraction is described. To 1 x $10^8$ CHO/GPR8 cells was added 10 ml of a homogenate buffer (10 mM $NaHCO_3$, 5 mM EDTA, 0.5 mM PMSF, 1 μg/ml pepstatin, 4 μg/ml E64, 20 μg/ml leupeptin), followed by cell disruption with a polytron (12,000 rpm, 1 minute). The disrupted cells were then centrifuged (1,000 g, 15 minutes) to give the supernatant. Next, the supernatant was subjected to ultracentrifugation (Beckman type 30 rotor, 30,000 rpm, 1 hour). The resulting precipitate was used as a membrane fraction of GPR8 expressing CHO cell.

The GTPγS binding activity was assayed as follows. The membrane fraction of the GPR8 expressing CHO cell was diluted with a buffer for membrane dilution (50 mM Tris-hydrochloride buffer (pH 7.4), 5 mM $MgCl_2$, 150 mM NaCl, 1 μM GDP) to make a cell membrane fraction solution for assay having a protein concentration of 30 mg/ml. To 200 μl of the cell membrane fraction solution for assay were added 2 μl of 51.5 nM [$^{35}$S]-guanosine 5'-(γ-thio) triphosphate (NEN) and sample. The resulting solution mixture was kept at 25°C for an hour. The mixture was filtrated through a filter. After washing twice with 1.5 ml of a washing buffer (50 mM Tris-hydrochloride buffer (pH 7.4), 5 mM $MgCl_2$, 1

mM EDTA, 0.1% BSA), radioactivity of the filter was measured using a liquid scintillation counter.

REFERENCE EXAMPLE 7

Detection of an activity exhibiting an inhibition of cAMP production and a promotion of GTPγS binding specific to CHO/GPR8 cell line, which is contained in porcine hypothalamus extract

Fractions of the porcine hypothalamus extract by high performance liquid chromatography (HPLC) were prepared by the method described below. Porcine hypothalamus, 500 g (corresponding to 30 pigs), which had been purchased from Tokyo Shibaura Zoki Co. and kept under ice cooling after the hypothalamus was withdrawn from porcine on the day of their sacrifice, was homogenized, immediately put into 2.0 liters of boiling distilled water and boiled for 10 minutes. Immediately after the boiling, the homogenate was ice-cooled and 120 ml of acetic acid was added to the homogenate to make the final concentration 1.0 M. Using a polytron (20,000 rpm, 6 minutes), the mixture was homogenized. The homogenate was centrifuged (8,000 rpm, 30 minutes) and the supernatant was taken out. After 2.0 liters of 1.0 M acetic acid was added to the precipitate, the mixture was again homogenized by a polytron. The homogenate was stirred for overnight and then centrifuged (8,000 rpm, 30 minutes) to obtain the supernatant. After 2-fold volume of chilled acetone was slowly added dropwise to the supernatant at 4°C, the supernatant obtained by the first centrifugation was stirred for overnight and, the supernatant obtained by the second centrifugation was stirred for 4 hours. The acetone-added extract was centrifuged (8,000 rpm, 30 minutes) to remove the precipitate and acetone was evaporated off in vacuum from the supernatant, using an evaporator. An equal volume of diethyl ether was added to the acetone-removed extract, the ethereal layer containing lipids was separated using a separating funnel to recover the aqueous layer. After the lipids were removed with ether, the extract was concentrated in vacuum using an evaporator to completely remove the ether. The concentrate was filtrated through a glass fiber filter paper (Advantech, DP70 (90 mmΦ)) and the filtrate was charged in a glass-made column (30Φ x 240 mm) packed with C18 column (YMC, YMCgel ODS-AM 120-S50). After washing with 400 ml of 1.0 M acetic acid, the column was eluted with 500 ml of 60% acetonitrile containing 0.1% trifluoroacetic acid. The eluate was concentrated in vacuum, the solvent was distilled off and then the concentrate was lyophilized. About 0.5 g of the lyophilized product was dissolved in 30 ml of 10% acetonitrile containing 0.1% trifluoroacetic acid. An aliquot of 10 ml each was subjected to HPLC on 10% to 60% acetonitrile containing 0.1% trifluoroacetic acid by concentration gradient elution using C18 column (Toso, TSKgel ODS-80TS (21.5Φ x 300 mm)). HPLC was performed three times. The eluate was fractionated into 60 fractions and the eluates in three runs were collected. Each fraction was concentrated and evaporated to dryness in vacuum. The residue was dissolved in 0.5 ml of dimethylsulfoxide (DMSO).

The DMSO solution of HPLC fraction obtained as described above was administered to the CHO/GPR8 cells by the method shown in Reference Example 5, and a level of intracellular cAMP production was measured. As a result, it was perceived that fraction number 30 has a significant inhibiting activity of cAMP production. In addition, using GPR8 expressing CHO cells, for the same sample, promoting activity of GTPγS binding was investigated. The fraction around the number 30 was confirmed to have a significant activity. Since these activities were not found in other receptor expressing cells, it was shown that a substance having ligand activity specific to GPR8 exists in porcine hypothalamus extract.

REFERENCE EXAMPLE 8

Inactivation of an active substance exhibiting an inhibiting activity of intracellular cAMP production specific to GPR8 expressing CHO cells in porcinr hypothalamus extract

The HPLC fraction #30 which showed the inhibiting activity of intracellular cAMP production to the GPR8 expressing CHO cells in Reference Example 7 was treated with proteolytic enzyme, Pronase (Sigma, protease Type XIV (P5147)) to examine if the active substance is proteinaceous.

The HPLC fraction (#30), 2 μl, from the hypothalamus extract described above was added to 200 μl of 0.2 M ammonium acetate and 3 μg of Pronase was further added thereto. After incubation at 37°C for 2 hours, the culture was boiled in boiling water for 10 minutes to inactivate the Pronase. To the reaction solution was added 2 ml of distilled water containing 0.05 mg of BSA and 0.05 mg of CHAPS, followed by lyophilization. In order to examine if Pronase itself, or heating and lyophilization have an affect, Pronase alone, the HPLC fraction alone, and a mixture of the HPLC fraction with Pronase alone after its heating were treated in a similar manner and then lyophilized. Each sample fluid lyophilized was administered to the GPR8 expressing CHO cells in accordance with the method described in Reference Example 5, and the inhibiting activity of intracellular cAMP production was assayed. Since the active substance showing the inhibiting activity of intracellular cAMP production on the GPR8 expressing CHO cells in the porcine hypothalamus extract was completely inactivated by Pronase, the substance was shown to be proteins or peptides.

REFERENCE EXAMPLE 9

Purification of the active substances showing the promoting activity of GTPγS binding specific to the membrane fraction of GPR8 expressing CHO cells, from porcine hypothalamus extract

A representative example of purifying from porcine hypothalamus the active substance exhibiting a ligand activity specific to GPR8, using the promoting activity of GTPγS binding to the membrane fraction of GPR8 expressing CHO cells as an index, is specifically described below. In the same manner as described in Reference Example 7, the extract was extracted with 1.0 M acetic acid from 500 g of porcine hypothalamus (corresponding to 30 pigs). After acetone precipitation and defatting by ether, the extract was adsorpted to C18 column (YMC, YMCgel ODS-AM 120-S50), and was eluted with 60% acetonitrile containing 0.1% trifluoroacetic acid. The eluate was concentrated and lyophilized. Then, an active fraction was obtained by HPLC using C18 column (Toso, TSKgel ODS-80TS (21.5ϕ x 300 mm)). The activity was recovered in the fraction number 30. This fraction was further purified by the following method.

This fraction was dissolved in 10 ml of 10 mM ammonium formate containing 10% acetonitrile. The solution was loaded on a cationic exchange column (Toso, TSKgel SP-5PW (20 mmΦ x 150 mm)). Then the column was eluted with 10 mM to 2.0 M ammonium formate containing 10% acetonitrile by means of concentration gradient. The activity was recovered around 0.8 M ammonium formate. The active fraction was lyophilized followed by dissolving in 1.0 ml of 10% acetonitrile containing 0.1% trifluoroacetic acid. After passing the resulting solution through a CN column (Nomura Kagaku, Develosil CN-UG-5), elution was performed by concentration gradient with 21% to 26% acetonitrile containing 0.1% trifluoroacetic acid. The activity appeared around 22.1% acetonitrile. The active fraction was lyophilized and dissolved in 0.1 ml of DMSO. Further, 0.4 ml of 10% acetonitrile containing 0.1% trifluoroacetic acid was added to the above solution. After passing the solution through an ODS column (Wako Junyaku, Wakosil-II 3C18HG (2.0 mmΦ x 150 mm)), elution was performed by concentration gradient with 22.5% to 32.5% acetonitrile containing 0.1% trifluoroacetic acid. The activity appeared around 26.5% acetonitrile as a single peak.

REFERENCE EXAMPLE 10

Analysis of an amino acid sequence of amino-terminus of the active substance exhibiting a promoting activity of GTPγS binding specific to the membrane fraction of GPR8 expressing CHO cells, which was purified from porcine hypothalamus, and EST sequence presumed to be coded for a portion of precursor protein of human and rat homologue peptide of GPR8 ligand

Amino acid sequencing of the active substances showing the promoting activity of GTPγS binding specific to the membrane fraction of GPR8 expressing CHO cells, which was purified in Reference Example 9 was performed. Since it was presumed that the active substances would be proteins or peptides as shown in Reference Example 8, amino-terminal amino acid sequencing was conducted by use of Procise 494 Protein Sequencer available from Perkin-Elmer, using the eluates containing the active peaks. As a result, the sequence shown by SEQ ID NO: 14, which is from the amino terminus to the 17th residue, was obtained.

When gene database was screened based on the above sequence, some EST sequences were found, wherein the sequences or complement strands thereof were presumed to be encoding a portion of precursor protein of the peptide. Their accession numbers, origins, length of the sequences, and SEQ ID NOs are as follows: AW007531 (anaplastic oligodendroglioma, 438 bases, SEQ ID NO: 15); AI500303 (anaplastic oligodendroglioma, 264 basses, SEQ ID NO: 16); AI990964 (colonic mucosa frompatient of Crohn's disease, 424 bases, SEQ ID NO: 17); AA744804 (germinal center B cell, 375 bases, SEQ ID NO: 18); and H31598 (PC12 cells, 260 bases, SEQ ID NO: 19). The first four sequences are derived from human, and the last is derived from rat. The DNA sequences of these ESTs is highly identical to that of the region encoding an amino acid sequence, which corresponds to the sequence of active peptide isolated from porcine hypothalamus. Further, an amino acid sequence translated was nearly identical to that of the peptide, which was isolated from porcine hypothalamus and elucidated, excluding that Thr at the 5th residue is replaced to Val. From the fact described above, it was presumed that these EST is coded for a portion of the precursor protein of human and rat homologues of GPR8 ligand peptide.

REFERENCE EXAMPLE 11

Amplification of human cDNA encoding a portion of the GPR8 ligand peptide precursor and decoding of the amplified cDNA ssequence

Based on EST sequences presumed to be encoding a portion of the GPR8 ligand peptide precursor described in Reference Example 10, primers were designed, and from human brain-derived cDNA, cDNA encoding a portion of the GPR8 ligand peptide precursor was amplified by PCR.

Using human brain-derived polyA(+) RNA (CLONTECH) as a template, and random primers, a reverse transcription reaction was carried out. For the reverse transcription reaction, ReverTra Ace (TOYOBO), the reverse transcriptase derived from MMLV, which is deficient for RNase H activity, was used. Subsequently, using synthetic primers represented by SEQ ID NO: 20 and SEQ ID NO: 21, which were designed based on the EST sequences described in Reference Example 10, amplification was performed by PCR method. The reaction solution comprised of 2 μl of cDNA template, 0.5 μM each of synthetic DNA primers, 1.6 mM dNTPs, 0.2 μl of LATaq (Takara Shuzo) and a buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 72°C for 45 seconds, which was repeated 4 times, 96°C for 30 seconds followed by 70°C for 45 seconds, which was repeated 4 times, 96°C for 30 seconds followed by 68°C for 45 seconds, which was repeated 4 times, 96°C for 30 seconds followed by 64°C for 30 seconds and 72°C for 45 seconds, which was repeated 5 times, 96°C for 30 seconds followed by 60°C for 30 seconds and 72°C for 45 seconds, which was repeated 20 times, and finally, incubation at 72°C for 10 minutes. The amplified product was confirmed by 3% agarose gel electrophoresis and staining with ethidium bromide.

The PCR reaction solution was subjected to a 3% low melting agarose gel electrophoresis for isolation of the product band. After excision of the band by a razor, the DNA was recovered with QIAquick Gel Extraction Kit (Qiagen). According to the protocol of the TOPO TA Cloning Kit (Invitrogen), the recovered DNA was subcloned into a plasmid vector pCR2.1-TOPO. After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using DyeDeoxyTerminator Cycle Sequence Kit (PE Biosystems). As a result, after decoding with the fluorescent automated sequencer, the DNA sequence represented by SEQ ID NO: 22, was obtained. In a portion of GPR8 ligand peptide precursor protein translated from this sequence (SEQ ID NO: 23), a peptide sequence exists predictably, wherein the peptide corresponds to an active peptide isolated from porcine hypothalamus, which sequence was elucidated. In addition, at the C-terminus, 2 sites of Arg-Arg sequence (Seidah, N. G., et al., Ann. N. Y. Acad. Sci., 839, 9-24, 1998), which is predicted to be a site where, in general, physiologically active peptide is excised, were present. From this fact, it was presumed that the amino acid sequence of human homologue of GPR8 ligand peptide is either SEQ ID NO: 8 or SEQ ID NO: 9, or both.

REFERENCE EXAMPLE 12

Production of hGPR8L (1-23): Trp-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 8) and Fmoc-added hGPR8L (1-23): Fmoc-Trp-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 105)

Using 0.25 mmol of Fmoc-Leu-O-Clt resin (0.76 mmol/g) that Fmoc-Leu was introduced to a commercially available 2-chlorotrityl resin (Clt resin, 1.33 mmol/g) as a starting material, with Peptide Synthesizer ABI 433A, by Fmoc/DCC/HOBt method, Fmoc-Gly, Fmoc-Met, Fmoc-Leu, Fmoc-Leu, Fmoc-Gly, Fmoc-Ala, Fmoc-Ala, Fmoc-Arg (Pbf), Fmoc-Gly, Fmoc-Val, Fmoc-Thr ($Bu^t$), Fmoc-His (Trt), Fmoc-Tyr ($Bu^t$), Fmoc-Arg (Pbf), Fmoc-Pro, Fmoc-Ser ($Bu^t$), Fmoc-Ala, Fmoc-Val, Fmoc- His (Trt), Fmoc-Lys (Boc), Fmoc-Tyr ($Bu^t$), and Fmoc-Trp (Boc) was condensed in sequences to give a 830 mg of Fmoc-Trp-Tyr-Lys-His-Val-Ala-Ser-Pro-Arg-Tyr-His-Thr-Val-Gly-Arg-Ala-Ala-Gly-Leu-Leu-Met-Gly-Leu-O-Clt resin.

To 150 mg of the resin, 5 ml of TFA/thioanisole/m-cresol/triisopropylsilane/ethanedithiol (85/5/5/2.5/2.5) were added. After shaking at room temperature for 2 hours, the resin was removed by filtration, and a solvent was concentrated. Then ether was added for obtaining a crude Fmoc-Trp-Tyr-Lys-His-Val-Ala-Ser-Pro-Arg-Tyr-His-Thr-Val-Gly-Arg-Ala-Ala-Gly-Leu-Leu-Met-Gly-Leu as a precipitate. With a fractionated HPLC using YMC D-ODS-5-ST S-5 120A column (20 x 150 mm), an elution by linear concentration gradient (60 minutes) from A/B: 72/28 to 52/48 was performed, wherein eluent A and eluent B were 0.1% TFA-water and acetonitrile containing 0.1% TFA, respectively. Fractions containing a target were recovered and were lyophilized to give a 9.7 mg of white-colored powder.

To 5 mg of Fmoc-Trp-Tyr-Lys-His-Val-Ala-Ser-Pro-Arg-Tyr-His-Thr-Val-Gly-Arg-Ala-Ala-Gly-Leu-Leu-Met-Gly-Leu, 1 ml of 20% diethylamine/DMF was added, and the mixture was stirred at room temperature for 2 hours. After removal of solvent by distillation, with a fractionated HPLC using YMC D-ODS-5-ST S-5 120A column (20 x 150 mm), an elution by linear concentration gradient (60 minutes) from A/B: 74/26 to 64/36 was performed, wherein eluent A and eluent B were 0.1% TFA-water and acetonitrile containing 0.1% TFA, respectively. Fractions containing a target were recovered and were lyophilized to give a 1.2 mg of white-colored powder.
$(M+H)^+$ by mass spectrometry: 2583.6 (calculated value 2583.4)

Elution time on HPLC: 20.4 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 13

Production of hGPR8L (1-30): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr-Leu-Trp (SEQ ID NO: 9)

Using 0.25 mmol of Fmoc-Trp-O-Clt resin (0.64 mmol/g) that Fmoc-Trp (Boc) was introduced to a commercially available 2-chlorotrityl resin (Clt resin, 1.33 mmol/g) as a starting material, amino acids were condensed in sequences in the same manner as reference example 12. Fmoc group was removed from the resin prior to excision from the resin immediately after introduction of the last Trp. Then, excision from the resin and removal of protecting group of side chain were simultaneously performed by treatment of TFA/thioanisole/m-cresol/triisopropylsilane/ethanedithiol (85/5/5/2.5/2.5). Crude peptide was purified in a similar manner to Reference Example 12 to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr-Leu-Trp.
$(M+H)^+$ by mass spectrometry: 3543.4 (calculated value 3544.2)
Elution time on HPLC: 21.5 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 14

Action of GPR8 ligand on feeding behavior

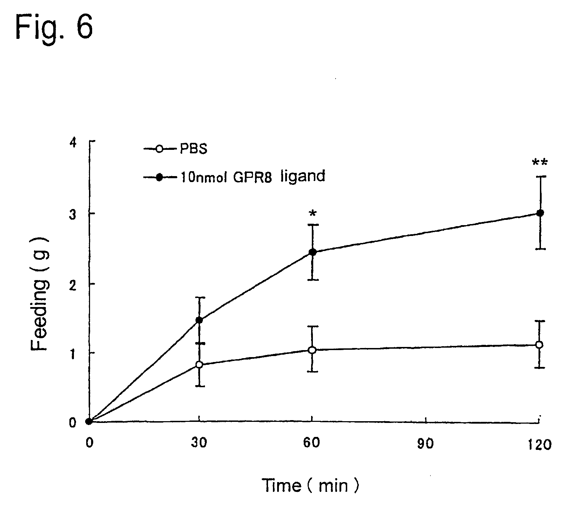

Into lateral venticle of Wistar male rat (9 weeks old) (AP: 8.1, L: 1.8, H: 7.1 mm), under anesthesia with pentobarbital, a guide cannula (AG-8) was inserted. Experment was done after recovery for one week or more. In process of recovery, handling was carried out in order to reduce a stress against rat at the time when the cannula was administered intraventricularly.
Feeding experiment was started at 15:00. On rat, under non-anesthesia and nonrestraint state, microinjection cannula was attached. Then, a peptide dissolved in PBS, which was obtained in Reference Example 12 (a peptide consisting of an amino acid sequence represented by SEQ ID NO: 8), or PBS solely was administered for 2 minutes at the rate of 5 μl/min. One minute after completion of administration, the microinjection cannula was removed, and preweighed foods (solid foods CE2: Nihon Crea) were freely given. An amount of feeding was calculated by weighing the rest of foods after 30, 60 and 120 minutes from starting of administration. The result is shown in FIG. 6.

REFERENCE EXAMPLE 15

Preparation of [$^{125}$I-Tyr$^2$]-hGPR8L (1-23) and [$^{125}$I-Tyr$^{10}$]-hGPR8L (1-23) using the lactoperoxidase method

One nmol of hGPR8L (1-23) dissolved in 5 μl of DMSO was mixed with 5 μl of 1 M nickel chloride, 10 μl of 0.001% hydrogen peroxide dissolved in 0.1 M HEPES (pH7), 10 μl of 10 μg/ml lactoperoxidase (Sigma) dissolved in 0.1 M HEPES (pH7), and 10 μl of NaI (37 MBq, NEN Life Science Products). The reaction mixture was incubated at room temperature for 60 minutes, and then was fractionated by HPLC under the following conditions.
As a column, ODS-80TM (4.6 mm x 15 cm) (Toso) was used, and as an eluent A and eluent B, 10% acetonitrile/0.1% TFA and 60% acetonitrile/0.1% TFA were used, respectively. Elution was performed by gradient elution of 0 - 0 (2 minutes), 0 - 30 (3 minutes), 30 - 38 (5 minutes), and 38 - 43 (55 minutes) of %B/A+B. Flow rate was 1 mL/min. Column temperature was 25°C. Detection of absorbance at 220 nm was used.
Since hGPR8L (1-23) has two tyrosine residues, by iodization, [$^{125}$I-Tyr$^2$]-hGPR8L (1-23) and [$^{125}$I-Tyr$^{10}$]-hGPR8L (1-23) were formed. Under the condition utilized, [$^{125}$I-Tyr$^2$]-hGPR8L (1-23) was eluted at around 30 minutes and [$^{125}$I-Tyr$^{10}$]-hGPR8L (1-23) at around 32 minutes.

REFERENCE EXAMPLE 16

Production of human GPR8 ligand (1-29): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr-Leu (SEQ ID NO: 128)

Using the resin in Reference Example 12, just like Reference Example 13, condensation of amino acids and excision from the resin in the sequence order, and purification were carried out to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr-Leu.

REFERENCE EXAMPLE 17

Production of human GPR8 ligand (1-28): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr (SEQ ID NO: 129)

Fmoc-Tyr($Bu^t$) was introduced to commercially available 2-chlorotrityl resin (Clt resin, 1.33 mmol/g). Then, just like Reference Example 13, condensation of amino acids and excision from the resin in the sequence order, and purification were carried out to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr.

REFERENCE EXAMPLE 18

Production of human GPR8 ligand (1-27): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro (SEQ ID NO: 130)

Fmoc-Pro was introduced to commercially available 2-chlorotrityl resin (Clt resin, 1.33 mmol/g). Then, just like Reference Example 13, condensation of amino acids and excision from the resin in the sequence order, and purification were carried out to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro.

REFERENCE EXAMPLE 19

Production of human GPR8 ligand (1-26): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser (SEQ ID NO: 131)

Fmoc-Ser ($Bu^t$) was introduced to commercially available 2-chlorotrityl resin (Clt resin, 1.33 mmol/g). Then, just like Reference Example 13, condensation of amino acids and excision from the resin in the sequence order, and purification were carried out to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser.

REFERENCE EXAMPLE 20

Production of human GPR8 ligand (1-25): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg (SEQ ID NO: 24)

Fmoc-Arg (Pbf) was introduced to commercially available 2-chlorotrityl resin (Clt resin, 1.33 mmol/g). Then, just like Reference Example 13, condensation of amino acids and excision from the resin in the sequence order, and purification were carried out to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg.

REFERENCE EXAMPLE 21

Production of human GPR8 ligand (1-24): Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg (SEQ ID NO: 25)

Fmoc-Arg (Pbf) was introduced to commercially available 2-chlorotrityl resin (Clt resin, 1.33 mmol/g). Then, just like Reference Example 13, condensation of amino acids and excision from the resin in the sequence order, and purification were carried out to give Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg.

REFERENCE EXAMPLE 22

Cloning of 5' upstream end of the cDNA encoding human TGR8 ligand precursor protein

By 5' RACE PCR using primers prepared based on human cDNA sequence (SEQ ID NO: 22) encoding a portion of the precursor protein of human homologue of GPR8 ligand peptide described in Reference Example 11 (hereafter, sometimes referred to as human GPR8 ligand), and human hypothalamus cDNA as a template, a base sequence of 5' upstream region of cDNA encoding human GPR8 ligand precursor protein was elucidated. The 5' RACE PCR cloning was accomplished by PCR reaction using human hypothalamus Marathon-Ready cDNA (CLONTECH) as a template, AP1 primer attached to the kit, and synthetic primer represented by SEQ ID NO: 33 followed by PCR reaction using the above PCR reaction mixture as a template, AP2 primer attached to the kit, and synthetic primer represented by SEQ ID NO: 34. The composition of the reaction solution and the conditions for PCR are as follows. The reaction solution comprised of 4 μl of human hypothalamus cDNA, 0.5 μM of AP1 primer, 0.5 μM of synthetic DNA primer represented by SEQ ID NO: 33, 0.4 mM dNTPs, 0.2 μl of LATaq Polymerase (Takara Shuzo) and a GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 240 seconds, which was repeated 30 times, and finally, incubation at 72°C for 10 minutes. Subsequently, the reaction solution comprised of 2 μl of the above PCR reaction solution diluted to 50-fold with Tricine-EDTA Buffer attached to the kit, 0.5 μM of AP2 primer, 0.5 μM of synthetic DNA primer represented by SEQ ID NO: 34, 0.4 mM dNTPs, 0.2 μl of LATaq Polymerase (Takara Shuzo) and GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 72°C for 180 seconds, which was repeated 4 times, 96°C for 30 seconds followed by 70°C for 180 seconds, which was repeated 4 times, 96°C for 30 seconds followed by 68°C for 180 seconds, which was repeated 17 times, and finally, incubation at 72°C for 10 minutes. After isolating the amplified DNA by 1.2% agarose gel electrophoresis, DNA having about 1200 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 35, was obtained.

REFERENCE EXAMPLE 23

Preparation of human brain cDNA

Human brain cDNA was prepared from human brain poly A(+) RNA (CLONTECH) using Marathon™ cDNA Amplification Kit (CLONTCH). The cDNA used for PCR was prepared according to protocol attached to the kit except 1st strand cDNA synthesis. The 1st strand cDNA was synthesized using reverse transcriptase MMLV (-RNAse H) (RevTraAce, TOYOBO) as substitute for reverse transcriptase AMV attached to the kit from 1 μg of human brain poly A(+) RNA.

REFERENCE EXAMPLE 24

Cloning of 3' downstream end of the cDNA encoding human TGR8 ligand precursor protein

By 3' RACE PCR using a primer prepared based on the base sequence of human cDNA encoding a portion of human GPR8 ligand precursor protein (SEQ ID NO: 22), a base sequence of 3' downstream region of cDNA encoding human GPR8 ligand precursor protein was elucidated. The 3' RACE PCR cloning was accomplished by PCR reaction using human brain cDNA as a template, AP1 primer attached to the kit, and synthetic primer represented by SEQ ID NO: 36 followed by PCR reaction using the above PCR reaction mixture as a template, AP2 primer attached to the kit, and synthetic primer represented by SEQ ID NO: 37. The composition of the reaction solution and the conditions for PCR are as follows. The reaction solution comprised of 1 μl of human brain cDNA diluted 50-fold with Tricine-EDTA Buffer attached to the kit, 0.5 μM of AP1 primer, 0.5 μM of synthetic DNA primer represented by SEQ ID NO: 36, 0.4 mM dNTPs, 0.2 μl of LATaq Polymerase (Takara Shuzo) and GC (I) buffer attached to the enzyme to make the total

volume 20 μl. The PCR reaction was carried out using a thermal cycler (PE Biosystems) by heating of 96°C for 120 seconds, then a cycle set to include 96°C for 30 seconds followed by 68°C for 240 seconds, which was repeated 30 times, and finally, incubation at 72°C for 10 minutes. Subsequently, the reaction solution comprised of 1 μl of the above PCR reaction solution diluted to 50-fold with Tricine-EDTA Buffer attached to the kit, 0.5 μM of AP2 primer, 0.5 μM of synthetic DNA primer represented by SEQ ID NO: 37, 0.4 mM dNTPs, 0.2 μl of LATaq Polymerase (Takara Shuzo) and GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 72°C for 180 seconds, which was repeated 4 times, 96°C for 30 seconds followed by 70°C for 180 seconds, which was repeated 4 times, 96°C for 30 seconds followed by 68°C for 180 seconds, which was repeated 17 times, and finally, incubation at 72°C for 10 minutes. After isolating the amplified DNA by 1.5% agarose gel electrophoresis, DNA having about 600 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 38, was obtained.

REFERENCE EXAMPLE 25

Cloning of cDNA encoding human GPR8 ligand precursor protein

By PCR amplification using a human hypothalamus cDNA as a template, a primer based on 5' upstream base sequence of the cDNA encoding human GPR8 ligand precursor protein and a primer based on 3' downstream base sequence of the cDNA encoding human GPR8 ligand precursor protein, cDNA encoding human GPR8 ligand precursor protein was cloned. The composition of the reaction solution and the conditions for PCR are as follows. The reaction solution comprised of 1 μl of human hypothalamus Marathon-Ready cDNA, 0.5 μM of synthetic DNA primer represented by SEQ ID NO: 39, 0.5 μM of synthetic DNA primer represented by SEQ ID NO: 40, 0.4 mM dNTPs, 2.5 mM $MgCl_2$, 5% DMSO, 0.2 μl of LATaq Polymerase (Takara Shuzo) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 96°C for 30 seconds followed by 64°C for 30 seconds and 72°C for 120 seconds, which was repeated 35 times, and finally, incubation at 72°C for 10 minutes. After isolating the amplified DNA by 1.5% agarose gel electrophoresis, DNA having about 700 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 41, was obtained.

Since this sequence (SEQ ID NO: 41) is coded for human GPR8 ligand precursor protein, Escherichia coli transformed with a plasmid containing this DNA was designated Escherichia coli TOP 10/pCR2.1-TOPO Human GPR8 Ligand Precursor.

While the DNA sequence shown by SEQ ID NO: 41 has a frame encoding an amino acid sequence of the human GPR8 ligand peptide described in Reference Example 11, there exists noATG predicted to be an initiation codon for protein translation in the 5' upstream reagion. However, it has been reported that in some proteins, codons other than ATG is predicted to be an initiation codon [human basic fibroblast growth factor (H. Prats et al., Proc. Natl. Acad. Sci. USA, 86, 1836-1840, 1989; R. Z. Florkiewicz and A. Sommer, Proc. Natl. Acad. Sci. USA, 86, 3978-3981, 1989); mouse retinoic acid receptor β4 (S. Nagpal et al., Proc. Natl. Acad. Sci. USA, 89, 2718, 1992); human phosphoribosyl pyrophosphoric acid synthetase (M. Taira et al., J. Biol. Chem., 265, 16491-16497, 1990); drosophila choline acetyltransferase (H. Sugihara et al., J. Biol. Chem., 265, 21714-21719, 1990)].

In many of these cases, CTG encoding Leu is assumed to be an initiation codon instead of ATG. Then, in the human GPR8 ligand precursor protein, it was also considered to be the same. Thus, by contrast with porcine or rat homologue of the GPR8 ligand precursor protein, CTG codon presented in the position nearly corresponding to ATG, which is predicted to be an initiation codon in these precursor protein, was assumed to be an initiation codon, and a sequence of the precursor protein was presumed. An amino acid sequence of the virtual human GPR8 ligand precursor protein was shown in SEQ ID NO: 42.

REFERENCE EXAMPLE 26

Preparation of porcine spinal cord cDNA

Porcine spinal cord cDNA was prepared using Marathon™ cDNA Amplification Kit (CLONTECH) from porcine spinal cord poly A(+) RNA. The porcine spinal cord poly A(+) RNA was prepared as follows. Porcine spinal cord was perfectly disrupted with Polytron homogenizer in ISOGEN (Nippon Gene). From this disrupted solution, porcine spinal cord total RNA was obtained according to the preparation method for total RNA using ISOGEN solution. Then, from the porcine total RNA, 7 μg of porcine spinal cord poly A(+) RNA was obtained by performing twice chromatography with oligo dT cellulose column attached to the mRNA Purification Kit (Amersham Pharmacia Biotech). The cDNA used for PCR was prepared according to protocol attached to the kit except 1st strand cDNA synthesis. The 1st strand cDNA was synthesized using reverse transcriptase MMLV (-RNAse H) (RevTraAce, TOYOBO) as substitute for reverse transcriptase AMV attached to the kit from 1 μg of porcine spinal cord poly A(+) RNA.

REFERENCE EXAMPLE 27

Cloning of 5' upstream end of the cDNA encoding porcine TGR8 ligand precursor protein

By the 1st 5' RACE PCR cloning and the 2nd 5' RACE PCR cloning, for which a base sequence of the PCR amplified DNA was utilized, a base sequence of 5' upstream region of cDNA encoding a precursor protein of porcine homologue of GPR8 ligand peptide (hereafter, sometimes referred to as porcine GPR8 ligand) was elucidated.

The 1st 5' RACE PCR cloning was accomplished by PCR reaction using porcine spinal cord cDNA as a template, AP1 primer attached to the kit, and synthetic primer represented by SEQ ID NO: 43 followed by PCR reaction using the above PCR reaction mixture as a template, AP2 primer attached to the kit, and synthetic primer represented by SEQ ID NO: 44. The composition of the reaction solution and the conditions for PCR are as follows. The reaction solution comprised of 4 μl of porcine spinal cord cDNA diluted 50-fold with Tricine-EDTA Buffer attached to the kit, 0.5 μM of AP1 primer, 0.5 μM of synthetic DNA primer represented by SEQ ID NO: 43, 0.4 mM dNTPs, 0.2 μl of LATaq Polymerase (Takara Shuzo) and a GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 180 seconds, which was repeated 30 times, and finally, incubation at 72°C for 10 minutes. Subsequently, the reaction solution comprised of 1 μl of the above PCR reaction solution diluted to 100-fold with Tricine-EDTA Buffer attached to the kit, 0.5 μM of AP2 primer, 0.5 μM of synthetic DNA primer represented by SEQ ID NO: 44, 0.4 mM dNTPs, 0.2 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 72°C for 180 seconds, which was repeated 3 times, 96°C for 30 seconds followed by 70°C for 180 seconds, which was repeated 3 times, 96°C for 30 seconds followed by 68°C for 180 seconds, which was repeated 4 times, 96°C for 30 seconds followed by 64°C for 30 seconds and 72°C for 180 seconds, which was repeated 15 times, and finally, incubation at 72°C for 10 minutes. After isolating the amplified DNA by 1.2% agarose gel electrophoresis, DNA having about 300 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10F' competent cell (Invitrogen) by introducing the above-mentioned vector, clones harboring cDNA insert fragment was selected on LB agar medium containing ampicillin, IPTG and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 45, was obtained.

The 2nd 5' RACE PCR cloning was accomplished by PCR reaction using porcine spinal cord cDNA as a template, AP1 primer attached to the kit, and synthetic primer represented by SEQ ID NO: 46 followed by PCR reaction using the above PCR reaction mixture as a template, AP2 primer attached to the kit, and synthetic primer represented by SEQ ID NO: 47. The composition of the reaction solution and the conditions for PCR are as follows. The reaction solution comprised of 1 μl of porcine spinal cord cDNA diluted 50-fold with Tricine-EDTA Buffer attached to the kit, 0.5 μM of AP1 primer, 0.5 μM of synthetic DNA primer represented by SEQ ID NO: 46, 0.4 mM dNTPs, 0.2 μl of Advan-

tage-GC 2 Polymerase (CLONTECH) and a buffer attached to the enzyme to make the total volume 20 µl. The PCR reaction was carried out using a thermal cycler (PE Biosystems) by heating of 96°C for 60 seconds, then a cycle set to include 96°C for 30 seconds followed by 72°C for 180 seconds, which was repeated 5 times, 96°C for 30 seconds followed by 70°C for 180 seconds, which was repeated 5 times, 96°C for 30 seconds followed by 68°C for 180 seconds, which was repeated 20 times, and finally, incubation at 72°C for 10 minutes. Subsequently, the reaction solution comprised of 1 µl of the above PCR reaction solution diluted to 100-fold with Tricine-EDTA Buffer attached to the kit, 0.5 µM of AP2 primer, 0.5 µM of synthetic DNA primer represented by SEQ ID NO: 47, 0.4 mM dNTPs, 0.2 µl of Advantage-GC 2 Polymerase (CLONTECH) and a buffer attached to the enzyme to make the total volume 20 µl. The PCR reaction was carried out using a thermal cycler (PE Biosystems) by heating of 96°C for 60 seconds, then a cycle set to include 96°C for 30 seconds followed by 68°C for 180 seconds, which was repeated 31 times, and finally, incubation at 72°C for 10 minutes. After isolating the amplified DNA by 2.0% agarose gel electrophoresis, DNA having about 200 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10F' competent cell (Invitrogen) by introducing the above-mentioned vector, clones harboring cDNA insert fragment was selected on LB agar medium containing ampicillin, IPTG and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 48, was obtained.

REFERENCE EXAMPLE 28

Cloning of 3' downstream end of the cDNA encoding porcine TGR8 ligand precursor protein

By 3' RACE PCR cloning using a primer prepared based on the base sequence of 5' upstream region of cDNA encoding porcine GPR8 ligand precursor protein, a base sequence of 3' downstream region of cDNA encoding porcine TGR8 ligand precursor protein was elucidated. The 3' RACE PCR cloning was accomplished by PCR reaction using porcine spinal cord cDNA as a template, AP1 primer attached to the kit, and synthetic primer represented by SEQ ID NO: 49 followed by PCR reaction using the above PCR reaction mixture as a template, AP2 primer attached to the kit, and synthetic primer represented by SEQ ID NO: 50. The composition of the reaction solution and the conditions for PCR are as follows. The reaction solution comprised of 1 µl of porcine spinal cord cDNA diluted 50-fold with Tricine-EDTA Buffer attached to the kit, 0.5 µM of AP1 primer, 0.5 µM of synthetic DNA primer represented by SEQ ID NO: 49, 0.4 mM dNTPs, 0.2 µl of Advantage-GC 2 Polymerase (CLONTECH) and a buffer attached to the enzyme to make the total volume 20 µl. The PCR reaction was carried out using a thermal cycler (PE Biosystems) by heating of 96°C for 60 seconds, then a cycle set to include 96°C for 30 seconds followed by 72°C for 120 seconds, which was repeated 5 times, 96°C for 30 seconds followed by 70°C for 120 seconds, which was repeated 5 times, 96°C for 30 seconds followed by 68°C for 120 seconds, which was repeated 20 times, and finally, incubation at 72°C for 10 minutes. Subsequently, the reaction solution comprised of 1 µl of the above PCR reaction solution diluted to 100-fold with Tricine-EDTA Buffer attached to the kit, 0.5 µM of AP2 primer, 0.5 µM of synthetic DNA primer represented by SEQ ID NO: 50, 0.4 mM dNTPs, 0.2 µl of Advantage-GC 2 Polymerase (CLONTECH) and a buffer attached to the enzyme to make the total volume 20 µl. The PCR reaction was carried out using a thermal cycler (PE Biosystems) by heating of 96°C for 120 seconds, then a cycle set to include 96°C for 30 seconds followed by 68°C for 120 seconds, which was repeated 31 times, and finally, incubation at 72°C for 10 minutes. After isolating the amplified DNA by 2.0% agarose gel electrophoresis, DNA having about 650 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10F' competent cell (Invitrogen) by introducing the above-mentioned vector, clones harboring cDNA insert fragment was selected on LB agar medium containing ampicillin, X-gal and IPTG. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 51, was obtained.

REFERENCE EXAMPLE 29

Cloning of cDNA encoding porcine GPR8 ligand precursor protein

By PCR amplification using a porcine spinal cord cDNA as a template, a primer based on 5' upstream base sequence of the cDNA encoding porcine GPR8 ligand precursor protein and a primer based on 3' downstream base sequence of the cDNA encoding porcine GPR8 ligand precursor protein, cDNA encoding porcine GPR8 ligand precursor protein was cloned. The composition of the reaction solution and the conditions for PCR are as follows. The reaction solution comprised of 1 μl of porcine spinal cord cDNA diluted 50-fold with Tricine-EDTA Buffer attached to the kit, 0.5 μM of synthetic DNA primer represented by SEQ ID NO: 52, 0.5 μM of synthetic DNA primer represented by SEQ ID NO: 53, 0.4 mM dNTPs, 0.2 μl of Advantage 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 72°C for 75 seconds, which was repeated 4 times, 96°C for 30 seconds followed by 70°C for 75 seconds, which was repeated 4 times, 96°C for 30 seconds followed by 68°C for 75 seconds, which was repeated 4 times, 96°C for 30 seconds followed by 64°C for 30 seconds and 72°C for 45 seconds, which was repeated 5 times, 96°C for 30 seconds followed by 60°C for 30 seconds and 72°C for 45 seconds, which was repeated 20 times, and finally, incubation at 72°C for 10 minutes. After isolating the amplified DNA by 1.2% agarose gel electrophoresis, DNA having about 600 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 54, was obtained. Since this sequence (SEQ ID NO: 54) is coded for porcine GPR8 ligand precursor protein, Escherichia coli transformed with a plasmid containing this DNA was designated Escherichia coli TOP10/pCR2.1-TOPO Porcine GPR8 Ligand Precursor.

An amino acid sequence of porcine GPR8 ligand precursor protein encoded by the DNA sequence represented by SEQ ID NO: 54 was shown in SEQ ID NO: 55. In this amino acid sequence of the precursor protein, there is a sequence from the amino-terminus to the 17th residue, which was elucidated by analysis of amino acid sequence of the GPR8 ligand peptide isolated from porcine hypothalamus by assaying a GTPγS binding activity to the membrane fraction of GPR8 expressing CHO cells as an index as described in Reference Example 10. Further, as well as the case of human homologue of the GPR8 ligand peptide precursor protein, at the carboxyl-terminus, 2 sites of Arg-Arg sequence (Seidah, N. G., et al., Ann. N. Y. Acad. Sci., 839, 9-24, 1998), which is predicted to be a site where, in general, physiologically active peptide is excised, were present. From this fact, it was presumed that the amino acid sequence of porcine homologue of GPR8 ligand peptide is either SEQ ID NO: 56 or SEQ ID NO: 57, or both.

REFERENCE EXAMPLE 30

Cloning of cDNA encoding a portion of rat GPR8 ligand precursor protein

As described in Reference Example 10, where based on the sequence consisting of 17 amino acid residues (SEQ ID NO: 14) from the amino-terminus of peptide purified from porcine hypothalamus by GTPγS binding activity to the membrane fraction of GPR8 expressing cells as an index, database retrieval was done, rat EST base sequence (accession number: H31598) identical to the base sequence represented by SEQ ID NO: 19 was found. This DNA sequence has a translation frame, wherein a sequence of 15 amino acids is identical to the amino acid sequence (SEQ ID NO: 14) of the peptide purified from porcine hypothalamus. The H31598 is an EST sequence derived from cDNA library prepared from rat PC12 cells, and consists of 260 bases containing 7 unidentified bases. The H31598 was predicted to be coding for a portion of precursor protein of homologue of rat GPR8 ligand peptide (hereafter, referred to as rat GPR8 ligand). Therefore, in order to determine a precise sequence, using respective primers prepared based on the 5' base sequence and the 3' base sequence of H31598 and rat brain Marathon-Ready cDNA (CLONTECH) as a template, PCR cloning was carried out. The composition of the reaction solution and the conditions for PCR are as follows. The reaction solution comprised of 2 μl of rat brain Marathon cDNA, 0.5 μM of synthetic DNA primer represented by SEQ ID NO: 60, 0.5 μM of synthetic DNA primer represented by SEQ ID NO: 61, 0.4 mM dNTPs, 0.2 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 60°C for 30 seconds and 72°C for 60 seconds, which was repeated 35 times, and finally, incubation at 72°C for 10 minutes. After isolating the amplified DNA by 4.0% agarose gel electrophoresis, DNA having about 250 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 62, was obtained. By comparison between the base sequence of DNA by PCR cloning (SEQ ID NO: 62) and the base sequence of H31598, it was found that the base sequence of H31598 has an error for one base deletion.

REFERENCE EXAMPLE 31

Cloning of 5' upstream end of the cDNA encoding rat TGR8 ligand precursor protein

By 5' RACE PCR cloning, a base sequence of 5' upstream region of cDNA encoding rat TGR8 ligand precursor protein was elucidated. The 5' RACE PCR cloning was accomplished by PCR reaction using rat brain Marathon-Ready cDNA (CLONTECH) as a template, AP1 primer attached to the kit, and synthetic primer represented by SEQ ID NO: 63 followed by PCR reaction using the above PCR reaction mixture as a template, AP2 primer attached to the kit, and synthetic primer represented by SEQ ID NO: 64. The composition of the reaction solution and the conditions for PCR are as follows. The reaction solution comprised of 2 μl of rat brain Marathon cDNA, 0.5 μM of AP1 primer, 0.5 μM of synthetic DNA primer represented by SEQ ID NO: 63, 0.4 mM dNTPs, 0.2 μl of LATaq Polymerase (Takara Shuzo) and a GC (I) buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 68°C for 120 seconds, which was repeated 30 times, and finally, incubation at 72°C for 10 minutes. Subsequently, the reaction solution comprised of 2 μl of the above PCR reaction solution diluted to 200-fold with Tricine-EDTA Buffer attached to the kit, 0.5 μM of AP2 primer, 0.5 μM of synthetic DNA primer represented by SEQ ID NO: 64, 0.4 mM dNTPs, 0.2 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 68°C for 120 seconds, which was repeated 31 times, and finally, incubation at 72°C for 10 minutes. After isolating the amplified DNA by 1.2% agarose gel electrophoresis, DNA having about 600 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 65, was obtained.

REFERENCE EXAMPLE 32

Cloning of 3' downstream end of the cDNA encoding rat TGR8 ligand precursor protein

By 3' RACE PCR cloning using a primer prepared based on the base sequence of 5' upstream end of cDNA encoding rat GPR8 ligand precursor protein and the sequence of cDNA fragment encoding a portion of rat GPR8 ligand precursor protein, a base sequence of 3' downstream region of cDNA encoding rat TGR8 ligand precursor protein was elucidated. The 3' RACE PCR cloning was accomplished by PCR reaction using rat brain Marathon-Ready cDNA as a template, AP1 primer attached to the kit, and synthetic primer represented by SEQ ID NO: 66 followed by PCR reaction using the above PCR reaction mixture as a template, AP2 primer attached to the kit, and synthetic primer represented by SEQ ID NO: 67. The composition of the reaction solution and the conditions for PCR are as follows. The reaction solution comprised of 2 μl of rat brain Marathon-Ready cDNA, 0.5 μM of AP1 primer, 0.5 μM of synthetic DNA primer represented by SEQ ID NO: 66, 0.4 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 68°C for 180 seconds, which was repeated 30 times, and finally, incubation at 72°C for 10 minutes. Subsequently, the reaction solution comprised of 2 μl of the above PCR reaction solution diluted to 200-fold with Tricine-EDTA Buffer attached to the kit, 0.5 μM of AP2 primer, 0.5 μM of synthetic DNA primer represented by SEQ ID NO: 67, 0.4 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 68°C for 180 seconds, which was repeated 30 times, and finally, incubation at 72°C for 10 minutes. After isolating the amplified DNA by 1.2% agarose gel electrophoresis, DNA having about 600 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 68, was obtained.

REFERENCE EXAMPLE 33

Cloning of cDNA encoding rat GPR8 ligand precursor protein

By PCR amplification using a rat brain cDNA as a template, a primer based on 5' upstream base sequence of the cDNA encoding rat GPR8 ligand precursor protein and a primer based on 3' downstream base sequence of the cDNA encoding rat GPR8 ligand precursor protein, cDNA encoding rat GPR8 ligand precursor protein was cloned. The composition of the reaction solution and the conditions for PCR are as follows. The reaction solution comprised of 1 μl of rat brain Marathon-Ready cDNA, 0.5 μM of synthetic DNA primer represented by SEQ ID NO: 69, 0.5 μM of synthetic DNA primer represented by SEQ ID NO: 70, 0.4 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 60 seconds, then a cycle set to include 96°C for 30 seconds followed by 60°C for 30 seconds and 72°C for 60 seconds, which was repeated 35 times, and finally, incubation at 72°C for 10 minutes. After isolating the amplified DNA by 1.2% agarose gel electrophoresis, DNA having about 750 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 71, was obtained. Since this sequence (SEQ ID NO: 71) is coded for rat GPR8 ligand precursor protein, Escherichia coli transformed with a plasmid containing this DNA was designated Escherichia coli TOP10/pCR2.1-TOPO Rat GPR8 Ligand Precursor.

An amino acid sequence of rat GPR8 ligand precursor protein encoded by the DNA sequence represented by SEQ ID NO: 71 was shown in SEQ ID NO: 72. In this amino acid sequence of the precursor protein, there are a sequence from the amino-terminus to the 17th residue, which was elucidated by analysis of amino acid sequence of the GPR8 ligand peptide isolated from porcine hypothalamus by assaying a GTPγS binding activity to the membrane fraction of GPR8 expressing CHO cells as an index as described in Reference Example 10, and a similar sequence including different amino acid residues at the 5th and 17th positions. In addition, as well as the case of human or porcine homologue of the GPR8 ligand peptide precursor protein, at the carboxyl-terminus, 2 sites of Arg-Arg sequence (Seidah, N. G., et al., Ann. N. Y. Acad. Sci., 839, 9-24, 1998), which is predicted to be a site where, in general, physiologically active peptide is excised, were present. From this fact, it was presumed that the amino acid sequence of human homologue of GPR8 ligand peptide is either SEQ ID NO: 73 or SEQ ID NO: 74, or both.

REFERENCE EXAMPLE 34

Cloning of cDNA encoding a portion of mouse GPR8 ligand precursor protein

Based on the base sequence encoding porcine GPR8 ligand peptide, which is consisting of 23 amino acid residues represented by SEQ ID NO: 58, database retrieval was done. As a result of retrieval on mouse genome database, a sequence of mouse genome fragment represented by SEQ ID NO: 77 containing a base sequence similar to the sequence represented by SEQ ID NO: 58 was found. It was predicted that this sequence is a sequence of genome fragment encoding a portion of precursor protein of mouse homologue of GPR8 ligand peptide (hereafter, sometimes referred to as mouse GPR8 ligand).

Using mouse testis cDNAa as a template, and primers prepared based on the sequence of mouse genome fragment, PCR amplification was performed. Then, a base sequence of the amplified DNA was determined. The composition of reaction solution and the reaction conditions for PCR are as follows. The reaction solution comprised of 1 μl of mouse testis cDNA (CLONTECH), 0.5 μM of synthetic DNA primer represented by SEQ ID NO: 78, 0.5 μM of synthetic DNA primer represented by SEQ ID NO: 79, 0.4 mM dNTPs, 0.2 μl of LATaq Polymerase (Takara Shuzo) and GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 120 seconds, which was repeated 10 times, 96°C for 30 seconds followed by 64°C for 30 seconds and 72°C for 120 seconds, which was repeated 25 times, and finally, incubation at 72°C for 10 minutes. After isolating the amplified DNA by 1.5% agarose gel electrophoresis, DNA having about 350 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence was obtained (SEQ ID NO: 80). The base sequence of cDNA thus obtained by PCR cloning (SEQ ID NO: 80) was completely identical to the base sequence of mouse genome fragment interleaving two base sequences, which were used as primers represented SEQ ID NO: 78 and SEQ ID NO: 79.

REFERENCE EXAMPLE 35

Cloning of cDNA encoding a portion of rat GPR8 ligand precursor protein

Based on the base sequence encoding porcine GPR8 ligand peptide, which is consisting of 23 amino acid residues represented by SEQ ID NO: 58, database retrieval was done. As a result of retrieval on mouse genome database of Celera, a sequence of mouse genome fragment represented by SEQ ID NO: 77 containing a base sequence similar to the sequence represented by SEQ ID NO: 58 was found. It was predicted that this sequence is a sequence of genome fragment encoding a portion of precursor protein of mouse homologue of GPR8 ligand peptide (hereafter, sometimes referred to as mouse GPR8 ligand).

Using mouse testis cDNAa as a template, and primers prepared based on the sequence of mouse genome fragment, PCR amplification was performed. Then, a base sequence of the amplified DNA was determined. The composition of reaction solution and the reaction conditions for PCR are as follows. The reaction solution comprised of 1 μl of mouse testis cDNA (CLONTECH), 0.5 μM of synthetic DNA primer represented by SEQ ID NO: 78, 0.5 μM of synthetic DNA primer represented by SEQ ID NO: 79, 0.4 mM dNTPs, 0.2 μl of LATaq Polymerase (Takara Shuzo) and GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 120 seconds, which was repeated 10 times, 96°C for 30 seconds followed by 64°C for 30 seconds and 72°C for 120 seconds, which was repeated 25 times, and finally, incubation at 72°C for 10 minutes. After isolating the amplified DNA by 1.5% agarose gel electrophoresis, DNA having about 350 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence was obtained (SEQ ID NO: 80). The base sequence of cDNA thus obtained by PCR cloning (SEQ ID NO: 80) was completely identical to the base sequence of mouse genome fragment interleaving two base sequences, which were used as primers represented SEQ ID NO: 78 and SEQ ID NO: 79.

REFERENCE EXAMPLE 36

Pereparation of mouse brain cDNA

The mouse brain cDNA was prepared from mouse brain polyA(+) RNA (CLONTECH) using SMART™ RACE cDNAAmplification Kit (CLONTECH) in accordance with the protocol attached the kit. The solution containing the synthesized 1st strand cDNA was diluted to 10-fold with Tricine-EDTA Buffer attached to the kit, and used for RACE PCR reaction.

REFERENCE EXAMPLE 37

Cloning of 5' upstream end of the cDNA encoding mouse TGR8 ligand precursor protein

By 5' RACE PCR cloning, a base sequence of 5' upstream region of cDNA encoding mouse TGR8 ligand precursor protein was elucidated. The 5' RACE PCR cloning was accomplished by PCR reaction using mouse brain cDNA as a template, Universal Primer Mix attached to SMART™ RACE cDNAAmplification Kit, and synthetic primer represented by SEQ ID NO: 81 followed by PCR reaction using the above PCR reaction mixture as a template, Nested Universal Primer attached to the kit, and synthetic primer represented by SEQ ID NO: 82. The composition of the reaction solution and the conditions for PCR are as follows. The reaction solution comprised of 1 μl of mouse brain cDNA, 2 μl of Universal Primer Mix, 0.2 μM of synthetic DNA primer represented by SEQ ID NO: 81, 0.8 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 120 seconds, which was repeated 30 times, and finally, incubation at 72°C for 10 minutes. Subsequently, the reaction solution comprised of 0.5 μl of the above PCR reaction solution diluted to 50-fold with Tricine-EDTA Buffer attached to the kit, 0.5 μM of Nested Universal Primer, 0.5 μM of synthetic DNA primer represented by SEQ ID NO: 82, 0.8 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 30 seconds and 72°C for 120 seconds, which was repeated 30 times, and finally, incubation at 72°C for 10 minutes. After isolating the amplified DNA by 1.5% agarose gel electrophoresis, DNA having about 300 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 83, was obtained.

REFERENCE EXAMPLE 38

Cloning of 3' downstream end of the cDNA encoding mouse TGR8 ligand precursor protein

By 3' RACE PCR cloning, a base sequence of 3' downstream region of cDNA encoding mouse TGR8 ligand precursor protein was elucidated. The 3' RACE PCR cloning was accomplished by PCR reaction using mouse brain cDNA as a template, Universal Primer Mix attached to SMART™ RACE cDNAAmplification Kit, and synthetic primer represented by SEQ ID NO: 84 followed by PCR reaction using the above PCR reaction mixture as a template, Nested Universal Primer attached to the kit, and synthetic primer represented by SEQ ID NO: 85. The composition of the reaction solution and the conditions for PCR are as follows. The reaction solution comprised of 1 μl of mouse brain cDNA, 2 μl of Universal Primer Mix, 0.2 μM of synthetic DNA primer represented by SEQ ID NO: 84, 0.8 mM dNTPs, 0.4 μl of Advantage-GC 2 Polymerase (CLONTECH) and a buffer attached to the enzyme to make the total volume

20 μl. The PCR reaction was carried out using a thermal cycler (PE Biosystems) by heating of 96°C for 120 seconds, then a cycle set to include 96°C for 30 seconds followed by 68°C for 120 seconds, which was repeated 30 times, and finally, incubation at 72°C for 10 minutes. Subsequently, the reaction solution comprised of 0.5 μl of the above PCR reaction solution diluted to 50-fold with Tricine-EDTA Buffer attached to the kit, 0.5 μM of Nested Universal Primer, 0.5 μM of synthetic DNA primer represented by SEQ ID NO: 85, 0.8 mM dNTPs, 0.4 μl of Advantage-GC 2 Polymerase (CLONTECH) and a buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 68°C for 30 seconds and 72°C for 120 seconds, which was repeated 30 times, and finally, incubation at 72°C for 10 minutes. After isolating the amplified DNA by 1.5% agarose gel electrophoresis, DNA having about 700 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 86, was obtained.

REFERENCE EXAMPLE 39

Cloning of cDNA encoding mouse GPR8 ligand precursor protein

By PCR amplification using a mouse brain cDNA as a template, a primer based on 5' upstream base sequence of the cDNA encoding mouse GPR8 ligand precursor protein and a primer based on 3' downstream base sequence of the cDNA encoding mouse GPR8 ligand precursor protein, cDNA encoding mouse GPR8 ligand precursor protein was cloned. The composition of the reaction solution and the conditions for PCR are as follows. The reaction solution comprised of 1 μl of mouse brain cDNA, 0.5 μM of synthetic DNA primer represented by SEQ ID NO: 87, 0.5 μM of synthetic DNA primer represented by SEQ ID NO: 88, 1.6 mM dNTPs, 0.2 μl of LATaq Polymerase (Takara Shuzo) and GC (I) buffer attached to the enzyme to make the total volume 20 μl. The PCR reaction was carried out using a thermal cycler (PE Biosystems) by heating of 96°C for 120 seconds, then a cycle set to include 96°C for 30 seconds followed by 64°C for 30 seconds and 72°C for 120 seconds, which was repeated 40 times, and finally, incubation at 72°C for 10 minutes. After isolating the amplified DNA by 1.5% agarose gel electrophoresis, DNA having about 700 bases length was excised with razor, and was recovered using QIAquick Gel Extraction Kit (Qiagen). This DNA was cloned into pCR2.1-TOPO vector according to the protocol attached to the TOPO TA Cloning Kit (Invitrogen). After transformation of Escherichia coli TOP10 competent cell (Invitrogen) by introducing the above-mentioned vector, clones harboring cDNA insert fragment was selected on LB agar medium containing ampicillin and X-gal. All the white-colored clones were isolated with sterilized toothpick, and then the transformants were obtained. Respective clones were cultured in LB medium containing ampicillin for overnight. Subsequently, the plasmid DNA was prepared using QIAwell 8 Plasmid Kit (Qiagen). The reaction for determination of the base sequence was carried out using BigDye Terminator Cycle Sequencing Ready Reaction Kit (PE Biosystems). As a result, after decoding with the fluorescent automated sequencer, the DNA sequence represented by SEQ ID NO: 89, was obtained. Since this sequence (SEQ ID NO: 89) is coded for rat GPR8 ligand precursor protein, Escherichia coli transformed with a plasmid containing this DNA was designated Escherichia coli TOP10/pCR2.1-TOPO Mouse GPR8 Ligand Precursor.

An amino acid sequence of rat GPR8 ligand precursor protein encoded by the DNA sequence represented by SEQ ID NO: 71 was shown in SEQ ID NO: 72. In this amino acid sequence of the precursor protein, there are frames coding for a sequence from the amino-terminus to the 17th residue, which was elucidated by analysis of amino acid sequence of the GPR8 ligand peptide isolated from porcine hypothalamus by assaying a GTPγS binding activity to the membrane fraction of GPR8 expressing CHO cells as an index as described in Reference Example 10, and a similar sequence including different amino acid residues at the 5th and 17th positions. However, as well as the case of human GPR8 ligand precursor, at the 5' upstream, there exists no ATG, which is predicted to be a initiation codon of the protein translation. Thus, as presumed in the case of human GPR8 ligand precursor protein, by comparison with porcine or rat homologue of GPR8 ligand precursor protein, CTG codon, which exists in the position nearly corresponding to that of ATG predictable to be a initiation codon of the precursor protein, was assumed to be a initiation codon. Then, a sequence of the mouse GPR8 ligand precursor protein was presumed. The assumptive amino acid sequence of the mouse GPR8 ligand precursor protein was shown in SEQ ID NO: 90. As well as the case of human, porcine or rat homologue of the GPR8 ligand peptide precursor protein, at the carboxyl-terminus, 2 sites of Arg-Arg sequence (Seidah, N. G., et al., Ann. N. Y. Acad. Sci., 839, 9-24, 1998), which is predicted to be a site where, in general, physiologically active peptide is excised, were present. From these facts, it was presumed that the amino acid sequence of mouse homologue of GPR8 ligand peptide is either SEQ ID NO: 91 or SEQ ID NO: 92, or both. In addition, an amino acid sequence of the mouse GPR8 ligand consisting of 23 residues, which is represented by SEQ ID NO: 91, is identical to the amino acid sequence of the rat GPR8 ligand consisting of 23 residues (SEQ ID NO: 73).

REFERENCE EXAMPLE 40

Production of human GPR8 ligand (1-23) oxidant: Trp-Tyr-Lys-His-Val-Ala-Ser-Pro-Arg-Tyr-His-Thr-Val-Gly-Arg-Ala-Ala-Gly-Leu-Leu-Met(O)-Gly-Leu (SEQ ID NO: 95)

0.45 g of the compound in Reference Example 12 was dissolved in 0.5 ml of 50% acetic acid water, and further 0.3% hydrogen peroxide solution was added followed by leaving to stand at room temperature for 8 hours. After vacuum concentration, the solution was purified by SepPak to give 0.443 mg of a white-colored powder.
$(M+H)^+$ by mass spectrometry: 2599.2 (calculated value 2599.4)
Elution time on HPLC: 19.1 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 0/70 (35 minutes)
Flow rate: 1.0 ml/minute

REFERENCE EXAMPLE 41

Production of human GPR8 ligand (1-22): Trp-Tyr-Lys-His-Val-Ala-Ser-Pro-Arg-Tyr-His-Thr-Val-Gly-Arg-Ala-Ala-Gly-Leu-Leu-Met-Gly (SEQ ID NO: 96)

Fmoc-Gly was introduced to commercially available 2-chlorotrityl resin (Clt resin, 1.33 mmol/g). Then, just like Reference Example 13, condensation of amino acids and excision from the resin in the sequence order, and purification were carried out to give a target.

REFERENCE EXAMPLE 42

Production of human GPR8 ligand (1-21): Trp-Tyr-Lys-His-Val-Ala-Ser-Pro-Arg-Tyr-His-Thr-Val-Gly-Arg-Ala-Ala-Gly-Leu-Leu-Met (SEQ ID NO: 97)

Fmoc-Met was introduced to commercially available 2-chlorotrityl resin (Clt resin, 1.33 mmol/g). Then, just like Reference Example 13, condensation of amino acids and excision from the resin in the sequence order, and purification were carried out to give a target.

REFERENCE EXAMPLE 43

Production of human GPR8 ligand (1-20): Trp-Tyr-Lys-His-Val-Ala-Ser-Pro-Arg-Tyr-His-Thr-Val-Gly-Arg-Ala-Ala-Gly-Leu-Leu (SEQ ID NO: 98)

Fmoc-Leu was introduced to commercially available 2-chlorotrityl resin (Clt resin, 1.33 mmol/g). Then, just like Reference Example 13, condensation of amino acids and excision from the resin in the sequence order, and purification were carried out to give a target.
$(M+H)^+$ by mass spectrometry: 2282.8 (calculated value 2282.6)
Elution time on HPLC: 17.2 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 0/70 (35 minutes)
Flow rate: 1.0 ml/minute

REFERENCE EXAMPLE 44

Production of human GPR8 ligand (1-19): Trp-Tyr-Lys-His-Val-Ala-Ser-Pro-Arg-Tyr-His-Thr-Val-Gly-Arg-Ala-Ala-Gly-Leu (SEQ ID NO: 99)

Fmoc-Leu was introduced to commercially available 2-chlorotrityl resin (Clt resin, 1.33 mmol/g). Then, just like Reference Example 13, condensation of amino acids and excision from the resin in the sequence order, and purification were carried out to give a target.
$(M+H)^+$ by mass spectrometry: 2169.6 (calculated value 2169.5)
Elution time on HPLC: 16.4 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 0/70 (35 minutes)
Flow rate: 1.0 ml/minute

REFERENCE EXAMPLE 45

Production of human GPR8 ligand (1-18): Trp-Tyr-Lys-His-Val-Ala-Ser-Pro-Arg-Tyr-His-Thr-Val-Gly-Arg-Ala-Ala-Gly (SEQ ID NO: 100)

Fmoc-Gly was introduced to commercially available 2-chlorotrityl resin (Clt resin, 1.33 mmol/g). Then, just like Reference Example 13, condensation of amino acids and excision from the resin in the sequence order, and purification were carried out to give a target.
$(M+H)^+$ by mass spectrometry: 2056.8 (calculated value 2056.3)
Elution time on HPLC: 14.2 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 0/70 (35 minutes)
Flow rate: 1.0 ml/minute

REFERENCE EXAMPLE 46

Production of human GPR8 ligand (1-17): Trp-Tyr-Lys-His-Val-Ala-Ser-Pro-Arg-Tyr-His-Thr-Val-Gly-Arg-Ala-Ala (SEQ ID NO: 101)

Fmoc-Ala was introduced to commercially available 2-chlorotrityl resin (Clt resin, 1.33 mmol/g). Then, just like Reference Example 13, condensation of amino acids and excision from the resin in the sequence order, and purification were carried out to give a target.

REFERENCE EXAMPLE 47

Production of human GPR8 ligand (1-16): Trp-Tyr-Lys-His-Val-Ala-Ser-Pro-Arg-Tyr-His-Thr-Val-Gly-Arg-Ala (SEQ ID NO: 102)

Fmoc-Ala was introduced to commercially available 2-chlorotrityl resin (Clt resin, 1.33 mmol/g). Then, just like Reference Example 13, condensation of amino acids and excision from the resin in the sequence order, and purification were carried out to give a target.

REFERENCE EXAMPLE 48

Production of porcine GPR8 ligand (1-23): Trp-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 56)

Fmoc-Leu was introduced to commercially available 2-chlorotrityl resin (Clt resin, 1.33 mmol/g). Then, just like Reference Example 13, condensation of amino acids and excision from the resin in the sequence order, and purification were carried out to give a target.

$(M+H)^+$ by mass spectrometry: 2585.2 (calculated value 2585.4)
Elution time on HPLC: 20.2 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 0/70 (35 minutes)
Flow rate: 1.0 ml/minute

REFERENCE EXAMPLE 49

Production of rat/mouse GPR8 ligand (1-23): Trp-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 73 and SEQ ID NO: 91)

Just like Reference Example 48, condensation of amino acids and excision from the resin in the sequence order, and purification were carried out to give a target.

REFERENCE EXAMPLE 50

Production of porcine GPR8 ligand (1-23) oxidant: Trp-Tyr-Lys-His-Val-Ala-Ser-Pro-Arg-Tyr-His-Thr-Val-Gly-Arg-Ala-Ala-Gly-Leu-Leu-Met(O)-Gly-Leu (SEQ ID NO: 103)

Using a compound described in Reference Example 48, a compound was oxidized in a similar manner to Reference Example 40 to give a target.
$(M+H)^+$ by mass spectrometry: 2601.3 (calculated value 2601.4)
Elution time on HPLC: 18.9 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 0/70 (35 minutes)
Flow rate: 1.0 ml/minute

REFERENCE EXAMPLE 51

Production of rat/mouse GPR8 ligand (1-23) oxidant: Trp-Tyr-Lys-His-Val-Ala-Ser-Pro-Arg-Tyr-His-Thr-Val-Gly-Arg-Ala-Ala-Gly-Leu-Leu-Met(O)-Gly-Leu (SEQ ID NO: 104)

Using a compound described in Reference Example 48, a compound was oxidized in a similar manner to Reference Example 40 to give a target.

REFERENCE EXAMPLE 52

Production of [$N^{\alpha}$-Acetyl-$Trp^1$]-human GPR8 ligand (1-23): Ac-Trp-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 106)

Fmoc group was removed from the resin prepared in Reference Example 12, and the terminus was acetylated with acetic anhydride. Then, excision from the resin and removal of protecting group of side chain were simultaneously performed by treatment of TFA/thioanisole/m-cresol/triisopropylsilane/ethanedithiol (85/5/5/2.5/2.5). Crude peptide was purified in a similar manner to Reference Example 12 to give a target. $(M+H)^+$ by mass spectrometry: 2626.12625.8 (calculated value 2627.12626.1)
Elution time on HPLC: 21.4 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 53

Production of human GPR8 ligand (2-23): Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 107)

In a similar manner to Reference Example 12, desired amino acid sequence was introduced to the resin. After removal of the last Tyr, Fmoc group was removed from the resin. Then, by treatment of TFA/thioanisole/m-cresol/triisopropylsilane/ethanedithiol (85/5/5/2.5/2.5), excision from the resin and removal of protecting group of side chain were simultaneously performed. Crude peptide was purified in a similar manner to Reference Example 12 to givea target.
$(M+H)^+$ by mass spectrometry: 2397.1 (calculated value 2397.3)
Elution time on HPLC: 19.9 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 54

Production of human GPR8 ligand (4-23): His-Val-Ala-Ser-Pro-Arg-Tyr-His-Thr-Val-Gly-Arg-Ala-Ala-Gly-Leu-Leu-Met-Gly-Leu (SEQ ID NO: 108)

In a similar manner to Reference Example 12, desired amino acid sequence was introduced to the resin. After removal of the last His, Fmoc group was removed from the resin. Then, by treatment of TFA/thioanisole/m-cresol/triisopropylsilane/ethanedithiol (85/5/5/2.5/2.5), excision from the resin and removal of protecting group of side chain were simultaneously performed. Crude peptide was purified in a similar manner to Reference Example 12 to givea target.
$(M+H)^+$ by mass spectrometry: 2106.0 (calculated value 2106.1)
Elution time on HPLC: 20.0 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 55

Production of human GPR8 ligand (9-23): Arg-Tyr-His-Thr-Val-Gly-Arg-Ala-Ala-Gly-Leu-Leu-Met-Gly-Leu (SEQ ID NO: 109)

In a similar manner to Reference Example 12, desired amino acid sequence was introduced to the resin. After removal of the last Arg, Fmoc group was removed from the resin. Then, by treatment of TFA/thioanisole/m-cresol/triisopropylsilane/ethanedithiol (85/5/5/2.5/2.5), excision from the resin and removal of protecting group of side chain were simultaneously performed. Crude peptide was purified in a similar manner to Reference Example 12 to givea target.
$(M+H)^+$ by mass spectrometry: 1615.0 (calculated value 1614.9)
Elution time on HPLC: 20.2 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 56

Production of human GPR8 ligand (15-23): Arg-Ala-Ala-Gly-Leu-Leu-Met-Gly-Leu (SEQ ID NO: 110)

In a similar manner to Reference Example 12, desired amino acid sequence was introduced to the resin. After removal of the last Arg, Fmoc group was removed from the resin. Then, by treatment of TFA/thioanisole/m-cresol/triisopropylsilane/ethanedithiol (85/5/5/2.5/2.5), excision from the resin and removal of protecting group of side chain were simultaneously performed. Crude peptide was purified in a similar manner to Reference Example 12 to givea target.
$(M+H)^+$ by mass spectrometry: 901.4 (calculated value 901.5)
Elution time on HPLC: 20.2 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 57

Production of [N-Acetyl-Tyr$^2$]-human GPR8 ligand (2-23): Ac-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 111)

After completion of acetylation of the resin prepared in Reference Example 53 with acetic anhydride, the resin was treated in a similar manner to Reference Example 53 and the peptide was purified to give a target.
$(M+H)^+$ by mass spectrometry: 2439.3 (calculated value 2439.3)
Elution time on HPLC: 20.2 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 58

Production of [D-Trp$^1$]-human GPR8 ligand (1-23): D-Trp-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 112)

Using Fmoc-D-Trp(Boc) instead of Fmoc-Trp(Boc) in Reference Example 12, a target was obtained in a similar manner.
$(M+H)^+$ by mass spectrometry: 2583.4 (calculated value 2583.4)
Elution time on HPLC: 20.6 minutes
Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 59

Production of [N-3-Indolepropanoyl-Tyr$^2$]-human GPR8 ligand (2-23): 3-Indolepropanoyl-Tyr-Lys-His-Val-Ala-Ser-Pro-Arg-Tyr-His-Thr-Val-Gly-Arg-Ala-Ala-Gly-Leu-Leu-Met-Gly-Leu (SEQ ID NO: 113)

Using 3-Indolepropionic acid instead of Fmoc-Trp(Boc) in Reference Example 12, a target was obtained. By treatment of TFA/thioanisole/m-cresol/triisopropylsilane/ethanedithiol (85/5/5/2.5/2.5), an excision of the peptide from the resin and removal of side chain protecting group were carried out at the same time. The crude peptide was purified in a similar way as described in Reference Example 12 to get a target.
$(M+H)^+$ by mass spectrometry: 2568.4 (calculated value 2568.4)
Elution time on HPLC: 21.7 minutes

Conditions for elution
Column: Wakosil-II 5C18HG (4.6 x 100 mm)
Eluant: using Solution A: 0.1% TFA-water and Solution B: acetonitrile containing 0.1% TFA, elution by linear concentration gradient from A/B: 100/0 to 30/70 (35 minutes)
Flow rate: 1.0 ml/minute

REFERENCE EXAMPLE 60

1) Amplification of human GPR7 DNA using human chromosomal DNA by PCR method

Subsequently, using human chromosomal DNA as a template and two synthetic DNA primers (SEQ ID NO: 190 and SEQ ID NO: 191), amplification by PCR method was performed. The synthetic primers were constructed to allow a region of the gene to be translated to the receptor protein to amplify. Therewith, at the 5' end of the gene, the base sequence recognized by restriction enzyme ClaI was added, and at the 3' end, the base sequence recognized by restriction enzyme SpeI was added. The reaction solution in the above reaction comprised of 0.5 µg of human chromosomal DNA (Takara Shuzo), 1 µM each of synthetic DNA primers, 0.8 mM dNTPs, 1 mM $MgCl_2$, 1 µl of KOD Polymerase (TOYOBO) and a buffer attached to the enzyme to make the total volume 50 µl. The PCR reaction was carried out using a thermal cycler (PE Biosystems) by heating of 94°C for 60 seconds, then a cycle set to include 98°C for 15 seconds followed by 65°C for 2 seconds and 74°C for 30 seconds, which was repeated 35 times. The amplified product was confirmed by 0.8% agarose gel electrophoresis follwed by ethidium bromide staining.

2) Subcloning of the PCR product into plasmid vector and confirmation of a sequence of the amplified DNA by decoding of the base sequence of inserted DNA region

Using the PCR reaction solution in 1) described above, DNA was isolated by 0.8% low melting agarose gel electrophoresis. The DNA band was excised from the gel with razor, and was recovered by crashing the pieces of agarose, phenol extraction, phenol-chroloform extraction and ethanol precipitation. In the manner prescribed in PCR-Script™ Amp SK(+) Cloning Kit (Stratagene), the recovered DNA was subcloned to plasmid vector pCR-Script Amp SK(+). After transformation of Escherichia coli DH5α competent cell (TOYOBO) by introducing the above-mentioned vector, clones harboring cDNA insert fragment was selected on LB agar medium containing ampicillin, IPTG and X-gal. All the white-colored clones were isolated with sterilized toothpick, and then the transformant E. coli DH5α/GPR7 was obtained. Respective clones were cultured in LB medium containing ampicillin for overnight. Subsequently, the plasmid DNA was prepared using QIAwell 8 Plasmid Kit (Qiagen). A portion of the prepared DNA was cleaved with the restriction enzymes ClaI and SpeI, and a size of the receptor cDNA fragment inserted was confirmed. The reaction for determination of the base sequence was carried out using DyeDeoxy Terminator Cycle Sequence Kit (PE Biosystems PE Biosystems). As a result, after decoding with the fluorescent automated sequencer, the DNA sequence was obtained (SEQ ID NO: 183). The pCR-Script Amp SK(+) plasmid harboring a DNA having the base sequence represented by SEQ ID NO: 183 was designated pCR-Script human GPR7. An amino acid sequence of human GPR7 encoded by DNA having the base sequence represented by SEQ ID NO: 183 was shown by SEQ ID NO: 182. Two bases in the DNA sequence of human GPR7 determined hereinabove was different from the DNA sequence described in O'Dowd's report (O'Dowd, B. F., et al., Genomics 28, 84-91, 1995). These two bases correspond to the 893rd and the 894th base in the sequence of SEQ ID NO: 183. These are C and G in O'Dowd's report, respectively, whereas G and C in the reference example, respectively. Therefore, for the amino acid sequence to be translated, the 296th amino acid of SEQ ID NO: 182 changes Thr in O'Dowd's report to Ser in the example.

REFERENCE EXAMPLE 61

Acquisition of GPR7 ligand precursor gene from human whole brain cDNA by PCR method and construction of expression plasmid

Using human whole brain cDNA as a template, and two synthetic DNA as described below, amplification was done by PCR:

GSF1: 5'-GTCGACATGGCCCGGTCCGCGACACTGGCGGCC-3' (SEQ ID NO: 184)

GSR2: 5'-GCTAGCAGCGGTGCCAGGAGAGGTCCGGGCTCA-3' (SEQ ID NO: 185)

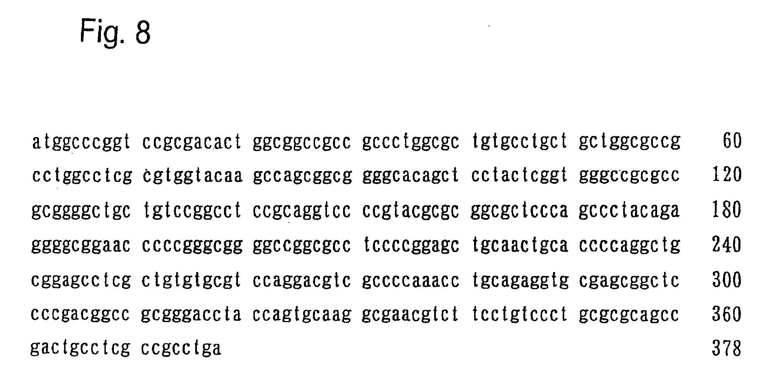

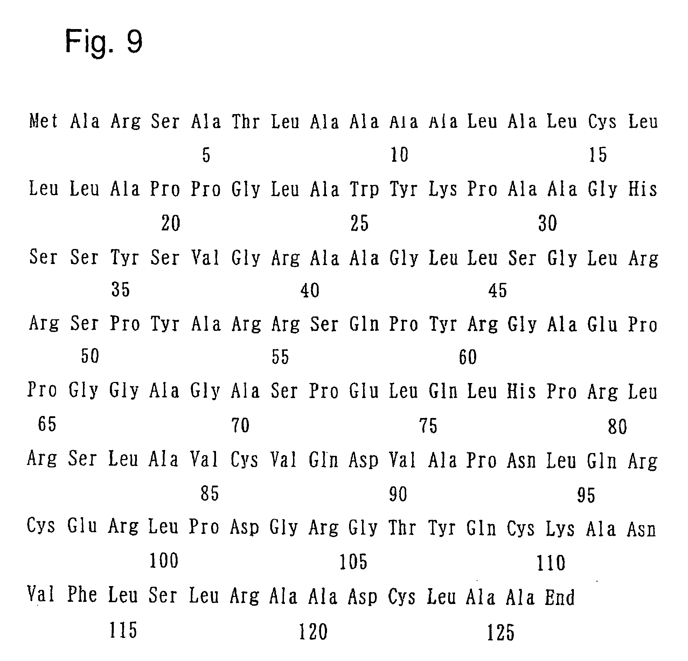

The reaction solution comprised of 1 µl of cDNA solution, 0.5 µl of GSF1 (10 µM), 0.5 µl of GSR2 (10 µM), 2.5µl of 10 x reaction solution attached, 2.5µl of dNTPs (10 mM), 0.5 µl of KlenTaq (CLONTECH) and 17.5 µl of Ohtsuka distilled water to the enzyme to make the total volume 25 µl. The PCR reaction was carried out using Thermal Cycler 9600 by heating of 95°C for 2 minutes, then a cycle set to include 98°C for 10 seconds followed by 60°C for 20 seconds and 72°C for 20 seconds, which was repeated 35 times. Using a portion of the PCR product, an amplification of the PCR product consisting of about 400 bp was confirmed by electrophoresis. Then, the PCR product was purified using QIAGEN PCR Purification Kit. Directly the sequencing was done, and a sequence shown in FIG. 8 was obtained. An amino acid sequence deduced from the DNA sequence shown in FIG. 8 was shown in FIG. 9. Subsequently, the PCR product recovered from the gel was subcloned into Escherichia coli JM109 using the TA Cloning Kit (Invitrogen) to get Escherichia coli JM109/pTAhGPR7-1. From Escherichia coli obtained by subcloning, plasmid pTAhGPR7-1 was extracted with plasmid extraction instrument (Kurabo). A base sequence of inserted fragment was determined and was confirmed to be a human GPR7 ligand cDNA identical to that shown in FIG. 8. Then, from the plasmid, after digestion by restriction enzymes Sall and Nhel, about 0.4 kb of human GPR7 ligand cDNA fragment was obtained. Further, a vector region of the expression vector for animal cells, pAKKO-111H was recovered by digestion of restriction sites, i. e. Sail site and Nhel site in the multicloning site, and electrophoresis. The human GPR7 ligand cDNA fragment prepared as described above and expression vector were ligated by ligation. Escherichia coli JM109 was transformed with this plasmid to obtain E.coli JM109/pAK-S64.

Transformant Escherichia coli JM109/pAK-S64 was cultured for preparation of pAK-S64 plasmid DNA in large quantities.

REFERENCE EXAMPLE 62

Acquisition of GPR7 ligand precursor gene from mouse whole brain cDNA by PCR method

Using mouse whole brain cDNA as a template, and two synthetic DNA as described below, amplification was done by PCR:

MFSAL1: 5'-GTCGACAGCTCCATGGCCCGGTGTAGGACGCTG-3' (SEQ ID NO: 186)

MRNHE1: 5'-GCTAGCTCAGGTGCTCTGGCAATCAGTCTCGTG-3' (SEQ ID NO: 187)

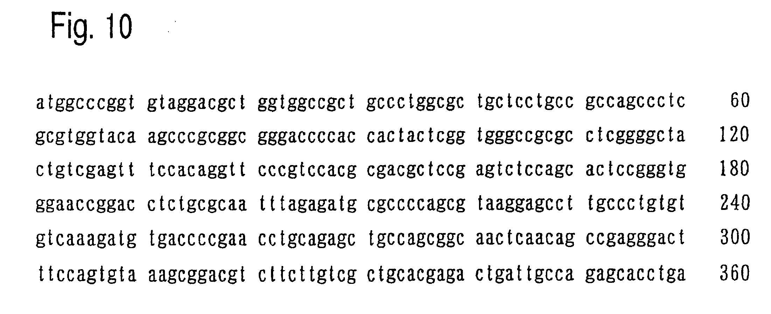

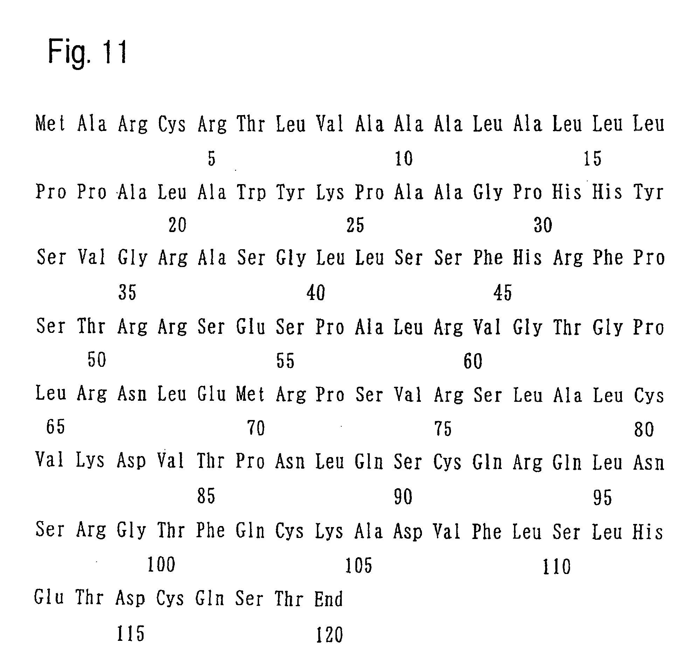

The reaction solution comprised of 1 µl of cDNA solution, 0.5 µl of MFSAL1 (10 µM), 0.5 µl of MRNHE1 (10 µM), 2.5µl of 10 x reaction solution attached, 2.5µl of dNTPs (10 mM), 0.5 µl of KlenTaq (CLONTECH) and 17.5 µl of Ohtsuka distilled water to the enzyme to make the total volume 25 µl. The PCR reaction was carried out using Thermal Cycler 9600 by heating of 95°C for 2 minutes, then a cycle set to include 98°C for 10 seconds followed by 60°C for 20 seconds and 72°C for 20 seconds, which was repeated 35 times. Using a portion of the PCR product, an amplification of the PCR product consisting of about 400 bp was confirmed by electrophoresis. Then, the PCR product was purified using QIAGEN PCR Purification Kit. Directly the sequencing was done, and a sequence shown in FIG. 10 was obtained. An amino acid sequence deduced from the DNA sequence shown in FIG. 10 was shown in FIG. 11. Subsequently, the PCR product recovered from the gel was subcloned into Escherichia coli JM109 using the TA Cloning Kit (Invitrogen) to get Escherichia coli JM109/pTAmGPR7-1. From Escherichia coli obtained by subcloning, plasmid pTAmGPR7-1 was extracted with plasmid extraction instrument (Kurabo). A base sequence of inserted fragment was determined and was confirmed to be a mouse GPR7 ligand cDNA identical to that shown in FIG. 10.

REFERENCE EXAMPLE 63

Acquisition of GPR7 ligand precursor gene from rat whole brain cDNA by PCR method

Using rat whole brain cDNA as a template, and two synthetic DNA as described below, amplification was done by PCR:

RF: 5'-CACGGCTCCATGGTCCGGTGTAGGACG-3' (SEQ ID NO: 188)

RR: 5'-CAGCGTCGAGGTTTGGGTTGGGGTTCA-3' (SEQ ID NO: 189)

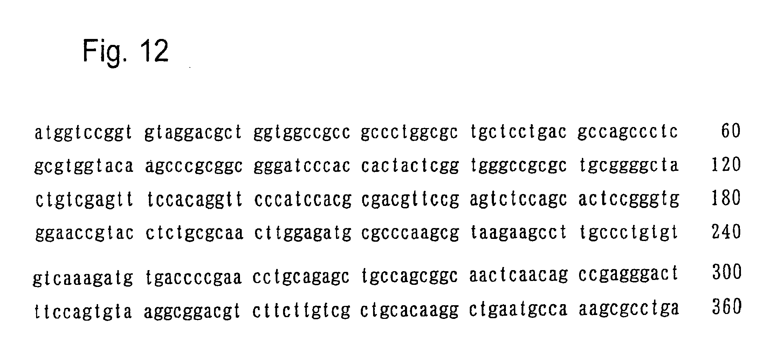

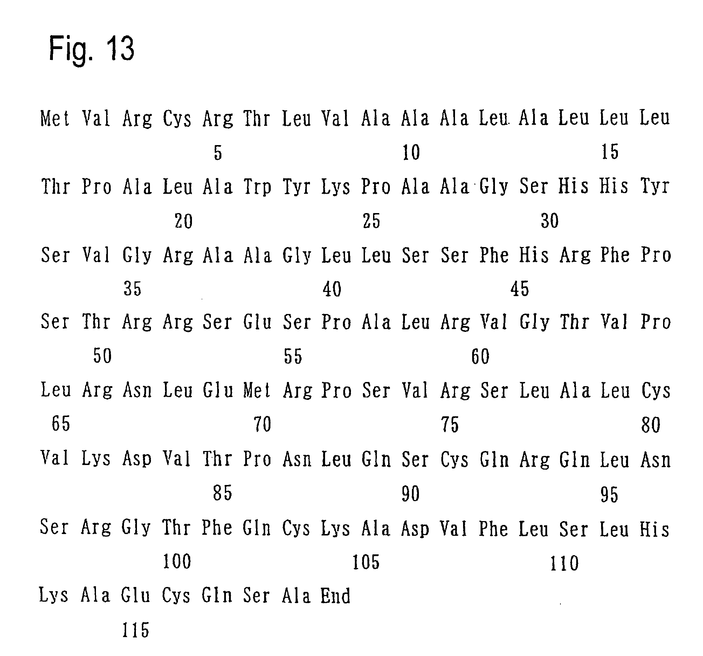

The reaction solution comprised of 1 µl of cDNA solution, 0.5 µl of RF (10 µM), 0.5 µl of RR (10 µM), 2.5µl of 10 x reaction solution attached, 2.5µl of dNTPs (10 mM), 0.5 µl of KlenTaq (CLONTECH) and 17.5 µl of Ohtsuka distilled water to the enzyme to make the total volume 25 µl. The PCR reaction was carried out using Thermal Cycler 9600 by heating of 95°C for 2 minutes, then a cycle set to include 98°C for 10 seconds followed by 60°C for 20 seconds and 72°C for 20 seconds, which was repeated 35 times. Using a portion of the PCR product, an amplification of the PCR product consisting of about 400 bp was confirmed by electrophoresis. Then, the PCR product was purified using QIAGEN PCR Purification Kit. Directly the sequencing was done, and a sequence shown in FIG. 12 was obtained. An amino acid sequence deduced from the DNA sequence shown in FIG. 12 was shown in FIG. 13.

Subsequently, the PCR product recovered from the gel was subcloned into Escherichia coli JM109 using the TA Cloning Kit (Invitrogen) to get Escherichia coli JM109/pTArGPR7-1. From Escherichia coli obtained by subcloning, plasmid pTArGPR7-1 was extracted with plasmid extraction instrument (Kurabo). A base sequence of inserted fragment was determined and was confirmed to be a rat GPR7 ligand cDNA identical to that shown in FIG 12.

REFERENCE EXAMPLE 64

Transient expression of GPR7 expression plasmid and reporter plasmid in Chinese hamster ovary (CHO) cells

Using a plasmid inserted the human GPR7 DNA obtained in the above reference example into pAKKO-111H, the expression plasmid for animal cells, by the publicly known method, Escherichia coli JM109 was transformed. A colony obtained was isolated and cultured for preparation of GPR7 expression plasmid DNA with QIAGEN Plasmid Maxi Kit (Qiagen). Further, plasmid DNA of pCRE-Luc (CLONTECH), in which luciferase gene is ligated as areporter downstream CAMP response element (CRE), was prepared in a similar method.

GPR7 expression plasmid and pCRE-Luc were transiently expressed in CHO cells, in which an expression vector without a receptor gene was introduced. The CHO cells were seeded in 96-well plate (Corning Star) at 40,000 cells/well and 100 µl of culture volume, and were cultured at 37°C for overnight. For a culture on the plate, DMEM (Dulbecco's modified Eagle's medium, CibcoBRL) supplemented with nothing but 10% fetal bovine serum was used.

Each plasmid was diluted to 240 ng/µl and was added to 240 µl of Opti-MEM-I (GibcoBRL) at the ratio of 9 µl of GPR7 expression plasmid and 1 µl of pCRE-Luc. This solution was mixed with the same volume of solution wherein 10 µl of Lipofectamine 2000 was added to 240 µl of Opti-MEM-I to form a complex of liposome and plasmid DNA according to the method described in the manual attached to Lipofectamine 2000. To culture of CHO cells, 25 µl/well of the above-mentioned solution was added. After 4 hours, a culture broth was replaced to an assay buffer (DMEM supplemented with 0.1% bovine serum albumin) to be serum-free. Then, it was cultured at 37°C for overnight.

REFERENCE EXAMPLE 65

Expression of ligand gene in CHO cells

The expression plasmid for animal cells, pAK-S64, in which human ligand cDNA prepared in the above-mentioned reference example was inserted, was transiently expressed in CHO cells by the same method as described above. The cells were seeded at 600,000 cells/well in 6-well plate (Falcon). After cultivation for overnight, a plasmid having ligand gene was introduced. Ten microliters of plasmid diluted to 240 ng/µl was added to 240 µl of Opti-MEM-I. This solution was mixed with the same volume of solution wherein 10 µl of Lipofectamine 2000 was added to 240 µl of Opti-MEM-I to form a complex of liposome and plasmid DNA according to the method described in the manual attached to Lipofectamine 2000. To culture of CHO cells, 500 µl/well of the above-mentioned solution was added. After 4 hours, a culture broth was replaced to an assay buffer to be serum-free. After 18 hours of replacement of medium, a medium in each well was recovered to acquire a culture supernatant of CHO cells containing ligand peptide.

REFERENCE EXAMPLE 66

1) Detection for an inhibition of luciferase activity in CHO cells, in which GPR7 is transiently expressed, by supernatant of S64 expressing cells

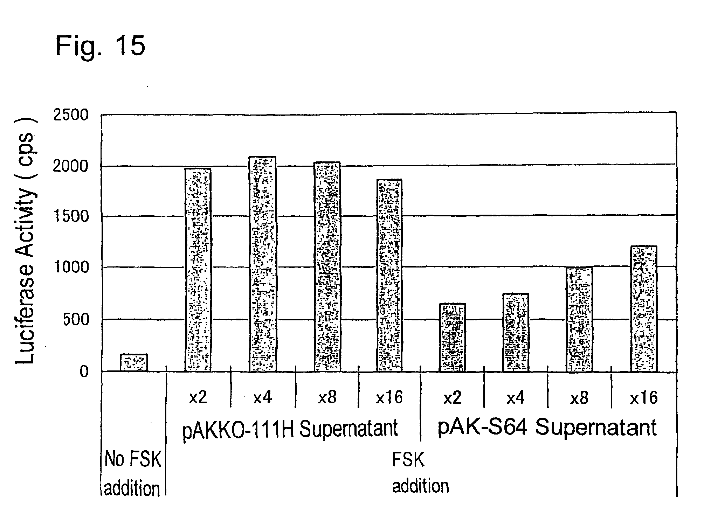

According to the method of Reference Example 64, to a culture of CHO cells, in which GPR7 was transiently expressed, a culture supernatant prepared in Reference Example 65, in which pAK-S64 was expressed, and forskolin to be 2 μM at a final concentration were added. Further, a culture supernatant of CHO cells, in which a raw expression vector (pAKKO-111H), in which no gene for ligand was inserted, was transiently expressed, was added in a similar manner. On these occasions, the supernatant, in which the DNA was expressed, was diluted to 2-fold, 4-fold, 8-fold and 16-fold with assay buffer. Incubation at 37°C for 4 hours after adding the supernatant was performed, and then enhancement or inhibition of transcription and/or translation of the reporter (luciferase) gene derived from intracellular signal transduction reised by agonist activity of ligand, which is mediated by receptor, was induced. After completion of incubation, an assay buffer in each well was removed, and a 50 μl of luminescent substrate of PicaGene LT2.0 (TOYO Ink) was added to each well. After lysing cells and fully mixing with substrate, a level of luminescence derived from an induction level of expression for the reporter gene in each well was assayed using a plate reader (ARVOsx multilabel counter, Perkin Elmer). As a result, only when a culture supernatant of pAK-S64 was added, an inhibition of expression of the reporter gene was detected as a decrease of luciferase activity (FIG. 15). In addition, an extent of the inhibition was dependent on the concentration of culture supernatant of pAK-S64. This shows that a product expressed by the plasmid inserted in pAK-S64 transduced an intracellular signal mediated by GPR7, i.e., the product acted as a ligand to GPR7.

2) Detection for an inhibition of luciferase activity in CHO cells, in which GPR7 is transiently expressed, by supernatant of S64 expressing cells

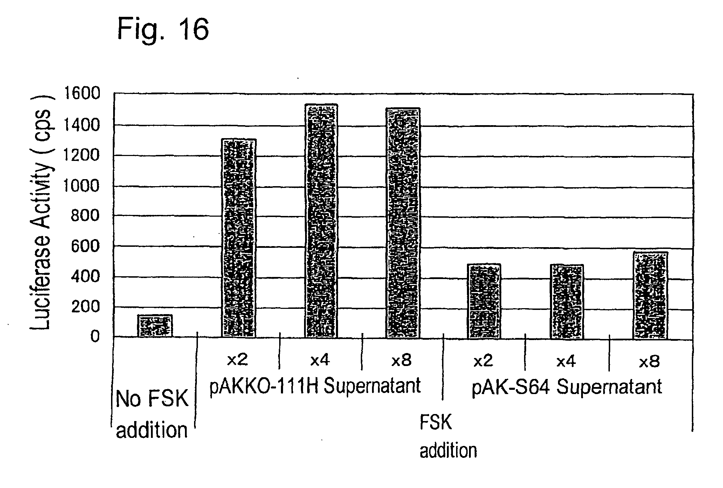

Using a plasmid inserted TGR26 DNA obtained in Reference Example 63 into pAKKO-111H, the expression plasmid for animal cells by the publicly known method, TGR26 expressing plasmid DNA was prepared in the same manner as Reference Example 64. This DNA was transiently co-expressed with luciferase gene in CHO cells by the method shown in Reference Example 64. By adding a culture supernatant prepared in Reference Example 65, in which pAK-S64 was expressed, culture supernatant of cells, in which a raw expression vector was expressed, and forskolin to be 2 μM at a final concentration to the cells, a ligand activity was detected in the same manner as 1) described above. As a result, by adding a supernatant of pAK-S64, luciferase activity, which is increased by forskolin, was decreased depending on the concentration (FIG. 16).

INDUSTRIAL APPLICABILITY

The G protein-coupled receptor protein of the present invention, its partial peptides, or salts thereof and the polynucleotides encoding the receptor protein or its partial peptide (e.g. DNA, RNA, and its derivatives) can be used for; 1) determination of ligands (agonists); 2) preparation of antibodies and antisera; 3) construction of recombinant receptor protein expression systems; 4) development of the receptor binding assay systems using the expression systems and screening of pharmaceutical candidate compounds; 5) effecting drug design based on comparison with structurally similar ligand receptors; 6) reagents for preparation of probes and PCR primers for gene diagnosis; 7) production of transgenic animals; and 8) pharmaceutical drugs for the gene prophylaxis and gene therapy.

## Claims

1. A G protein-coupled receptor protein containing the same or substantially the same amino acid sequence as the amino acid sequence represented by SEQ ID NO: 1 or SEQ ID NO: 138, or a salt thereof.

2. A G protein-coupled receptor protein according to claim 1, or a salt thereof, which comprises containing an amino acid sequence represented by SEQ ID NO: 1.

3. A G protein-coupled receptor protein according to claim 1, or a salt thereof, which comprises containing an amino acid sequence represented by SEQ ID NO: 138.

4. A partial peptide of the G protein-coupled receptor protein according to claim 1, or a salt thereof.

5. A polynucleotide containing a polynucleotide encoding the G protein-coupled protein according to claim 1.

6. A polynucleotide according to claim 5, which is DNA.

7. A DNA according to claim 6, which is represented by SEQ ID NO: 2 or SEQ ID NO: 139.

8. A recombinant vector containing the polynucleotide according to claim 5.

**9.** A transformant transformed with the recombinant vector according to claim 8.

**10.** A method of manufacturing the G protein-coupled receptor protein or its salt according to claim 1, which comprises culturing the transformant according to claim 9 and accumulating the G protein-coupled receptor protein according to claim 1.

**11.** An antibody to the G protein-coupled receptor protein according to claim 1, the partial peptide according to claim 4, or a salt of said protein or partial peptide.

**12.** An antibody according to claim 11, which is a neutralizing antibody capable of inactivating signal transduction of the G protein-coupled receptor protein according to claim 1.

**13.** A diagnostic composition comprising an antibody according to claim 11.

**14.** A ligand to the G protein-coupled receptor protein or its salt according to claim 1, which is obtainable using the G protein-coupled receptor protein according to claim 1 or the partial peptide according to claim 4, or a salt of said protein or partial peptide.

**15.** A pharmaceutical composition comprising the ligand to the G protein-coupled receptor according to claim 14.

**16.** A method of determining a ligand to the G protein-coupled receptor protein or its salt according to claim 1, which comprises using the G protein-coupled receptor protein according to claim 1 or the partial peptide according to claim 4, or a salt of said protein or partial peptide.

**17.** A method of screening a compound that alters the binding property between a ligand and the G protein-coupled receptor protein or its salt according to claim 1, which comprises using the G protein-coupled receptor protein according to claim 1 or the partial peptide according to claim 4, or a salt of said protein or partial peptide.

**18.** A kit for screening a compound or its salt that alters the binding property between a ligand and the G protein-coupled receptor protein or its salt according to claim 1, comprising the G protein-coupled receptor protein according to claim 1 or the partial peptide according to claim 4, or a salt of said protein or partial peptide.

**19.** A compound or its salt that alters the binding property between a ligand and the G protein-coupled receptor protein or its salt according to claim 1, which is obtainable using the screening method according to claim 17 or the screening kit according to claim 18.

**20.** A pharmaceutical composition comprising a compound or its salt that alters the binding property between a ligand and the G protein-coupled receptor protein or its salt according to claim 1, which is obtainable using the screening method according to claim 17 or the screening kit according to claim 18.

**21.** A polynucleotide that hybridizes to the polynucleotide according to claim 5 under a highly stringent condition.

**22.** A polynucleotide comprising a base sequence complementary to the polynucleotide according to claim 5 or a part of the base sequence.

**23.** A method of quantifying mRNA of the G protein-coupled receptor protein according to claim 1, which comprises using the polynucleotide according to claim 5 or a part of the polynucleotide.

**24.** A method of quantifying the G protein-coupled receptor protein according to claim 1, which comprises using the antibody according to claim 11.

**25.** A diagnostic method for a disease associated with functions of the G protein-coupled receptor protein according to claim 1, which comprises using the quantification method according to claim 23 or claim 24.

**26.** A method of screening a compound or its salt that alters the expression level of the G protein-coupled receptor protein according to claim 1, which comprises using the quantification method according to claim 23.

**27.** A method of screening a compound or its salt that alters the amount of the G protein-coupled receptor protein according to claim 1 in cell membrane, which comprises using the quantification method according to claim 24.

**28.** A compound or its salt that alters the expression level of the G protein-coupled receptor protein according to claim 1, which is obtainable using the screening method according to claim 26.

**29.** A compound or its salt that alters the amount of the G protein-coupled receptor protein according to claim 1 in cell membrane, which is obtainable using the screening method according to claim 27.

**30.** The screening method according to claim 17, which comprises comparing (i) the case where the G protein-coupled receptor protein according to claim 1 or its salt, or the partial peptide according to claim 4 or its salt is contacted with ligand, with (ii) the case where the G protein-coupled receptor protein according to claim 1 or its salt, or the partial peptide according to claim 4 or its salt is contacted with ligand and test compound.

**31.** The screening method according to claim 30, wherein the ligand is the polypeptide containing an identical or substantially identical amino acid sequence to that represented by SEQ ID NO: 8.

**32.** The screening method according to claim 30, wherein the ligand is the polypeptide containing an amino acid sequence represented by SEQ ID NO: 8.

**33.** The screening method according to claim 30, wherein the ligand is the polypeptide having an amino acid sequence represented by SEQ ID NO: 8.

**34.** The screening method according to claim 30, wherein the ligand is the polypeptide having an amino acid sequence represented by SEQ ID NO: 9.

**35.** The diagnostic according to claim 13, which is a diagnostic for obesity.

**36.** The medicament according to claim 15 or claim 20, which is an anti-obesity drug.

**37.** The medicament according to claim 15 or claim 20, which is an appetite enhancer.

**38.** The medicament according to claim 15 or claim 20, which is an inhibitor of prolactin production..

**39.** A method for prevention and/or treatment of obesity, which comprises administrating an effective amount of a compound or a salt thereof that alters a binding property between the ligand according to claim 19 and the G protein-coupled receptor protein according to claim 1 or a salt thereof, to mammal.

**40.** A method for enhancing appetite, which comprises administrating an effective amount of a compound or a salt thereof that alters a binding property between the ligand according to claim 19 and the G protein-coupled receptor protein according to claim 1 or a salt thereof, to mammal.

**41.** A method for inhibiting a prolactin production, which comprises administrating an effective amount of a compound or a salt thereof that alters a binding property between the ligand according to claim 19 and the G protein-coupled receptor protein according to claim 1 or a salt thereof, to mammal.

**42.** Use of a compound or a salt thereof that alters a binding property between the ligand according to claim 19 and the G protein-coupled receptor protein according to claim 1 or a salt thereof for manufacturing an anti-obesity drug.

**43.** Use of a compound or a salt thereof that alters a binding property between the ligand according to claim 19 and the G protein-coupled receptor protein according to claim 1 or a salt thereof for manufacturing an appetite enhancer.

**44.** Use of a compound or a salt thereof that alters a binding property between the ligand according to claim 19 and the G protein-coupled receptor protein according to claim 1 or a salt thereof for manufacturing an inhibitor of prolactin production.

**45.** A non-human transgenic animal, which has exogenous DNA encoding the G protein-coupled receptor protein according to claim 1 or mutated DNA thereof.

**46.** The animal according to claim 45, wherein the non-human animal is a rodent.

**47.** The animal according to claim 46, wherein the rodent is mouse or rat.

**48.** A recombinant vector, which contains exogenous DNA encoding the G protein-coupled receptor protein according to claim 1 or mutated DNA thereof, and is capable of expressing in non-human animal.

**49.** A non-human mammalian embryonic stem cell, wherein the DNA encoding the G protein-coupled receptor protein according to claim 1 is inactivated.

**50.** The embryonic stem cell according to claim 49, wherein the non-human mammal is a rodent.

**51.** The embryonic stem cell according to claim 50, wherein the rodent is mouse.

**52.** A non-human mammal barely expressing DNA, wherein the DNA encoding the G protein-coupled receptor protein according to claim 1.

**53.** The non-human mammal according to claim 52, wherein the non-human mammal is a rodent.

**54.** The non-human mammal according to claim 53, wherein the rodent is mouse.

# Fig. 1

10 20 30 40 50 60
ATGCACAACTTGTCGCTCTTCGAGCCTGGCAGGGGCAATGTGTCTTGCGGCGGCCCATTT
MetHisAsnLeuSerLeuPheGluProGlyArgGlyAsnValSerCysGlyGlyProPhe

70 80 90 100 110 120
TTGGGCTGTCCTAACGAGTCGAACCCAGCGCCTCTGCCACTGCCGCAGCCTCTGGCGGTA
LeuGlyCysProAsnGluSerAsnProAlaProLeuProLeuProGlnProLeuAlaVal

130 140 150 160 170 180
GCAGTGCCTGTGGTCTACGGGGTGATCTGCGCGGTGGGACTGGCGGGCAACTCCGCGGTG
AlaValProValValTyrGlyValIleCysAlaValGlyLeuAlaGlyAsnSerAlaVal

190 200 210 220 230 240
CTGTACGTACTGCTGCGCACGCCGCGCATGAAGACTGTTACCAACGTGTTCATTCTCAAC
LeuTyrValLeuLeuArgThrProArgMetLysThrValThrAsnValPheIleLeuAsn

250 260 270 280 290 300
CTGGCTATCGCGGACGAGCTCTTCACCCTCGTGCTGCCCATCAACATCGCGGACTTCCTG
LeuAlaIleAlaAspGluLeuPheThrLeuValLeuProIleAsnIleAlaAspPheLeu

310 320 330 340 350 360
CTGAGGCGCTGGCCCTTCGGGGAAGTCATGTGCAAGCTCATCGTGGCTGTCGACCAGTAC
LeuArgArgTrpProPheGlyGluValMetCysLysLeuIleValAlaValAspGlnTyr

370 380 390 400 410 420
AACACTTTCTCTAGCCTCTACTTCCTCGCCGTCATGAGCGCAGACCGCTACCTGGTTGTC
AsnThrPheSerSerLeuTyrPheLeuAlaValMetSerAlaAspArgTyrLeuValVal

430 440 450 460 470 480
CTGGCCACAGCCGAGTCGCGCCGGGTGTCCGGGCGCACTTATGGTGCAGCGCGGGCTGTC
LeuAlaThrAlaGluSerArgArgValSerGlyArgThrTyrGlyAlaAlaArgAlaVal

490 500 510 520 530 540
AGTCTGGCGGTGTGGGCGCTGGTGACATTGGTCGTGCTGCCTTTTGCGGTATTCGCCCGG
SerLeuAlaValTrpAlaLeuValThrLeuValValLeuProPheAlaValPheAlaArg

# Fig. 2

550 560 570 580 590 600
CTGGACGAAGAGCAGGGTCGGCGTCAGTGCGTGCTGGTCTTCCCGCAGCCTGAGGCCTTC
LeuAspGluGluGlnGlyArgArgGlnCysValLeuValPheProGlnProGluAlaPhe

610 620 630 640 650 660
TGGTGGCGCGCCAGCCGTCTGTACACTCTAGTGTTGGGCTTCGCCATCCCGGTGTCCACC
TrpTrpArgAlaSerArgLeuTyrThrLeuValLeuGlyPheAlaIleProValSerThr

670 680 690 700 710 720
ATCTGCGCCCTCTATATCACCCTGTTGTGCCGACTGCGTGCTATCCAGCTAGACAGCCAC
IleCysAlaLeuTyrIleThrLeuLeuCysArgLeuArgAlaIleGlnLeuAspSerHis

730 740 750 760 770 780
GCCAAGGCCCTGGACCGTGCCAAGAAGCGCGTGACCTTGTTGGTGGTGGCGATTCTGGCT
AlaLysAlaLeuAspArgAlaLysLysArgValThrLeuLeuValValAlaIleLeuAla

790 800 810 820 830 840
GTGTGCCTCCTCTGCTGGACACCGTACCACCTGAGCACCATAGTGGCGCTCACCACCGAC
ValCysLeuLeuCysTrpThrProTyrHisLeuSerThrIleValAlaLeuThrThrAsp

850 860 870 880 890 900
CTCCCGCAAACACCGTTGGTCATCGGCATCTCTTACTTCATCACCAGTCTGAGCTATGCC
LeuProGlnThrProLeuValIleGlyIleSerTyrPheIleThrSerLeuSerTyrAla

910 920 930 940 950 960
AACAGCTGCCTCAACCCTTTCCTCTATGCCTTCCTGGACGACAGCTTCCGCAGGAGCCTG
AsnSerCysLeuAsnProPheLeuTyrAlaPheLeuAspAspSerPheArgArgSerLeu

970 980 990
CGGCAGCTGGTGTCATGCCGCACAGCCTGA
ArgGlnLeuValSerCysArgThrAla***

Fig. 3

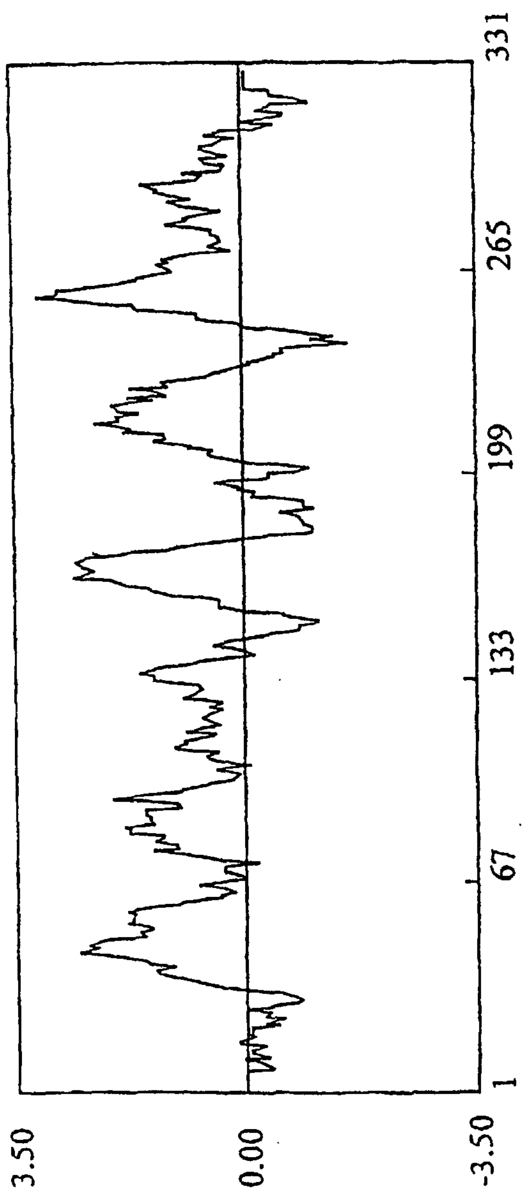

Fig. 4

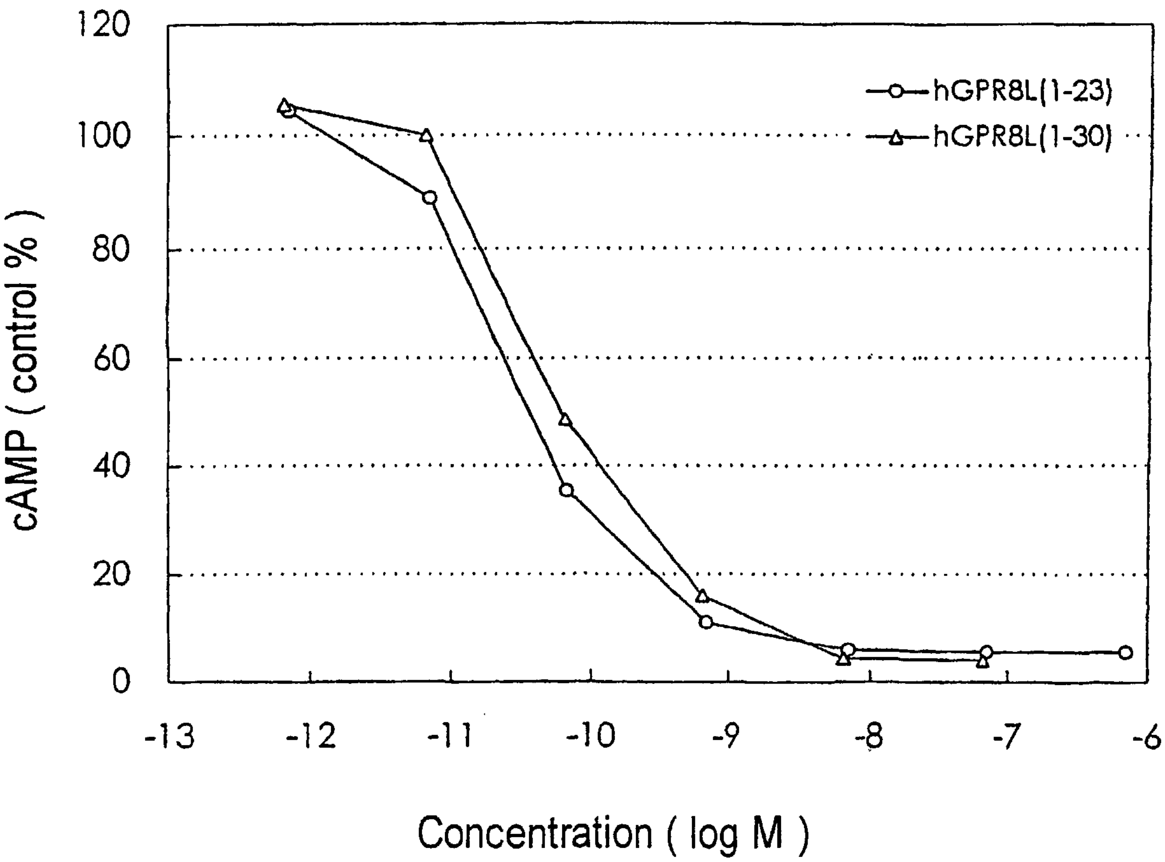
120
100
80
60
40
20
0
cAMP ( control % )
-13
-12
-11
-10
-9
-8
-7
-6
Concentration ( log M )
hGPR8L(1-23)
hGPR8L(1-30)

Fig. 5

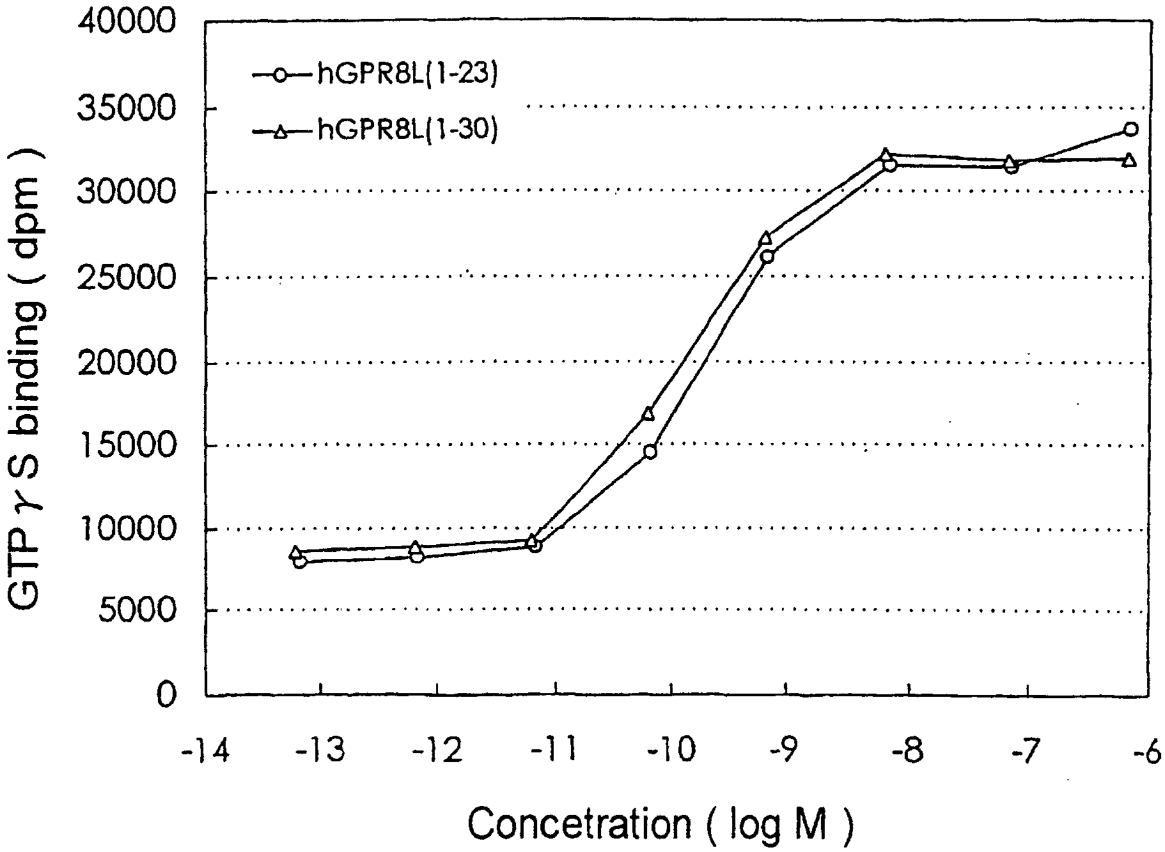
40000
35000
30000
25000
20000
15000
10000
5000
0
GTP γ S binding ( dpm )
hGPR8L(1-23)
hGPR8L(1-30)
-14
-13
-12
-11
-10
-9
-8
-7
-6
Concetration ( log M )

Fig. 6

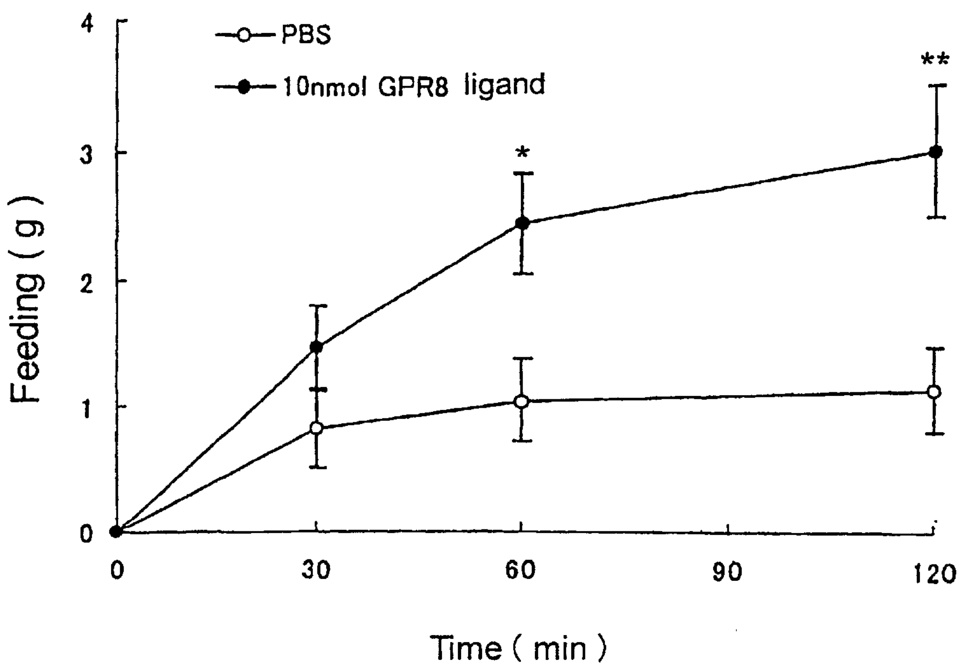

Fig. 7

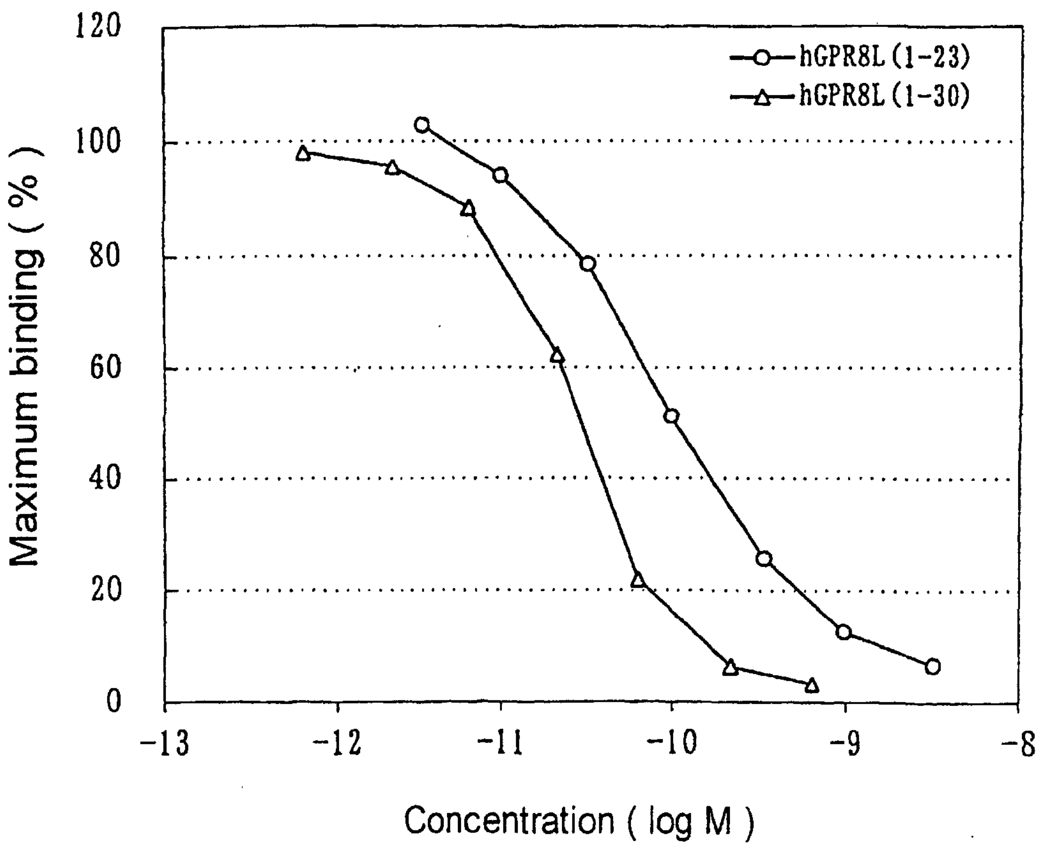
120
100
80
60
40
20
0
Maximum binding ( % )
hGPR8L (1-23)
hGPR8L (1-30)
-13
-12
-11
-10
-9
-8
Concentration ( log M )

Fig. 8

```
atggcccggt ccgcgacact ggcggccgcc gccctggcgc tgtgcctgct gctggcgccg    60
cctggcctcg cgtggtacaa gccagcggcg gggcacagct cctactcggt gggccgcgcc   120
gcggggctgc tgtccggcct ccgcaggtcc ccgtacgcgc ggcgctccca gccctacaga   180
ggggcggaac ccccgggcgg ggccggcgcc tccccggagc tgcaactgca ccccaggctg   240
cggagcctcg ctgtgtgcgt ccaggacgtc gccccaaacc tgcagaggtg cgagcggctc   300
cccgacggcc gcgggaccta ccagtgcaag gcgaacgtct tcctgtccct gcgcgcagcc   360
gactgcctcg ccgcctga                                                 378
```

# Fig. 9

```
Met Ala Arg Ser Ala Thr Leu Ala Ala Ala Ala Leu Ala Leu Cys Leu
                5                   10                  15
Leu Leu Ala Pro Pro Gly Leu Ala Trp Tyr Lys Pro Ala Ala Gly His
            20                  25                  30
Ser Ser Tyr Ser Val Gly Arg Ala Ala Gly Leu Leu Ser Gly Leu Arg
        35                  40                  45
Arg Ser Pro Tyr Ala Arg Arg Ser Gln Pro Tyr Arg Gly Ala Glu Pro
    50                  55                  60
Pro Gly Gly Ala Gly Ala Ser Pro Glu Leu Gln Leu His Pro Arg Leu
65                  70                  75                  80
Arg Ser Leu Ala Val Cys Val Gln Asp Val Ala Pro Asn Leu Gln Arg
                85                  90                  95
Cys Glu Arg Leu Pro Asp Gly Arg Gly Thr Tyr Gln Cys Lys Ala Asn
            100                 105                 110
Val Phe Leu Ser Leu Arg Ala Ala Asp Cys Leu Ala Ala End
        115                 120                 125
```

Fig. 10

```
atggcccggt gtaggacgct ggtggccgct gccctggcgc tgctcctgcc gccagccctc    60
gcgtggtaca agcccgcggc gggaccccac cactactcgg tgggccgcgc ctcggggcta   120
ctgtcgagtt tccacaggtt cccgtccacg cgacgctccg agtctccagc actccgggtg   180
ggaaccggac ctctgcgcaa tttagagatg cgccccagcg taaggagcct tgccctgtgt   240
gtcaaagatg tgaccccgaa cctgcagagc tgccagcggc aactcaacag ccgagggact   300
ttccagtgta aagcggacgt cttcttgtcg ctgcacgaga ctgattgcca gagcacctga   360
```

## Fig. 11

```
Met Ala Arg Cys Arg Thr Leu Val Ala Ala Ala Leu Ala Leu Leu Leu
                 5                  10                  15
Pro Pro Ala Leu Ala Trp Tyr Lys Pro Ala Ala Gly Pro His His Tyr
             20                  25                  30
Ser Val Gly Arg Ala Ser Gly Leu Leu Ser Ser Phe His Arg Phe Pro
         35                  40                  45
Ser Thr Arg Arg Ser Glu Ser Pro Ala Leu Arg Val Gly Thr Gly Pro
     50                  55                  60
Leu Arg Asn Leu Glu Met Arg Pro Ser Val Arg Ser Leu Ala Leu Cys
 65                  70                  75                  80
Val Lys Asp Val Thr Pro Asn Leu Gln Ser Cys Gln Arg Gln Leu Asn
                 85                  90                  95
Ser Arg Gly Thr Phe Gln Cys Lys Ala Asp Val Phe Leu Ser Leu His
            100                 105                 110
Glu Thr Asp Cys Gln Ser Thr End
        115                 120
```

Fig. 12

```
atggtccggt gtaggacgct ggtggccgcc gccctggcgc tgctcctgac gccagccctc    60
gcgtggtaca agcccgcggc gggatcccac cactactcgg tgggccgcgc tgcggggcta   120
ctgtcgagtt tccacaggtt cccatccacg cgacgttccg agtctccagc actccgggtg   180
ggaaccgtac ctctgcgcaa cttggagatg cgcccaagcg taagaagcct tgccctgtgt   240
gtcaaagatg tgaccccgaa cctgcagagc tgccagcggc aactcaacag ccgagggact   300
ttccagtgta aggcggacgt cttcttgtcg ctgcacaagg ctgaatgcca aagcgcctga   360
```

# Fig. 13

Met Val Arg Cys Arg Thr Leu Val Ala Ala Ala Leu Ala Leu Leu Leu
5 10 15
Thr Pro Ala Leu Ala Trp Tyr Lys Pro Ala Ala Gly Ser His His Tyr
20 25 30
Ser Val Gly Arg Ala Ala Gly Leu Leu Ser Ser Phe His Arg Phe Pro
35 40 45
Ser Thr Arg Arg Ser Glu Ser Pro Ala Leu Arg Val Gly Thr Val Pro
50 55 60
Leu Arg Asn Leu Glu Met Arg Pro Ser Val Arg Ser Leu Ala Leu Cys
65 70 75 80
Val Lys Asp Val Thr Pro Asn Leu Gln Ser Cys Gln Arg Gln Leu Asn
85 90 95
Ser Arg Gly Thr Phe Gln Cys Lys Ala Asp Val Phe Leu Ser Leu His
100 105 110
Lys Ala Glu Cys Gln Ser Ala End
115

Fig. 14

```
Human  MARSATLAAAALALCLLLAPPGLAWYKPAA   30
Mouse  MARCRTLVAAALAL---LLPPALAWYKPAA   27
Rat    MVRCRTLVAAALAL---LLTPALAWYKPAA   27

Human  GHSSYSVGRAAGLLSGLRRSPYARRSQPYR   60
Mouse  GPHHYSVGRASGLLSSFHRFPSTRRSES--   55
Rat    GSHHYSVGRAAGLLSSFHRFPSTRRSES--   55

Human  GAEPPGGAGASPELQLHPRLRSLAVCVQDV   90
Mouse  -PALRVGTGPLRNLEMRPSVRSLALCVKDV   84
Rat    -PALRVGTVPLRNLEMRPSVRSLALCVKDV   84

Human  APNLQRCERLPDGRGTYQCKANVFLSLRAA  120
Mouse  TPNLQSCQRQLNSRGTFQCKADVFLSLHET  114
Rat    TPNLQSCQRQLNSRGTFQCKADVFLSLHKA  114

Human  DCLAA                          125
Mouse  DCQST                          119
Rat    ECQSA                          119
```

Fig. 15

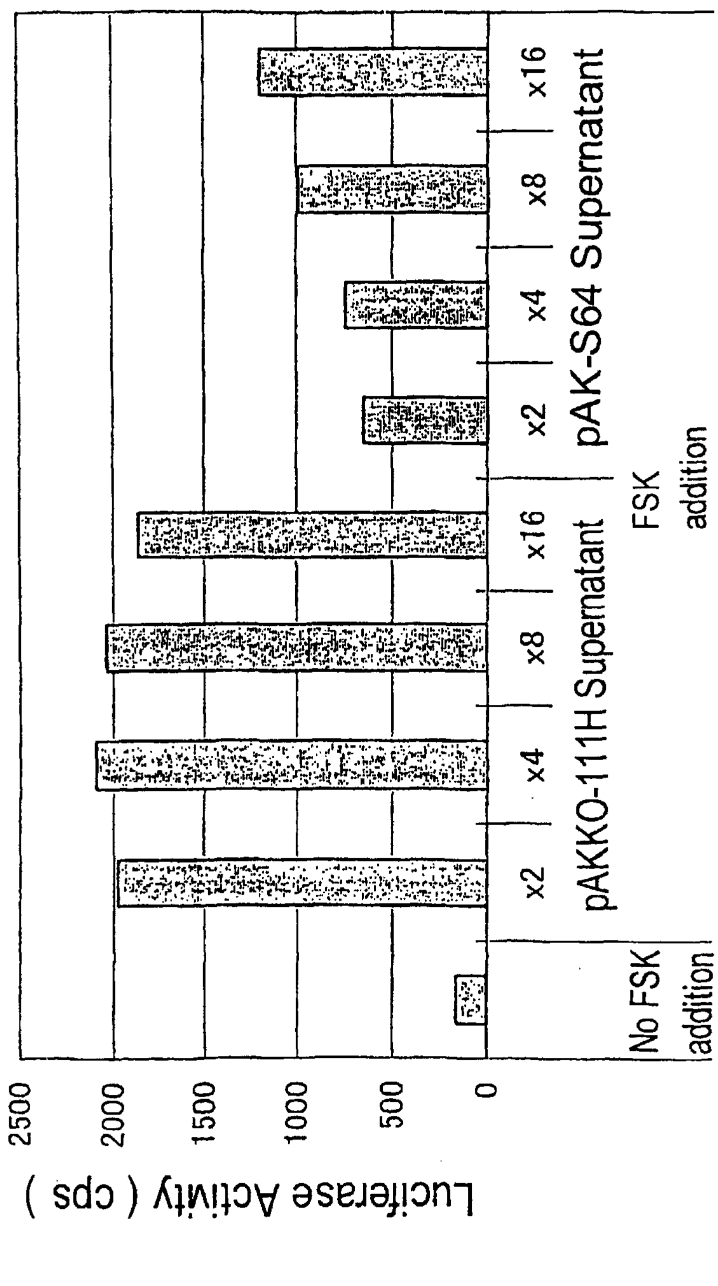
Luciferase Activity ( cps )
2500
2000
1500
1000
500
0
No FSK addition
x2
x4
x8
x16
pAKKO-111H Supernatant
x2
x4
x8
x16
pAK-S64 Supernatant
FSK addition

Fig. 16

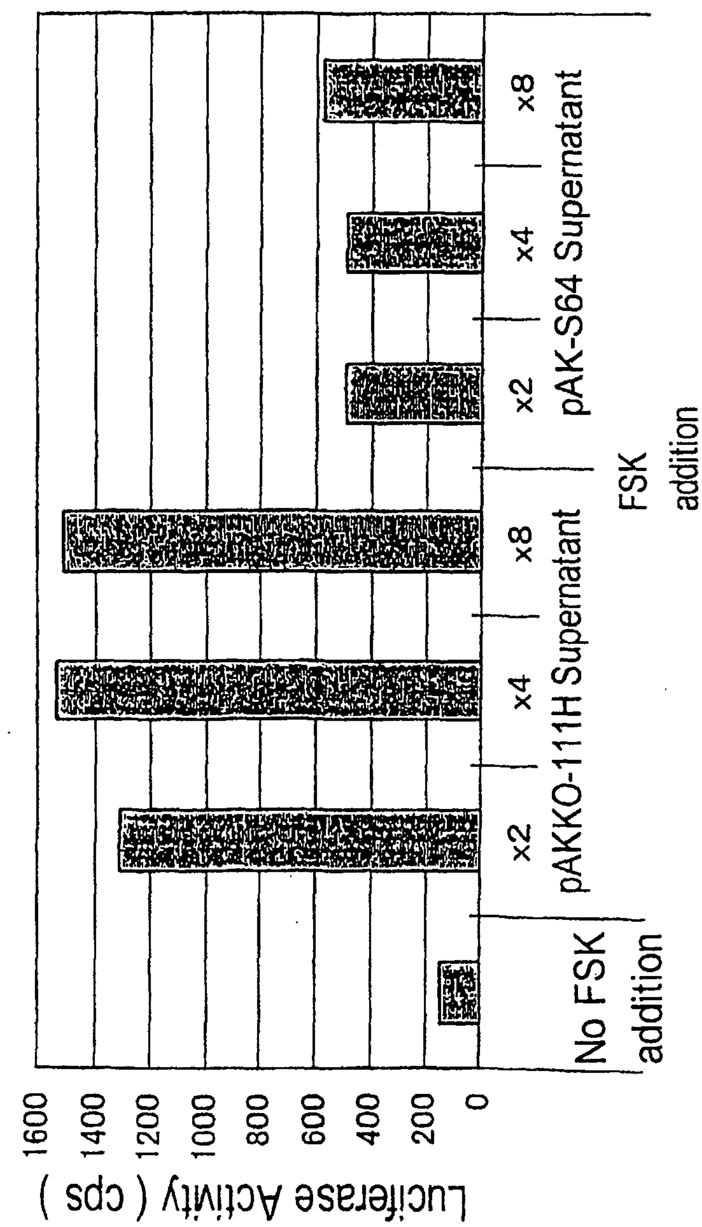
Luciferase Activity ( cps )
1600
1400
1200
1000
800
600
400
200
0
No FSK addition
x2
x4
x8
pAKKO-111H Supernatant
x2
x4
x8
pAK-S64 Supernatant
FSK addition

**INTERNATIONAL SEARCH REPORT**

International application No.
PCT/JP01/10418

A. CLASSIFICATION OF SUBJECT MATTER
Int.Cl[7] C12N15/12, C07K14/705, C07K16/28, C12P21/02, C12Q1/68, A61K45/00, A61P25/00, A61P29/00, A61P9/00, A61P35/00, A61P3/00, A61P37/02, A61P1/00

According to International Patent Classification (IPC) or to both national classification and IPC

B. FIELDS SEARCHED

Minimum documentation searched (classification system followed by classification symbols)
Int.Cl[7] C12N15/12, C07K14/705, C07K16/28, C12P21/02, C12Q1/68, A61K45/00, A61P25/00, A61P29/00, A61P9/00, A61P35/00, A61P3/00, A61P37/02, A61P1/00

Documentation searched other than minimum documentation to the extent that such documents are included in the fields searched

Electronic data base consulted during the international search (name of data base and, where practicable, search terms used)
Genbank/EMBL/DDBJ/GeneSeq, SwissProt/PIR/GeneSeq, MEDLINE (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br><br><br>A | O' DOWD, B. F, et al., "The cloning and chromosomal mapping of two novel human opioid-somatostatin-like receptor genes, GPR7 and GPR8, expressed in discrete areas of the brain", Genomics, (1995), Vol.28, No.1, pages 84 to 91 | 1,4<br>2,3,5-13,16, 21-24,26,27, 45-54<br>31-35 |
| X<br><br>Y<br><br><br>A | WO 95/12670 A1 (Alcoholism & Drug Addiction Res. Found.), 11 May, 1995 (11.05.1995), & EP 726949 A1 & US 5591602 A & JP 9-507022 A | 1,4-6,8-12, 16,21,22,48<br>2,3,7,13,23, 24,26,27, 45-47,49-54<br>31-35 |
| X<br><br>Y<br><br><br>A | WO 00/22129 A (Arena Pharmaceuticals, Inc.), 20 April, 2000 (20.04.2000), & AU 9964307 A & EP 1121431 A | 1,4-6,8,9,21, 22,48<br>2,3,7,10-13, 16,23,24,26, 27,45-47,49-54<br>31-35 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>13 February, 2002 (13.02.02) | Date of mailing of the international search report<br>26 February, 2002 (26.02.02) |
|---|---|
| Name and mailing address of the ISA/<br>Japanese Patent Office<br><br>Facsimile No. | Authorized officer<br><br><br>Telephone No. |

Form PCT/ISA/210 (second sheet) (July 1992)

## INTERNATIONAL SEARCH REPORT

International application No.
PCT/JP01/10418

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | JP 9-121865 A (Takeda Chemical Industries, Ltd.), 13 May, 1997 (13.05.1997), (Family: none) | 1-13,16,21, 22,24,45-48<br>23,26,27,49-54<br>31-35 |
| Y<br><br><br>A | LIBERT, F., et al., "Selective amplification and cloning of four new members of the G protein-coupled receptor family", Science, (1989), Vol.244, No.4904, pages 569 to 572 | 2,3,5-13,16, 21-24,26,27, 45-54<br>31-35 |

Form PCT/ISA/210 (continuation of second sheet) (July 1992)

**INTERNATIONAL SEARCH REPORT**

International application No.
PCT/JP01/10418

**Box I Observations where certain claims were found unsearchable (Continuation of item 1 of first sheet)**

This international search report has not been established in respect of certain claims under Article 17(2)(a) for the following reasons:

1. ☒ Claims Nos.: 25, 39-41
because they relate to subject matter not required to be searched by this Authority, namely:

Claims 25 and 39 to 41 pertain to diagnostic methods to be practiced on the human body and methods for treatment of the human body by therapy and thus relate to a subject matter which this International Searching Authority is not required, under the provisions of Article 17(2)(a) of the PCT and Rule 39.1(iv) of the Regulations under the PCT, to search.

2. ☒ Claims Nos.: 14,15,19,20,28-30,36-38,42-44
because they relate to parts of the international application that do not comply with the prescribed requirements to such an extent that no meaningful international search can be carried out, specifically:

Concerning the inventions relating to "a ligand against a receptor protein or its salt", "a compound capable of altering the biding properties of a ligand to a receptor protein" and "a compound or its salt - - - - - which may be obtained by using the screening method" as set forth in claims 14, 15, 17 to 19, 20, 28 to 30, 36 to 38 and 42 to 44, it is completely unknown what specific compounds are involved in the scopes thereof and what are not. Namely, these claims are described in an extremely unclear manner.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

SEQUENCE LISTING

<110> Takeda Chemical Industries, Ltd.

<120> Novel G Protein-Coupled Receptor Proteins and DNAs thereof

<130> P01-0255PCT

<140> PCT/JP01/10418
<141> 2001-11-29

<150> JP 2000-364801
<151> 2000-11-30

<150> JP 2001-087482
<151> 2001-03-26

<150> JP 2001-145434
<151> 2001-05-15

<150> JP 2001-270838
<151> 2001-09-06

<160> 191

<210> 1
<211> 329
<212> PRT
<213> Rat

<400> 1
```
Met His Asn Leu Ser Leu Phe Glu Pro Gly Arg Gly Asn Val Ser Cys
                5                   10                  15
Gly Gly Pro Phe Leu Gly Cys Pro Asn Glu Ser Asn Pro Ala Pro Leu
            20                  25                  30
Pro Leu Pro Gln Pro Leu Ala Val Ala Val Pro Val Val Tyr Gly Val
```

35 40 45

Ile Cys Ala Val Gly Leu Ala Gly Asn Ser Ala Val Leu Tyr Val Leu

50 55 60

Leu Arg Thr Pro Arg Met Lys Thr Val Thr Asn Val Phe Ile Leu Asn

65 70 75 80

Leu Ala Ile Ala Asp Glu Leu Phe Thr Leu Val Leu Pro Ile Asn Ile

85 90 95

Ala Asp Phe Leu Leu Arg Arg Trp Pro Phe Gly Glu Val Met Cys Lys

100 105 110

Leu Ile Val Ala Val Asp Gln Tyr Asn Thr Phe Ser Ser Leu Tyr Phe

115 120 125

Leu Ala Val Met Ser Ala Asp Arg Tyr Leu Val Val Leu Ala Thr Ala

130 135 140

Glu Ser Arg Arg Val Ser Gly Arg Thr Tyr Gly Ala Ala Arg Ala Val

145 150 155 160

Ser Leu Ala Val Trp Ala Leu Val Thr Leu Val Val Leu Pro Phe Ala

165 170 175

Val Phe Ala Arg Leu Asp Glu Glu Gln Gly Arg Arg Gln Cys Val Leu

180 185 190

Val Phe Pro Gln Pro Glu Ala Phe Trp Trp Arg Ala Ser Arg Leu Tyr

195 200 205

Thr Leu Val Leu Gly Phe Ala Ile Pro Val Ser Thr Ile Cys Ala Leu

210 215 220

Tyr Ile Thr Leu Leu Cys Arg Leu Arg Ala Ile Gln Leu Asp Ser His

225 230 235 240

Ala Lys Ala Leu Asp Arg Ala Lys Lys Arg Val Thr Leu Leu Val Val

245 250 255

Ala Ile Leu Ala Val Cys Leu Leu Cys Trp Thr Pro Tyr His Leu Ser

260 265 270

Thr Ile Val Ala Leu Thr Thr Asp Leu Pro Gln Thr Pro Leu Val Ile

275 280 285

Gly Ile Ser Tyr Phe Ile Thr Ser Leu Ser Tyr Ala Asn Ser Cys Leu

290 295 300

Asn Pro Phe Leu Tyr Ala Phe Leu Asp Asp Ser Phe Arg Arg Ser Leu

305 310 315 320

Arg Gln Leu Val Ser Cys Arg Thr Ala

325 329

<210> 2
<211> 987
<212> DNA
<213> Rat

<400> 2
atgcacaact tgtcgctctt cgagcctggc aggggcaatg tgtcttgcgg cggcccattt 60
ttgggctgtc ctaacgagtc gaacccagcg cctctgccac tgccgcagcc tctggcggta 120
gcagtgcctg tggtctacgg ggtgatctgc gcggtgggac tggcgggcaa ctccgcggtg 180
ctgtacgtac tgctgcgcac gccgcgcatg aagactgtta ccaacgtgtt cattctcaac 240
ctggctatcg cggacgagct cttcaccctc gtgctgccca tcaacatcgc ggacttcctg 300
ctgaggcgct ggcccttcgg ggaagtcatg tgcaagctca tcgtggctgt cgaccagtac 360
aacactttct ctagcctcta cttcctcgcc gtcatgagcg cagaccgcta cctggttgtc 420
ctggccacag ccgagtcgcg ccgggtgtcc gggcgcactt atggtgcagc gcgggctgtc 480
agtctggcgg tgtgggcgct ggtgacattg gtcgtgctgc cttttgcggt attcgcccgg 540
ctggacgaag agcagggtcg gcgtcagtgc gtgctggtct tcccgcagcc tgaggccttc 600
tggtggcgcg ccagccgtct gtacactcta gtgttgggct tcgccatccc ggtgtccacc 660
atctgcgccc tctatatcac cctgttgtgc cgactgcgtg ctatccagct agacagccac 720
gccaaggccc tggaccgtgc caagaagcgc gtgaccttgt tggtggtggc gattctggct 780
gtgtgcctcc tctgctggac accgtaccac ctgagcacca tagtggcgct caccaccgac 840
ctcccgcaaa caccgttggt catcggcatc tcttacttca tcaccagtct gagctatgcc 900
aacagctgcc tcaacccttt cctctatgcc ttcctggacg acagcttccg caggagcctg 960
cggcagctgg tgtcatgccg cacagcc 987

<210> 3
<211> 28
<212> DNA
<213> Artificial Sequence

<220>
<223> Primer

<400> 3
actgatatgc acaacttgtc gctcttcg 28

<210> 4
<211> 28
<212> DNA
<213> Artificial Sequence

<220>
<223> Primer

<400> 4
actagttcag gctgtgcggc atgacacc 28

<210> 5
<211> 19
<212> DNA
<213> Artificial Sequence

<220>
<223> Primer

<400> 5
gttggtggtg gcgattctg 19

<210> 6
<211> 19
<212> DNA
<213> Artificial Sequence

<220>
<223> Primer

<400> 6
tggtgagcgc cactatggt 19

<210> 7
<211> 27

<212> DNA
<213> Artificial Sequence

<220>
<223> Probe

<400> 7
tcctctgctg gacaccgtac cacctga 27

<210> 8
<211> 23
<212> PRT
<213> Human

<400> 8
Trp Tyr Lys His Val Ala Ser Pro Arg Tyr His Thr Val Gly Arg Ala
1 5 10 15
Ala Gly Leu Leu Met Gly Leu
20 23

<210> 9
<211> 30
<212> PRT
<213> Human

<400> 9
Trp Tyr Lys His Val Ala Ser Pro Arg Tyr His Thr Val Gly Arg Ala
1 5 10 15
Ala Gly Leu Leu Met Gly Leu Arg Arg Ser Pro Tyr Leu Trp
20 25 30

<210> 10
<211> 32
<212> DNA
<213> Artificial Sequence

<220>

<223> Primer

<400> 10

atcgattaca atgcaggccg ctgggcaccc ag 32

<210> 11

<211> 32

<212> DNA

<213> Artificial Sequence

<220>

<223> Primer

<400> 11

actagtgccc ttcagcaccg caatatgctg cg 32

<210> 12

<211> 1023

<212> DNA

<213> Human

<400> 12

atcgattaca atgcaggccg ctgggcaccc agagcccctt gacagcaggg gctccttctc 60
cctccccacg atgggtgcca acgtctctca ggacaatggc actggccaca atgccacctt 120
ctccgagcca ctgccgttcc tctatgtgct cctgcccgcc gtgtactccg ggatctgtgc 180
tgtggggctg actggcaaca cggccgtcat ccttgtaatc ctaagggcgc ccaagatgaa 240
gacggtgacc aacgtgttca tcctgaacct ggccgtcgcc gacgggctct tcacgctggt 300
actgcccgtc aacatcgcgg agcacctgct gcagtactgg cccttcgggg agctgctctg 360
caagctggtg ctggccgtcg accactacaa catcttctcc agcatctact tcctagccgt 420
gatgagcgtg gaccgatacc tggtggtgct ggccaccgtg aggtcccgcc acatgccctg 480
gcgcacctac cggggggcga aggtcgccag cctgtgtgtc tggctgggcg tcacggtcct 540
ggttctgccc ttcttctctt tcgctggcgt ctacagcaac gagctgcagg tcccaagctg 600
tgggctgagc ttcccgtggc ccgagcaggt ctggttcaag gccagccgtg tctacacgtt 660
ggtcctgggc ttcgtgctgc ccgtgtgcac catctgtgtg ctctacacag acctcctgcg 720
caggctgcgg gccgtgcggc tccgctctgg agccaaggct ctaggcaagg ccaggcggaa 780

ggtgaccgtc ctggtcctcg tcgtgctggc cgtgtgcctc ctctgctgga cgcccttcca 840
cctggcctct gtcgtggccc tgaccacgga cctgccccag accccactgg tcatcagtat 900
gtcctacgtc atcaccagcc tcagctacgc caactcgtgc ctgaacccct tcctctacgc 960
ctttctagat gacaacttcc ggaagaactt ccgcagcata ttgcggtgct gaagggcact 1020
agt 1023

<210> 13
<211> 687
<212> RNA
<213> Artificial Sequence

<220>
<223> Riboprobe

<400> 13
caaaagcugg agcuccaccg cgguggcggc cgcucuagcc cacuagugcc cuucagcacc 60
gcaauaugcu gcggaaguuc uuccggaagu ugucaucuag aaaggcguag aggaaggggu 120
ucaggcacga guuggcguag cugaggcugg ugaugacgua ggacauacug augaccagug 180
gggucugggg cagguccgug gucagggcca cgacagaggc cagguggaag ggcguccagc 240
agaggaggca cacggccagc acgacgagga ccaggacggu caccuuccgc cuggccuugc 300
cuagagccuu ggcuccagag cggagccgca cggcccgcag ccugcgcagg aggucugugu 360
agagcacaca gauggugcac acgggcagca cgaagcccag gaccaacgug uagacacggc 420
uggccuugaa ccagaccugc ucgggccacg ggaagcucag cccacagcuu gggaccugca 480
gcucguugcu guagacgcca gcgaaagaga agaagggcag aaccaggacc gugacgccca 540
gccagacaca caggcuggcg accuucgccc cccgguaggu gcgccagggc auguggcggg 600
accucacggu ggccagcacc accagguauc gguccacgcu caucacggcu aggaaguaga 660
ugcuggagaa gauguuguag uggucga 687

<210> 14
<211> 17
<212> PRT
<213> Porcine

<400> 14
Trp Tyr Lys His Thr Ala Ser Pro Arg Tyr His Thr Val Gly Arg Ala
1 5 10 15

Ala

17

<210> 15

<211> 438

<212> DNA

<213> Human

<220>

<221> misc_feature

<222> 408

<223> n is a, g, t or c

<400> 15

gccccatgag caggccagcg gcgcggccca ccgtgtggta gcggggactc gccacgtgct 60
tgtaccacgc gccggagggc agcggcagca ggagcagaag cagcagcagt gccagccgcg 120
gccggctcgc gggagccccc cgctcccctg ggcgccacgc cagggcgctc gcgtcgacgg 180
ccgcccggcg gggcgggcca cgaaccggct cggctggggt tgggcgcgca gtggagttgg 240
gacgcccagg taccggagcg caggaggctg gaggcgagcc gtgggtcccc tgcaggccca 300
gctataaccg ctcggtggcc ccgcctcgtt ccgccccctc agtaccgctg ggctccccag 360
atggggggag ggacggaggg aggagaggga accctggcag ctggcggngg acgtgggtac 420
ttgagcacct cactgagt 438

<210> 16

<211> 264

<212> DNA

<213> Human

<400> 16

gatagggtga gcgacgcagc cccatgagca ggccagcggc gcggcccacc gtgtggtagc 60
ggggactcgc cacgtgcttg taccacgcgc cggagggcag cggcagcagg agcagaagca 120
gcagcagtgc cagccgcggc cggctcgcgg gagccccccg ctcccctggg cgccacgcca 180
gggcgctcgc gtcgacggcc gcccggcggg gcgggccacg aaccggctcg gctgggtttg 240
ggcgcgcagt ggagttggga cgcc 264

<210> 17

<211> 424
<212> DNA
<213> Human

<400> 17
gatagggtga gcgacgcagc cccatgagca ggccagcggc gcggcccacc gtgtggtagc 60
ggggactcgc cacgtgcttg taccacgcgc cggagggcag cggcagcagg agcagaagca 120
gcagcagtgc cagccgcggc cggctcgcgg gagccccccg ctcccctggg cgcccacgcca 180
gggcgctcgc gtcgacggcc gcccggcggg gcgggccacg aaccggctcg gctgggtttg 240
ggcgcgcagt ggagttggga cgcccaggta ccggagcgca ggaggctgga ggcgagccgt 300
gggtcccctg caggcccagc tataaccgct cggtggcccc gcctcgttcc gccccctcag 360
taccgctggg ctccccagat ggggggaggg acggagggag gagagggaac cctggcagct 420
ggcg 424

<210> 18
<211> 375
<212> DNA
<213> Human

<400> 18
gcgcctcacc gtgtggtagc ggggactcgc cacgtgcttg taccacgcgc cggaggcagc 60
ggcacgagga gcagaagcag cagcagtgcc agccgcggcc ggctcgcggg agccccccgc 120
tcccctgggc gccacgcagg gctacagcgt cgacggccgc ccgcggggcc atcgcaaccg 180
gctcggctgg gtttgggcgc gcagtggagt tgggacgccc aggtaccgga gcgcaggagg 240
ctggaggcga gccgtgggtc ccctgcaggc ccagctataa ccgctcggtg gccccgcctc 300
gttccgcccc ctcagtaccg ctgggctccc cagaatgggg gagggacgga gggaggagag 360
ggaaccctgg cagct 375

<210> 19
<211> 260
<212> DNA
<213> Human

<220>
<221> misc_feature
<222> 2

<223> n is a, g, t or c

<220>
<221> misc_feature
<222> 61
<223> n is a, g, t or c

<220>
<221> misc_feature
<222> 147
<223> n is a, g, t or c

<220>
<221> misc_feature
<222> 189
<223> n is a, g, t or c

<220>
<221> misc_feature
<222> 213
<223> n is a, g, t or c

<220>
<221> misc_feature
<222> 237
<223> n is a, g, t or c

<220>
<221> misc_feature
<222> 249
<223> n is a, g, t or c

<400> 19
cnacgttctc ggggacataa accctgttct tgtcctaacc cgccaagggg ccatggactt 60
nagcgcgctg gcgtcgagca gagaagtacg gggccctggg ccggggctcc ggtgaaccgg 120
cccctgctac cgctactgct gcttctnctc ttgctacctc tgcccgccag cgcctggtac 180

aagcacgtng cgagccctcg ctatcacaca gtnggtcgtg cctccgggct gctcatnggg 240
ctgcgccgnt cgtcctacct 260

<210> 20
<211> 24
<212> DNA
<213> Artificial Sequence

<220>
<223> Primer

<400> 20
aactccactg cgcgcccaaa ccca 24

<210> 21
<211> 24
<212> DNA
<213> Artificial Sequence

<220>
<223> Primer

<400> 21
tctcccacag ctcctgaacc cacg 24

<210> 22
<211> 375
<212> DNA
<213> Human

<400> 22
aactccactg cgcgcccaaa cccagccgag ccggttcgtg gcccgccccg ccgggcggcc 60
gtcgacgcga gcgccctggc gtggcgccca ggggagcggg gggctcccgc gagccggccg 120
cggctggcac tgctgctgct tctgctcctg ctgccgctgc cctccggcgc gtggtacaag 180
cacgtggcga gtccccgcta ccacacggtg ggccgcgccg ctggcctgct catggggctg 240
cgtcgctcac cctatctgtg gcgccgcgcg ctgcgcgcgg ccgccgggcc cctggccagg 300

gacaccctct cccccgaacc cgcagcccgc gaggctcctc tcctgctgcc ctcgtgggtt 360
caggagctgt gggag 375

<210> 23
<211> 125
<212> PRT
<213> Human

<400> 23
Asn Ser Thr Ala Arg Pro Asn Pro Ala Glu Pro Val Arg Gly Pro Pro
1 5 10 15
Arg Arg Ala Ala Val Asp Ala Ser Ala Leu Ala Trp Arg Pro Gly Glu
20 25 30
Arg Gly Ala Pro Ala Ser Arg Pro Arg Leu Ala Leu Leu Leu Leu Leu
35 40 45
Leu Leu Leu Pro Leu Pro Ser Gly Ala Trp Tyr Lys His Val Ala Ser
50 55 60
Pro Arg Tyr His Thr Val Gly Arg Ala Ala Gly Leu Leu Met Gly Leu
65 70 75 80
Arg Arg Ser Pro Tyr Leu Trp Arg Arg Ala Leu Arg Ala Ala Ala Gly
85 90 95
Pro Leu Ala Arg Asp Thr Leu Ser Pro Glu Pro Ala Ala Arg Glu Ala
100 105 110
Pro Leu Leu Leu Pro Ser Trp Val Gln Glu Leu Trp Glu
115 120 125

<210> 24
<211> 25
<212> PRT
<213> Human

<400> 24
Trp Tyr Lys His Val Ala Ser Pro Arg Tyr His Thr Val Gly Arg Ala
1 5 10 15
Ala Gly Leu Leu Met Gly Leu Arg Arg
20 25

<210> 25
<211> 24
<212> PRT
<213> Human

<400> 25
Trp Tyr Lys His Val Ala Ser Pro Arg Tyr His Thr Val Gly Arg Ala
1 5 10 15
Ala Gly Leu Leu Met Gly Leu Arg
20 24

<210> 26
<211> 87
<212> DNA
<213> Human

<400> 26
tggtacaagc acgtggcgag tccccgctac cacacggtgg gccgcgccgc tggcctgctc 60
atggggctgc gtcgctcacc ctatctg 87

<210> 27
<211> 84
<212> DNA
<213> Human

<400> 27
tggtacaagc acgtggcgag tccccgctac cacacggtgg gccgcgccgc tggcctgctc 60
atggggctgc gtcgctcacc ctat 84

<210> 28
<211> 81
<212> DNA
<213> Human

<400> 28

tggtacaagc acgtggcgag tccccgctac cacacggtgg gccgcgccgc tggcctgctc 60
atggggctgc gtcgctcacc c 81

<210> 29
<211> 78
<212> DNA
<213> Human

<400> 29
tggtacaagc acgtggcgag tccccgctac cacacggtgg gccgcgccgc tggcctgctc 60
atggggctgc gtcgctca 78

<210> 30
<211> 75
<212> DNA
<213> Human

<400> 30
tggtacaagc acgtggcgag tccccgctac cacacggtgg gccgcgccgc tggcctgctc 60
atggggctgc gtcgc 75

<210> 31
<211> 72
<212> DNA
<213> Human

<400> 31
tggtacaagc acgtggcgag tccccgctac cacacggtgg gccgcgccgc tggcctgctc 60
atggggctgc gt 72

<210> 32
<211> 333
<212> PRT
<213> Human

<400> 32

Met Gln Ala Ala Gly His Pro Glu Pro Leu Asp Ser Arg Gly Ser Phe
1               5                   10                  15
Ser Leu Pro Thr Met Gly Ala Asn Val Ser Gln Asp Asn Gly Thr Gly
            20                  25                  30
His Asn Ala Thr Phe Ser Glu Pro Leu Pro Phe Leu Tyr Val Leu Leu
        35                  40                  45
Pro Ala Val Tyr Ser Gly Ile Cys Ala Val Gly Leu Thr Gly Asn Thr
    50                  55                  60
Ala Val Ile Leu Val Ile Leu Arg Ala Pro Lys Met Lys Thr Val Thr
65                  70                  75                  80
Asn Val Phe Ile Leu Asn Leu Ala Val Ala Asp Gly Leu Phe Thr Leu
                85                  90                  95
Val Leu Pro Val Asn Ile Ala Glu His Leu Leu Gln Tyr Trp Pro Phe
            100                 105                 110
Gly Glu Leu Leu Cys Lys Leu Val Leu Ala Val Asp His Tyr Asn Ile
        115                 120                 125
Phe Ser Ser Ile Tyr Phe Leu Ala Val Met Ser Val Asp Arg Tyr Leu
    130                 135                 140
Val Val Leu Ala Thr Val Arg Ser Arg His Met Pro Trp Arg Thr Tyr
145                 150                 155                 160
Arg Gly Ala Lys Val Ala Ser Leu Cys Val Trp Leu Gly Val Thr Val
                165                 170                 175
Leu Val Leu Pro Phe Phe Ser Phe Ala Gly Val Tyr Ser Asn Glu Leu
            180                 185                 190
Gln Val Pro Ser Cys Gly Leu Ser Phe Pro Trp Pro Glu Gln Val Trp
        195                 200                 205
Phe Lys Ala Ser Arg Val Tyr Thr Leu Val Leu Gly Phe Val Leu Pro
    210                 215                 220
Val Cys Thr Ile Cys Val Leu Tyr Thr Asp Leu Leu Arg Arg Leu Arg
225                 230                 235                 240
Ala Val Arg Leu Arg Ser Gly Ala Lys Ala Leu Gly Lys Ala Arg Arg
                245                 250                 255
Lys Val Thr Val Leu Val Leu Val Val Leu Ala Val Cys Leu Leu Cys
            260                 265                 270
Trp Thr Pro Phe His Leu Ala Ser Val Val Ala Leu Thr Thr Asp Leu
        275                 280                 285

Pro Gln Thr Pro Leu Val Ile Ser Met Ser Tyr Val Ile Thr Ser Leu
290 295 300

Ser Tyr Ala Asn Ser Cys Leu Asn Pro Phe Leu Tyr Ala Phe Leu Asp
305 310 315 320

Asp Asn Phe Arg Lys Asn Phe Arg Ser Ile Leu Arg Cys
325 330 333

<210> 33
<211> 24
<212> DNA
<213> Artificial Sequence

<220>
<223> Primer

<400> 33
tctcccacag ctcctgaacc cacg 24

<210> 34
<211> 24
<212> DNA
<213> Artificial Sequence

<220>
<223> Primer

<400> 34
acagataggg tgagcgacgc agcc 24

<210> 35
<211> 1102
<212> DNA
<213> Human

<400> 35
gccatttaag tggagtcttg aaggatgagt aggtgttagg cacagacgca cagaggcagg 60

caaagccaca ggctgttggt ttaggcaaaa attgagactg gctggataaa gtggtcttgg 120
gggaccatca ccagagagga ggcgctggag gtctgcaagg ccttgtcctg cccctccagg 180
ggtagaggtt ccaggagggg ctgacttttt ctcctggaag cctcacagaa ctgcagaccc 240
cacggatggc ttggtgttgc caacatgagg cttctaaggc ttctgcgggg agatgggttg 300
gtggggagaa gctgggggtg gcagtggaca ggacagggtg tggggacagc tttgggagct 360
atgctaggca aggacaaggg acaactcttg gggggactca cccagagggg tcttgaatgg 420
tgctgaaggc cccgacagc cctcctgcaa tagccactgt agctctgcct gcacctgggc 480
cttcgctctg ctgtcgtccc accggcagga gtctggctaa aggggcatcc ctcagcccta 540
ctccctcatc agtgttccca gtacccactc cctggcactt ccactcctag agggaggagg 600
ctgagcaggc agagaatggg acgtgtcccc tcagaggagc ctcgagccca gttccagcca 660
gcggcccact cagtgaggtg ctcaagtacc cacgtccccc gccagctgcc agggttccct 720
ctcctccctc cgtccctccc cccatctggg gagcccagcg gtactgaggg ggcggaacga 780
ggcggggcca ccgagcggtt atagctgggc ctgcagggga cccacggctc gcctccagcc 840
tcctgcgctc cggtacctgg gcgtcccaac tccactgcgc gcccaaaccc agccgagccg 900
gttcgtggcc cgccccgccg ggcggccgtc gacgcgagcg ccctggcgtg gcgcccaggg 960
gagcgggggg ctcccgcgag ccggccgcgg ctggcactgc tgctgcttct gctcctgctg 1020
ccgctgccct ccggcgcgtg gtacaagcac gtggcgagtc cccgctacca cacggtgggc 1080
cgcgccgctg gcctgctcat gg 1102

<210> 36
<211> 24
<212> DNA
<213> Artificial Sequence

<220>
<223> Primer

<400> 36
aactccactg cgcgcccaaa ccca 24

<210> 37
<211> 24
<212> DNA
<213> Artificial Sequence

<220>

<223> Primer

<400> 37
ctggcactgc tgctgcttct gctc 24

<210> 38
<211> 609
<212> DNA
<213> Human

<400> 38
ctgctgccgc tgccctccgg cgcgtggtac aagcacgtgg cgagtccccg ctaccacacg 60
gtgggccgcg ccgctggcct gctcatgggg ctgcgtcgct caccctatct gtggcgccgc 120
gcgctgcgcg cggccgccgg gcccctggcc agggacaccc tctcccccga acccgcagcc 180
cgcgaggctc ctctcctgct gccctcgtgg gttcaggagc tgtgggagac gcgacgcagg 240
agctcccagg cagggatccc cgtccgtgcg ccccggagcc cgcgcgcccc agagcctgcg 300
ctggaaccgg agtccctgga cttcagcgga gctggccaga gacttcggag agacgtctcc 360
cgcccagcgg tggaccccgc agcaaaccgc cttggcctgc cctgcctggc ccccggaccg 420
ttctgacagc gtcccccgcc cgcccgtggc gcctccgcgc ctgacccagg aggagtggcc 480
gcgcgcttcc aggagccgct catagacccc gcctgccgtc cggtcaataa aatccgcctg 540
actcctgcgc ccccgcatgc gtaaaaaaaa aaaaaaaaaa aaaaaaaaaa agcggccgct 600
gaattctag 609

<210> 39
<211> 24
<212> DNA
<213> Artificial Sequence

<220>
<223> Primer

<400> 39
agcggtactg agggggcgga acga 24

<210> 40
<211> 24

<212> DNA
<213> Artificial Sequence

<220>
<223> Primer

<400> 40
gggtctatga gcggctcctg gaag 24

<210> 41
<211> 719
<212> DNA
<213> Human

<400> 41
ggcggggcca ccgagcggtt atagctgggc ctgcagggga cccacggctc gcctccagcc 60
tcctgcgctc cggtacctgg gcgtcccaac tccactgcgc gcccaaaccc agccgagccg 120
gttcgtggcc cgccccgccg ggcggccgtc gacgcgagcg ccctggcgtg gcgcccaggg 180
gagcgggggg ctcccgcgag ccggccgcgg ctggcactgc tgctgcttct gctcctgctg 240
ccgctgccct ccggcgcgtg gtacaagcac gtggcgagtc cccgctacca cacggtgggc 300
cgcgccgctg gcctgctcat ggggctgcgt cgctcaccct atctgtggcg ccgcgcgctg 360
cgcgcggccg ccgggcccct ggccagggac accctctccc ccgaacccgc agcccgcgag 420
gctcctctcc tgctgccctc gtgggttcag gagctgtggg agacgcgacg caggagctcc 480
caggcaggga tccccgtccg tgcgccccgg agcccgcgcg ccccagagcc tgcgctggaa 540
ccggagtccc tggacttcag cggagctggc cagagacttc ggagagacgt ctcccgccca 600
gcggtggacc ccgcagcaaa ccgccttggc ctgccctgcc tggcccccgg accgttctga 660
cagcgtcccc cgcccgcccg tggcgcctcc gcgcctgacc caggaggagt ggccgcgcg 719

<210> 42
<211> 165
<212> PRT
<213> Human

<400> 42
Leu Ala Trp Arg Pro Gly Glu Arg Gly Ala Pro Ala Ser Arg Pro Arg
1 5 10 15

Leu Ala Leu Leu Leu Leu Leu Leu Leu Leu Pro Leu Pro Ser Gly Ala
20 25 30

Trp Tyr Lys His Val Ala Ser Pro Arg Tyr His Thr Val Gly Arg Ala
35 40 45

Ala Gly Leu Leu Met Gly Leu Arg Arg Ser Pro Tyr Leu Trp Arg Arg
50 55 60

Ala Leu Arg Ala Ala Ala Gly Pro Leu Ala Arg Asp Thr Leu Ser Pro
65 70 75 80

Glu Pro Ala Ala Arg Glu Ala Pro Leu Leu Leu Pro Ser Trp Val Gln
85 90 95

Glu Leu Trp Glu Thr Arg Arg Arg Ser Ser Gln Ala Gly Ile Pro Val
100 105 110

Arg Ala Pro Arg Ser Pro Arg Ala Pro Glu Pro Ala Leu Glu Pro Glu
115 120 125

Ser Leu Asp Phe Ser Gly Ala Gly Gln Arg Leu Arg Arg Asp Val Ser
130 135 140

Arg Pro Ala Val Asp Pro Ala Ala Asn Arg Leu Gly Leu Pro Cys Leu
145 150 155 160

Ala Pro Gly Pro Phe
165

<210> 43
<211> 24
<212> DNA
<213> Artificial Sequence

<220>
<223> Primer

<400> 43
acagataggg tgagcgacgc agcc 24

<210> 44
<211> 24
<212> DNA
<213> Artificial Sequence

<220>
<223> Primer

<400> 44
tgagcgacgc agccccatga gcag 24

<210> 45
<211> 235
<212> DNA
<213> Porcine

<400> 45
cgacacccct gcgcccagac cctccggagc cagttcctgg tccgccccgc cgggagccgt 60
cagcatgaac ccccgggcac gcggcatggg agcgcggggc ccgggaccgg gggccactgc 120
gaggcgccgg ctgctggcat tgctgttact gctgctgctg ctgccgctgc ccgccccgtgc 180
ctggtacaag cacacggcga gtccccgcta ccacacggtg ggccgcgccg cgggc 235

<210> 46
<211> 24
<212> DNA
<213> Artificial Sequence

<220>
<223> Primer

<400> 46
cagcggcagc agcagcagca gtaa 24

<210> 47
<211> 24
<212> DNA
<213> Artificial Sequence

<220>
<223> Primer

<400> 47
cagcagtaac agcaatgcca gcag 24

<210> 48
<211> 156
<212> DNA
<213> Porcine

<400> 48
ctgtagcctc ccgcgctgcg gcttcccgac acccctgcgc ccagaccctc cggagccagt 60
tcctggtccg ccccgccggg agccgtcagc atgaaccccc gggcacgcgg catgggagcg 120
cggggcccgg gaccgggggc cactgcgagg cgccgg 156

<210> 49
<211> 24
<212> DNA
<213> Artificial Sequence

<220>
<223> Primer

<400> 49
cggctgctgg cattgctgtt actg 24

<210> 50
<211> 23
<212> DNA
<213> Artificial Sequence

<220>
<223> Primer

<400> 50
cgcccgtgcc tggtacaagc aca 23

<210> 51
<211> 588
<212> DNA
<213> Porcine

<400> 51
cggcgagtcc ccgctaccac acggtgggcc gcgccgcggg cctgctcatg gggctgcgcc 60
gctcgcccta catgtggcgc cgcgcgctgc gcccggcggc cgggcccctg gcctgggaca 120
ctttcggcca ggacgtgccc cctcggggac cctccgccag gaacgccctc tctccggggc 180
ccgcccctcg cgacgctccg ctgcttcccc ccggggttca gacactgtgg caggtgcgac 240
gcggaagctt ccgctccggg atcccggtca gtgcgccccg cagcccgcgc gcccgggggt 300
ccgagccgca accggaattg ggcgcctctt cctggacctc ggcggagtag accagagcct 360
tcggagagtc ttcagctcag cggtggtctg cgcagggaac cgccttcgcc agccccccgcc 420
tcgccccagc gtcagagccg acctgatcgc ggccccggcg gcgcggcccc gcgcctggcc 480
cccgcggagt ctcttcgcgc ccccaggccg gccgtctggt caataaaacc cgcctagttc 540
ctgcgaaaaa aaaaaaaaaa aaaaaaaaaa aaaaaaaaaa aaaaaaa 588

<210> 52
<211> 24
<212> DNA
<213> Artificial Sequence

<220>
<223> Primer

<400> 52
ttcccgacac ccctgcgccc agac 24

<210> 53
<211> 24
<212> DNA
<213> Artificial Sequence

<220>
<223> Primer

<400> 53
gggctggcga aggcggttcc ctgc 24

<210> 54
<211> 565
<212> DNA
<213> Porcine

<400> 54
cctccggagc cagttcctgg tccgccccgc cgggagccgt cagcatgaac ccccgggcac 60
gcggcatggg agcgcggggc ccgggaccgg gggccactgc gaggcgccgg ctgctggcat 120
tgctgttact gctgctgctg ctgccgctgc ccgcccgtgc ctggtacaag cacacggcga 180
gtccccgcta ccacacggtg ggccgcgccg cgggcctgct catggggctg cgccgctcgc 240
cctacatgtg gcgccgcgcg ctgcgcccgg cggccgggcc cctggcctgg gacactttcg 300
gccaggacgt gccccctcgg ggaccctccg ccaggaacgc cctctctccg gggcccgccc 360
ctcgcgacgc tccgctgctt ccccccgggg ttcagacact gtggcaggtg cgacgcggaa 420
gcttccgctc cgggatcccg gtcagtgcgc cccgcagccc gcgcgcccgg gggtccgagc 480
cgcaaccgga attgggcgcc tcttcctgga cctcggcgga gtagaccaga gccttcggag 540
agtcttcagc tcagcggtgg tctgc 565

<210> 55
<211> 159
<212> PRT
<213> Porcine

<400> 55
Met Asn Pro Arg Ala Arg Gly Met Gly Ala Arg Gly Pro Gly Pro Gly
1 5 10 15
Ala Thr Ala Arg Arg Arg Leu Leu Ala Leu Leu Leu Leu Leu Leu Leu
20 25 30
Leu Pro Leu Pro Ala Arg Ala Trp Tyr Lys His Thr Ala Ser Pro Arg
35 40 45
Tyr His Thr Val Gly Arg Ala Ala Gly Leu Leu Met Gly Leu Arg Arg
50 55 60
Ser Pro Tyr Met Trp Arg Arg Ala Leu Arg Pro Ala Ala Gly Pro Leu
65 70 75 80

Ala Trp Asp Thr Phe Gly Gln Asp Val Pro Pro Arg Gly Pro Ser Ala
85 90 95

Arg Asn Ala Leu Ser Pro Gly Pro Ala Pro Arg Asp Ala Pro Leu Leu
100 105 110

Pro Pro Gly Val Gln Thr Leu Trp Gln Val Arg Arg Gly Ser Phe Arg
115 120 125

Ser Gly Ile Pro Val Ser Ala Pro Arg Ser Pro Arg Ala Arg Gly Ser
130 135 140

Glu Pro Gln Pro Glu Leu Gly Ala Ser Ser Trp Thr Ser Ala Glu
145 150 155 159

<210> 56
<211> 23
<212> PRT
<213> Porcine

<400> 56
Trp Tyr Lys His Thr Ala Ser Pro Arg Tyr His Thr Val Gly Arg Ala
1 5 10 15

Ala Gly Leu Leu Met Gly Leu
20 23

<210> 57
<211> 30
<212> PRT
<213> Porcine

<400> 57
Trp Tyr Lys His Thr Ala Ser Pro Arg Tyr His Thr Val Gly Arg Ala
1 5 10 15

Ala Gly Leu Leu Met Gly Leu Arg Arg Ser Pro Tyr Met Trp
20 25 30

<210> 58
<211> 69
<212> DNA

<213> Porcine

<400> 58
tggtacaagc acacggcgag tccccgctac cacacggtgg gccgcgccgc gggcctgctc 60
atggggctg 69

<210> 59
<211> 90
<212> DNA
<213> Porcine

<400> 59
tggtacaagc acacggcgag tccccgctac cacacggtgg gccgcgccgc gggcctgctc 60
atggggctgc gccgctcgcc ctacatgtgg 90

<210> 60
<211> 23
<212> DNA
<213> Artificial Sequence

<220>
<223> Primer

<400> 60
cgttctcggg gacataaacc ctg 23

<210> 61
<211> 23
<212> DNA
<213> Artificial Sequence

<220>
<223> Primer

<400> 61
atgagcagcc cggaggcacg acc 23

<210> 62
<211> 188
<212> DNA
<213> Rat

<400> 62
ttcttgtcct aacccgccaa ggggccatgg acttgagcgc gctggcgtcg agcagagaag 60
tacggggccc tgggcccggg gctccggtga accggcccct gctaccgcta ctgctgcttc 120
tgctcttgct acctctgccc gccagcgcct ggtacaagca cgtggcgagc cctcgctatc 180
acacagtg 188

<210> 63
<211> 23
<212> DNA
<213> Artificial Sequence

<220>
<223> Primer

<400> 63
atgagcagcc cggaggcacg acc 23

<210> 64
<211> 23
<212> DNA
<213> Artificial Sequence

<220>
<223> Primer

<400> 64
actgtgtgat agcgagggct cgc 23

<210> 65
<211> 615

<212> DNA
<213> Rat

<400> 65

ctcagagctg tactaggcag gaagagggac ggccctcagg gaagggtggc cctatgctta 60
aaactttcct gtctcctctc cataagtgct ccacttgtag caactcctac caagggggca 120
tccttttgcc cctggcagcc catccttgta ttctgagacc atgcatggta ccagaactcc 180
ctccctgaca gttcccttcc tgggggcgag gaaagggtaa gcaaggagat cccccactaa 240
agcttcaagc gcagtccagc ttgcgatcta ctcattggga ggcttctagc tacccgggtt 300
ccctcttctc cctccctctc catcctcctc tcccttgggc atgtgccgcg ggggcgagcc 360
ggggcggggc cattgagaag ctgtagtcgc accaactgac tagtctcttc catcctccgg 420
agctccgacg ttctcgggga cataaaccct gttcttgtcc taacccgcca aggggccatg 480
gacttgagcg cgctggcgtc gagcagagaa gtacggggcc ctgggcccgg ggctccggtg 540
aaccggcccc tgctaccgct actgctgctt ctgctcttgc tacctctgcc cgccagcgcc 600
tggtacaagc acgtg 615

<210> 66
<211> 23
<212> DNA
<213> Artificial Sequence

<220>
<223> Primer

<400> 66
cgttctcggg gacataaacc ctg 23

<210> 67
<211> 24
<212> DNA
<213> Artificial Sequence

<220>
<223> Primer

<400> 67

cgagccctcg ctatcacaca gtgg 24

<210> 68
<211> 497
<212> DNA
<213> Rat

<400> 68
gtcgtgcctc cgggctgctc atggggctgc gccgctcgcc ctacctgtgg cgccgtgcct 60
tgggtggggc cgctggaccg ctcgtggggc tcccgggaca gatggcccgc agcgctctcc 120
tgcttccttc ccccgggcag gagctgtggg aggtacgaag caggagttca ccggcaggac 180
ttcccgtgca tgcaacccgg agtctgcggg acctggaggg agccggccaa cctgagcagt 240
cgctaagctt tcagtcctgg acttcagcag agcccgctgc tagagccttc ggtgagacgc 300
ttcgtgccca gccatggttc ctgcagcaaa tcatctttgc cgatcctgtc aggctcgacg 360
accgtctcaa gaaccgatgg cgcccccgtg cttgacctaa gcaggagcac agcttgtagc 420
tccagtcagg tctcgttgtc tggtcaataa aatcactctg attcccaaaa aaaaaaaaaa 480
aaaaaaaaaa aaaaaaa 497

<210> 69
<211> 21
<212> DNA
<213> Artificial Sequence

<220>
<223> Primer

<400> 69
ggggcggggc cattgagaag c 21

<210> 70
<211> 21
<212> DNA
<213> Artificial Sequence

<220>
<223> Primer

<400> 70
tgaccagaca acgagacctg a 21

<210> 71
<211> 684
<212> DNA
<213> Rat

<400> 71
tgtagtcgca ccaactgact agtctcttcc atcctccgga gctccgacgt tctcggggac 60
ataaaccctg ttcttgtcct aacccgccaa ggggccatgg acttgagcgc gctggcgtcg 120
agcagagaag tacggggccc tgggcccggg gctccggtga accggcccct gctaccgcta 180
ctgctgcttc tgctcttgct acctctgccc gccagcgcct ggtacaagca cgtggcgagc 240
cctcgctatc acacagtggg tcgtgcctcc gggctgctca tggggctgcg ccgctcgccc 300
tacctgtggc gccgtgcctt gggtggggcc gctggaccgc tcgtggggct cccgggacag 360
atggcccgca gcgctctcct gcttccttcc cccgggcagg agctgtggga ggtacgaagc 420
aggagttcac cggcaggact tcccgtgcat gcaacccgga gtctgcggga cctggaggga 480
gccggccaac ctgagcagtc gctaagcttt cagtcctgga cttcagcaga gcccgctgct 540
agagccttcg gtgagacgct tcgtgcccag ccatggttcc tgcagcaaat catctttgcc 600
gatcctgtca ggctcgacga ccgtctcaag aaccgatggc gcccccgtgc ttgacctaag 660
caggagcaca gcttgtagct ccag 684

<210> 72
<211> 185
<212> PRT
<213> Rat

<400> 72
Met Asp Leu Ser Ala Leu Ala Ser Ser Arg Glu Val Arg Gly Pro Gly
1 5 10 15
Pro Gly Ala Pro Val Asn Arg Pro Leu Leu Pro Leu Leu Leu Leu Leu
20 25 30
Leu Leu Leu Pro Leu Pro Ala Ser Ala Trp Tyr Lys His Val Ala Ser
35 40 45
Pro Arg Tyr His Thr Val Gly Arg Ala Ser Gly Leu Leu Met Gly Leu

50 55 60

Arg Arg Ser Pro Tyr Leu Trp Arg Arg Ala Leu Gly Gly Ala Ala Gly
65 70 75 80

Pro Leu Val Gly Leu Pro Gly Gln Met Ala Arg Ser Ala Leu Leu Leu
85 90 95

Pro Ser Pro Gly Gln Glu Leu Trp Glu Val Arg Ser Arg Ser Ser Pro
100 105 110

Ala Gly Leu Pro Val His Ala Thr Arg Ser Leu Arg Asp Leu Glu Gly
115 120 125

Ala Gly Gln Pro Glu Gln Ser Leu Ser Phe Gln Ser Trp Thr Ser Ala
130 135 140

Glu Pro Ala Ala Arg Ala Phe Gly Glu Thr Leu Arg Ala Gln Pro Trp
145 150 155 160

Phe Leu Gln Gln Ile Ile Phe Ala Asp Pro Val Arg Leu Asp Asp Arg
165 170 175

Leu Lys Asn Arg Trp Arg Pro Arg Ala
180 185

<210> 73
<211> 23
<212> PRT
<213> Rat

<400> 73
Trp Tyr Lys His Val Ala Ser Pro Arg Tyr His Thr Val Gly Arg Ala
1 5 10 15

Ser Gly Leu Leu Met Gly Leu
20 23

<210> 74
<211> 30
<212> PRT
<213> Rat

<400> 74
Trp Tyr Lys His Val Ala Ser Pro Arg Tyr His Thr Val Gly Arg Ala

1 5 10 15

Ser Gly Leu Leu Met Gly Leu Arg Arg Ser Pro Tyr Leu Trp

20 25 30

<210> 75

<211> 69

<212> DNA

<213> Rat

<400> 75

tggtacaagc acgtggcgag ccctcgctat cacacagtgg gtcgtgcctc cgggctgctc 60

atggggctg 69

<210> 76

<211> 90

<212> DNA

<213> Rat

<400> 76

tggtacaagc acgtggcgag ccctcgctat cacacagtgg gtcgtgcctc cgggctgctc 60

atggggctgc gccgctcgcc ctacctgtgg 90

<210> 77

<211> 529

<212> DNA

<213> Mouse

<400> 77

acgtgctcgt tctcggagac ataaacccag ttcttgtcct aaccctccaa ggggcaattg 60

acgtgagcgc gctggcgtct aacagagaag tacggggccc tgggcccggg actcccagga 120

accggcccct gctgcccctg ctgctgcttc tgctcttgct accgctgccc gccagcgcct 180

ggtataagca cgtggcgagt ccccgctatc acacagtggg tcgtgcctcc gggctgctca 240

tggggctgcg ccgctcgccc taccagtggc gccgtgccct gggcggggct gctggacccc 300

tctcccggct cccaggaccg gtcgcccgcg gcgctctcct gcttccttcc tcagggcagg 360

agctgtggga ggtacgaagc aggagctcac ctgcagggct tcccgtccat gcaccctgga 420

gtccgcggga cctggaggga gtccgccaac cggagcagtc gctaagcctt cactcctgga 480

tgtcagagga gcccgctgat aggtaagtag gaaagagagg aggcgggcg 529

<210> 78
<211> 24
<212> DNA
<213> Artificial Sequence

<220>
<223> Primer

<400> 78
acccagttct tgtcctaacc ctcc 24

<210> 79
<211> 24
<212> DNA
<213> Artificial Sequence

<220>
<223> Primer

<400> 79
cctgcttcgt acctcccaca gctc 24

<210> 80
<211> 311
<212> DNA
<213> Mouse

<400> 80
aaggggcaat tgacgtgagc gcgctggcgt ctaacagaga agtacggggc cctgggcccg 60
ggactcccag gaaccggccc ctgctgcccc tgctgctgct tctgctcttg ctaccgctgc 120
ccgccagcgc ctggtataag cacgtggcga gtccccgcta tcacacagtg ggtcgtgcct 180
ccgggctgct catggggctg cgccgctcgc cctaccagtg gcgccgtgcc ctgggcgggg 240
ctgctggacc cctctcccgg ctcccaggac cggtcgcccg cggcgctctc ctgcttcctt 300
cctcagggca g 311

<210> 81
<211> 24
<212> DNA
<213> Artificial Sequence

<220>
<223> Primer

<400> 81
catgagcagc ccggaggcac gacc 24

<210> 82
<211> 24
<212> DNA
<213> Artificial Sequence

<220>
<223> Primer

<400> 82
gtgatagcgg ggactcgcca cgtg 24

<210> 83
<211> 237
<212> DNA
<213> Mouse

<400> 83
aaaggctgta gtcgcaccaa ctgactggtc tccatcctct ggagctccga cgtgctcgtt 60
ctcggagaca taaacccagt tcttgtccta accctccaag gggcaattga cgtgagcgcg 120
ctggcgtcta acagagaagt acggggccct gggcccggga ctcccaggaa ccggcccctg 180
ctgcccctgc tgctgcttct gctcttgcta ccgctgcccg ccagcgcctg gtataag 237

<210> 84
<211> 24

<212> DNA
<213> Artificial Sequence

<220>
<223> Primer

<400> 84
acccagttct tgtcctaacc ctcc 24

<210> 85
<211> 24
<212> DNA
<213> Artificial Sequence

<220>
<223> Primer

<400> 85
gggcaattga cgtgagcgcg ctgg 24

<210> 86
<211> 598
<212> DNA
<213> Mouse

<400> 86
cgtctaacag agaagtacgg ggccctgggc ccgggactcc caggaaccgg cccctgctgc 60
ccctgctgct gcttctgctc ttgctaccgc tgcccgccag cgcctggtat aagcacgtgg 120
cgagtccccg ctatcacaca gtgggtcgtg cctccgggct gctcatgggg ctgcgccgct 180
cgccctacca gtggcgccgt gccctgggcg gggctgctgg acccctctcc cggctcccag 240
gaccggtcgc ccgcggcgct ctcctgcttc cttcctcagg gcaggagctg tgggaggtac 300
gaagcaggag ctcacctgca gggcttcccg tccatgcacc ctggagtccg cgggacctgg 360
agggagtccg ccaaccggag cagtcgctaa gccttcactc ctggatctca gaggagcccg 420
ctgctagagc cttcggagag acgcttcgtg cccagccatg gttcctgcag caagtcatct 480
ttgccgatcc tgtcaggccc aagaaccgat ggcgccccca tgcttgacct aggcaggagc 540
acagcttgaa gctccagtca ggcctcgtgt ttctggtcaa taaaaccaac ctgattcc 598

<210> 87
<211> 21
<212> DNA
<213> Artificial Sequence

<220>
<223> Primer

<400> 87
aaaggctgta gtcgcaccaa c 21

<210> 88
<211> 21
<212> DNA
<213> Artificial Sequence

<220>
<223> Primer

<400> 88
accagaaaca cgaggcctga c 21

<210> 89
<211> 659
<212> DNA
<213> Mouse

<400> 89
tgactggtct ccatcctctg gagctccgac gtgctcgttc tcggagacat aaacccagtt 60
cttgtcctaa ccctccaagg ggcaattgac gtgagcgcgc tggcgtctaa cagagaagta 120
cggggccctg ggcccgggac tcccaggaac cggcccctgc tgcccctgct gctgcttctg 180
ctcttgctac cgctgcccgc cagcgcctgg tataagcacg tggcgagtcc ccgctatcac 240
acagtgggtc gtgcctccgg gctgctcatg gggctgcgcc gctcgcccta ccagtggcgc 300
cgtgccctgg gcggggctgc tggacccctc tcccggctcc caggaccggt cgcccgcggc 360
gctctcctgc ttccttcctc agggcaggag ctgtgggagg tacgaagcag gagctcacct 420

gcagggcttc ccgtccatgc accctggagt ccgcgggacc tggagggagt ccgccaaccg 480
gagcagtcgc taagccttca ctcctggatc tcagaggagc ccgctgctag agccttcgga 540
gagacgcttc gtgcccagcc atggttcctg cagcaagtca tctttgccga tcctgtcagg 600
cccaagaacc gatggcgccc ccatgcttga cctaggcagg agcacagctt gaagctcca 659

<210> 90
<211> 176
<212> PRT
<213> Mouse

<400> 90
Leu Ala Ser Asn Arg Glu Val Arg Gly Pro Gly Pro Gly Thr Pro Arg
1 5 10 15
Asn Arg Pro Leu Leu Pro Leu Leu Leu Leu Leu Leu Leu Leu Pro Leu
20 25 30
Pro Ala Ser Ala Trp Tyr Lys His Val Ala Ser Pro Arg Tyr His Thr
35 40 45
Val Gly Arg Ala Ser Gly Leu Leu Met Gly Leu Arg Arg Ser Pro Tyr
50 55 60
Gln Trp Arg Arg Ala Leu Gly Gly Ala Ala Gly Pro Leu Ser Arg Leu
65 70 75 80
Pro Gly Pro Val Ala Arg Gly Ala Leu Leu Leu Pro Ser Ser Gly Gln
85 90 95
Glu Leu Trp Glu Val Arg Ser Arg Ser Ser Pro Ala Gly Leu Pro Val
100 105 110
His Ala Pro Trp Ser Pro Arg Asp Leu Glu Gly Val Arg Gln Pro Glu
115 120 125
Gln Ser Leu Ser Leu His Ser Trp Ile Ser Glu Glu Pro Ala Ala Arg
130 135 140
Ala Phe Gly Glu Thr Leu Arg Ala Gln Pro Trp Phe Leu Gln Gln Val
145 150 155 160
Ile Phe Ala Asp Pro Val Arg Pro Lys Asn Arg Trp Arg Pro His Ala
165 170 175 176

<210> 91
<211> 23

<212> PRT
<213> Mouse

<400> 91
Trp Tyr Lys His Val Ala Ser Pro Arg Tyr His Thr Val Gly Arg Ala
1 5 10 15
Ser Gly Leu Leu Met Gly Leu
20 23

<210> 92
<211> 30
<212> PRT
<213> Mouse

<400> 92
Trp Tyr Lys His Val Ala Ser Pro Arg Tyr His Thr Val Gly Arg Ala
1 5 10 15
Ser Gly Leu Leu Met Gly Leu Arg Arg Ser Pro Tyr Gln Trp
20 25 30

<210> 93
<211> 69
<212> DNA
<213> Mouse

<400> 93
tggtataagc acgtggcgag tccccgctat cacacagtgg gtcgtgcctc cgggctgctc 60
atggggctg 69

<210> 94
<211> 90
<212> DNA
<213> Mouse

<400> 94
tggtataagc acgtggcgag tccccgctat cacacagtgg gtcgtgcctc cgggctgctc 60

atggggctgc gccgctcgcc ctaccagtgg 90

<210> 95
<211> 23
<212> PRT
<213> Artificial Sequence

<220>
<221> MUTAGEN
<222> 21
<223> Met on the 21st position is oxidised

<400> 95
Trp Tyr Lys His Val Ala Ser Pro Arg Tyr His Thr Val Gly Arg Ala
1 5 10 15
Ala Gly Leu Leu Met Gly Leu
20 23

<210> 96
<211> 22
<212> PRT
<213> Human

<400> 96
Trp Tyr Lys His Val Ala Ser Pro Arg Tyr His Thr Val Gly Arg Ala
1 5 10 15
Ala Gly Leu Leu Met Gly
20 22

<210> 97
<211> 21
<212> PRT
<213> Human

<400> 97
Trp Tyr Lys His Val Ala Ser Pro Arg Tyr His Thr Val Gly Arg Ala

1 5 10 15
Ala Gly Leu Leu Met
20 21

<210> 98
<211> 20
<212> PRT
<213> Human

<400> 98
Trp Tyr Lys His Val Ala Ser Pro Arg Tyr His Thr Val Gly Arg Ala
1 5 10 15
Ala Gly Leu Leu
20

<210> 99
<211> 19
<212> PRT
<213> Human

<400> 99
Trp Tyr Lys His Val Ala Ser Pro Arg Tyr His Thr Val Gly Arg Ala
1 5 10 15
Ala Gly Leu
19

<210> 100
<211> 18
<212> PRT
<213> Human

<400> 100
Trp Tyr Lys His Val Ala Ser Pro Arg Tyr His Thr Val Gly Arg Ala
1 5 10 15
Ala Gly
18

<210> 101
<211> 17
<212> PRT
<213> Human

<400> 101
Trp Tyr Lys His Val Ala Ser Pro Arg Tyr His Thr Val Gly Arg Ala
1 5 10 15
Ala
17

<210> 102
<211> 16
<212> PRT
<213> Human

<400> 102
Trp Tyr Lys His Val Ala Ser Pro Arg Tyr His Thr Val Gly Arg Ala
1 5 10 15 16

<210> 103
<211> 23
<212> PRT
<213> Artificial Sequence

<220>
<221> MUTAGEN
<222> 21
<223> Met on the 21st position is oxidised

<400> 103
Trp Tyr Lys His Thr Ala Ser Pro Arg Tyr His Thr Val Gly Arg Ala
1 5 10 15
Ala Gly Leu Leu Met Gly Leu
20 23

<210> 104
<211> 23
<212> PRT
<213> Artificial Sequence

<220>
<221> MUTAGEN
<222> 21
<223> Met on the 21st position is oxidised

<400> 104
Trp Tyr Lys His Val Ala Ser Pro Arg Tyr His Thr Val Gly Arg Ala
1 5 10 15
Ser Gly Leu Leu Met Gly Leu
20 23

<210> 105
<211> 23
<212> PRT
<213> Artificial Sequence

<220>
<221> MUTAGEN
<222> 1
<223> Trp on the 1st position is Fmoc-added Trp

<400> 105
Trp Tyr Lys His Val Ala Ser Pro Arg Tyr His Thr Val Gly Arg Ala
1 5 10 15
Ala Gly Leu Leu Met Gly Leu
20 23

<210> 106
<211> 23

<212> PRT
<213> Artificial Sequence

<220>
<221> ACETYLATION
<222> 1
<223> Trp on the 1st position is acetylated

<400> 106
Trp Tyr Lys His Val Ala Ser Pro Arg Tyr His Thr Val Gly Arg Ala
1 5 10 15
Ala Gly Leu Leu Met Gly Leu
20 23

<210> 107
<211> 22
<212> PRT
<213> Human

<400> 107
Tyr Lys His Val Ala Ser Pro Arg Tyr His Thr Val Gly Arg Ala Ala
1 5 10 15
Gly Leu Leu Met Gly Leu
20 22

<210> 108
<211> 20
<212> PRT
<213> Human

<400> 108
His Val Ala Ser Pro Arg Tyr His Thr Val Gly Arg Ala Ala Gly Leu
1 5 10 15
Leu Met Gly Leu
20

<210> 109
<211> 15
<212> PRT
<213> Human

<400> 109
Arg Tyr His Thr Val Gly Arg Ala Ala Gly Leu Leu Met Gly Leu
1 5 10 15

<210> 110
<211> 9
<212> PRT
<213> Human

<400> 110
Arg Ala Ala Gly Leu Leu Met Gly Leu
1 5 9

<210> 111
<211> 22
<212> PRT
<213> Artificial Sequence

<220>
<221> ACETYLATION
<222> 1
<223> Tyr on the 1st position is acetylated

<400> 111
Tyr Lys His Val Ala Ser Pro Arg Tyr His Thr Val Gly Arg Ala Ala
1 5 10 15
Gly Leu Leu Met Gly Leu
20 22

<210> 112
<211> 23

<212> PRT
<213> Artificial Sequence

<220>
<221> MUTAGEN
<222> 1
<223> Trp on the 1st position means D-Trp

<400> 112
Trp Tyr Lys His Val Ala Ser Pro Arg Tyr His Thr Val Gly Arg Ala
1 5 10 15
Ala Gly Leu Leu Met Gly Leu
20 23

<210> 113
<211> 22
<212> PRT
<213> Artificial Sequence

<220>
<221> MUTAGEN
<222> 1
<223> Tyr on the 1st position means 3-Indolepropanoyl Tyr

<400> 113
Tyr Lys His Val Ala Ser Pro Arg Tyr His Thr Val Gly Arg Ala Ala
1 5 10 15
Gly Leu Leu Met Gly Leu
20 22

<210> 114
<211> 66
<212> DNA
<213> Human

<400> 114

tggtacaagc acgtggcgag tccccgctac cacacggtgg gccgcgccgc tggcctgctc 60
atgggg 66

<210> 115
<211> 63
<212> DNA
<213> Human

<400> 115
tggtacaagc acgtggcgag tccccgctac cacacggtgg gccgcgccgc tggcctgctc 60
atg 63

<210> 116
<211> 60
<212> DNA
<213> Human

<400> 116
tggtacaagc acgtggcgag tccccgctac cacacggtgg gccgcgccgc tggcctgctc 60

<210> 117
<211> 57
<212> DNA
<213> Human

<400> 117
tggtacaagc acgtggcgag tccccgctac cacacggtgg gccgcgccgc tggcctg 57

<210> 118
<211> 54
<212> DNA
<213> Human

<400> 118
tggtacaagc acgtggcgag tccccgctac cacacggtgg gccgcgccgc tggc 54

<210> 119
<211> 51
<212> DNA
<213> Human

<400> 119
tggtacaagc acgtggcgag tccccgctac cacacggtgg gccgcgccgc t 51

<210> 120
<211> 48
<212> DNA
<213> Human

<400> 120
tggtacaagc acgtggcgag tccccgctac cacacggtgg gccgcgcc 48

<210> 121
<211> 66
<212> DNA
<213> Human

<400> 121
tacaagcacg tggcgagtcc ccgctaccac acggtgggcc gcgccgctgg cctgctcatg 60
gggctg 66

<210> 122
<211> 60
<212> DNA
<213> Human

<400> 122
cacgtggcga gtccccgcta ccacacggtg ggccgcgccg ctggcctgct catggggctg 60

<210> 123
<211> 45
<212> DNA

<213> Human

<400> 123
cgctaccaca cggtgggccg cgccgctggc ctgctcatgg ggctg 45

<210> 124
<211> 27
<212> DNA
<213> Human

<400> 124
cgcgccgctg gcctgctcat ggggctg 27

<210> 125
<211> 51
<212> DNA
<213> Porcine

<400> 125
tggtacaagc acacggcgag tccccgctac cacacggtgg gccgcgccgc g 51

<210> 126
<211> 69
<212> DNA
<213> Human

<400> 126
tggtacaagc acgtggcgag tccccgctac cacacggtgg gccgcgccgc tggcctgctc 60
atggggctg 69

<210> 127
<211> 90
<212> DNA
<213> Human

<400> 127

tggtacaagc acgtggcgag tccccgctac cacacggtgg gccgcgccgc tggcctgctc 60
atggggctgc gtcgctcacc ctatctgtgg 90

<210> 128
<211> 29
<212> PRT
<213> Human

<400> 128
Trp Tyr Lys His Val Ala Ser Pro Arg Tyr His Thr Val Gly Arg Ala
1 5 10 15
Ala Gly Leu Leu Met Gly Leu Arg Arg Ser Pro Tyr Leu
20 25 29

<210> 129
<211> 28
<212> PRT
<213> Human

<400> 129
Trp Tyr Lys His Val Ala Ser Pro Arg Tyr His Thr Val Gly Arg Ala
1 5 10 15
Ala Gly Leu Leu Met Gly Leu Arg Arg Ser Pro Tyr
20 25 28

<210> 130
<211> 27
<212> PRT
<213> Human

<400> 130
Trp Tyr Lys His Val Ala Ser Pro Arg Tyr His Thr Val Gly Arg Ala
1 5 10 15
Ala Gly Leu Leu Met Gly Leu Arg Arg Ser Pro
20 25 27

<210> 131
<211> 26
<212> PRT
<213> Human

<400> 131
Trp Tyr Lys His Val Ala Ser Pro Arg Tyr His Thr Val Gly Arg Ala
1 5 10 15
Ala Gly Leu Leu Met Gly Leu Arg Arg Ser
20 25 26

<210> 132
<211> 24
<212> DNA
<213> Artificial Sequence

<220>
<223> Primer

<400> 132
gtccgcgatg ttgatgggca gcac 24

<210> 133
<211> 24
<212> DNA
<213> Artificial Sequence

<220>
<223> Primer

<400> 133
gaagagctca tcggcgatag ccag 24

<210> 134
<211> 440
<212> DNA

<213> Mouse

<400> 134
taagcagtgg taacaacgca gagtacgcgg gggcgcataa gcagtggtaa caacgcagag 60
tcacgcgggg agtgcctggg tgcagatccc tgtaaacgtg ggcgcataaa cctcgagttt 120
cgcggggctg ctgagtggaa tcctggtggt cgcctgctct ccagccctct ccaagatgca 180
taacttaacg cttttcgagt ctggagggga caacgtgtct tgcggcggct catctttggg 240
ctgtcccaac gggtccagcc tggctcctct gccgctgccg cagccactgg cggtagcagt 300
gcctgtcgtc tacggggtaa tttgcgccgt gggactggct ggcaactctg cggtgctgta 360
cgtactgctg cgcacgccgc gcatgaagac tgtcaccaac gtgttcatcc tcaacctggc 420
tatcgccgat gagctcttca 440

<210> 135
<211> 24
<212> DNA
<213> Artificial Sequence

<220>
<223> Primer

<400> 135
tttcgcgggg ctgctgagtg gaat 24

<210> 136
<211> 24
<212> DNA
<213> Artificial Sequence

<220>
<223> Primer

<400> 136
agtgctgcct gcggtggaaa gagg 24

<210> 137
<211> 1083

<212> DNA
<213> Mouse

<400> 137

```
tttcgcgggg ctgctgagtg gaatcctggt ggtcgcctgc tctccagccc tctccaagat   60
gcataactta acgcttttcg agtctggagg ggacaacgtg tcttgcggcg gctcatcttt  120
gggctgtccc aacgggtcca gcctggctcc tctgccgctg ccgcagccac tggcggtagc  180
agtgcctgtc gtctacgggg taatttgcgc cgtgggactg gctggcaact ctgcggtgct  240
gtacgtactg ctgcgcacgc cgcgcatgaa gactgtcacc aacgtgttca tcctcaacct  300
ggctatcgcc gatgagctct tcaccctcgt gctgcccatc aacatcgcgg acttcctgct  360
gaggcgctgg cccttcgggg aggtcatgtg caagctcatt gtagccgtcg accagtacaa  420
cactttctct agcctctact tcctcgccgt catgagcgcc gaccgatacc tggtggttct  480
ggccacagca gagtcgcgcc gggtgtccgg gcgcacttac ggtgcagcgc gtgctgtcag  540
tctggcggtg tgggcgctgg tgacgctggt cgtgctgccc tttgcggtat tcgctcggct  600
ggacgaggag cagggtcggc gccagtgcgt gctggtcttc ccgcagcccg aggccttctg  660
gtggcgtgcc agccgtctct acacactagt attgggcttt gccatcccgg tgaccaccat  720
ctgtgctctc tataccactc tgctctgccg actgcgtgct atccagctag atagccacgc  780
caaggccctg gatcgtgcca agaagcgcgt gaccttgttg gtggcggcga ttctggctgt  840
gtgcctcctc tgctggacgc cttatcacct gagtaccata gtggccctca ccaccgacct  900
cccgcaaacg ccgctggtca tcggcatctc ttacttcatc accagcctga gctatgctaa  960
cagctgcctc aaccctttcc tctatgcctt cctggacgac agcttccgca gaagcctccg 1020
gcaattggtg tcatgccgtt cagcctgatg ccctttccac ctctttccac cgcaggcagc 1080
act                                                               1083
```

<210> 138
<211> 329
<212> PRT
<213> Mouse

<400> 138

```
Met His Asn Leu Thr Leu Phe Glu Ser Gly Gly Asp Asn Val Ser Cys
                    5                   10                  15
Gly Gly Ser Ser Leu Gly Cys Pro Asn Gly Ser Ser Leu Ala Pro Leu
            20                  25                  30
Pro Leu Pro Gln Pro Leu Ala Val Ala Val Pro Val Val Tyr Gly Val
        35                  40                  45
```

Ile Cys Ala Val Gly Leu Ala Gly Asn Ser Ala Val Leu Tyr Val Leu
50 55 60
Leu Arg Thr Pro Arg Met Lys Thr Val Thr Asn Val Phe Ile Leu Asn
65 70 75 80
Leu Ala Ile Ala Asp Glu Leu Phe Thr Leu Val Leu Pro Ile Asn Ile
85 90 95
Ala Asp Phe Leu Leu Arg Arg Trp Pro Phe Gly Glu Val Met Cys Lys
100 105 110
Leu Ile Val Ala Val Asp Gln Tyr Asn Thr Phe Ser Ser Leu Tyr Phe
115 120 125
Leu Ala Val Met Ser Ala Asp Arg Tyr Leu Val Val Leu Ala Thr Ala
130 135 140
Glu Ser Arg Arg Val Ser Gly Arg Thr Tyr Gly Ala Ala Arg Ala Val
145 150 155 160
Ser Leu Ala Val Trp Ala Leu Val Thr Leu Val Val Leu Pro Phe Ala
165 170 175
Val Phe Ala Arg Leu Asp Glu Glu Gln Gly Arg Arg Gln Cys Val Leu
180 185 190
Val Phe Pro Gln Pro Glu Ala Phe Trp Trp Arg Ala Ser Arg Leu Tyr
195 200 205
Thr Leu Val Leu Gly Phe Ala Ile Pro Val Thr Thr Ile Cys Ala Leu
210 215 220
Tyr Thr Thr Leu Leu Cys Arg Leu Arg Ala Ile Gln Leu Asp Ser His
225 230 235 240
Ala Lys Ala Leu Asp Arg Ala Lys Lys Arg Val Thr Leu Leu Val Ala
245 250 255
Ala Ile Leu Ala Val Cys Leu Leu Cys Trp Thr Pro Tyr His Leu Ser
260 265 270
Thr Ile Val Ala Leu Thr Thr Asp Leu Pro Gln Thr Pro Leu Val Ile
275 280 285
Gly Ile Ser Tyr Phe Ile Thr Ser Leu Ser Tyr Ala Asn Ser Cys Leu
290 295 300
Asn Pro Phe Leu Tyr Ala Phe Leu Asp Asp Ser Phe Arg Arg Ser Leu
305 310 315 320
Arg Gln Leu Val Ser Cys Arg Ser Ala
325 329

<210> 139
<211> 987
<212> DNA
<213> Mouse

<400> 139
atgcataact taacgctttt cgagtctgga ggggacaacg tgtcttgcgg cggctcatct 60
ttgggctgtc ccaacgggtc cagcctggct cctctgccgc tgccgcagcc actggcggta 120
gcagtgcctg tcgtctacgg ggtaatttgc gccgtgggac tggctggcaa ctctgcggtg 180
ctgtacgtac tgctgcgcac gccgcgcatg aagactgtca ccaacgtgtt catcctcaac 240
ctggctatcg ccgatgagct cttcaccctc gtgctgccca tcaacatcgc ggacttcctg 300
ctgaggcgct ggcccttcgg ggaggtcatg tgcaagctca ttgtagccgt cgaccagtac 360
aacactttct ctagcctcta cttcctcgcc gtcatgagcg ccgaccgata cctggtggtt 420
ctggccacag cagagtcgcg ccgggtgtcc gggcgcactt acggtgcagc gcgtgctgtc 480
agtctggcgg tgtgggcgct ggtgacgctg gtcgtgctgc cctttgcggt attcgctcgg 540
ctggacgagg agcagggtcg gcgccagtgc gtgctggtct tcccgcagcc cgaggccttc 600
tggtggcgtg ccagccgtct ctacacacta gtattgggct ttgccatccc ggtgaccacc 660
atctgtgctc tctataccac tctgctctgc cgactgcgtg ctatccagct agatagccac 720
gccaaggccc tggatcgtgc caagaagcgc gtgaccttgt tggtggcggc gattctggct 780
gtgtgcctcc tctgctggac gccttatcac ctgagtacca tagtggccct caccaccgac 840
ctcccgcaaa cgccgctggt catcggcatc tcttacttca tcaccagcct gagctatgct 900
aacagctgcc tcaacccttt cctctatgcc ttcctggacg acagcttccg cagaagcctc 960
cggcaattgg tgtcatgccg ttcagcc 987

<210> 140
<211> 23
<212> PRT
<213> Human

<400> 140
Trp Tyr Lys Pro Ala Ala Gly His Ser Ser Tyr Ser Val Gly Arg Ala
1 5 10 15
Ala Gly Leu Leu Ser Gly Leu
20

<210> 141
<211> 24
<212> PRT
<213> Mouse

<400> 141
Trp Tyr Lys Pro Ala Ala Gly Pro His His Tyr Ser Val Gly Arg Ala
5 10 15
Ser Gly Leu Leu Ser Ser Phe His
20

<210> 142
<211> 24
<212> PRT
<213> Rat

<400> 142
Trp Tyr Lys Pro Ala Ala Gly Ser His His Tyr Ser Val Gly Arg Ala
5 10 15
Ala Gly Leu Leu Ser Ser Phe His
20

<210> 143
<211> 29
<212> PRT
<213> Human

<400> 143
Trp Tyr Lys Pro Ala Ala Gly His Ser Ser Tyr Ser Val Gly Arg Ala
5 10 15
Ala Gly Leu Leu Ser Gly Leu Arg Arg Ser Pro Tyr Ala
20 25

<210> 144
<211> 29
<212> PRT

<213> Mouse

<400> 144

Trp Tyr Lys Pro Ala Ala Gly Pro His His Tyr Ser Val Gly Arg Ala
5 10 15

Ser Gly Leu Leu Ser Ser Phe His Arg Phe Pro Ser Thr
20 25

<210> 145

<211> 29

<212> PRT

<213> Rat

<400> 145

Trp Tyr Lys Pro Ala Ala Gly Ser His His Tyr Ser Val Gly Arg Ala
5 10 15

Ala Gly Leu Leu Ser Ser Phe His Arg Phe Pro Ser Thr
20 25

<210> 146

<211> 13

<212> PRT

<213> Human

<400> 146

Trp Tyr Lys Pro Ala Ala Gly His Ser Ser Tyr Ser Val
5 10

<210> 147

<211> 14

<212> PRT

<213> Human

<400> 147

Trp Tyr Lys Pro Ala Ala Gly His Ser Ser Tyr Ser Val Gly
5 10

<210> 148
<211> 13
<212> PRT
<213> Mouse

<400> 148
Trp Tyr Lys Pro Ala Ala Gly Pro His His Tyr Ser Val
5 10

<210> 149
<211> 14
<212> PRT
<213> Mouse

<400> 149
Trp Tyr Lys Pro Ala Ala Gly Pro His His Tyr Ser Val Gly
5 10

<210> 150
<211> 13
<212> PRT
<213> Rat

<400> 150
Trp Tyr Lys Pro Ala Ala Gly Ser His His Tyr Ser Val
5 10

<210> 151
<211> 14
<212> PRT
<213> Rat

<400> 151
Trp Tyr Lys Pro Ala Ala Gly Ser His His Tyr Ser Val Gly
5 10

<210> 152
<211> 70
<212> PRT
<213> Human

<400> 152
Ser Gln Pro Tyr Arg Gly Ala Glu Pro Pro Gly Gly Ala Gly Ala Ser
5 10 15
Pro Glu Leu Gln Leu His Pro Arg Leu Arg Ser Leu Ala Val Cys Val
20 25 30
Gln Asp Val Ala Pro Asn Leu Gln Arg Cys Glu Arg Leu Pro Asp Gly
35 40 45
Arg Gly Thr Tyr Gln Cys Lys Ala Asn Val Phe Leu Ser Leu Arg Ala
50 55 60
Ala Asp Cys Leu Ala Ala
65 70

<210> 153
<211> 67
<212> PRT
<213> Mouse

<400> 153
Ser Glu Ser Pro Ala Leu Arg Val Gly Thr Gly Pro Leu Arg Asn Leu
5 10 15
Glu Met Arg Pro Ser Val Arg Ser Leu Ala Leu Cys Val Lys Asp Val
20 25 30
Thr Pro Asn Leu Gln Ser Cys Gln Arg Gln Leu Asn Ser Arg Gly Thr
35 40 45
Phe Gln Cys Lys Ala Asp Val Phe Leu Ser Leu His Glu Thr Asp Cys
50 55 60
Gln Ser Thr
65

<210> 154

<211> 67
<212> PRT
<213> Rat

<400> 154
Ser Glu Ser Pro Ala Leu Arg Val Gly Thr Val Pro Leu Arg Asn Leu
5 10 15
Glu Met Arg Pro Ser Val Arg Ser Leu Ala Leu Cys Val Lys Asp Val
20 25 30
Thr Pro Asn Leu Gln Ser Cys Gln Arg Gln Leu Asn Ser Arg Gly Thr
35 40 45
Phe Gln Cys Lys Ala Asp Val Phe Leu Ser Leu His Lys Ala Glu Cys
50 55 60
Gln Ser Ala
65

<210> 155
<211> 44
<212> PRT
<213> Human

<400> 155
Ser Leu Ala Val Cys Val Gln Asp Val Ala Pro Asn Leu Gln Arg Cys
5 10 15
Glu Arg Leu Pro Asp Gly Arg Gly Thr Tyr Gln Cys Lys Ala Asn Val
20 25 30
Phe Leu Ser Leu Arg Ala Ala Asp Cys Leu Ala Ala
35 40

<210> 156
<211> 44
<212> PRT
<213> Mouse

<400> 156
Ser Leu Ala Leu Cys Val Lys Asp Val Thr Pro Asn Leu Gln Ser Cys

5 10 15
Gln Arg Gln Leu Asn Ser Arg Gly Thr Phe Gln Cys Lys Ala Asp Val
20 25 30
Phe Leu Ser Leu His Glu Thr Asp Cys Gln Ser Thr
35 40

<210> 157
<211> 44
<212> PRT
<213> Rat

<400> 157
Ser Leu Ala Leu Cys Val Lys Asp Val Thr Pro Asn Leu Gln Ser Cys
5 10 15
Gln Arg Gln Leu Asn Ser Arg Gly Thr Phe Gln Cys Lys Ala Asp Val
20 25 30
Phe Leu Ser Leu His Lys Ala Glu Cys Gln Ser Ala
35 40

<210> 158
<211> 69
<212> DNA
<213> Human

<400> 158
tggtacaagc cagcggcggg gcacagctcc tactcggtgg gccgcgccgc ggggctgctg 60
tccggcctc 69

<210> 159
<211> 72
<212> DNA
<213> Mouse

<400> 159
tggtacaagc ccgcggcggg accccaccac tactcggtgg gccgcgcctc ggggctactg 60
tcgagtttcc ac 72

<210> 160
<211> 72
<212> DNA
<213> Rat

<400> 160
tggtacaagc ccgcggcggg atcccaccac tactcggtgg gccgcgctgc ggggctactg 60
tcgagtttcc ac 72

<210> 161
<211> 87
<212> DNA
<213> Human

<400> 161
tggtacaagc cagcggcggg gcacagctcc tactcggtgg gccgcgccgc ggggctgctg 60
tccggcctcc gcaggtcccc gtacgcg 87

<210> 162
<211> 87
<212> DNA
<213> Mouse

<400> 162
tggtacaagc ccgcggcggg accccaccac tactcggtgg gccgcgcctc ggggctactg 60
tcgagtttcc acaggttccc gtccacg 87

<210> 163
<211> 87
<212> DNA
<213> Rat

<400> 163
tggtacaagc ccgcggcggg atcccaccac tactcggtgg gccgcgctgc ggggctactg 60
tcgagtttcc acaggttccc atccacg 87

<210> 164
<211> 39
<212> DNA
<213> Human

<400> 164
tggtacaagc cagcggcggg gcacagctcc tactcggtg 39

<210> 165
<211> 42
<212> DNA
<213> Human

<400> 165
tggtacaagc cagcggcggg gcacagctcc tactcggtgg gc 42

<210> 166
<211> 39
<212> DNA
<213> Mouse

<400> 166
tggtacaagc ccgcggcggg accccaccac tactcggtg 39

<210> 167
<211> 42
<212> DNA
<213> Mouse

<400> 167
tggtacaagc ccgcggcggg accccaccac tactcggtgg gc 42

<210> 168
<211> 39
<212> DNA

<213> Rat

<400> 168
tggtacaagc ccgcggcggg atcccaccac tactcggtg 39

<210> 169
<211> 42
<212> DNA
<213> Rat

<400> 169
tggtacaagc ccgcggcggg atcccaccac tactcggtgg gc 42

<210> 170
<211> 210
<212> DNA
<213> Human

<400> 170
tcccagccct acagaggggc ggaacccccg ggcggggccg gcgcctcccc ggagctgcaa 60
ctgcacccca ggctgcggag cctcgctgtg tgcgtccagg acgtcgcccc aaacctgcag 120
aggtgcgagc ggctccccga cggccgcggg acctaccagt gcaaggcgaa cgtcttcctg 180
tccctgcgcg cagccgactg cctcgccgcc 210

<210> 171
<211> 201
<212> DNA
<213> Mouse

<400> 171
tccgagtctc cagcactccg ggtgggaacc ggacctctgc gcaatttaga gatgcgcccc 60
agcgtaagga gccttgccct gtgtgtcaaa gatgtgaccc cgaacctgca gagctgccag 120
cggcaactca acagccgagg gactttccag tgtaaagcgg acgtcttctt gtcgctgcac 180
gagactgatt gccagagcac c 201

<210> 172

<211> 201
<212> DNA
<213> Rat

<400> 172
tccgagtctc cagcactccg ggtgggaacc gtacctctgc gcaacttgga gatgcgccca 60
agcgtaagaa gccttgccct gtgtgtcaaa gatgtgaccc cgaacctgca gagctgccag 120
cggcaactca acagccgagg gactttccag tgtaaggcgg acgtcttctt gtcgctgcac 180
aaggctgaat gccaaagcgc c 201

<210> 173
<211> 132
<212> DNA
<213> Human

<400> 173
agcctcgctg tgtgcgtcca ggacgtcgcc ccaaacctgc agaggtgcga gcggctcccc 60
gacggccgcg ggacctacca gtgcaaggcg aacgtcttcc tgtccctgcg cgcagccgac 120
tgcctcgccg cc 132

<210> 174
<211> 132
<212> DNA
<213> Mouse

<400> 174
agccttgccc tgtgtgtcaa agatgtgacc ccgaacctgc agagctgcca gcggcaactc 60
aacagccgag ggactttcca gtgtaaagcg gacgtcttct tgtcgctgca cgagactgat 120
tgccagagca cc 132

<210> 175
<211> 132
<212> DNA
<213> Rat

<400> 175

agccttgccc tgtgtgtcaa agatgtgacc ccgaacctgc agagctgcca gcggcaactc 60
aacagccgag ggactttcca gtgtaaggcg gacgtcttct tgtcgctgca caaggctgaa 120
tgccaaagcg cc 132

<210> 176
<211> 125
<212> PRT
<213> Human

<400> 176
Met Ala Arg Ser Ala Thr Leu Ala Ala Ala Ala Leu Ala Leu Cys Leu
5 10 15
Leu Leu Ala Pro Pro Gly Leu Ala Trp Tyr Lys Pro Ala Ala Gly His
20 25 30
Ser Ser Tyr Ser Val Gly Arg Ala Ala Gly Leu Leu Ser Gly Leu Arg
35 40 45
Arg Ser Pro Tyr Ala Arg Arg Ser Gln Pro Tyr Arg Gly Ala Glu Pro
50 55 60
Pro Gly Gly Ala Gly Ala Ser Pro Glu Leu Gln Leu His Pro Arg Leu
65 70 75 80
Arg Ser Leu Ala Val Cys Val Gln Asp Val Ala Pro Asn Leu Gln Arg
85 90 95
Cys Glu Arg Leu Pro Asp Gly Arg Gly Thr Tyr Gln Cys Lys Ala Asn
100 105 110
Val Phe Leu Ser Leu Arg Ala Ala Asp Cys Leu Ala Ala
115 120 125

<210> 177
<211> 119
<212> PRT
<213> Mouse

<400> 177
Met Ala Arg Cys Arg Thr Leu Val Ala Ala Ala Leu Ala Leu Leu Leu
5 10 15
Pro Pro Ala Leu Ala Trp Tyr Lys Pro Ala Ala Gly Pro His His Tyr

20 25 30

Ser Val Gly Arg Ala Ser Gly Leu Leu Ser Ser Phe His Arg Phe Pro
35 40 45

Ser Thr Arg Arg Ser Glu Ser Pro Ala Leu Arg Val Gly Thr Gly Pro
50 55 60

Leu Arg Asn Leu Glu Met Arg Pro Ser Val Arg Ser Leu Ala Leu Cys
65 70 75 80

Val Lys Asp Val Thr Pro Asn Leu Gln Ser Cys Gln Arg Gln Leu Asn
85 90 95

Ser Arg Gly Thr Phe Gln Cys Lys Ala Asp Val Phe Leu Ser Leu His
100 105 110

Glu Thr Asp Cys Gln Ser Thr
115 119

<210> 178
<211> 119
<212> PRT
<213> Rat

<400> 178

Met Val Arg Cys Arg Thr Leu Val Ala Ala Ala Leu Ala Leu Leu Leu
5 10 15

Thr Pro Ala Leu Ala Trp Tyr Lys Pro Ala Ala Gly Ser His His Tyr
20 25 30

Ser Val Gly Arg Ala Ala Gly Leu Leu Ser Ser Phe His Arg Phe Pro
35 40 45

Ser Thr Arg Arg Ser Glu Ser Pro Ala Leu Arg Val Gly Thr Val Pro
50 55 60

Leu Arg Asn Leu Glu Met Arg Pro Ser Val Arg Ser Leu Ala Leu Cys
65 70 75 80

Val Lys Asp Val Thr Pro Asn Leu Gln Ser Cys Gln Arg Gln Leu Asn
85 90 95

Ser Arg Gly Thr Phe Gln Cys Lys Ala Asp Val Phe Leu Ser Leu His
100 105 110

Lys Ala Glu Cys Gln Ser Ala
115 119

<210> 179
<211> 375
<212> DNA
<213> Human

<400> 179
atggcccggt ccgcgacact ggcggccgcc gccctggcgc tgtgcctgct gctggcgccg 60
cctggcctcg cgtggtacaa gccagcggcg gggcacagct cctactcggt gggccgcgcc 120
gcggggctgc tgtccggcct ccgcaggtcc ccgtacgcgc ggcgctccca gccctacaga 180
ggggcggaac ccccgggcgg ggccggcgcc tccccggagc tgcaactgca ccccaggctg 240
cggagcctcg ctgtgtgcgt ccaggacgtc gccccaaacc tgcagaggtg cgagcggctc 300
cccgacggcc gcgggaccta ccagtgcaag gcgaacgtct tcctgtccct gcgcgcagcc 360
gactgcctcg ccgcc 375

<210> 180
<211> 357
<212> DNA
<213> Mouse

<400> 180
atggcccggt gtaggacgct ggtggccgct gccctggcgc tgctcctgcc gccagccctc 60
gcgtggtaca agcccgcggc gggaccccac cactactcgg tgggccgcgc ctcggggcta 120
ctgtcgagtt tccacaggtt cccgtccacg cgacgctccg agtctccagc actccgggtg 180
ggaaccggac ctctgcgcaa tttagagatg cgccccagcg taaggagcct tgccctgtgt 240
gtcaaagatg tgaccccgaa cctgcagagc tgccagcggc aactcaacag ccgagggact 300
ttccagtgta aagcggacgt cttcttgtcg ctgcacgaga ctgattgcca gagcacc 357

<210> 181
<211> 357
<212> DNA
<213> Rat

<400> 181
atggtccggt gtaggacgct ggtggccgcc gccctggcgc tgctcctgac gccagccctc 60
gcgtggtaca agcccgcggc gggatcccac cactactcgg tgggccgcgc tgcggggcta 120

ctgtcgagtt tccacaggtt cccatccacg cgacgttccg agtctccagc actccgggtg 180
ggaaccgtac ctctgcgcaa cttggagatg cgcccaagcg taagaagcct tgccctgtgt 240
gtcaaagatg tgaccccgaa cctgcagagc tgccagcggc aactcaacag ccgagggact 300
ttccagtgta aggcggacgt cttcttgtcg ctgcacaagg ctgaatgcca aagcgcc 357

<210> 182
<211> 328
<212> PRT
<213> Human

<400> 182

Met Asp Asn Ala Ser Phe Ser Glu Pro Trp Pro Ala Asn Ala Ser Gly
1 5 10 15
Pro Asp Pro Ala Leu Ser Cys Ser Asn Ala Ser Thr Leu Ala Pro Leu
20 25 30
Pro Ala Pro Leu Ala Val Ala Val Pro Val Val Tyr Ala Val Ile Cys
35 40 45
Ala Val Gly Leu Ala Gly Asn Ser Ala Val Leu Tyr Val Leu Leu Arg
50 55 60
Ala Pro Arg Met Lys Thr Val Thr Asn Leu Phe Ile Leu Asn Leu Ala
65 70 75 80
Ile Ala Asp Glu Leu Phe Thr Leu Val Leu Pro Ile Asn Ile Ala Asp
85 90 95
Phe Leu Leu Arg Gln Trp Pro Phe Gly Glu Leu Met Cys Lys Leu Ile
100 105 110
Val Ala Ile Asp Gln Tyr Asn Thr Phe Ser Ser Leu Tyr Phe Leu Thr
115 120 125
Val Met Ser Ala Asp Arg Tyr Leu Val Val Leu Ala Thr Ala Glu Ser
130 135 140
Arg Arg Val Ala Gly Arg Thr Tyr Ser Ala Ala Arg Ala Val Ser Leu
145 150 155 160
Ala Val Trp Gly Ile Val Thr Leu Val Val Leu Pro Phe Ala Val Phe
165 170 175
Ala Arg Leu Asp Asp Glu Gln Gly Arg Arg Gln Cys Val Leu Val Phe
180 185 190
Pro Gln Pro Glu Ala Phe Trp Trp Arg Ala Ser Arg Leu Tyr Thr Leu

195 200 205
Val Leu Gly Phe Ala Ile Pro Val Ser Thr Ile Cys Val Leu Tyr Thr
210 215 220
Thr Leu Leu Cys Arg Leu His Ala Met Arg Leu Asp Ser His Ala Lys
225 230 235 240
Ala Leu Glu Arg Ala Lys Lys Arg Val Thr Phe Leu Val Val Ala Ile
245 250 255
Leu Ala Val Cys Leu Leu Cys Trp Thr Pro Tyr His Leu Ser Thr Val
260 265 270
Val Ala Leu Thr Thr Asp Leu Pro Gln Thr Pro Leu Val Ile Ala Ile
275 280 285
Ser Tyr Phe Ile Thr Ser Leu Ser Tyr Ala Asn Ser Cys Leu Asn Pro
290 295 300
Phe Leu Tyr Ala Phe Leu Asp Ala Ser Phe Arg Arg Asn Leu Arg Gln
305 310 315 320
Leu Ile Thr Cys Arg Ala Ala Ala
325 328

<210> 183
<211> 1000
<212> DNA
<213> Human

<400> 183
atcgatatgg acaacgcctc gttctcggag ccctggcccg ccaacgcatc gggcccggac 60
ccggcgctga gctgctccaa cgcgtcgact ctggcgccgc tgccggcgcc gctggcggtg 120
gctgtaccag ttgtctacgc ggtgatctgc gccgtgggtc tggcgggcaa ctccgccgtg 180
ctgtacgtgt tgctgcgggc gccccgcatg aagaccgtca ccaacctgtt catcctcaac 240
ctggccatcg ccgacgagct cttcacgctg gtgctgccca tcaacatcgc cgacttcctg 300
ctgcggcagt ggcccttcgg ggagctcatg tgcaagctca tcgtggctat cgaccagtac 360
aacaccttct ccagcctcta cttcctcacc gtcatgagcg ccgaccgcta cctggtggtg 420
ttggccactg cggagtcgcg ccgggtggcc ggccgcacct acagcgccgc gcgcgcggtg 480
agcctggccg tgtgggggat cgtcacactc gtcgtgctgc ccttcgcagt cttcgcccgg 540
ctagacgacg agcagggccg gcgccagtgc gtgctagtct ttccgcagcc cgaggccttc 600
tggtggcgcg cgagccgcct ctacacgctc gtgctgggct tcgccatccc cgtgtccacc 660
atctgtgtcc tctataccac cctgctgtgc cggctgcatg ccatgcggct ggacagccac 720

gccaaggccc tggagcgcgc caagaagcgg gtgaccttcc tggtggtggc aatcctggcg 780
gtgtgcctcc tctgctggac gccctaccac ctgagcaccg tggtggcgct caccaccgac 840
ctcccgcaga cgccgctggt catcgctatc tcctacttca tcaccagcct gagctacgcc 900
aacagctgcc tcaacccctt cctctacgcc ttcctggacg ccagcttccg caggaacctc 960
cgccagctga taacttgccg cgcggcagcc tgacactagt 1000

<210> 184
<211> 33
<212> DNA
<213> Artificial Sequence

<220>
<223> Primer

<400> 184
gtcgacatgg cccggtccgc gacactggcg gcc 33

<210> 185
<211> 33
<212> DNA
<213> Artificial Sequence

<220>
<223> Primer

<400> 185
gctagcagcg gtgccaggag aggtccgggc tca 33

<210> 186
<211> 33
<212> DNA
<213> Artificial Sequence

<220>
<223> Primer

<400> 186
gtcgacagct ccatggcccg gtgtaggacg ctg 33

<210> 187
<211> 33
<212> DNA
<213> Artificial Sequence

<220>
<223> Primer

<400> 187
gctagctcag gtgctctggc aatcagtctc gtg 33

<210> 188
<211> 27
<212> DNA
<213> Artificial Sequence

<220>
<223> Primer

<400> 188
cacggctcca tggtccggtg taggacg 27

<210> 189
<211> 27
<212> DNA
<213> Artificial Sequence

<220>
<223> Primer

<400> 189
cagcgtcgag gtttgggttg gggttca 27

<210> 190
<211> 32
<212> DNA
<213> Artificial Sequence

<220>
<223> Primer

<400> 190
atcgatatgg acaacgcctc gttctcggag cc 32

<210> 191
<211> 32
<212> DNA
<213> Artificial Sequence

<220>
<223> Primer

<400> 191
actagtgtca ggctgccgcg cggcaagtta tc 32